(19) Europäisches Patentamt
European Patent Office
Office européen des brevets

(11) EP 3 027 707 B1

(12) **EUROPÄISCHE PATENTSCHRIFT**

(45) Veröffentlichungstag und Bekanntmachung des Hinweises auf die Patenterteilung:
**11.12.2019 Patentblatt 2019/50**

(21) Anmeldenummer: **14739675.8**

(22) Anmeldetag: **07.07.2014**

(51) Int Cl.:
***C09K 11/06*** *(2006.01)* ***C07D 471/04*** *(2006.01)*
***C07D 487/04*** *(2006.01)* ***H05B 33/10*** *(2006.01)*

(86) Internationale Anmeldenummer:
**PCT/EP2014/001860**

(87) Internationale Veröffentlichungsnummer:
**WO 2015/014434 (05.02.2015 Gazette 2015/05)**

(54) **MATERIALIEN FÜR ELEKTRONISCHE VORRICHTUNGEN**

MATERIALS FOR ELECTRONIC DEVICES

MATÉRIAUX POUR DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2013 EP 13003800**

(43) Veröffentlichungstag der Anmeldung:
**08.06.2016 Patentblatt 2016/23**

(73) Patentinhaber: **Merck Patent GmbH**
**64293 Darmstadt (DE)**

(72) Erfinder:
- **PARHAM, Amir, Hossain**
  **60486 Frankfurt am Main (DE)**
- **MARTYNOVA, Irina**
  **64347 Griesheim (DE)**
- **JATSCH, Anja**
  **60489 Frankfurt am Main (DE)**
- **EBERLE, Thomas**
  **76829 Landau (DE)**
- **KROEBER, Jonas, Valentin**
  **60311 Frankfurt am Main (DE)**
- **PFLUMM, Christof**
  **64291 Darmstadt (DE)**

(56) Entgegenhaltungen:
**EP-A1- 1 956 022**
**EP-A1- 2 873 667**
**EP-A2- 2 123 733**
**WO-A1-2011/136156**
**US-A1- 2011 006 670**
**US-A1- 2012 085 997**
**US-A1- 2012 273 766**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 027 707 B1

Printed by Jouve, 75001 PARIS (FR)

## Beschreibung

Die Vorliegende Erfindung betrifft cyclische Verbindungen mit einer spezifischen Anordung von elektronenleitenden und lochleitenden Gruppen, deren Verwendung in organischen Elektrolumineszenzvorrichtungen, deren Herstellung sowie organischen Elektrolumineszenzvorrichtungen.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746,

EP 0906947, EP 0908787, EP 0906948).

Für fluoreszierende OLEDs werden gemäß dem Stand der Technik vor allem kondensierte Aromaten, insbesondere Anthracenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart. Weitere Anthracenderivate sind in WO 01/076323, in WO 01/021729, in WO 2004/013073, in WO 2004/018588, in WO 2003/087023 oder in

WO 2004/018587 offenbart. Host-Materialien, basierend auf Arylsubstituierten Pyrenen und Chrysenen, werden in WO 2004/016575 offenbart. Host-Materialien basierend auf Benzanthracenderivaten werden in WO 2008/145239 offenbart. Es ist für hochwertige Anwendungen wünschenswert, verbesserte Host-Materialien zur Verfügung zu haben.

Im Stand der Technik ist die Verwendung von Verbindungen enthaltend eine oder mehrere Carbazolgruppen in elektronischen Vorrichtungen, beispielsweise aus WO 2005/039246, US 2005/0069729, JP 2004/288381, EP 1205527 oder WO 2008/086851 bekannt.

Weiterhin im Stand der Technik bekannt ist die Verwendung von Verbindungen enthaltend eine oder mehrere Indenocarbazolgruppen in elektronischen Vorrichtungen, beispielsweise aus WO 2010/136109 und WO 2011/000455.

Weiterhin im Stand der Technik bekannt ist die Verwendung von Verbindungen enthaltend eine oder mehrere elektronenarme heteroaromatische Sechsringe in elektronischen Vorrichtungen, beispielsweise aus WO 2010/015306, WO 2007/063754 und WO 2008/056746.

WO 2009/069442 offenbart Tricyclen, wie Carbazol, Dibenzofuran oder Dibenzothiophen, die hochgradig mit elektronenarmen Heteroaromaten (z.B. Pyridin, Pyrimidin oder Triazin) substituiert sind. Mit lochleitenden Gruppen, d.h. elektronenreichen Gruppen, sind die Tricyclen nicht substituiert.

JP 2009-21336 offenbart substituierte Carbazole als Matrixmaterialien, wobei die Carbazole mit einer elektronenleitenden und mit einer lochleitenden Gruppe substituiert sind. Die Verbindungen weisen allerdings keine face-to-face Substitution auf.

WO 2011/057706 offenbart substituierte Carbazole als Matrixmaterialien, wobei die Carbazole mit einer elektronenleitenden und mit einer lochleitenden Gruppe substituiert sind. Allerdings weisen die meisten der offenbarten Carbazole keine face-to-face Substitution auf. Bei den vereinzelt offenbarten face-to-face Anordnungen ist die loch- bzw. elektronenleitende Gruppe jedoch direkt an den Tricyclus gebunden.

EP 2873667 A1 offenbart Naphthaline, die in den Positionen 1 und 8 mit Elektronentransportgruppen substituiert sind, sowie deren Verwendung in organischen elektronischen Vorrichtungen.

US 2011/006670 A1 offenbart Dibenzofurane und Dibenzothiophene, die in den Positionen 1 und 5 mit Lochleitergruppen substituiert sind, sowie deren Verwendung in organischen elektrolumineszierenden Vorrichtungen.

Allerdings besteht bei Verwendung dieser Materialien ebenso wie bei anderen Materialien noch Verbesserungsbedarf, insbesondere in Bezug auf die Effizienz und die Lebensdauer der Vorrichtung.

Aufgabe der vorliegenden Erfindung ist daher die Bereitstellung von Verbindungen, welche sich für den Einsatz in einer fluoreszierenden oder phosphoreszierenden OLED eignen, beispielsweise als Host- und/oder Matrixmaterial

oder als Lochtransport-/Elektronenblockiermaterial bzw. Exzitonenblockiermaterial oder als Elektronentransport- bzw. Lochblockiermaterial, und welche bei Verwendung in einer OLED zu guten Device-Eigenschaften führen, sowie die Bereitstellung der entsprechenden organischen Elektrolumineszenzvorrichtungen.

Überraschend wurde gefunden, dass bestimmte, unten näher beschriebene Verbindungen diese Aufgaben lösen und zu guten Eigenschaften der organischen Elektrolumineszenzvorrichtung führen, insbesondere hinsichtlich der Lebensdauer, der Effizienz und der Betriebsspannung. Organische Elektrolumineszenzvorrichtungen, welche derartige Verbindungen enthalten, sowie die entsprechenden bevorzugten Verbindungen sind daher der Gegenstand der vorliegenden Erfindung. Die überraschenden Effekte werden durch eine spezielle Anordnung ("face-to-face", d.h. einander gegenüberliegende Anordnung von Gruppen) elektronenleitender und lochleitender Gruppen in Verbindungen der unten aufgeführten Formeln erreicht. Ohne an eine Theorie gebunden zu sein, könnte der schnelle Ladungstransport an der relativ wohldefinierten (hochgeordneten) Parallelausrichtung der Moleküle (*face-to-face* Anordnung) liegen, in der eine gewisse Nahordnung der Moleküle vorliegt. Bedingt durch die geringen Abstände der Gruppen zueinander könnten zwischenmolekulare Wechselwirkungen, wie beispielsweise direkte $\pi$-$\pi$-Wechselwirkung für den schnellen Ladungstransfer mit ursächlich sein.

Die erfindungsgemäßen Verbindungen weisen auch eine hohe Glasübergangstempertur ($T_g$) auf, was vorteilhaft ist hinsichtlich der Prozessierung der Verbindungen bei der Herstellung elektronischer Vorrichtungen. Die hohe Glasübergangstemperatur der Verbindungen gestattet auch die Verwendung der Verbindungen in dünnen amorphen organischen Schichten.

Weiterhin erlauben die erfindungsgemäßen Verbindungen eine Stabilisierung der Ladungsträger im angeregten Zustand und weisen eine ausreichend hohe Triplett Energie auf, was für phosphoreszierende Vorrichtungen eine wichtige Vorraussetzung darstellt. Ferner zeigen die erfindungsgemäßen Verbindungen verbesserte Leistungsdaten in OLEDs gegenüber den Verbindungen aus dem Stand der Technik.

Gegenstand der vorliegenden Erfindung sind daher Verbindungen der allgemeinen Formel (1) gemäß Anspruch 1.

Demnach gilt, beispielsweise, für die Verbindungen der allgemeinen Formel (1), dass im Fall m = n = 1 und V = W = Einfachbindung die allgemeine Formel wie folgt lautet

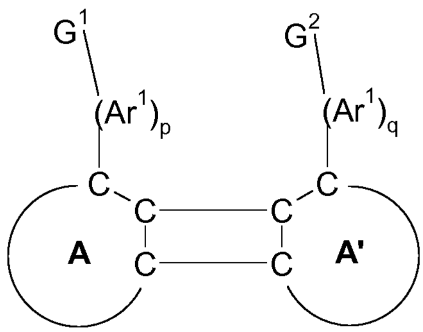

Weiterhin gilt, beispielsweise, für die Verbindungen der allgemeinen Formel (1), dass im Fall m = n = 1 und V = N-$Ar^3$ und W = Einfachbindung die allgemeine Formel wie folgt lautet

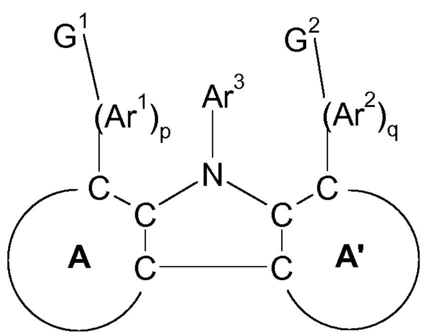

Weiterhin gilt, beispielsweise, für die Verbindungen der allgemeinen Formel (1), dass im Fall m = n = 0 die allgemeine Formel wie folgt lautet

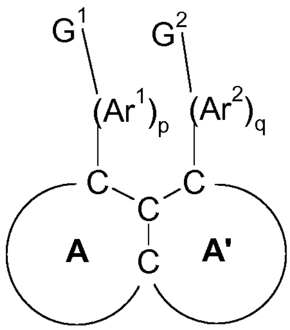

In einer bevorzugten Ausführungsform der vorliegenden Erfindung enthalten die Gruppen $G^1$ und $G^2$ ausschließlich ladungstransportierende Einheiten einer Art. Das bedeutet, beispielsweise,dass sofern $G^1$ eine ETG ist, $G^1$ keine Gruppen enthalten kann, die lochleitend sind.

Sofern Triazine als ETGs verwendet werden, gilt in einer weiteren bevorzugten Ausführungsform, dass die Triazine substituiert vorliegen, d.h., keines der Triazine einer ETG darf noch direkt an das Triazin gebundene Wasserstatome aufweisen. Weisen die Triazine noch direkt an das Triazin gebundene Wasserstoffatome auf, führt dies zu verschlechterten Leistungsdaten elektronischer, insbesondere elektrolumineszierender Vorrichtungen gegenüber Triazinen, die keine direkt an das Triazin gebundene Wasserstoffatome mehr aufweisen.

In einer bevorzugten Ausführungsform ist die Verbindung ausgewählt aus der allgemeinen Formel (2)

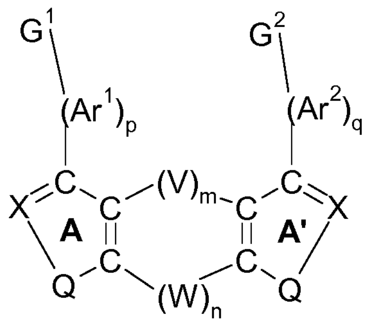

Formel (2)

wobei für die zusätzlich verwendeten Symbole gilt:

X ist gleich oder verscheiden bei jeden Auftreten N oder $CR^1$;

Q ist gleich oder verschieden bei jedem Auftreten X=X, S, O oder $NR^1$, bevorzugt X=X, S und O, ganz bevorzugt X=X und S und ganz besonders bevorzugt X=X.

Ganz bevorzugt ist demnach eine Verbindung der allgemeinen Formeln (3) bis (11)

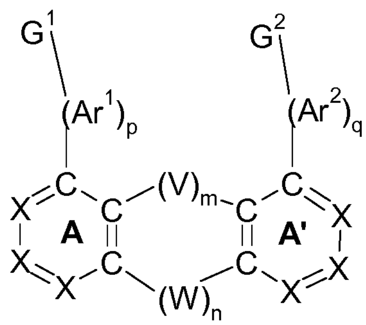

Formel (3)

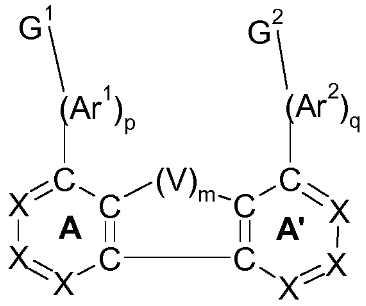

Formel (4)

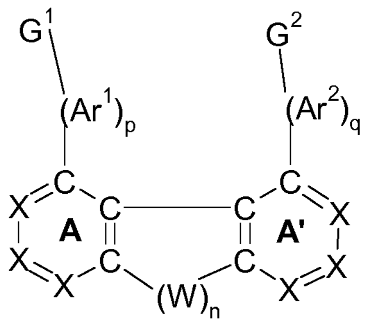

Formel (5)

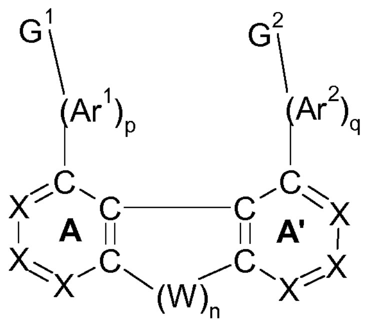

Formel (6)

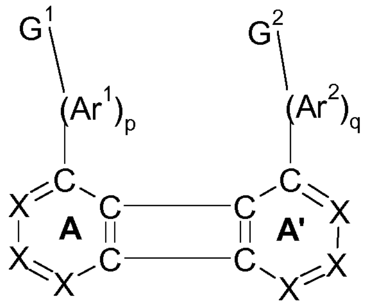

Formel (7)

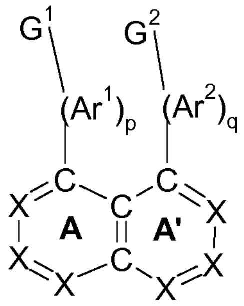

Formel (8)

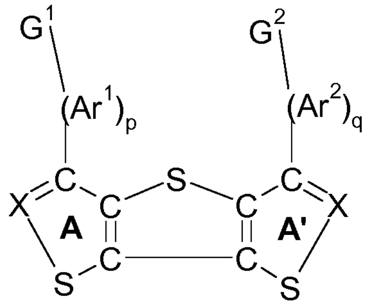

Formel (9)

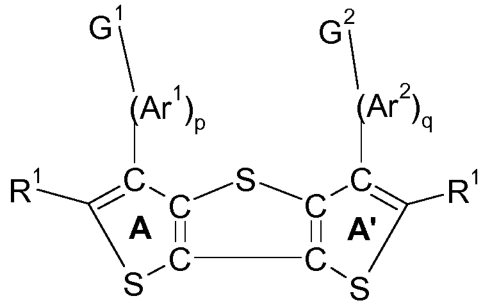

Formel (10)

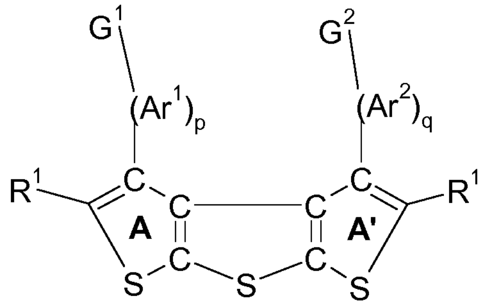

Formel (11)

wobei eine Verbindung der allgemeinen Formeln (3) bis (8) ganz besonders bevorzugt ist und eine Verbindung der allgemeinen Formel (4) insbesondere bevorzugt ist.

Es ist weiterhin ganz besonders bevorzugt, wenn X in den Formeln (1) bis (11) gleich $CR^1$ ist.

In einer bevorzugten Ausführungsform betrifft die vorliegende Erfindung eine Verbindung der Formel (4), bevorzugt eine Verbindung der Formel (4) mit X gleich $CR^1$ und m=1, ganz bevorzugt eine Verbindung der Formel (4) mit X gleich $CR^1$, m=1 und V gleich O, wobei für die anderen Symbole und Indizes obige Definitionen und bevorzugte

Ausführungsformen gelten.

In einer weiteren bevorzugten Ausführungsform betrifft die vorliegende Erfindung eine Verbindung der Formel (4) mit X gleich $CR^1$, m=1 und V gleich N-$Ar^3$, wobei für die anderen Symbole und Indizes obige Definitionen und bevorzugte Ausführungsformen gelten.

In einer weiteren bevorzugten Ausführungsform betrifft die vorliegende Erfindung eine Verbindung der allgemeinen Formel (12)

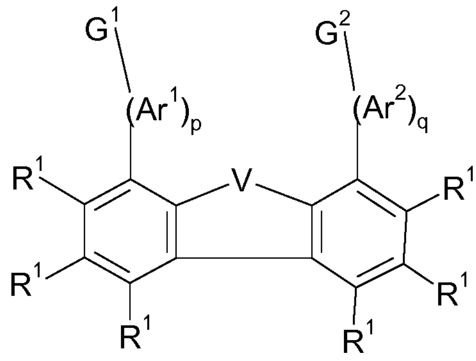

Formel (12)

wobei V gleich O oder S ist und wobei für die verwendeten Indizes und Symbole die hierin aufgeführten Definitionen und bevorzugten Ausführungsformen gelten. Es ist ganz bevorzugt, wenn V in de Verbindung der Formel (12) gleich O ist.

In einer weiteren bevorzugten Ausführungsform betrifft die vorliegende Erfindung eine Verbindung der allgemeinen Formel (13)

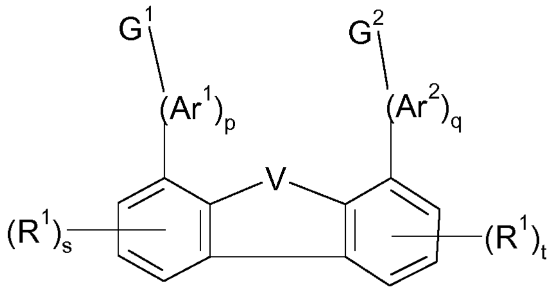

Formel (13)

wobei V gleich O oder S ist und wobei für die verwendeten Indizes und Symbole die hierin aufgeführten Definitionen und bevorzugten Ausführungsformen gelten und wobei die aromatischen Ringe A und A' jeweils maximal einen Substituenten $R^1$ haben , d.h, s ist gleich 0 oder 1 und t ist gleich 0 oder 1, wobei s + t gleich 0, 1 oder 2 sein kann. Es ist ganz bevorzugt, wenn V in de Verbindung der Formel (13) gleich O ist.

In einer ganz bevorzugten Ausführungsform betrifft die vorliegende Erfindung eine Verbindung der allgemeinen Formel (14)

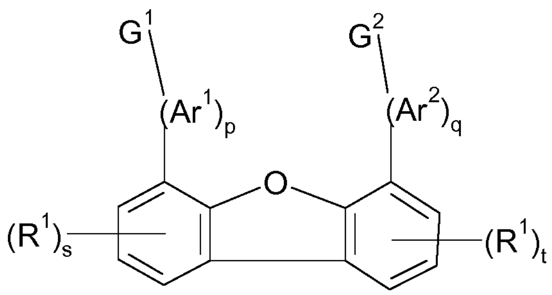

Formel (14)

wobei für die verwendeten Indizes und Symbole die hierin aufgeführten Definitionen und bevorzugten Ausführungsformen gelten und wobei die aromatischen Ringe A und A' jeweils maximal einen Substituenten $R^1$ haben.

In einer ganz besonders bevorzugten Ausführungsform betrifft die vorliegende Erfindung eine Verbindung der allgemeinen Formel (15)

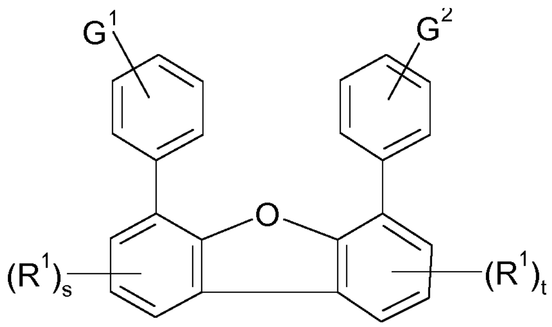

Formel (15)

wobei für die verwendeten Symbole die hierin aufgeführten Definitionen und bevorzugten Ausführungsformen gelten und wobei die aromatischen Ringe jeweils maximal einen Substituenten $R^1$ haben.

In einer weiteren ganz besonders bevorzugten Ausführungsform betrifft die vorliegende Erfindung eine Verbindung der allgemeinen Formel (16)

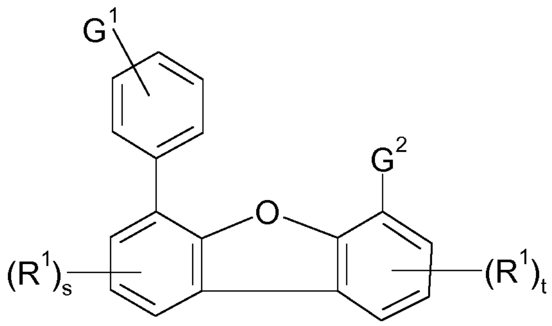

Formel (16)

wobei für die verwendeten Symbole die hierin aufgeführten Definitionen und bevorzugten Ausführungsformen gelten und wobei die aromatischen Ringe jeweils maximal einen Substituenten $R^1$ haben.

Es ist weiterhin insbesondere bevorzugt, wenn $R^1$ in den Ringen A und A' in den Verbindungen der Formeln (12) bis (16) gleich H ist.

In einer bevorzugten Ausführungsform ist die Summe der beiden Indizes p und q in den Formeln (12) bis (16) gleich 1.

In einer anderen bevorzugten Ausführungsform ist die Summe der beiden Indizes p und q in den Formeln (12)

bis (16) gleich 2.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

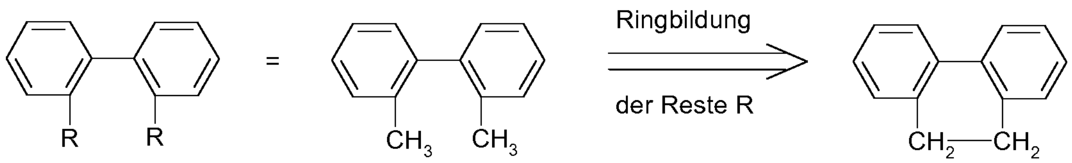


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

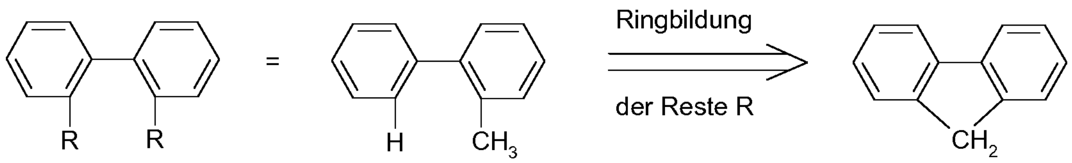


Unter einer kondensierten Arylgruppe wird dabei eine Arylgruppe verstanden, welche zwei oder mehr aromatische Ringe enthält, die miteinander kondensiert sind, d. h. eine oder mehr aromatische Bindungen miteinander teilen. Eine entsprechende Definition gilt für Heteroarylgruppen. Beispiele für kondensierte Arylgruppen ungeachtet der Zahl ihrer Ringatome sind Naphthyl, Anthracenyl, Pyrenyl, Phenanthrenyl und Perylenyl. Beispiele für kondensierte Heteroarylgruppen sind Chinolinyl, Indolyl, Carbazolyl, und Acridinyl.

Es folgen allgemeine Definitionen für chemische Gruppen im Rahmen der vorliegenden Anmeldung:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie dies beispielsweise in Benzimidazol, Chinolin oder Phenanthrolin der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Wenn $G^1$ oder $G^2$ eine elektronentransportierende Gruppe (ETG) ist, dann kann die Gruppe eine elektronenarme heteroaromatische Gruppe sein, die mit einem oder mehreren Resten $R^1$ substituiert sein kann. Sie kann auch eine heteroaromatische Gruppe mit 6 aromatischen Ringatomen sein, von denen mindestens eines ein N-Atom ist, oder heteroaromatische Gruppen mit 5 aromatischen Ringatomen, von denen mindestens 2 Heteroatome sind, oder mindestens eines davon ein N-Atom ist, das mit $R^1$ substituiert sein kann, wobei an diese Gruppen jeweils auch weitere Aryl- oder Heteroarylgruppen ankondensiert sein können.

Beispiele für elektronenarme heteroaromatische Gruppen sind die folgenden Gruppen.

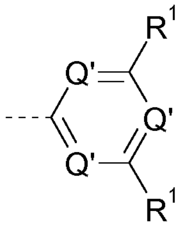

Formel (E-1)

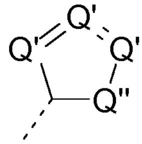

Formel (E-2)

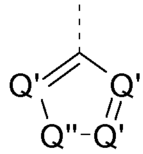

Formel (E-3)

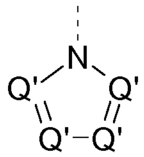

Formel (E-4)

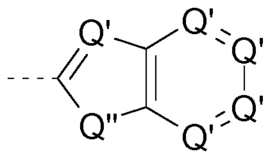

Formel (E-5)

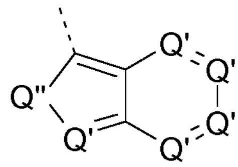

Formel (E-6)

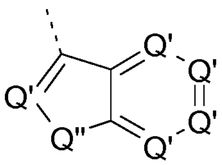

Formel (E-7)

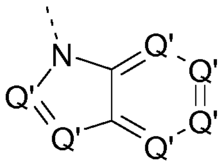

Formel (E-8)

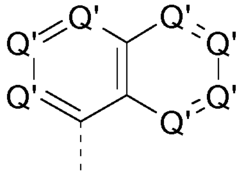

Formel (E-9)

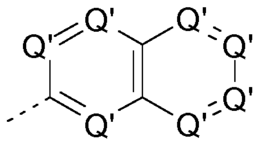

Formel (E-10),

wobei die gestrichelte Bindung die Anbindungsposition markiert, $R^1$ wie oben definiert ist und

Q’ bei jedem Auftreten gleich oder verschieden $CR^1$ oder N darstellt, und

Q" $NR^1$, O oder S darstellt;

wobei wenigstens ein Q’ gleich N und/oder wenigstens ein Q" gleich $NR^1$ ist.

Weitere Beispiele für elektronenarme heteroaromatische Gruppen sind: Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole, die jeweils mit $R^1$ substituiert sein können. Noch mehr bevorzugt ist die elektronentransportierende Gruppe ein mit einem oder mehreren Resten $R^1$ substituiertes Pyridin, Pyrazin, Pyrimidin, Pyridazin und 1,3,5-Triazin.

Ganz bevorzugte elektronenarme heteroaromatische Gruppen sind ausgewählt aus den folgenden Gruppen

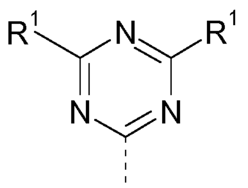

Formel (E-11)

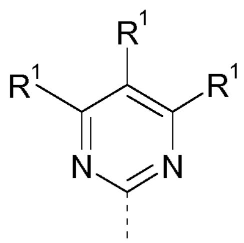

Formel (E-12)

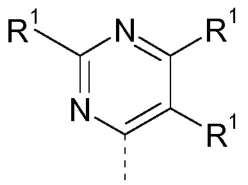

Formel (E-13)

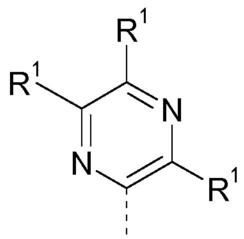

Formel (E-14)

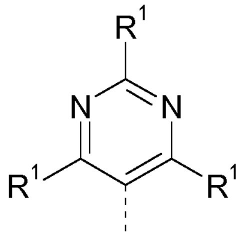

Formel (E-15)

Die Substituenten $R^1$ in der ETG sind vorzugsweise ausgewählt aus der Gruppe bestehend aus H oder ein aromatisches oder heteroaromatisches Ringsystem mit 5 bis 60 aromatischen Ringatomen, das jeweils durch einen oder mehrere Reste $R^2$ substituiert sein kann.

Beispiele ganz besonders bevorzugter ETGs mit Resten $R^1$ sind die folgenden Gruppen, die mit einem oder mehreren voneinander unabhängigen Resten $R^2$ substituiert sein können, wobei die gestrichelten Bindungen die Bindungspositionen zu den Gruppen $Ar^1$ und $Ar^2$ kennzeichnen.

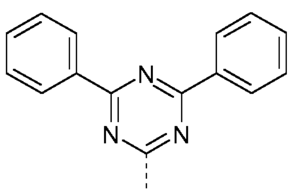

Formel (E-17)

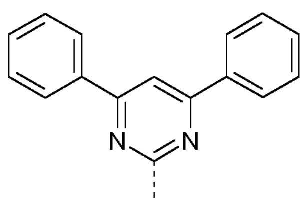

Formel (E-18)

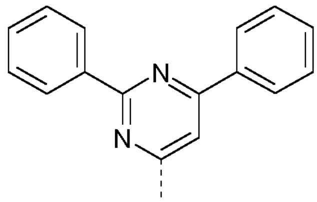

Formel (E-19)

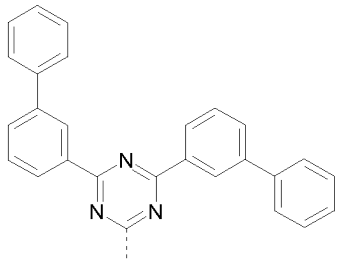

Formel (E-22)

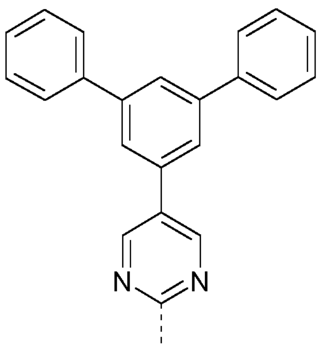

Formel (E-23)

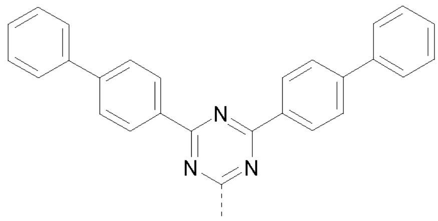

Formel (E-24)

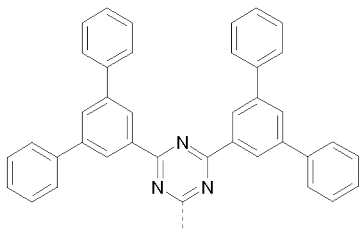

Formel (E-25)

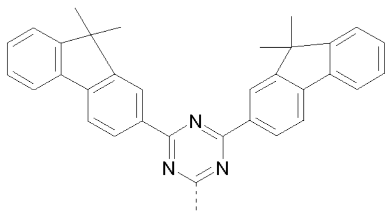

Formel (E-26)

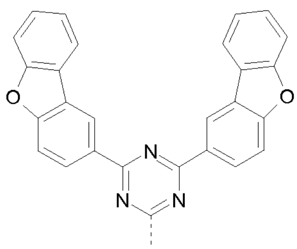

Formel (E-27)

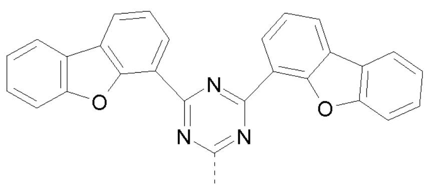

Formel (E-28)

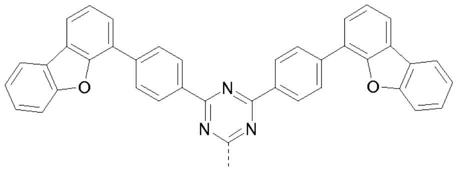

Formel (E-29)

Die Elektronentransportgruppe weist bevorzugt eine LUMO (lowest unoccupied molecular orbital) Energie auf, die niedriger als -1.3 eV ist, ganz bevorzugt niedriger als -2.5 eV und ganz besonders bevorzugt niedriger als -2.7 eV.

HOMO- (highest occupied molecular orbital) und LUMO- (lowest unoccupied molecular orbital) Energieniveaus sowie die Energie des niedrigsten Triplettzustands $T_1$ bzw. die des niedrigsten angeregten Singulettzustands $S_1$ der Materialien werden über quantenchemische Rechnungen bestimmt. Hierzu wird das Programmpaket "Gaussian09W" (Gaussian Inc.) verwendet. Zur Berechnung organischer Substanzen wird zuerst eine Geometrieoptimierung mit der Methode "Ground State/Semiempirical/Default Spin/AM1/Charge 0/Spin Singlet" durchgeführt. Im Anschluss erfolgt auf Grundlage der optimierten Geometrie eine Energierechnung. Hierbei wird die Methode "TD-SFC/DFT/Default Spin/B3PW91" mit dem Basissatz "6-31G(d)" verwendet (Charge 0, Spin Singlet). Aus der Energie--rechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplett-zustands mit der niedrigsten Energie, der sich aus der beschriebenen quantenchemischen Rechnung ergibt.

Der niedrigste angeregte Singulettzustand $S_1$ ist definiert als die Energie des angeregten Singulett-zustands mit der niedrigsten Energie, der sich aus der beschriebenen quantenchemischen Rechnung ergibt.

Weiter bevorzugt ist dieElektronentransportgruppe dadurch charakterisiert, dass die Elektronenmobilität $\mu_-$ $10^{-6}$ $cm^2/(Vs)$ oder mehr beträgt, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

In den Verbindungen nach Formel (1) ist das LUMO bevorzugt auf der Elektronentransportgruppe lokalisiert sein. Ganz bevorzugt ist, wenn das LUMO mehr als 80 % auf elektrontransportierende Gruppe lokalisiert ist, noch bevorzugter, wenn das LUMO überhaupt nicht auf der LTG (bspw. eine Amin- oder Carbazolgruppe) lokalisiert ist. Insbesondere bevorzugt ist, wenn das HOMO und das LUMO der erfindungsgemäßen Verbindung überhaupt nicht überlappen. Der Fachmann hat keinerlei Schwierigkeiten die Überlappung der Orbitale zu ermitteln. Dazu wird das hierin angegebene Berechnungsverfahren verwendet und Orbitale mit einer Aufenthaltswahrscheinlichkeit von 90% angenommen. Die Überlappung kann durch Ermittlung von Überlappungsintegralen berechnet werden. Die Lochtransportgruppe hat vorzugsweise eine HOMO Energie ($HOMO_{LTG}$), die im Bereich der Elektronenausrittsarbeit der verwendeten Anode ($\phi_{Anode}$) zuzüglich +1.5 eV oder niedriger entspricht, d.h. es gilt:

$$HOMO_{LTG} \leq (\phi_{Anode} +1.5\ eV)$$

Wenn die verwendete Anode eine Elektronenaustrittsarbeit von -5 eV hat, so ist die HOMO Energie der Lochtransportgruppe -3.5 eV oder niedriger (d.h. negativer als -3.5 eV). Ganz bevorzugt ist, wenn die Lochtransportgruppe eine HOMO Energie aufweist, die gleich der Elektronenaustrittsarbeit der Anode oder niedriger ist, ganz besonders bevorzugt ist sie niedriger.

Weiter bevorzugt ist die Lochtransportgruppe dadurch charakterisiert, dass die Lochmobilität $\mu_+$ $10^{-6}$ $cm^2/(Vs)$ oder mehr beträgt, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

In den Verbindungen nach Formel (1) wird das HOMO maßgeblich auf der Lochtransportgruppe lokalisiert sein. Maßgeblich bedeutet hierbei, dass das HOMO zu 80% oder mehr auf der lochleitenden Gruppe lokalisiert ist oder ist

nicht auf elektronarmen elektronentransportierenden Gruppe lokalisiert.

Bevorzugt ist die LTG eine Gruppe gemäß der folgenden allgemeinen Formeln, wobei die mit der gestrichelten Bindung gekennzeichneten Positionen die Verknüpfungsposition zu $Ar^1$ oder $Ar^2$ darstellen und wobei die Gruppen mit einem oder meheren Resten $R^1$, die gleich oder verschiden bei jedem Auftreten sein können, substituiert sein können.

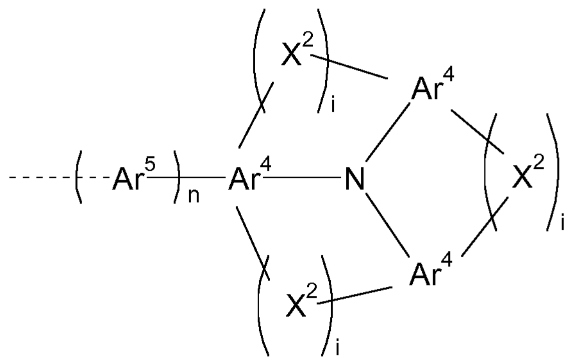

Formel (L-1)

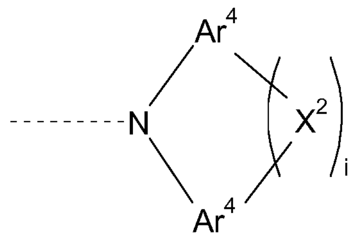

Formel (L-2)

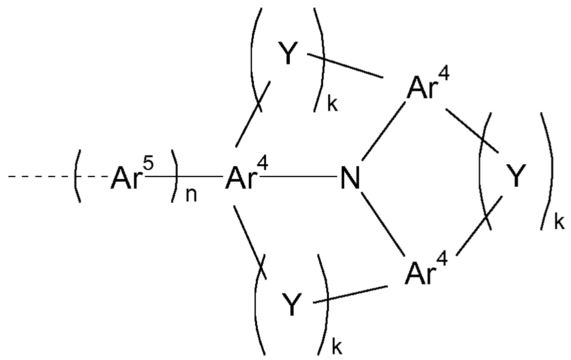

Formel (L-3)

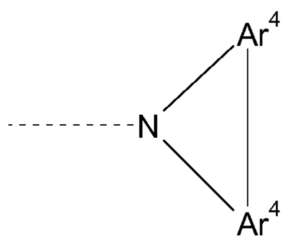

Formel (L-4)

wobei gilt:

$Ar^4$ ist bei jedem Auftreten gleich oder verschieden gewählt aus Aryl- oder Heteroarylgruppen mit 6 bis 13 aromatischen Ringatomen, die mit einem oder mehreren Resten $R^1$ substituiert sein können;

$Ar^5$ ist bei jedem Auftreten gleich oder verschieden gewählt aus Aryl- oder Heteroarylgruppen mit 6 bis 13 aromatischen Ringatomen, die mit einem oder mehreren Resten $R^1$ substituiert sein können;

$X^2$ ist bei jedem Auftreten gleich oder verschieden gewählt aus $C(R^2)_2$, $Si(R^2)_2$, C=O, O, S, S=O, $SO_2$ und $NR^2$;

Y ist eine Einfachbindung;

n ist bei jedem Auftreten gleich oder verschieden 0, 1, 2, 3 oder 4;

i ist bei jedem Auftreten gleich oder verschieden 0 oder 1;

k ist bei jedem Auftreten gleich oder verschieden 0 oder 1, wobei mindestens ein Index k pro Gruppe gleich 1 sein muss;

und wobei ansonsten obige Definitionen gelten.

Besonders bevorzugte Gruppen der Formel (L-1) sind solche der folgenden Formeln (L-5) bis (L-10)

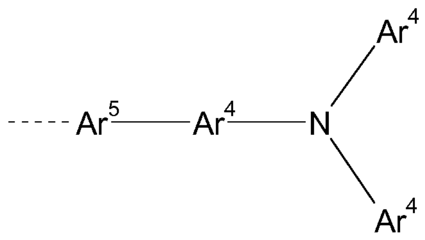

Formel (L-5)

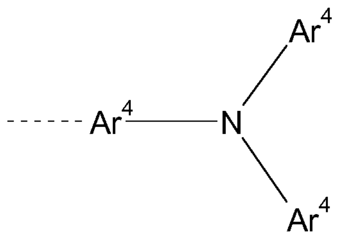

Formel (L-6)

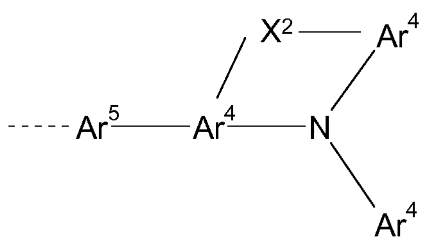

Formel (L-7)

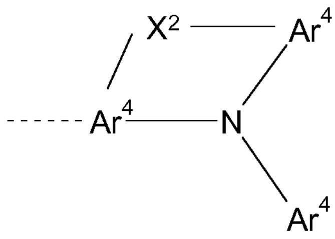

Formel (L-8)

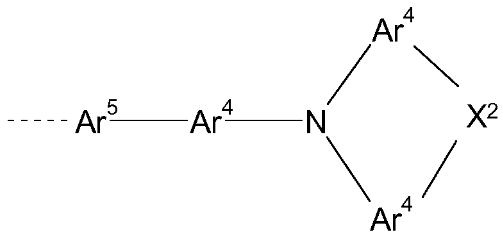

Formel (L-9)

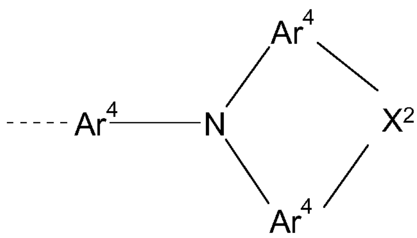

Formel (L-10)

wobei die auftretenden Symbole wie oben angegeben definiert sind. Die in der Anmeldung angegebenen bevorzugten Ausführungsformen von Gruppen gelten ebenfalls als bevorzugt.

Besonders bevorzugte Gruppen der Formel (L-2) sind solche der folgenden Formeln (L-11) bis (L-12)

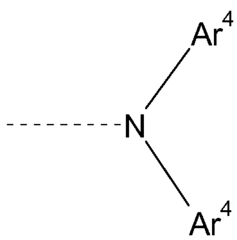

Formel (L-11)

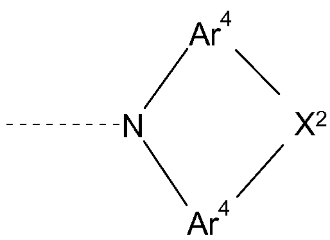

Formel (L-12)

wobei die auftretenden Symbole wie oben angegeben definiert sind. Die in der Anmeldung angegebenen bevorzugten Ausführungsformen von Gruppen gelten ebenfalls als bevorzugt.

Insbesondere ist es bevorzugt, dass in den Formeln (L-11) bis (L-12) $Ar^4$ gleich oder verschieden gewählt ist aus Phenyl, Naphthyl, Pyridyl, Pyrimidyl, Pyridazyl, Pyrazinyl und Triazinyl, die jeweils mit einem oder mehreren Resten $R^2$ substituiert sein können. Weiterhin ist es bevorzugt, dass die Gruppe X bei jedem Auftreten gleich oder verschieden gewählt ist aus $C(R^2)_2$, C=O, O, S und $NR^2$.

Besonders bevorzugt ist die Formel (L-11).

Besonders bevorzugte Gruppen der Formel (L-3) sind solche der folgenden Formeln.

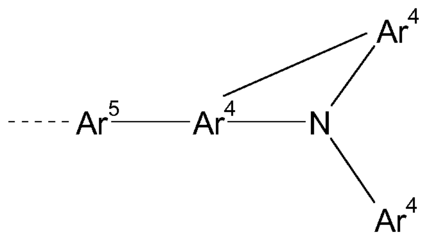

Formel (L-13)

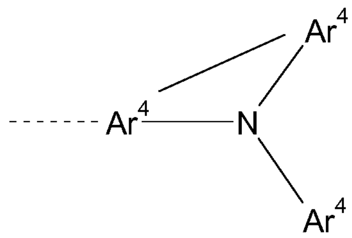

Formel (L-14)

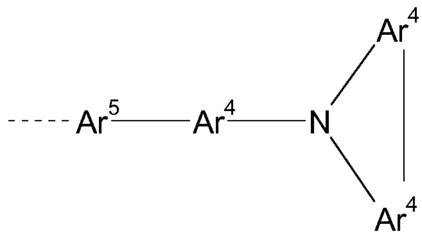

Formel (L-15)

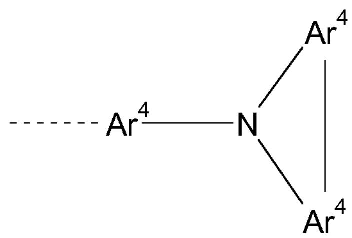

Formel (L-16)

wobei die auftretenden Symbole wie oben definiert sind. Die in der Anmeldung angegebenen bevorzugten Ausführungsformen von Gruppen gelten ebenfalls als bevorzugt.

Insbesondere ist es bevorzugt, dass in den Formeln (L-13) bis (L-16) $Ar^4$ und $Ar^5$ gleich oder verschieden gewählt sind aus Phenyl, Naphthyl, Pyridyl, Pyrimidyl, Pyridazyl, Pyrazinyl und Triazinyl, die jeweils mit einem oder mehreren Resten $R^2$ substituiert sein können.

Besonders bevorzugte Gruppen der Formel (L-4) sind solche der folgenden Formel.

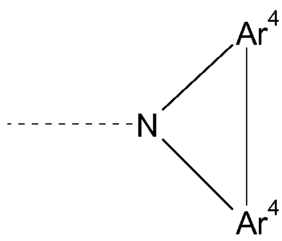

Formel (L-17)

wobei $Ar^4$ gleich oder verschieden gewählt ist aus Phenyl, Naphthyl, Pyridyl, Pyrimidyl, Pyridazyl, Pyrazinyl und Triazinyl, die jeweils mit einem oder mehreren Resten $R^2$ substituiert sein können.

Insbesondere ist es bevorzugt, dass in den Formeln (L-5) bis (L-10) $Ar^4$ und $Ar^5$ gleich oder verschieden gewählt sind aus Phenyl, Naphthyl, Pyridyl, Pyrimidyl, Pyridazyl, Pyrazinyl und Triazinyl, die jeweils mit einem oder mehreren Resten $R^2$ substituiert sein können. Weiterhin ist es bevorzugt, dass die Gruppe $X^2$ bei jedem Auftreten gleich oder verschieden gewählt ist aus $C(R^2)_2$, C=O, O, S und $NR^2$.

Besonders bevorzugt sind die Formeln (L-5) und (L-6).

Beispiele ganz besonders bevorzugter LTGs mit Resten $R^1$ sind die folgenden Gruppen, die mit einem oder mehreren voneinander unabhängigen Resten $R^2$ substituiert sein können, wobei die gestrichelten Bindungen die Bindungspositionen zu den Gruppen $Ar^1$ und $Ar^2$ kennzeichnen.

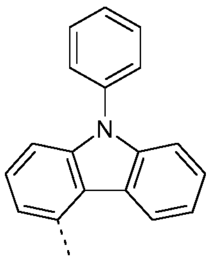

Formel (L-18)

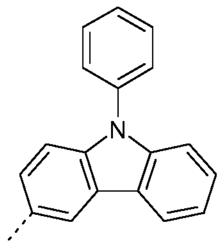

Formel (L-19)

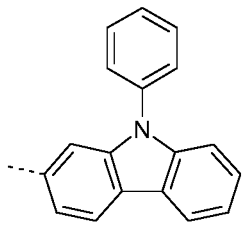

Formel (L-20)

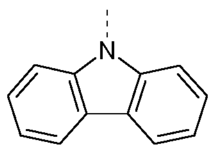

Formel (L-21)

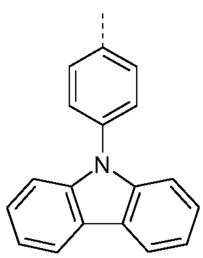

Formel (L-22)

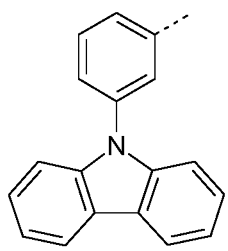

Formel (L-23)

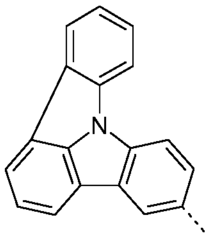

Formel (L-24)

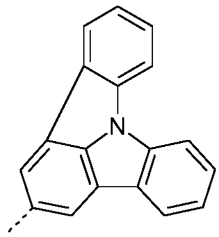

Formel (L-25)

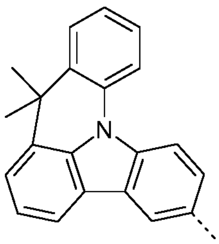

Formel (L-26)

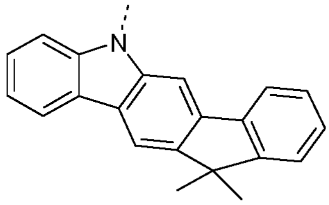

Formel (L-27)

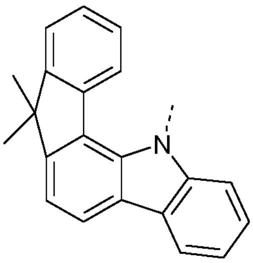

Formel (L-28)

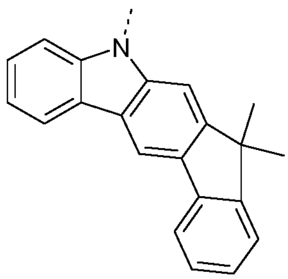

Formel (L-29)

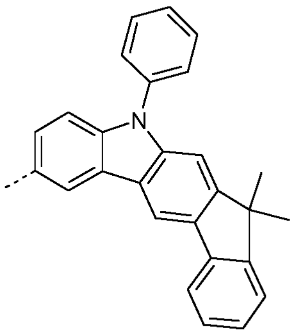

Formel (L-30)

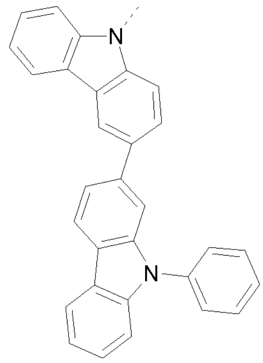

Formel (L-31)

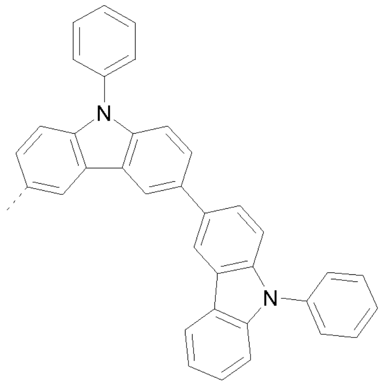

Formel (L-32)

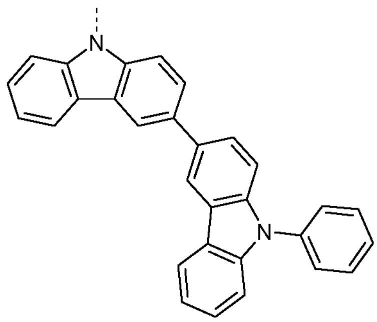

Formel (L-33)

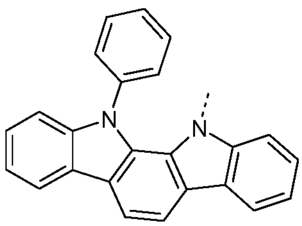

Formel (L-34)

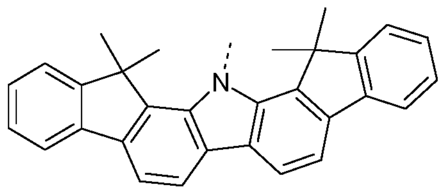

Formel (L-35)

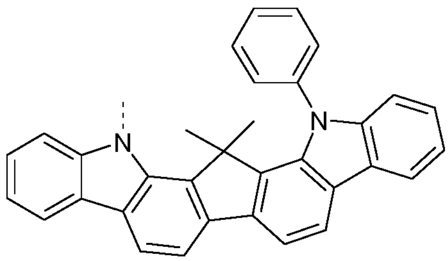

Formel (L-36)

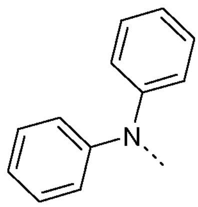

Formel (L-37)

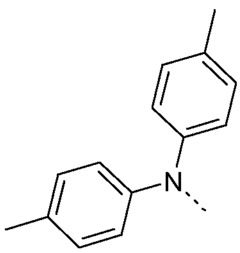

Formel (L-38)

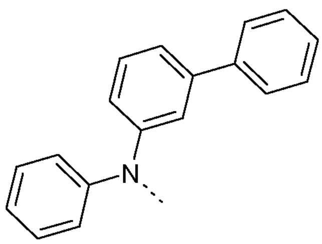

Formel (L-39)

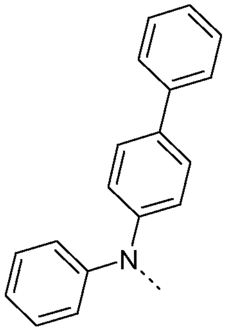

Formel (L-40)

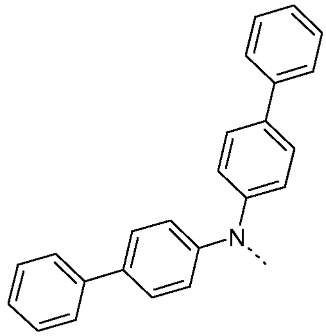

Formel (L-41)

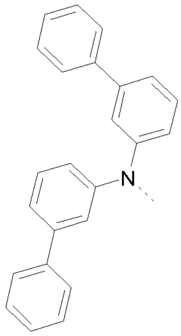

Formel (L-42)

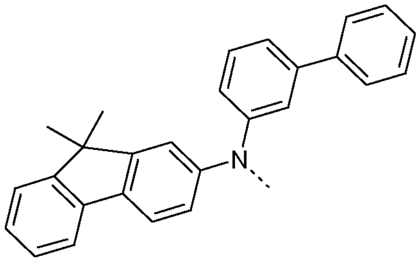

Formel (L-43)

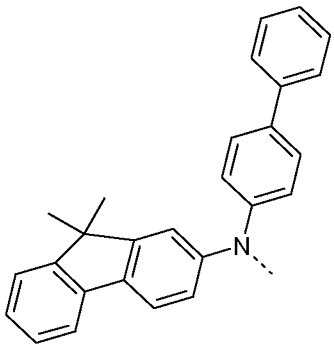

Formel (L-44)

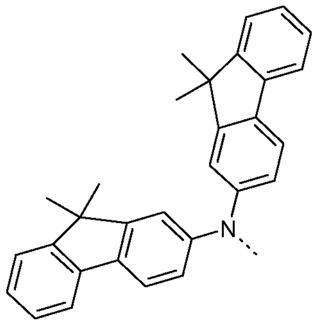

Formel (L-45)

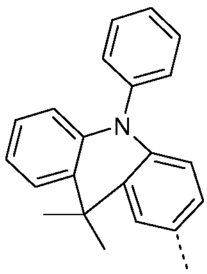

Formel (L-46)

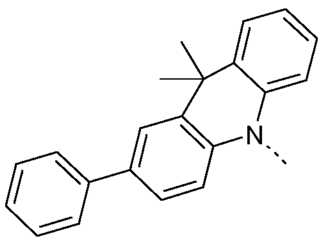

Formel (L-47)

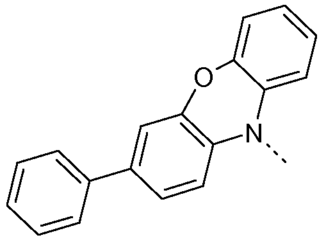

Formel (L-48)

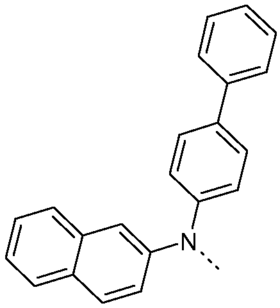

Formel (L-49)

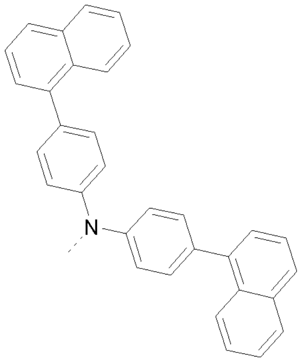

Formel (L-50)

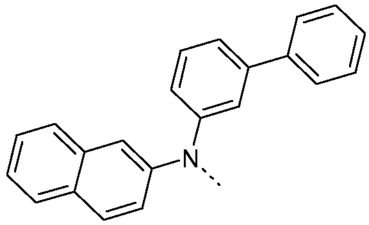

Formel (L-51)

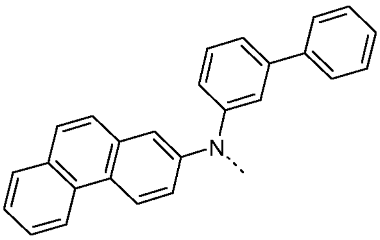

Formel (L-52)

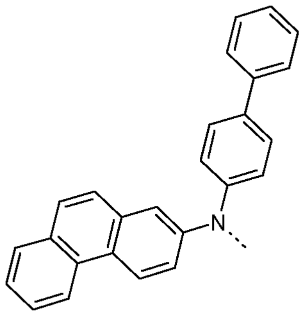

Formel (L-53)

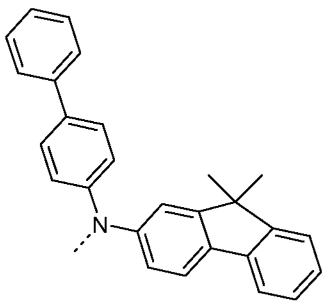

Formel (L-54)

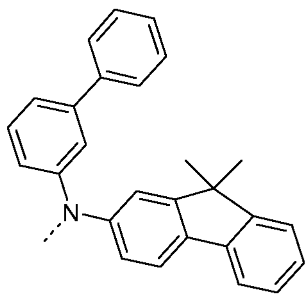

Formel (L-55)

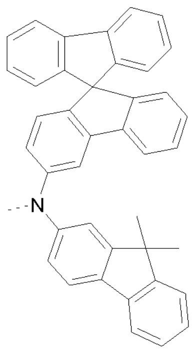

Formel (L-56)

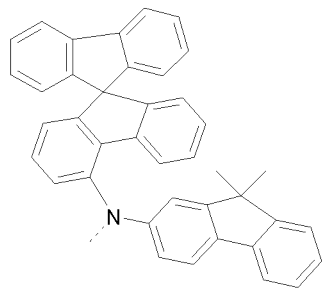

Formel (L-57)

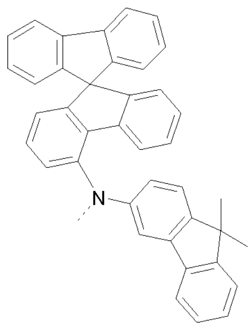

Formel (L-58)

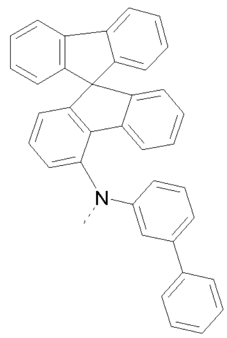

Formel (L-59)

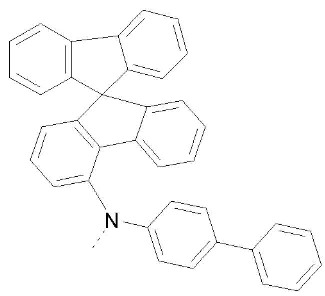

Formel (L-60)

Die erfindungsgemäßen Verbindungen können gemäß Schemata 1 und 2 dargestellt werden.

Die entsprechenden Monoboronsäuren (a) können durch Suzuki-Kupplung und anschließende Silylierung (Schema 1) hergestellt werden. Eine weitere Möglichkeit ist, ausgehend von dem den Monobromiden durch Buchwald-Kupplung und anschließende Silylierung die entsprechende Monoboronsäuren herzustellen (Schema 2). Die Reaktion von diesen Monoboronsäuren via Suzuki-Kupplung mit entsprechenden Arylbromiden oder Arylchloriden führt zu den Zielverbindungen.

## Schema 1

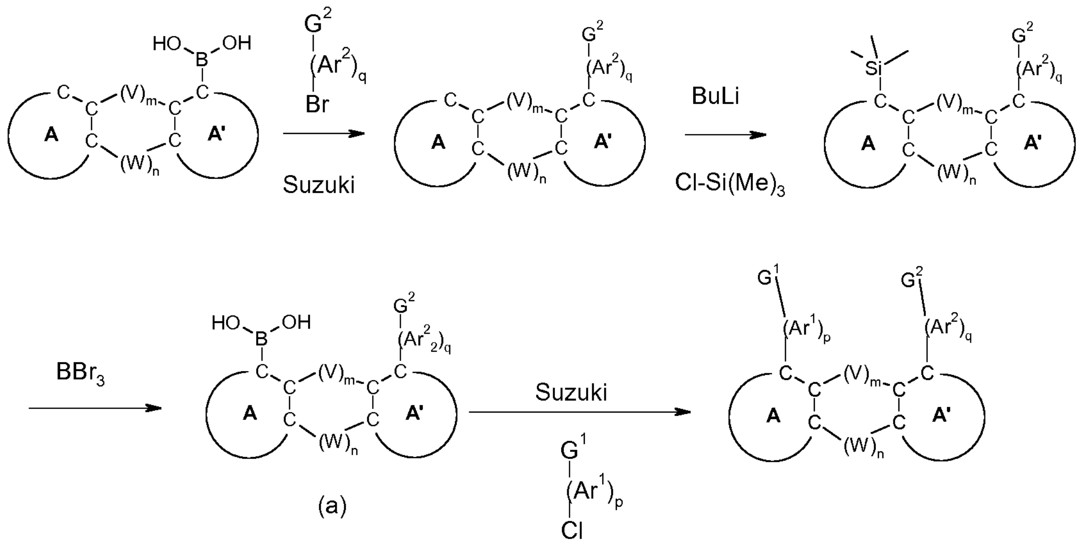


wobei für die verwendeten Indizes und Symbole die oben angegenenen Definitionen sowie deren bevorzugten Ausführungsformen gelten.

## Schema 2

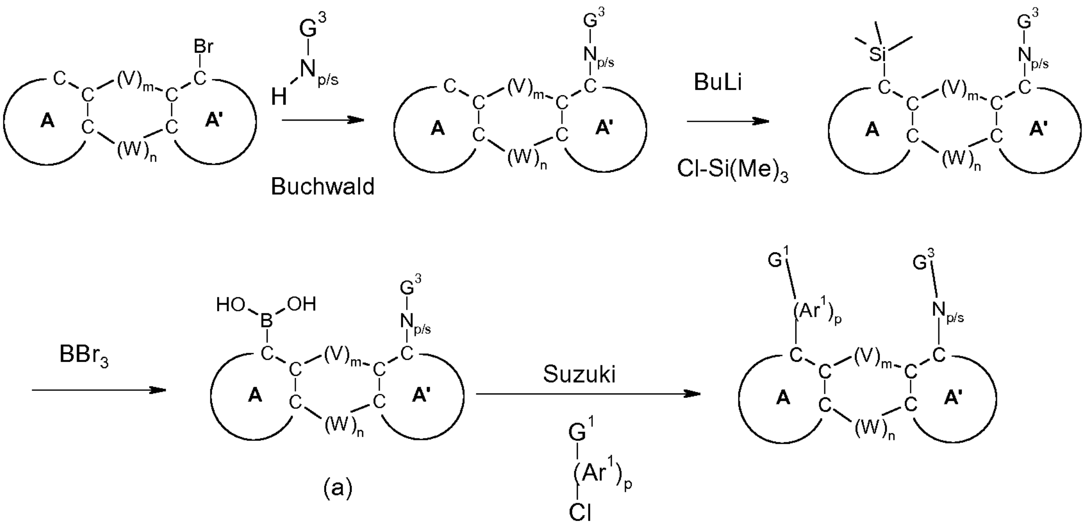


wobei die Gruppe $G^3$-$N_{p/s}$-H gleich der Gruppe $G^2$ ist und wobei die Gruppe $G^2$ ein primäres (p) oder sekundäres (s) Amin enthält und eine LTG darstellt. Die Gruppe $G^1$ ist in diesem Fall eine ETG ist. Weiterhin ist p gleich 1. Für die anderen verwendeten Indizes und Symbole gelten die oben angebenen Definitionen sowie deren bevorzugte Ausführungsformen.

Die Suzuki und die Buchwald Reaktion sind dem Fachmann gut bekannt und es bereitet ihm keinerlei Schwierigkeiten die Reaktionen sowie bekannte Variationen hiervon auf die erfindungsgemäßen Verbindungen anzuwenden, um sie, unter Berücksichtigung des allgemeinen Fachwissens, in der beanspruchten Breite herzustellen. Des Weiteren können sowohl bei der Suzuki- als auch bei der Buchwald-Reaktion die chemischen Funktionalitäten zwischen Substituent und der Struktur enthaltend die Ringe A und A' ausgetauscht werden. Das bedeutet, dass auch der Substituent enthaltend $G^1$ oder $G^2$ die Boronsäure enthalten kann, wohingegen die Struktur enthaltend die Ringe A und A' das Halogenid enthält. Die folgenden Schemata verdeutlichen beispielhaft anhand spezifischerer Fälle die Anwendung der genannten Verfahren, wobei obige Definitionen für die verwendeten Symbole und Indizes gelten. Die in den folgenden

Shemata aufgeführten Gruppen Ar sind gleich oder verschieden voneinander bei jedem Auftreten und repräsentierten aromatische oder heteroaromatische Gruppen, die so ausgewählt werden können, dass die obigen Definitionen für ETG, LTG und allgemein die von Formel (1) erfüllt sind. Hal steht für Halogenide ist bevorzugt Br oder I.

## Schema 3

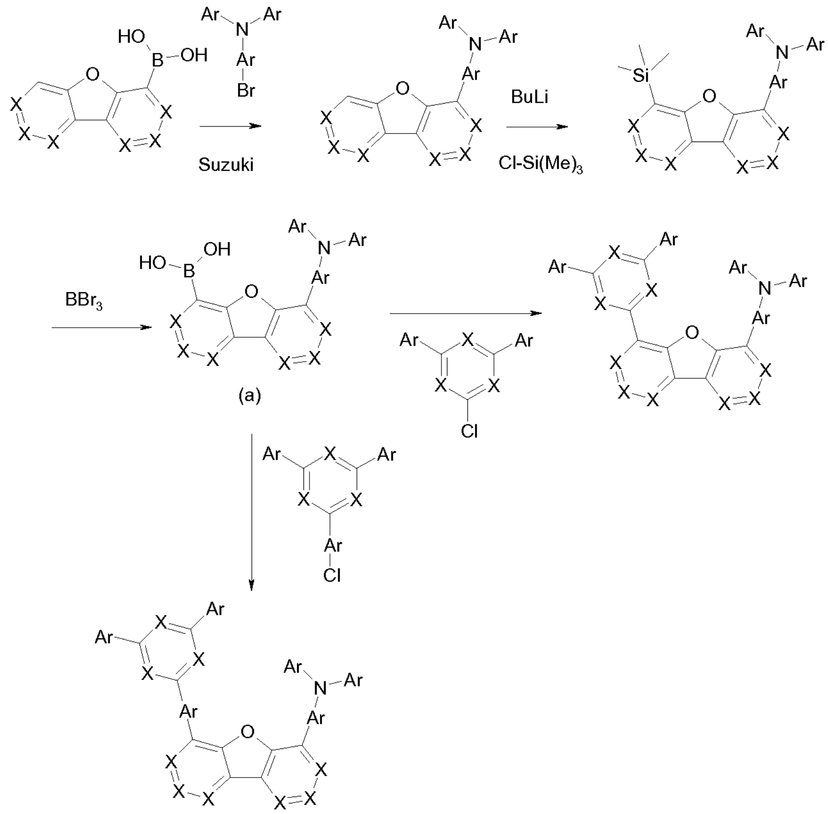


wobei die Gruppe enthaltend die Einheit $N(Ar_3)$ die LTG und die andere Gruppe die ETG darstellt.

## Schema 4

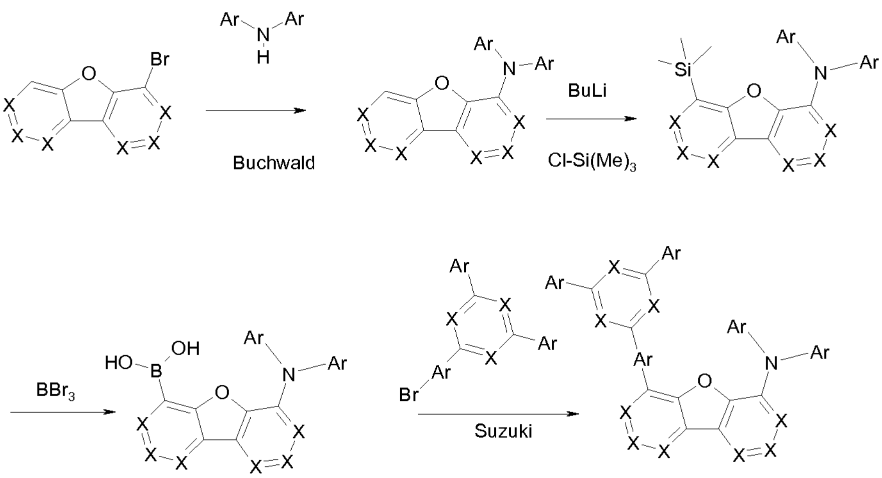

## Schema 5

Eine weitere Möglichkeit zur Herstellung der erfindungsgemäßen Verbindungen besteht in der Umsetzung eines Dihalogenids (Hal = Br, I) mit 1 eq der ensprechenden Boronsäure und anschließebe Szuki-Kupplung zum gewünschten Produckt, wobei sich der Syntheseablauf ähnlichen Schritten wie in Schema 1 gezeigt bedient.

Eine weitere Möglichkeit ist die Umsetzung des Dihalogenids mit 2 eq der Boronsäure der ETG.

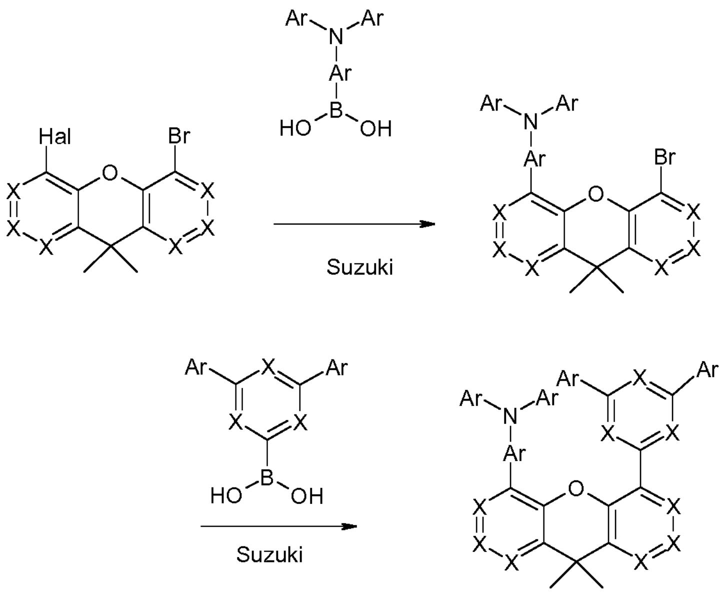

## Schema 6

Viele der Dihalogenide (a) oder Diboronsäuren (b) sind kommerziell erhältlich oder können wie in Schema 6 angegeben synthetisiert werden. Sie können anschließend über Suzuki-Kupplungen zu den gewünschten Produkten umgesetzt werden.

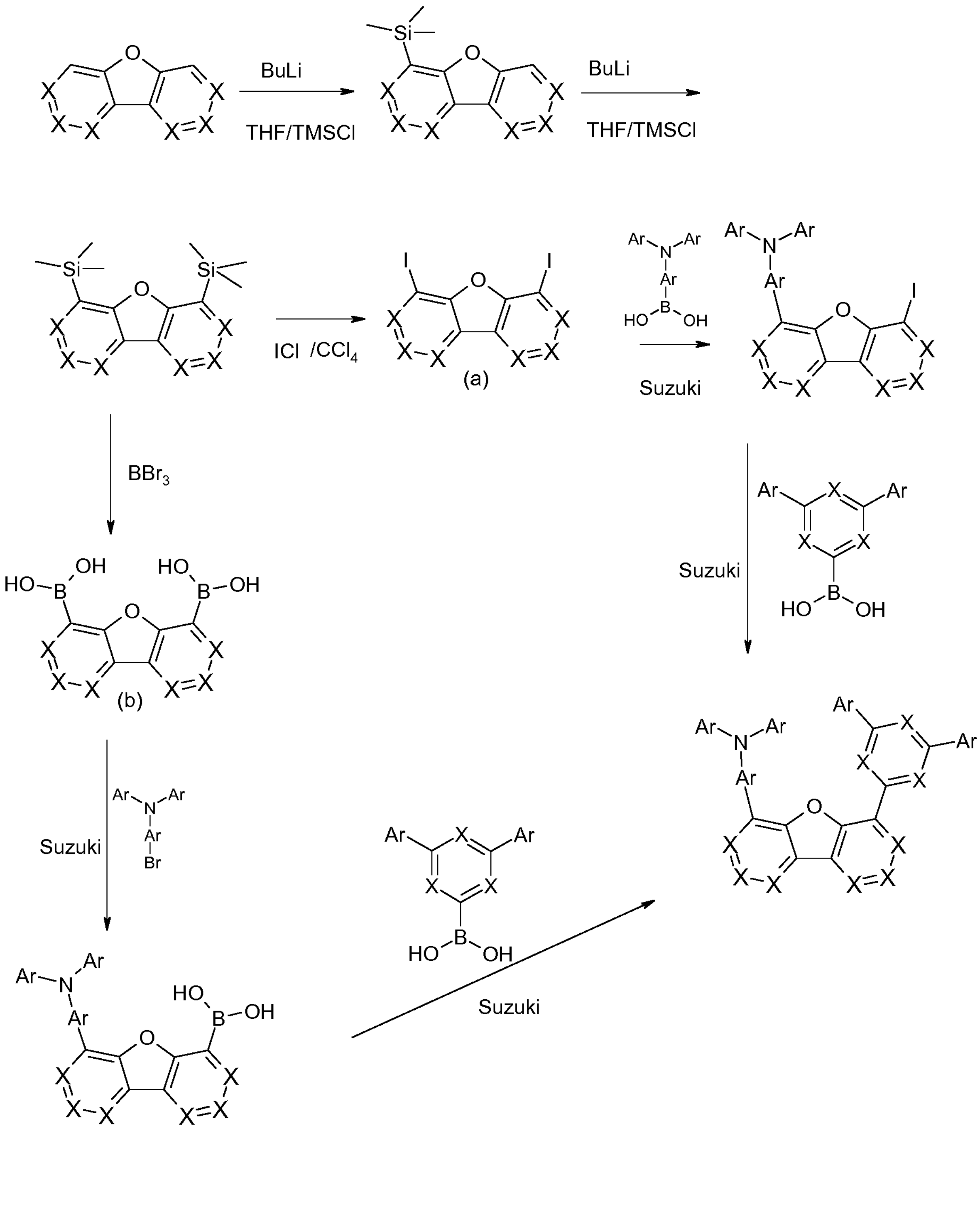

## Schema 7

Eine weitere Möglichkeit, erfindungsgemäße Verbindungen herzustellen ist die Umsetzung von Carbazol-Derivaten gefolgt von einer Ullmann- oder Buchwald-Kupplung.

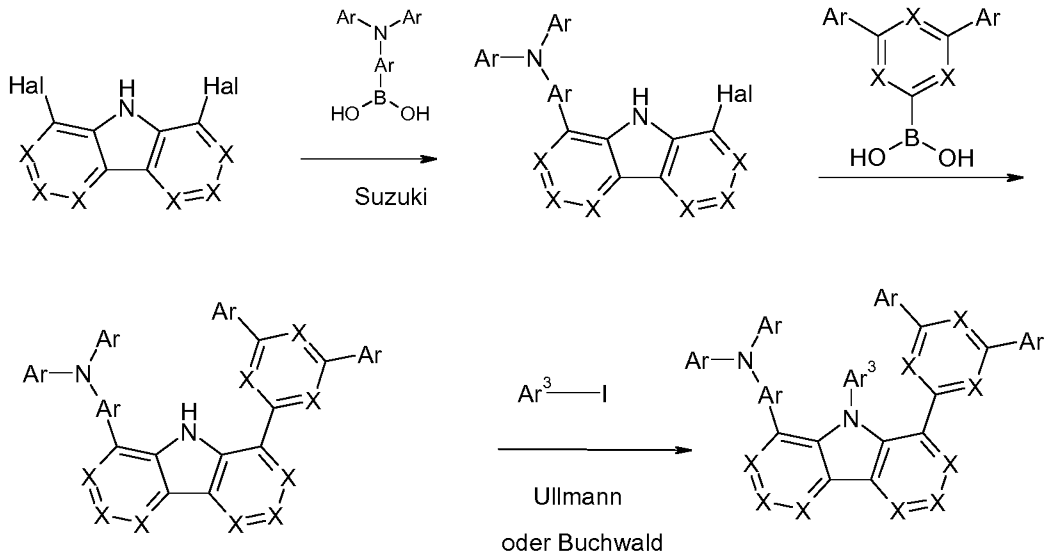


Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die folgende Übersicht enthält eine beispielhafte Darstellung erfindungsgemäßer Verbindungen, die nach einem der hierin beschriebenen Verfahren hergestellt werden können.

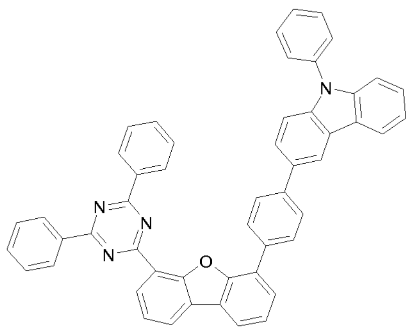

Formel (17)

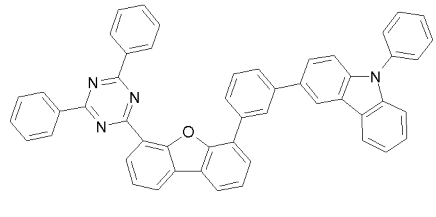

Formel (18)

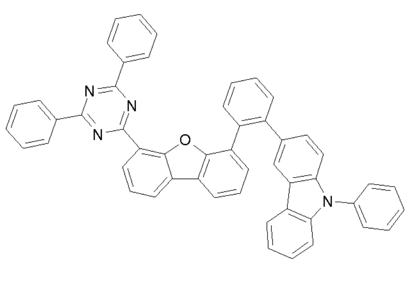

Formel (19)

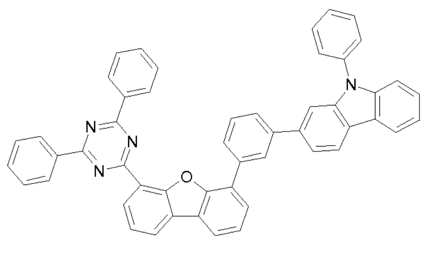

Formel (20)

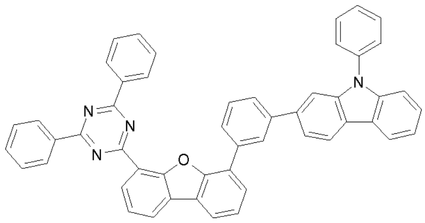

Formel (21)

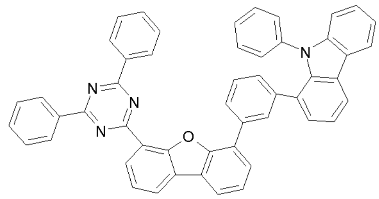

Formel (22)

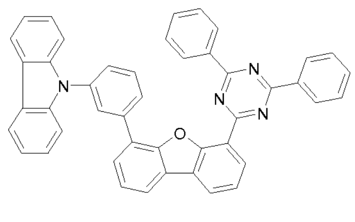

Formel (23)

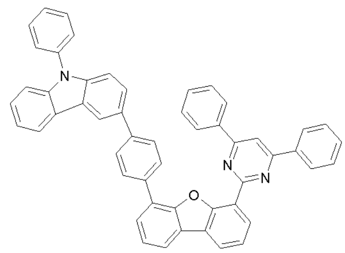

Formel (24)

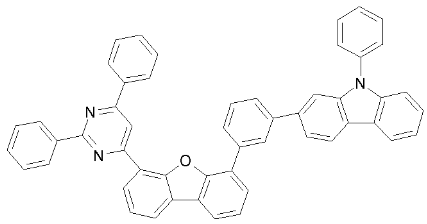

Formel (25)

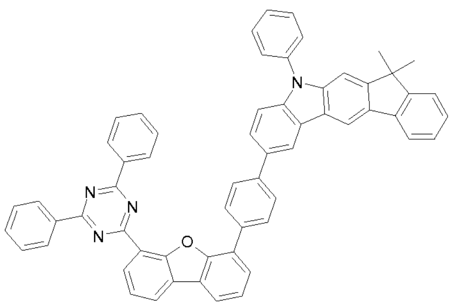

Formel (27)

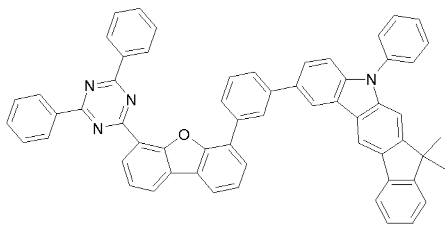

Formel (28)

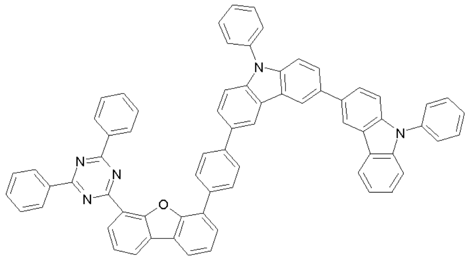

Formel (29)

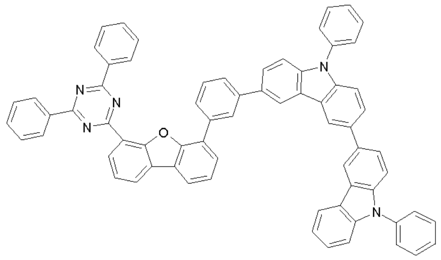

Formel (30)

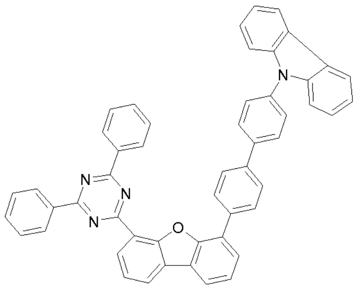

Formel (31)

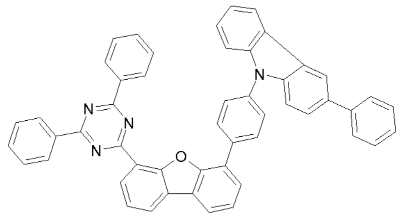

Formel (32)

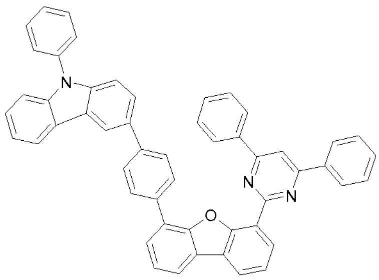

Formel (33)

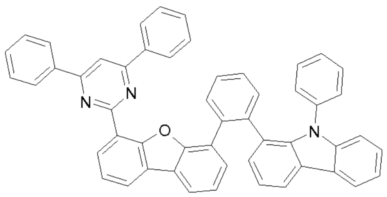

Formel (34)

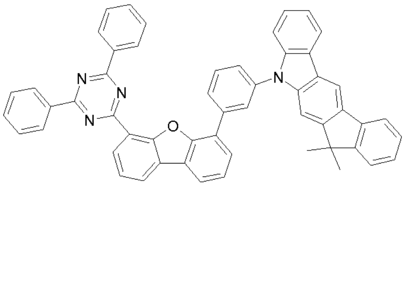

Formel (35)

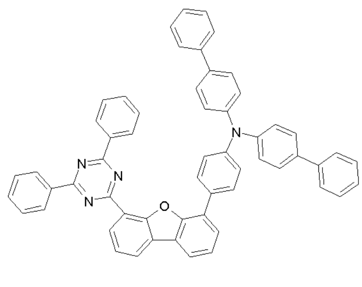

Formel (36)

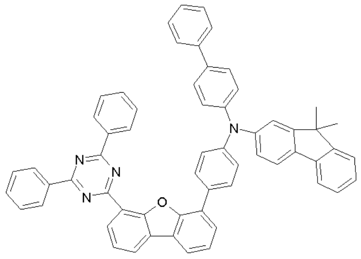

Formel (37)

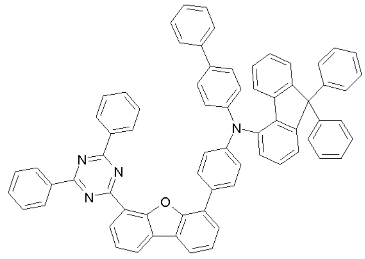

Formel (38)

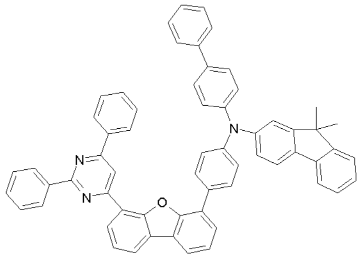

Formel (39)

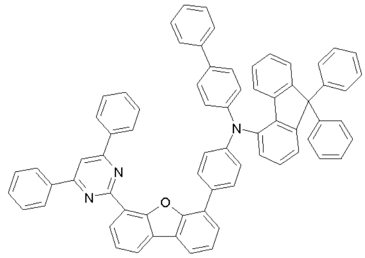

Formel (40)

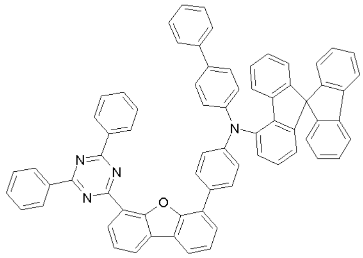

Formel (41)

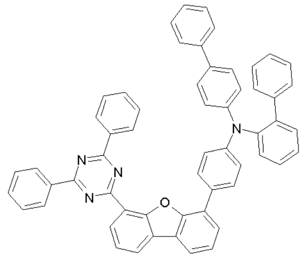

Formel (42)

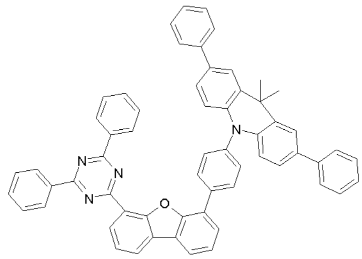

Formel (43)

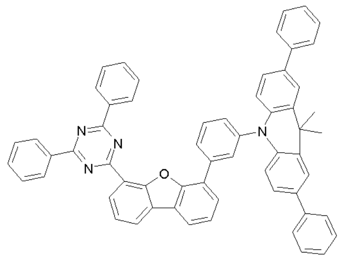

Formel (44)

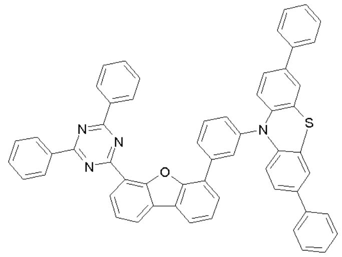

Formel (45)

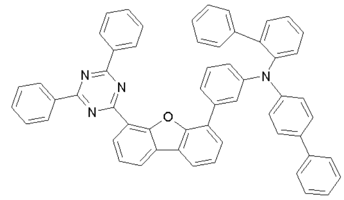

Formel (46)

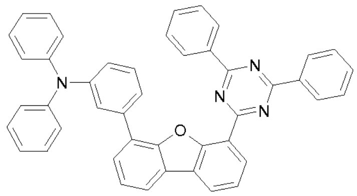

Formel (47)

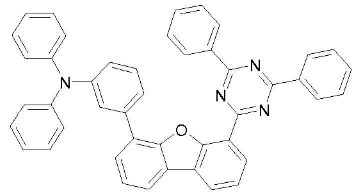

Formel (48)

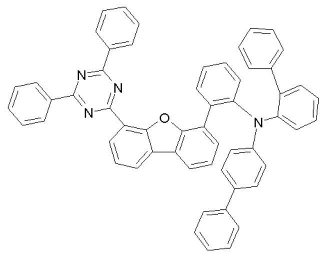

Formel (49)

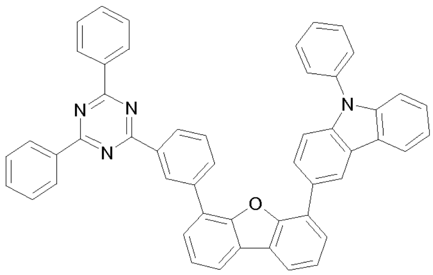

Formel (50)

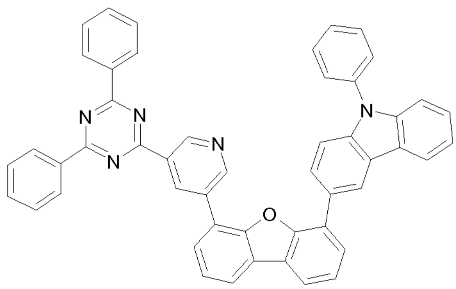

Formel (51)

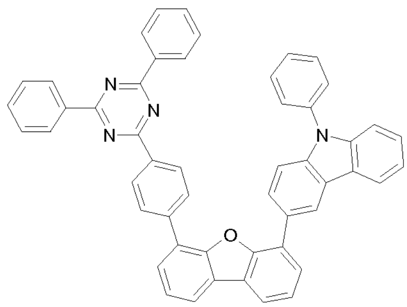

Formel (52)

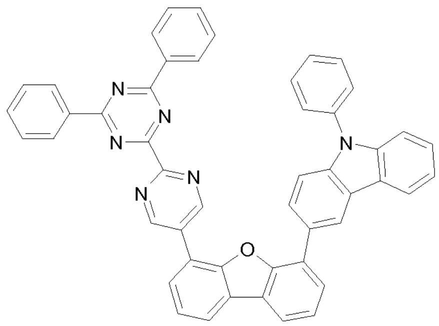

Formel (53)

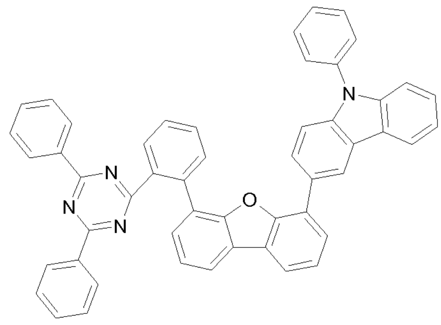

Formel (54)

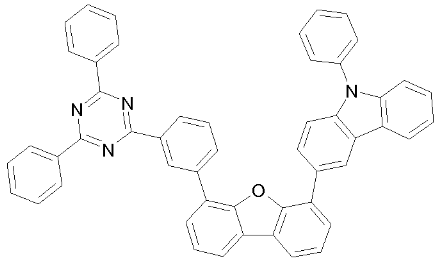

Formel (55)

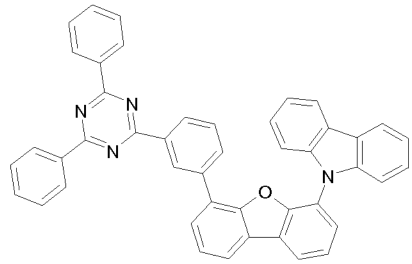

Formel (56)

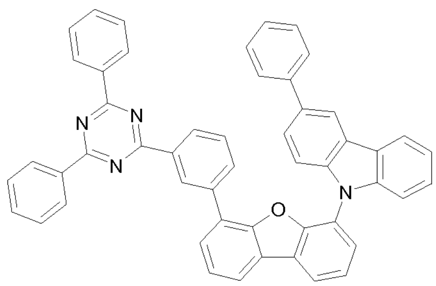

Formel (57)

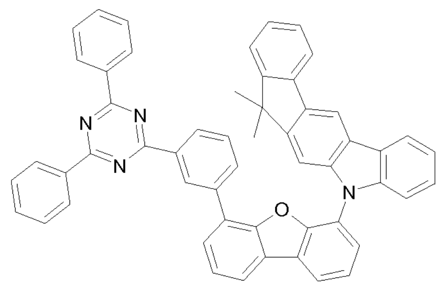

Formel (58)

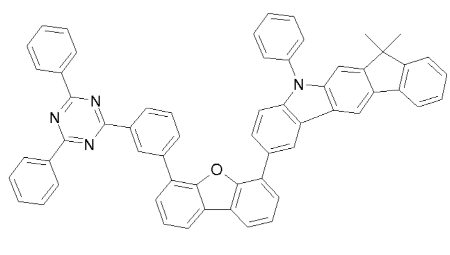

Formel (59)

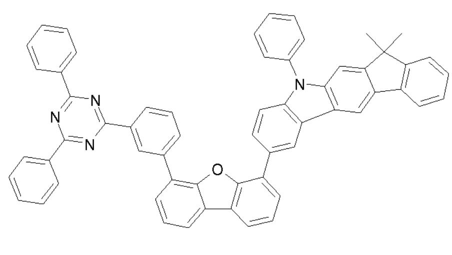

Formel (60)

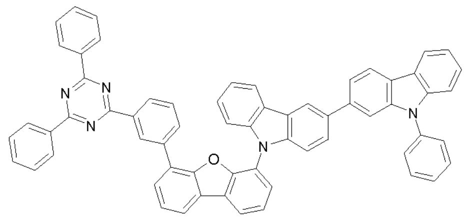

Formel (61)

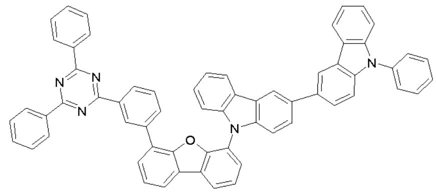

Formel (62)

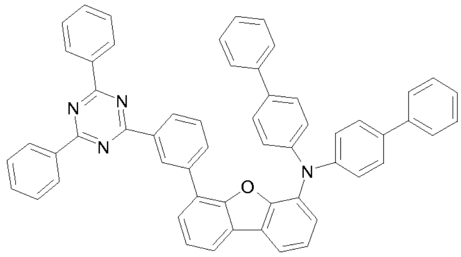

Formel (63)

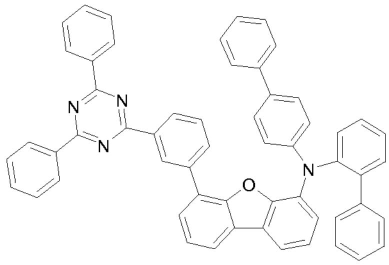

Formel (64)

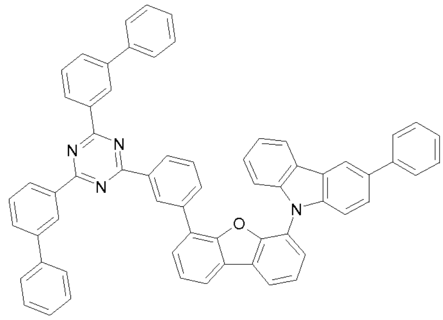

Formel (65)

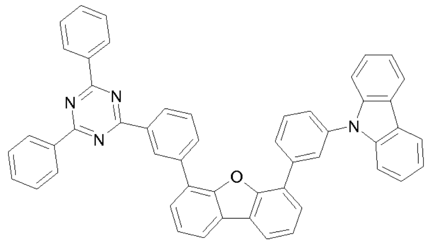

Formel (66)

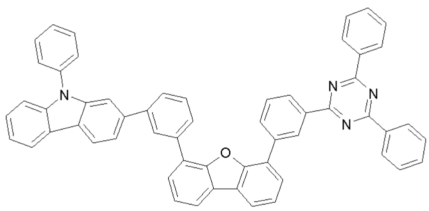

Formel (67)

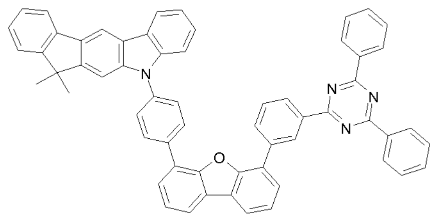

Formel (68)

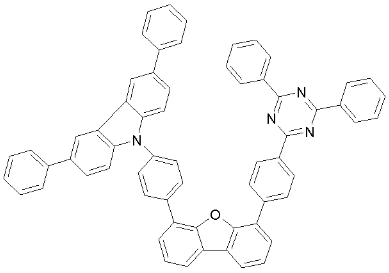

Formel (69)

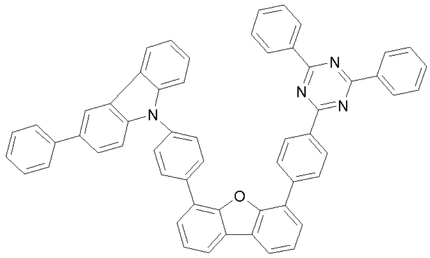

Formel (70)

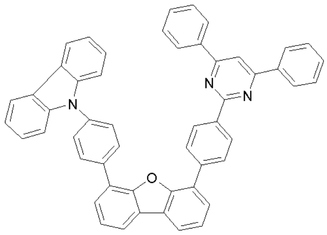

Formel (71)

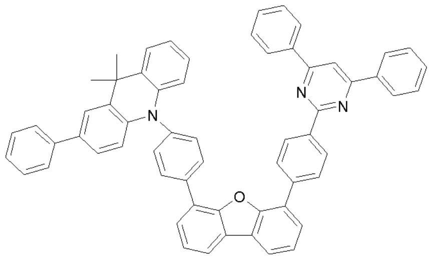

Formel (72)

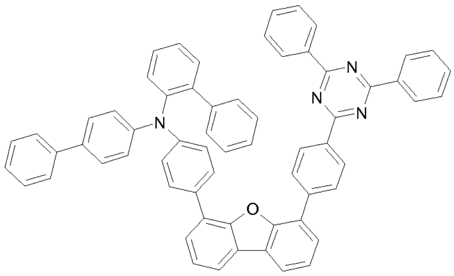

Formel (73)

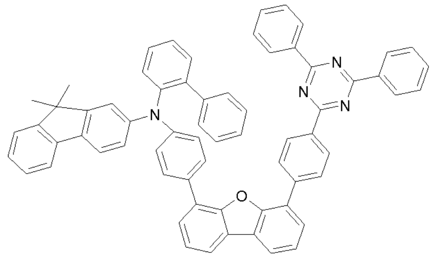

Formel (74)

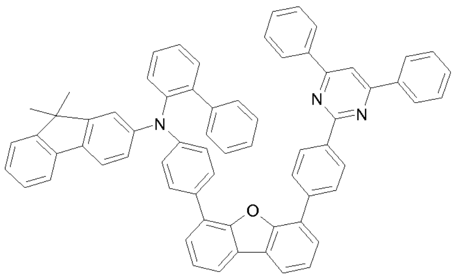

Formel (75)

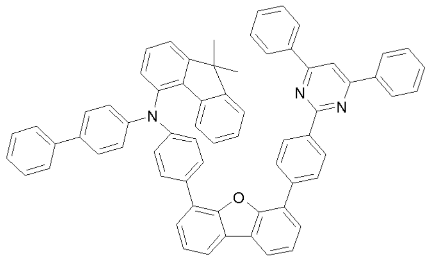

Formel (76)

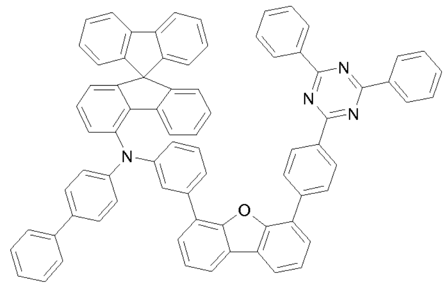

Formel (77)

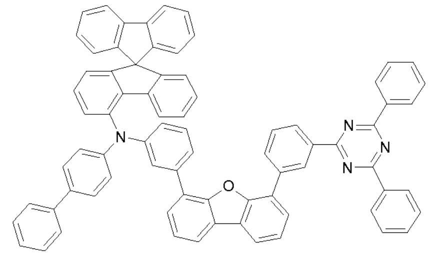

Formel (78)

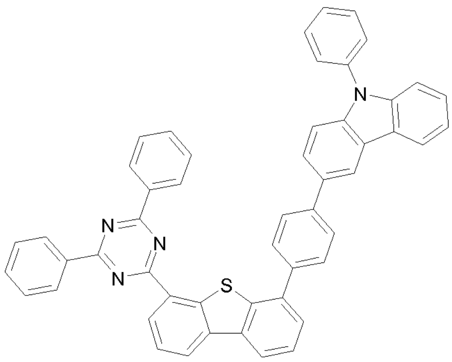

Formel (79)

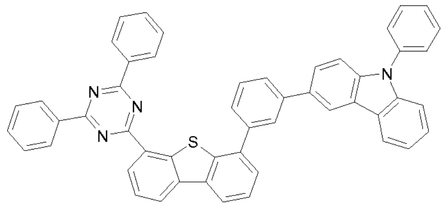

Formel (80)

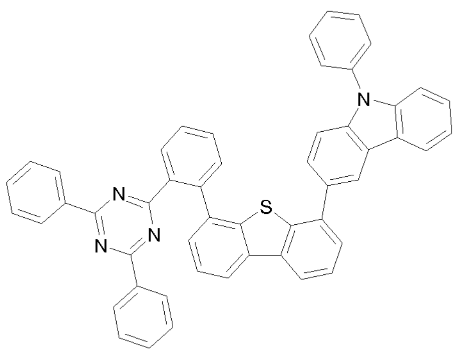

Formel (81)

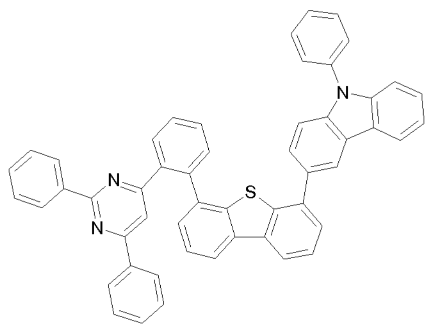

Formel (82)

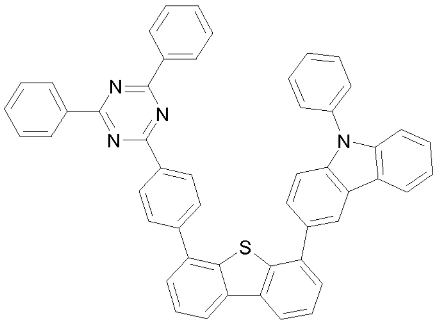

Formel (83)

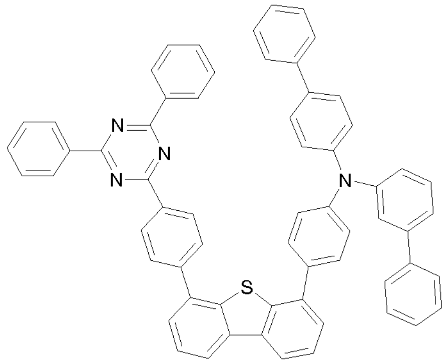

Formel (84)

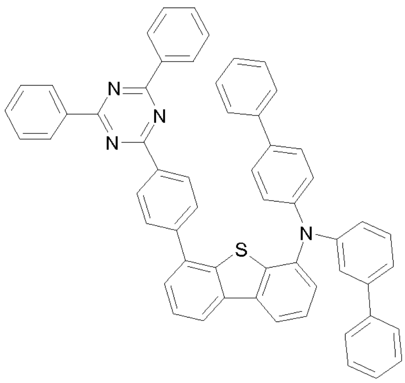

Formel (85)

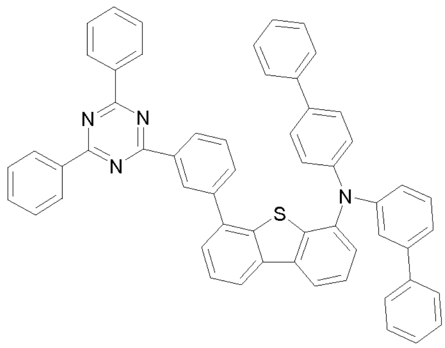

Formel (86)

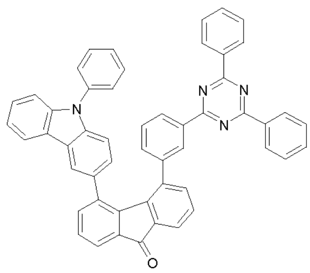

Formel (87)

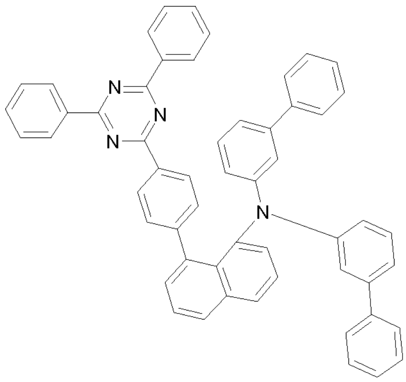

Formel (89)

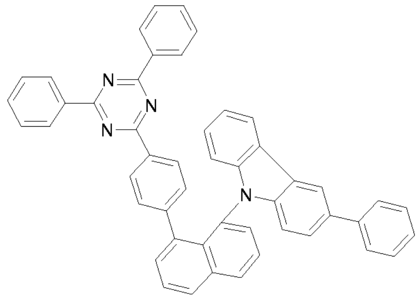

Formel (90)

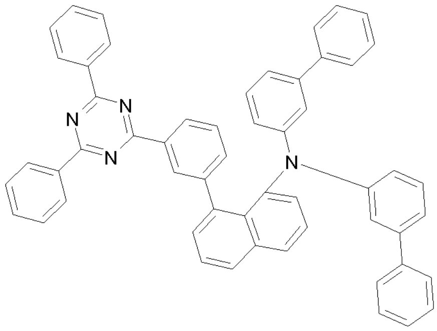

Formel (91)

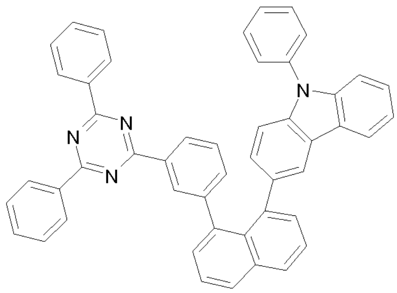

Formel (92)

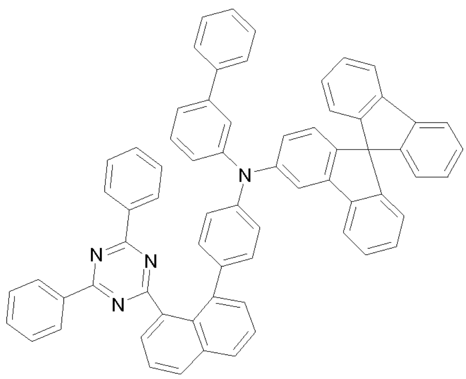

Formel (93)

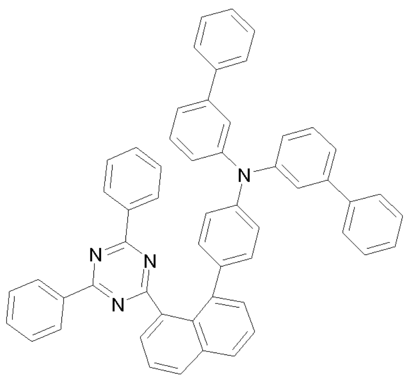

Formel (94)

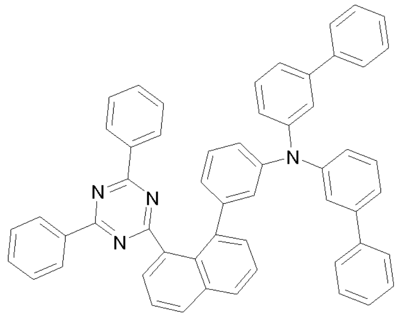

Formel (95)

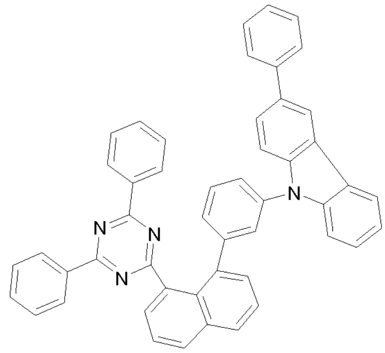

Formel (96)

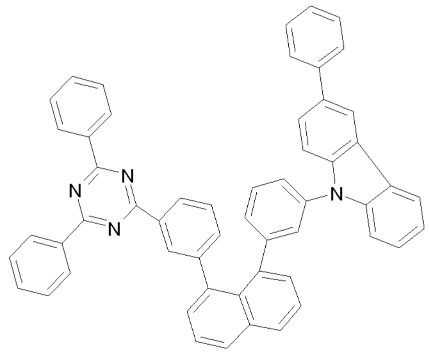

Formel (97)

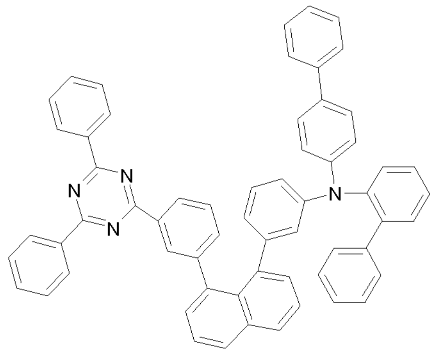

Formel (98)

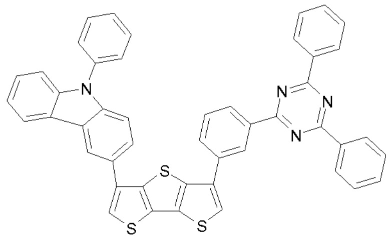

Formel (101)

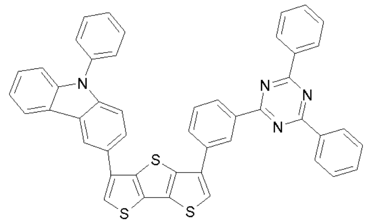

Formel (102)

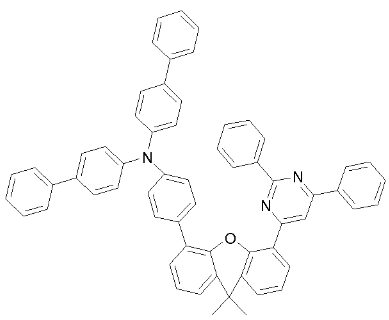

Formel (103)

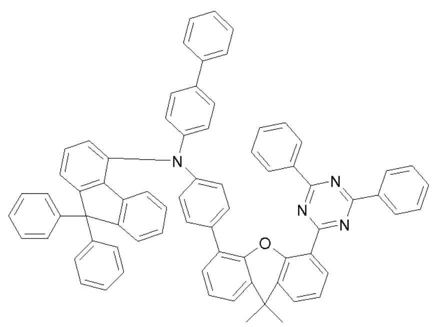

Formel (104)

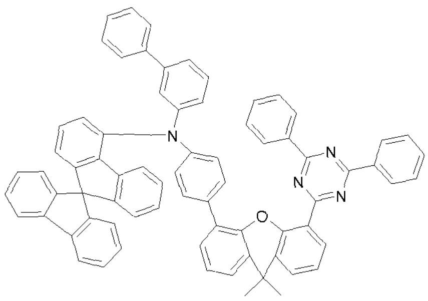

Formel (105)

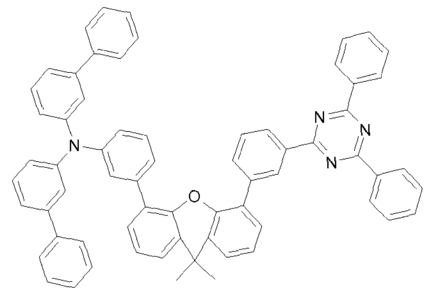

Formel (106)

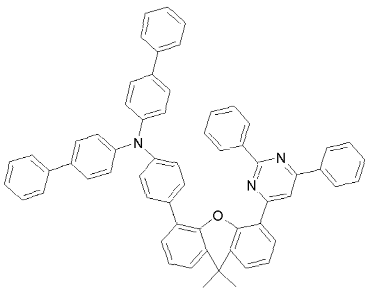

Formel (107)

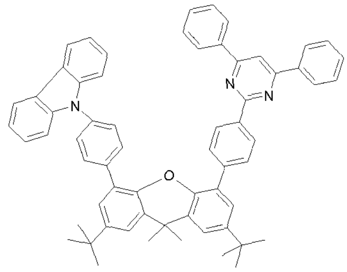

Formel (108)

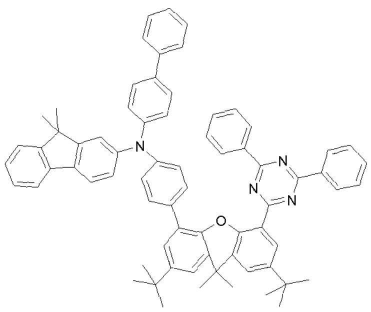

Formel (109)

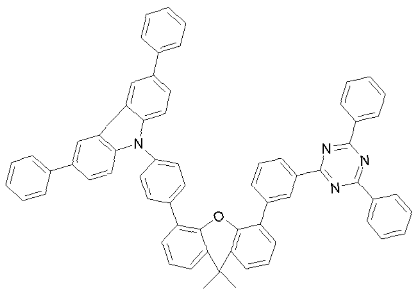

Formel (110)

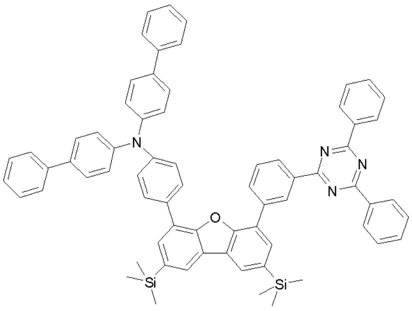

Formel (111)

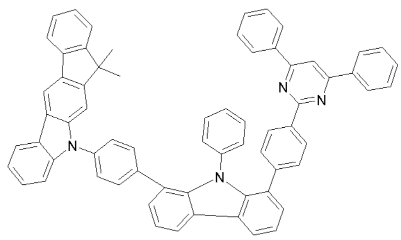

Formel (112)

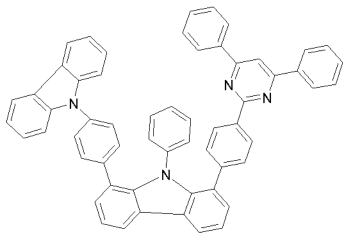

Formel (113)

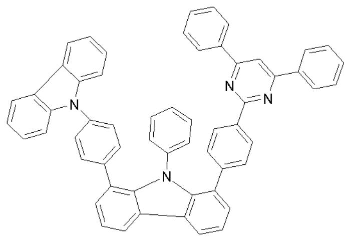

Formel (114)

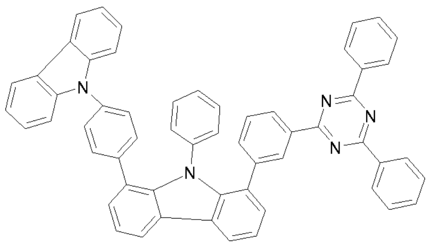

Formel (115)

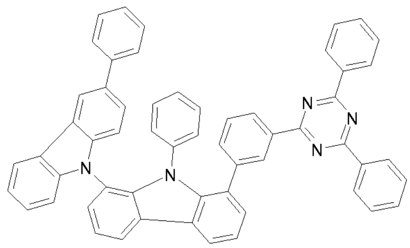

Formel (116)

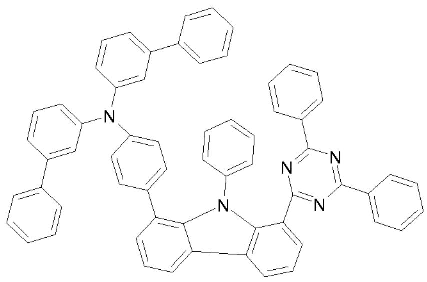

Formel (117)

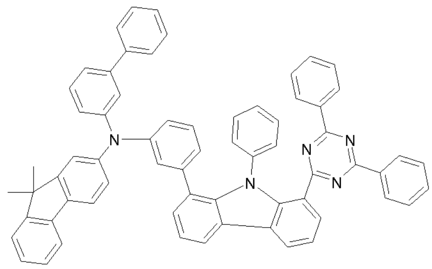

Formel (118)

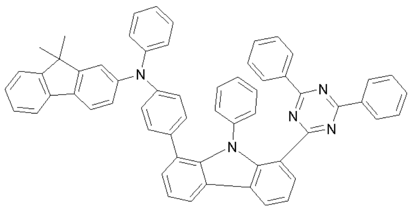

Formel (119)

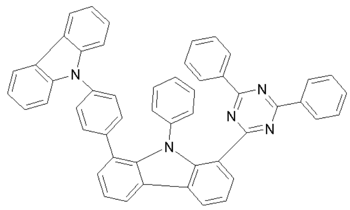

Formel (120)

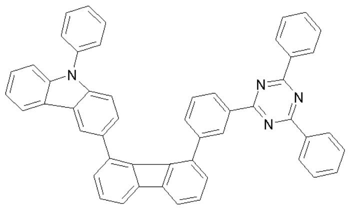

Formel (121)

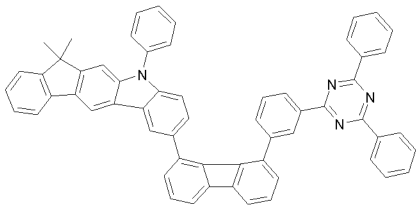

Formel (122)

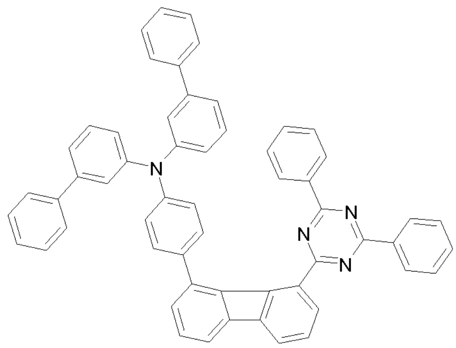

Formel (123)

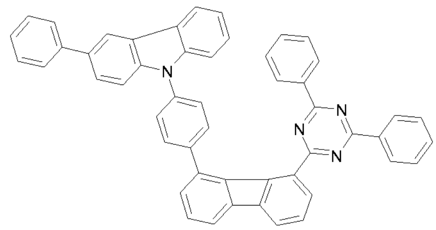

Formel (124)

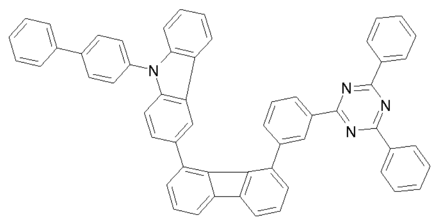

Formel (125)

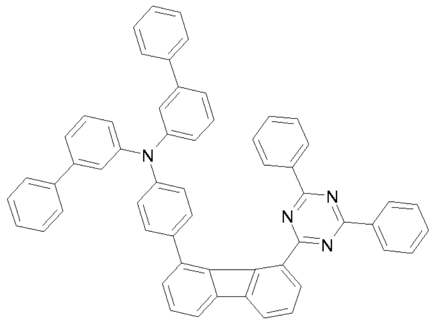

Formel (126)

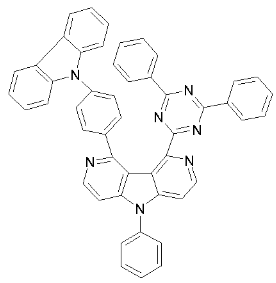

Formel (129)

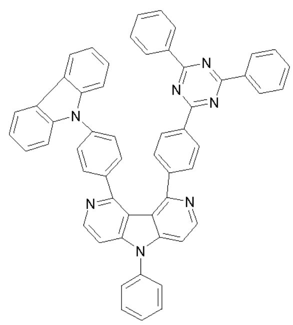

Formel (130)

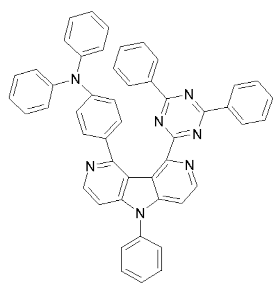

Formel (131)

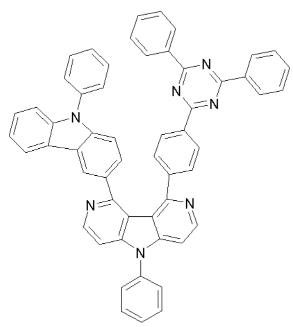

Formel (132)

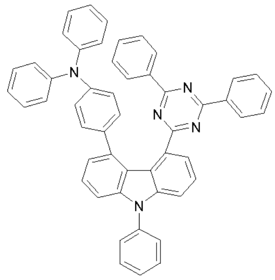

Formel (133)

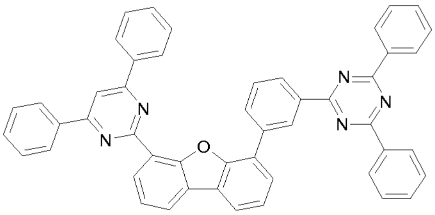

Formel (134)

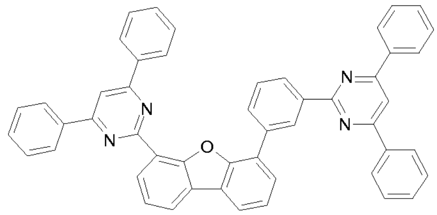

Formel (135)

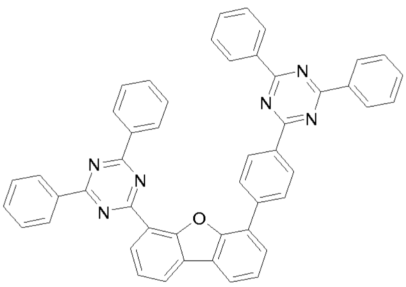

Formel (136)

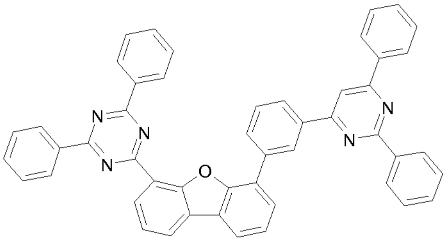

Formel (137)

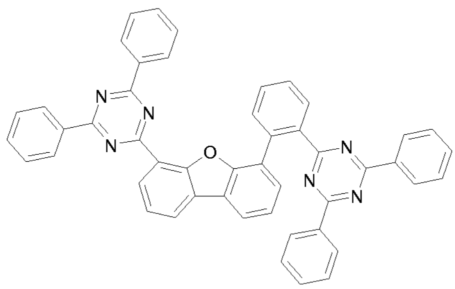

Formel (138)

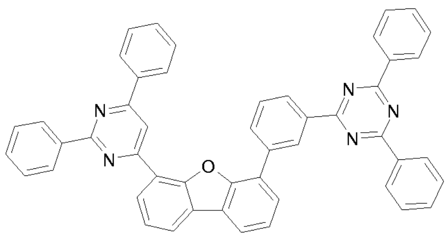

Formel (139)

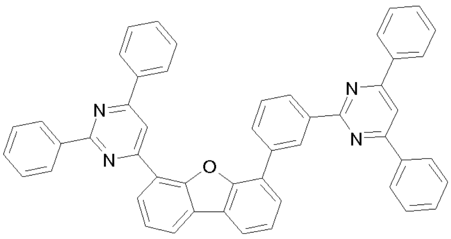

Formel (140)

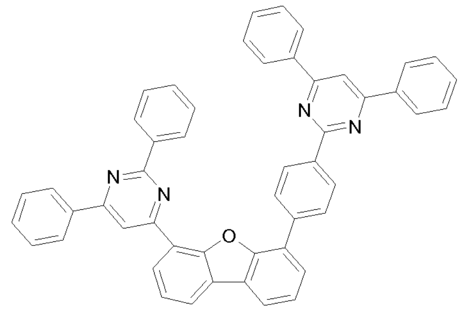

Formel (141)

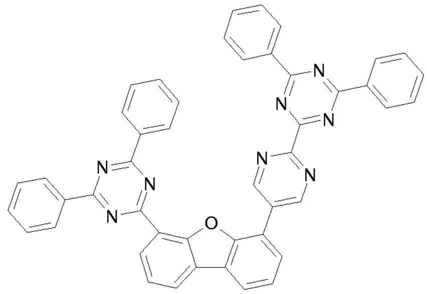

Formel (144)

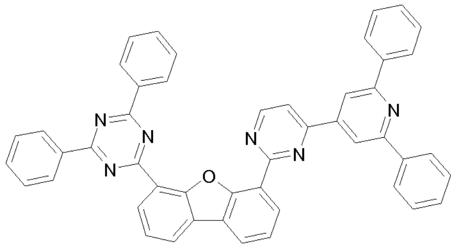

Formel (145)

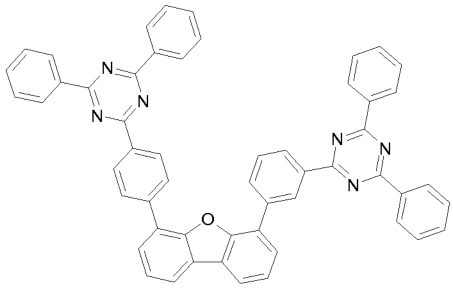

Formel (146)

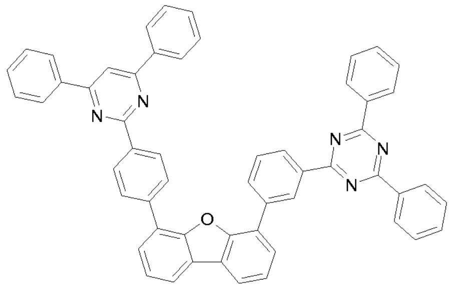

Formel (147)

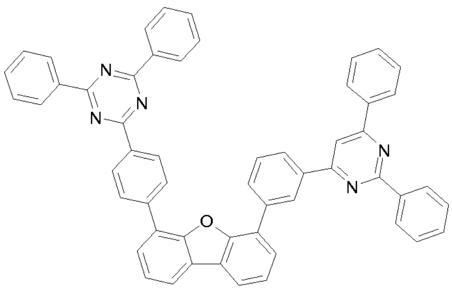

Formel (148)

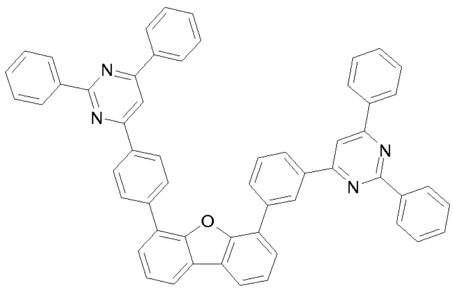

Formel (149)

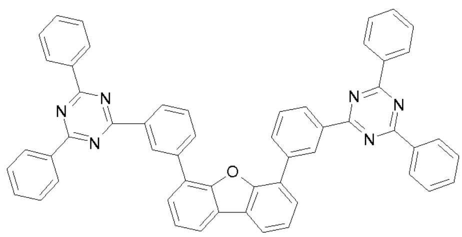

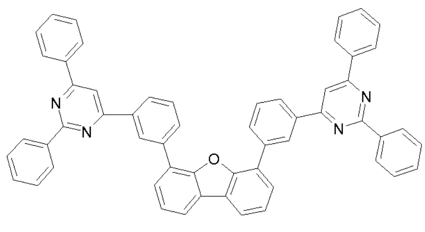

Formel (150) Formel (151)

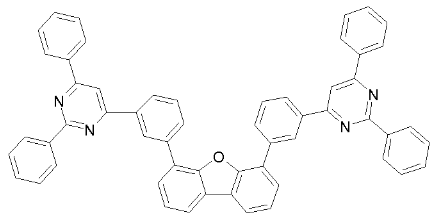

Formel (153)

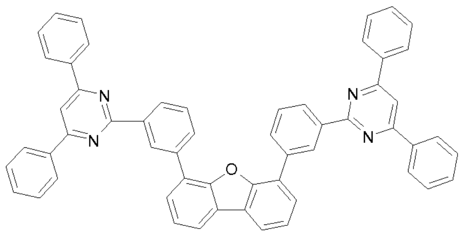

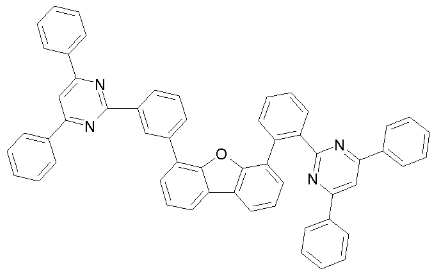

Formel (154) Formel (155)

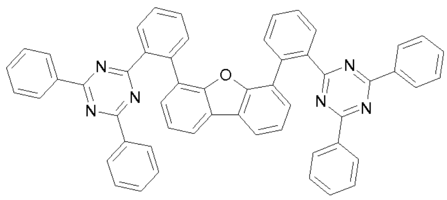

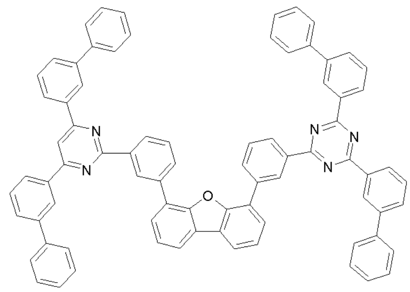

Formel (156) Formel (157)

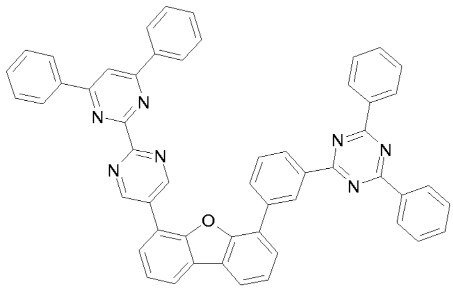

Formel (158)

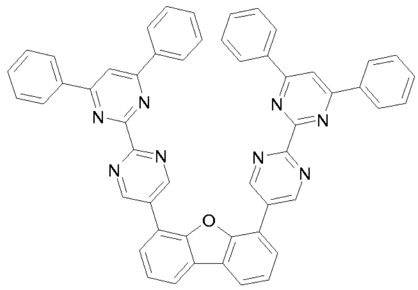

Formel (159)

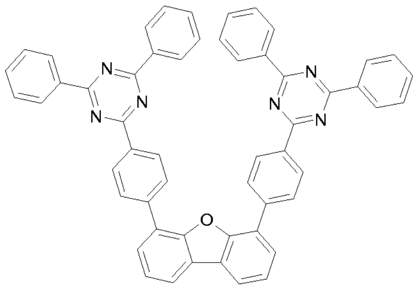

Formel (160)

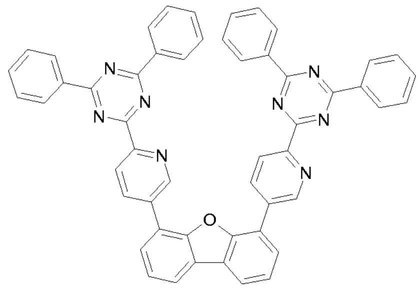

Formel (161)

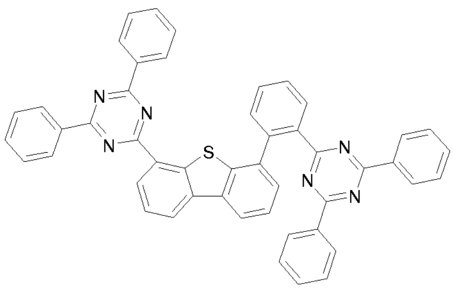

Formel (162)

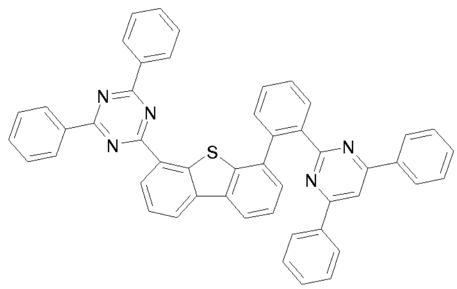

Formel (163)

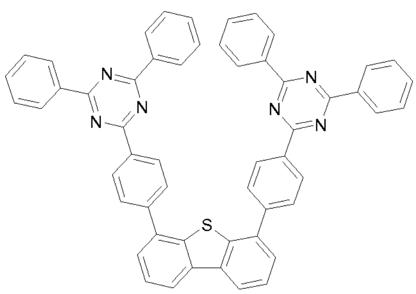

Formel (164)

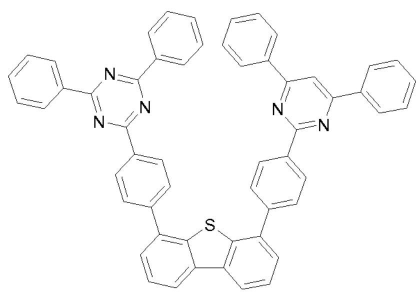

Formel (165)

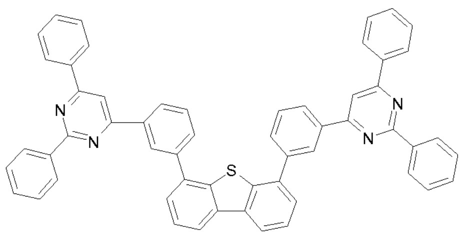

Formel (166)

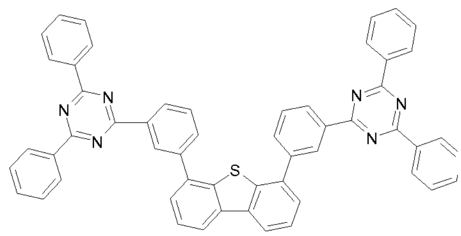

Formel (167)

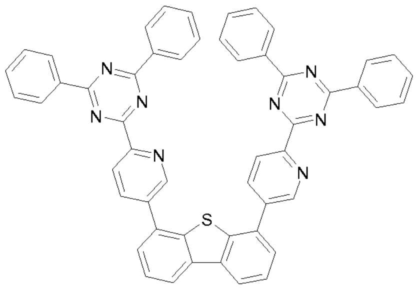

Formel (168)

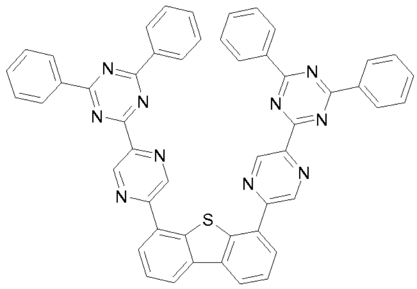

Formel (169)

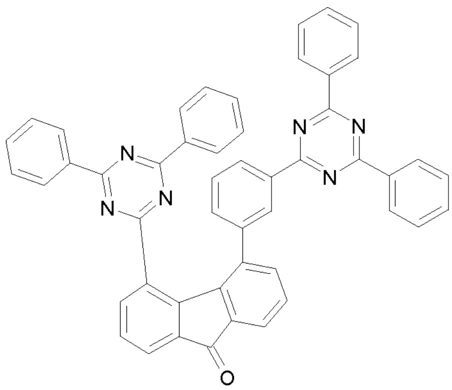

Formel (170)

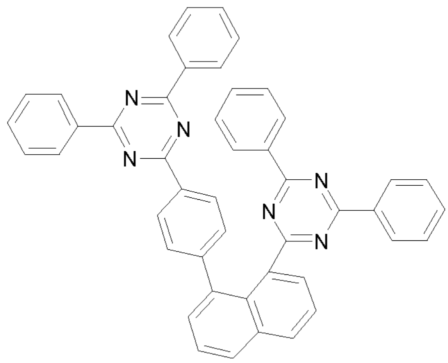

Formel (172)

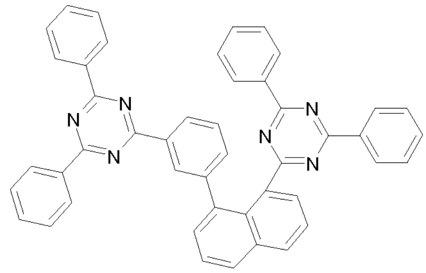

Formel (173)

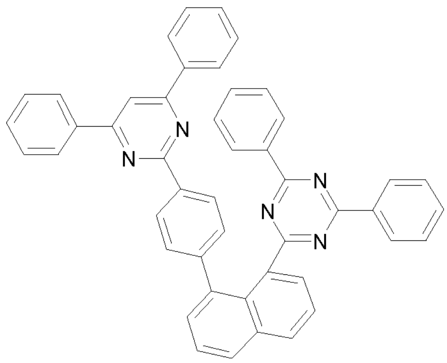

Formel (174)

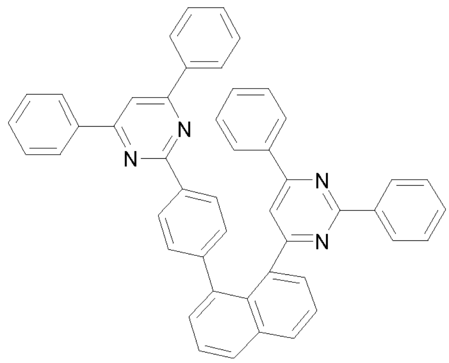

Formel (175)

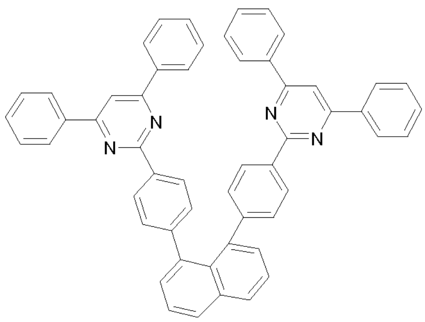

Formel (176)

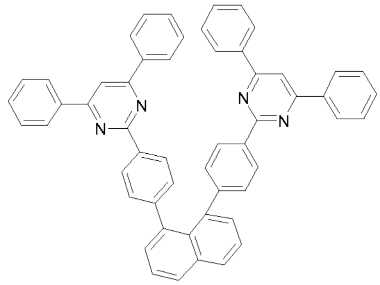

Formel (177)

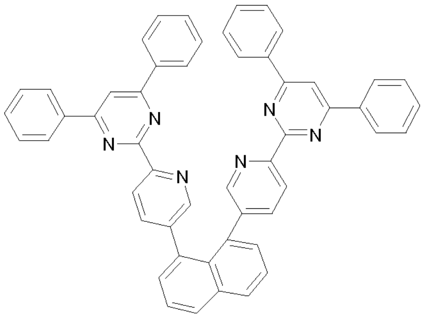

Formel (178)

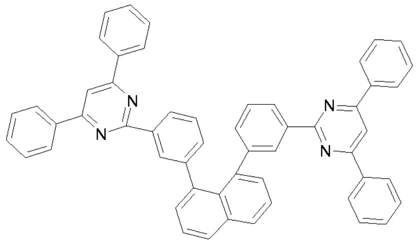

Formel (179)

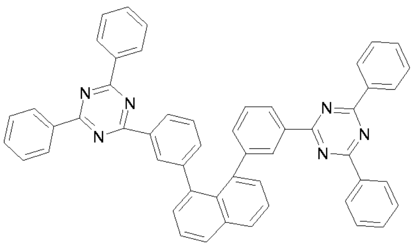

Formel (180)

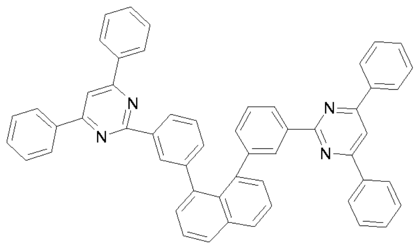

Formel (181)

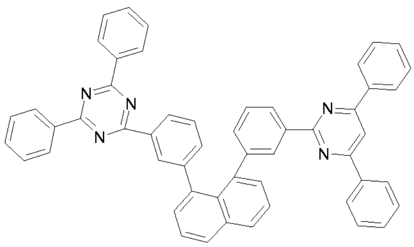

Formel (182)

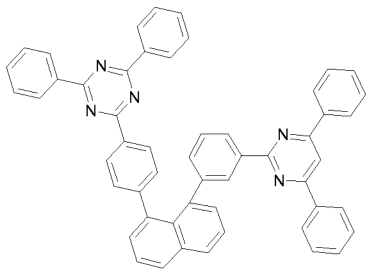

Formel (183)

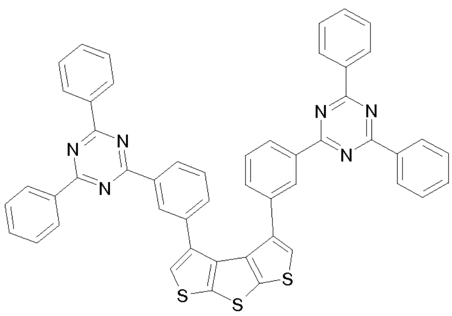

Formel (184)

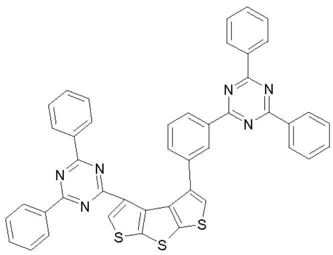

Formel (185)

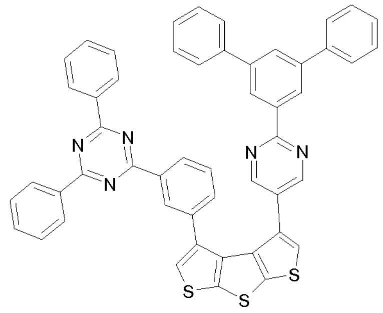

Formel (186)

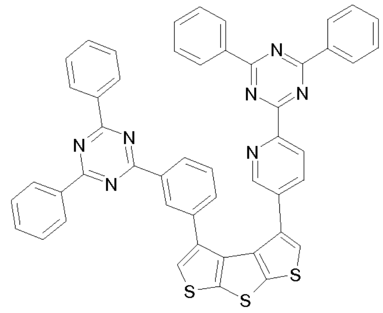

Formel (187)

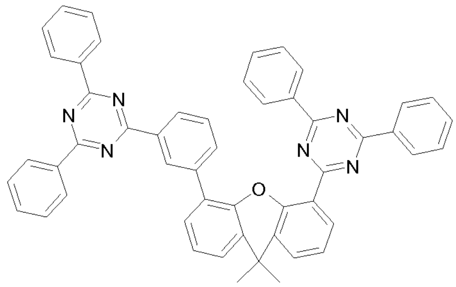

Formel (188)

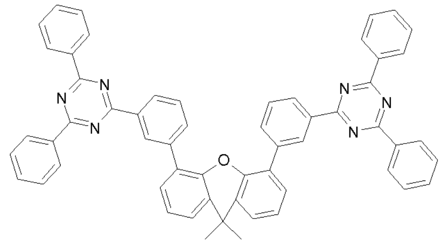

Formel (189)

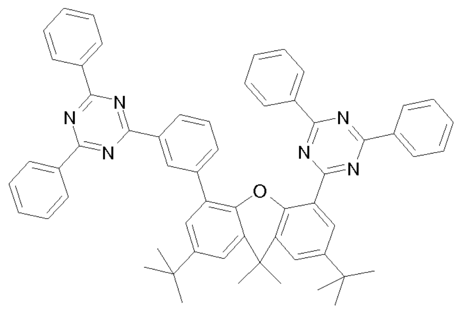

Formel (190)

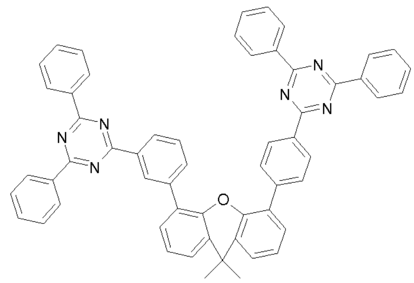

Formel (191)

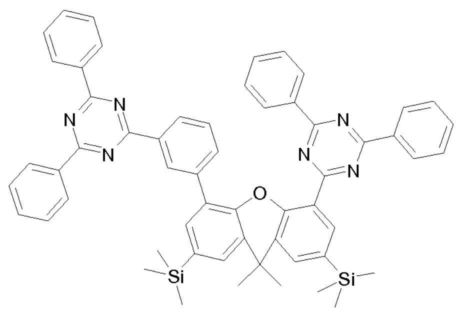

Formel (192)

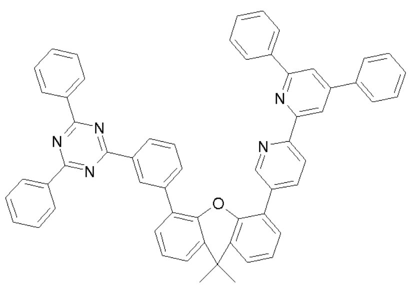

Formel (193)

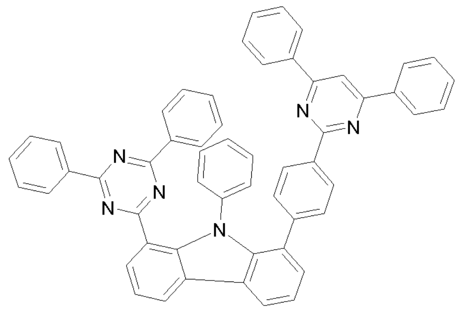

Formel (194)

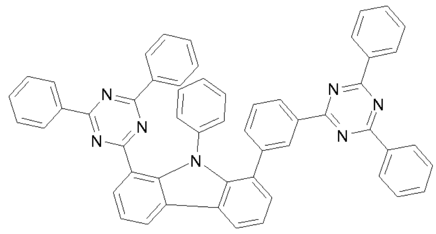

Formel (195)

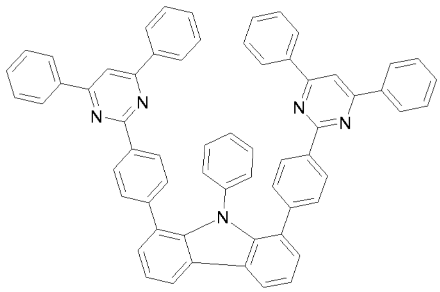

Formel (196)

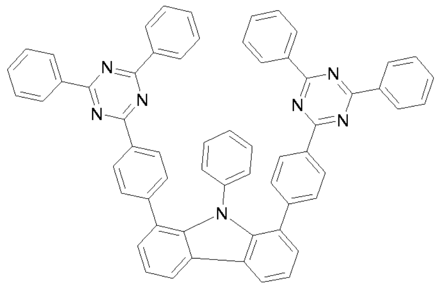

Formel (197)

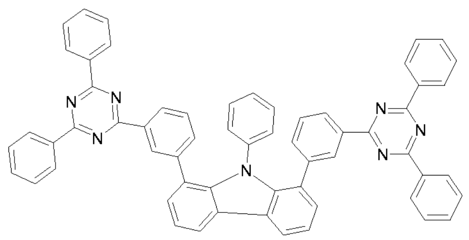

Formel (198)

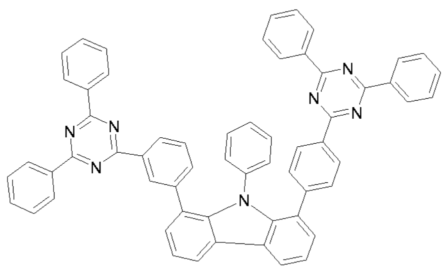

Formel (199)

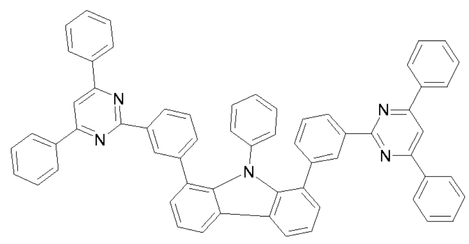

Formel (200)

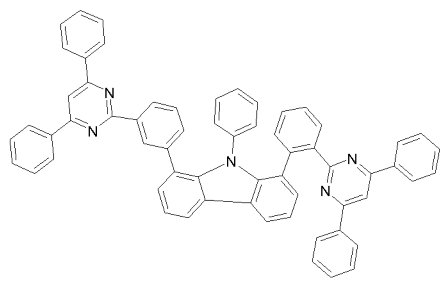

Formel (201)

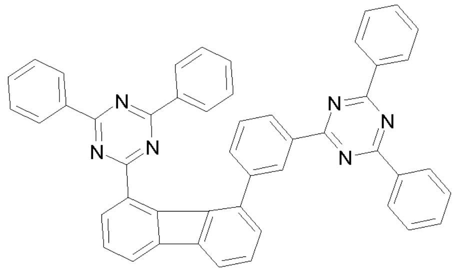

Formel (202)

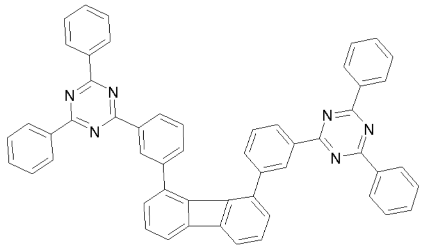

Formel (203)

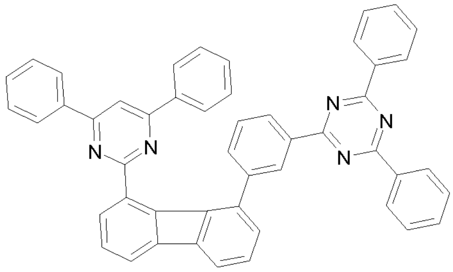

Formel (204)

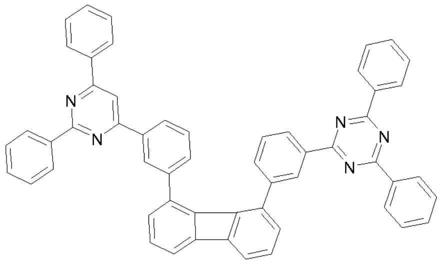

Formel (205)

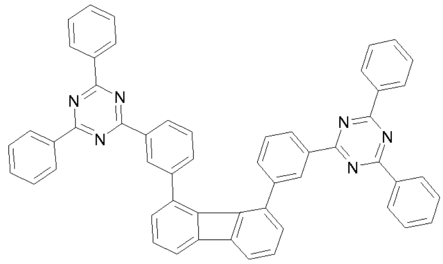

Formel (206)

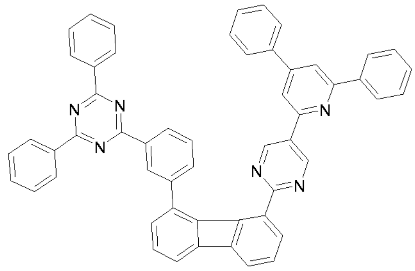

Formel (207)

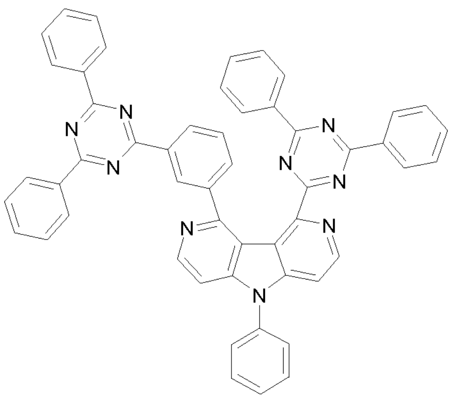

Formel (212)

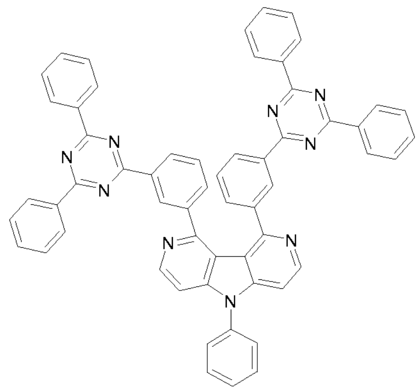

Formel (213)

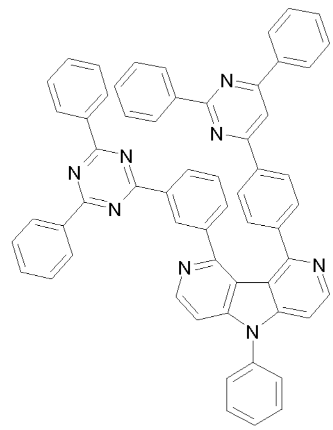

Formel (214)

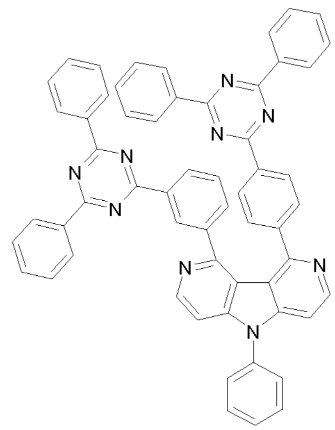

Formel (215)

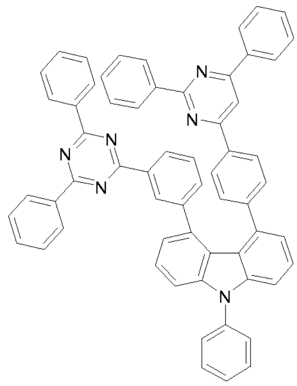

Formel (216)

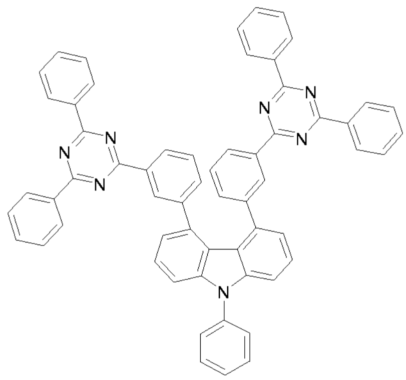

Formel (217)

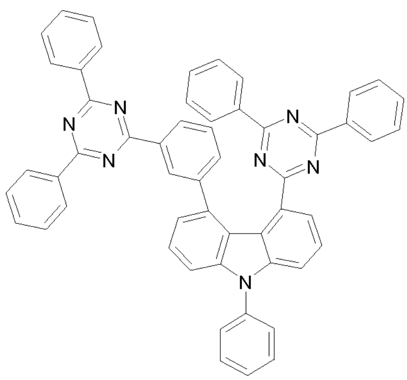

Formel (218)

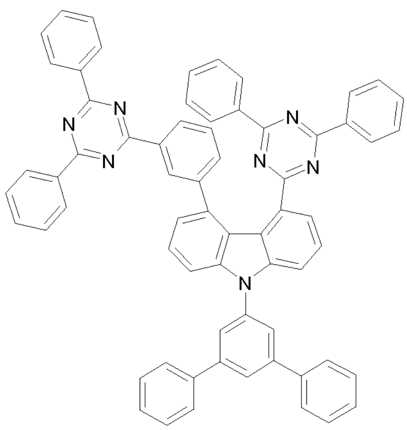

Formel (219)

Weiterer Gegenstand der Erfindung ist die Verwendung einer Verbindung der Formel (1) in einer elektronischen Vorrichtung, bevorzugt in einer elektronentransportierenden und/oder in einer emittierenden Schicht.

Die erfindungsgemäße elektronische Vorrichtung ist bevorzugt gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs, LECs oder LEECs), organischen Laserdioden (O-Laser) und organischen lichtemittierenden Dioden (OLEDs). Besonders bevorzugt sind die organischen Elektrolumineszenzvorrichtungen, ganz besonders bevorzugt die OLECs und OLEDs und insbesondere bevorzugt die OLEDs.

Die organische Schicht enthaltend die Verbindung der Formel (1) ist bevorzugt eine Schicht mit elektronentransportierender Funktion. Besonders bevorzugt ist sie eine Elektroneninjektionsschicht, Elektronentransportschicht, eine Lochblockierschicht oder eine emittierende Schicht.

In einer ganz besonders bevorzugten Ausführungsform sind sowohl $G^1$ als auch $G^2$ in der Verbindung der Formel (1) ETGs und die Verbindungen der Formel (1) sind dann ganz besonders bevorzugt in einer Schicht einer zuvor genannten elektronischen Vorrichtung mit elektronentransportierender Funktion, insbesondere bevorzugt befindet sich die Verbindung in einer Elektroneninjektionsschicht (EIL), Elektronentransportschicht (ETL), einer Lochblockierschicht (HBL) oder in einer emittierenden Schicht, wobei eine EIL und ETL insbesondere bevorzugt sind und eine ETL noch bevorzugter ist.

In einer weiteren ganz besonders bevorzugten Ausführungsform ist eine der beiden Gruppen $G^1$ und $G^2$ in der Verbindung der Formel (1) eine LTG und die andere Gruppe eine ETG und die Verbindung der Formel (1) liegt dann dann ganz besonders bevorzugt in einer Schicht einer zuvor genannten elektronischen Vorrichtung mit elektronentransportierender Funktion vor, insbesondere bevorzugt befindet sich die Verbindung in einer Elektroneninjektionsschicht, Elektronentransportschicht, einer Lochblockierschicht oder in einer emittierenden Schicht, wobei noch bevorzugter ist, wenn diese Verbindung in einer emittierenden Schicht vorliegt, insbesondere als Matrixmaterial.

Eine Lochtransportschicht gemäß der vorliegenden Anmeldung ist eine Schicht mit lochtransportierender Funktion, welche sich zwischen Anode und emittierender Schicht befindet.

Eine Elektronenransportschicht gemäß der vorliegenden Anmeldung ist eine Schicht mit elektronentransportierender Funktion, welche sich zwischen Kathode und emittierender Schicht befindet. Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wie oben bereits erwähnt, wird die Verbindung der Formel (1) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt. Dabei wird das Matrixmaterial der Formel (1) in der elektronischen Vorrichtung in Kombination mit einem oder mehreren Dotanden, vorzugsweise phosphoreszierenden Dotanden, vorhanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind

diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Die vorliegenden Erfindung betrifft daher auch eine Zusammensetzung enthaltend wenigstens eine Verbindung gemäß Formel (1) sowie wenigstens ein weiteres Matrixmaterial.

Die vorliegende Erfindung betrifft auch eine Zusammensetzung enthaltend wenigstens eine Verbindung gemäß Formel (1) sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrichtungen.

Weiterhin betrifft die vorliegende Erfindung eine Zusammensetzung enthaltend wenigstens eine Verbindung gemäß Formel (1) sowie wenigstens ein weiteres organisches Halbleitermaterial ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

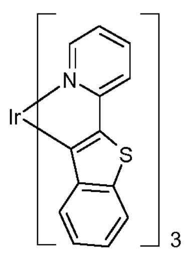
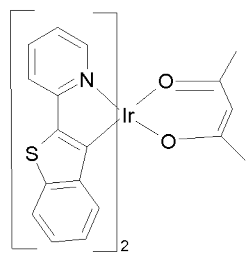
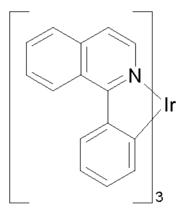

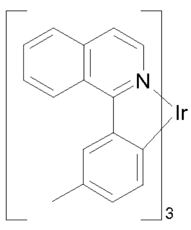
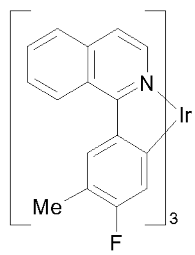
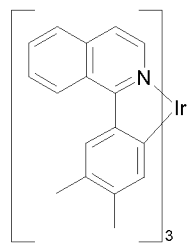

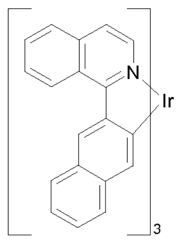

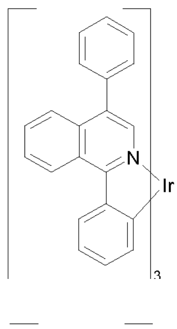

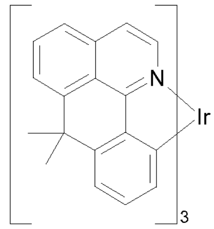

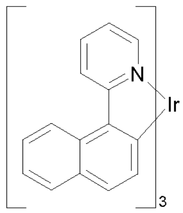

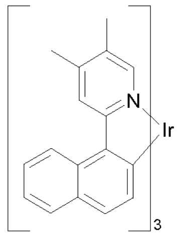

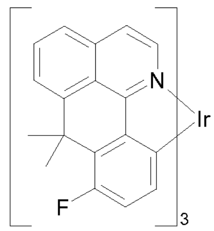

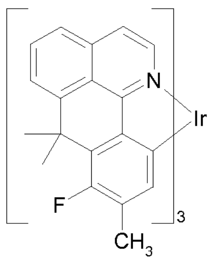

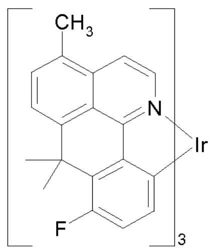

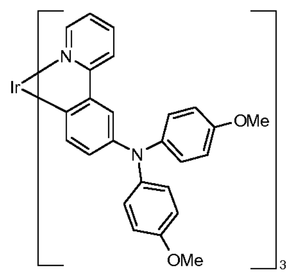

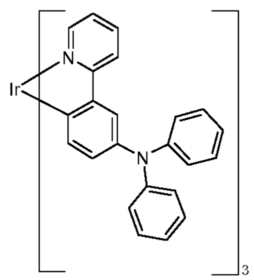

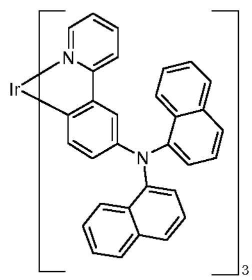

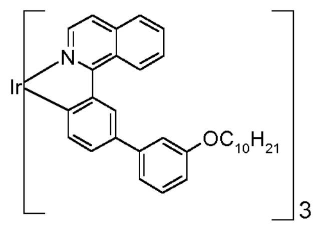

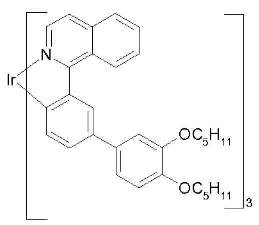

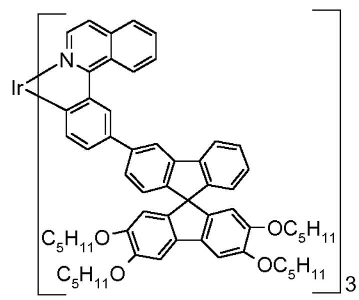

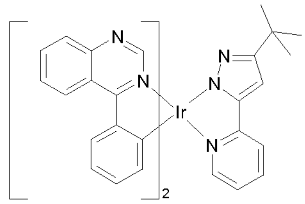

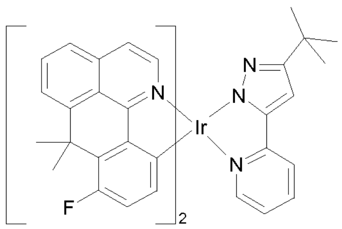

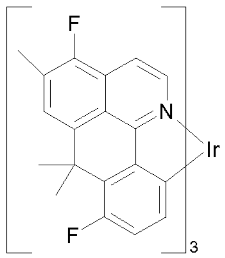

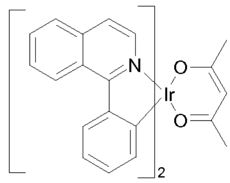

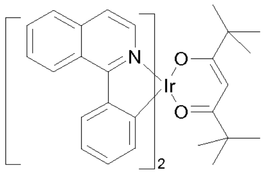

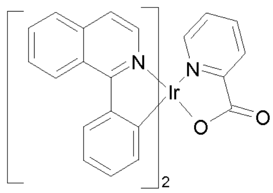

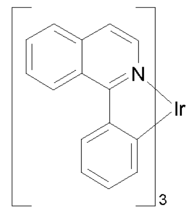

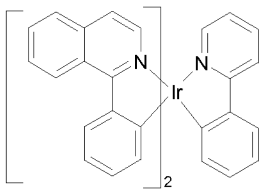

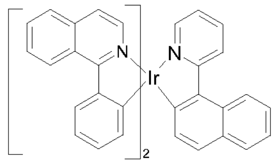

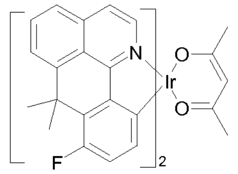

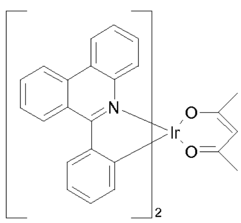

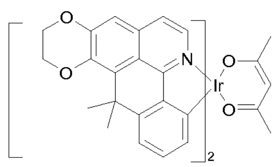

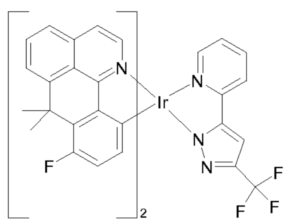

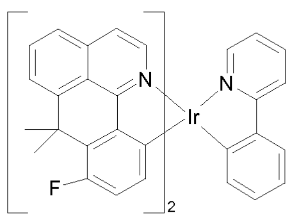

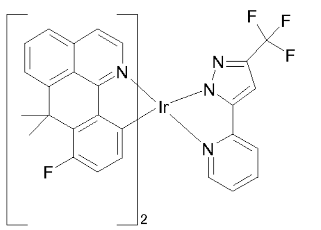

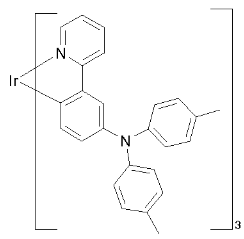

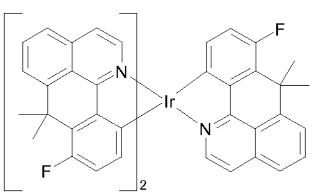

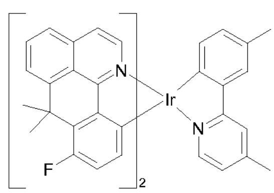

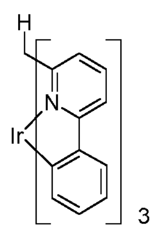

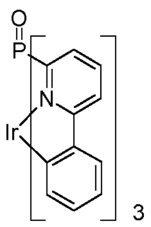
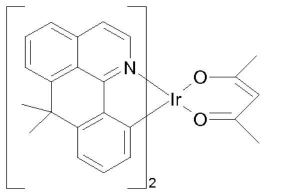
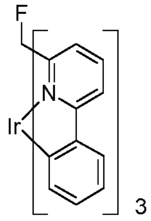

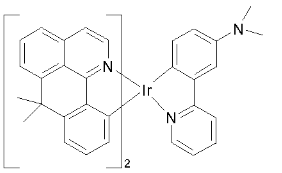
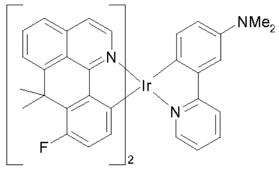
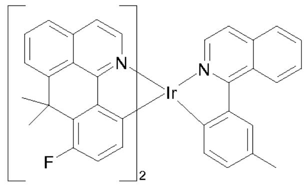

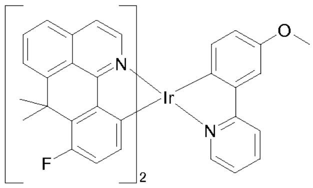
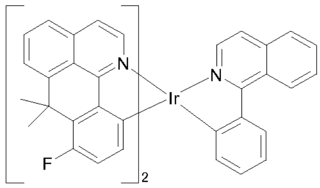
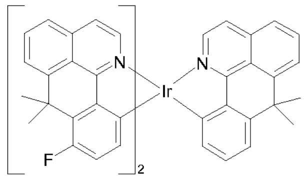

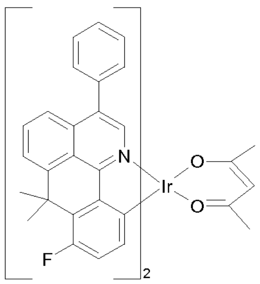
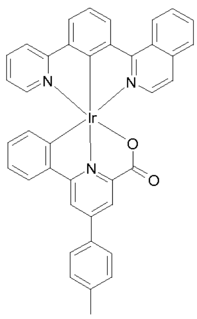
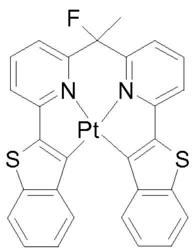

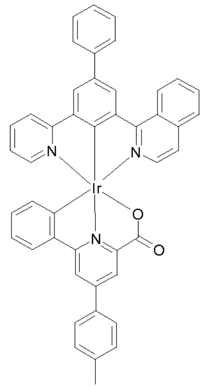
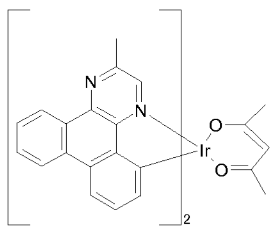
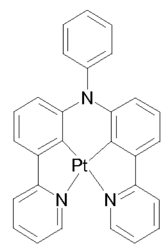

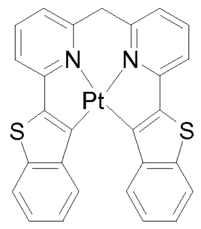
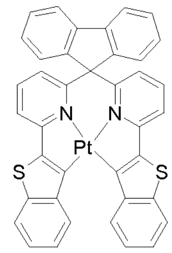
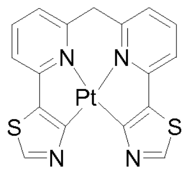
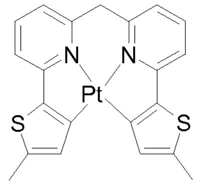
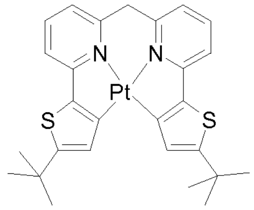
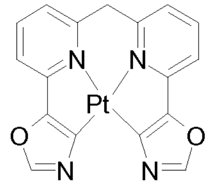
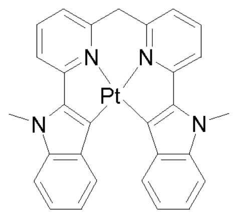
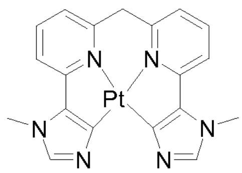
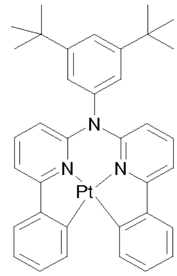
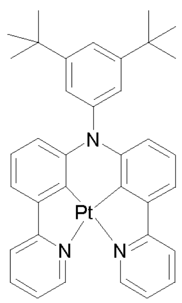
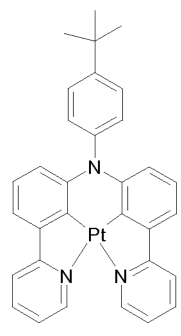
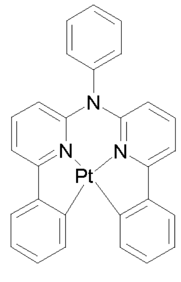
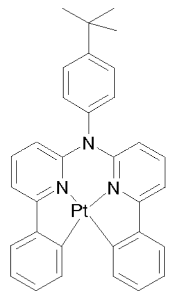
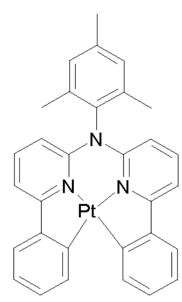
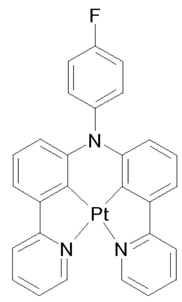

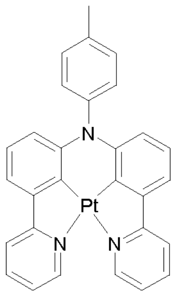
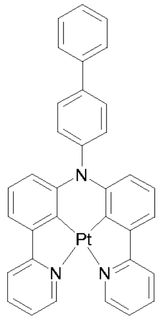
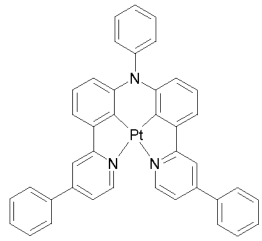
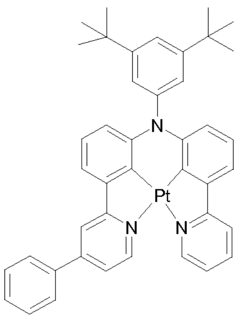
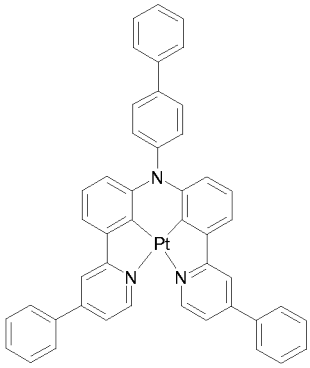
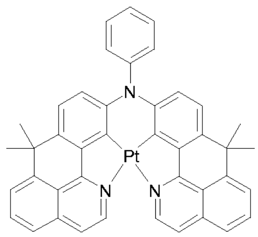
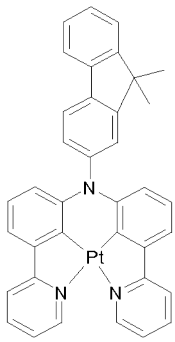
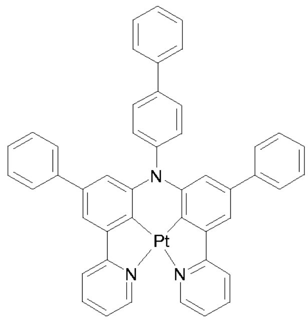
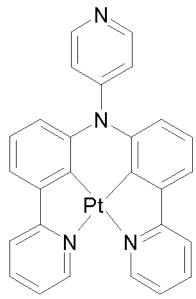
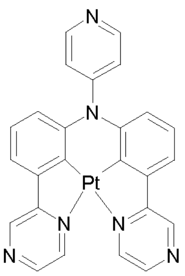
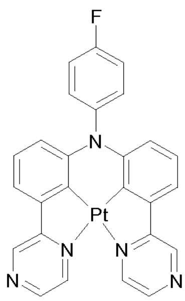
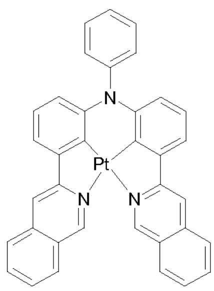
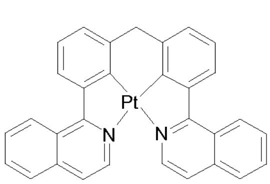
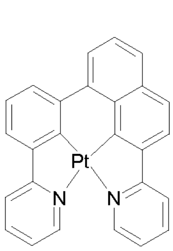
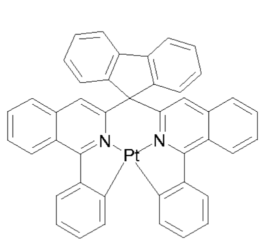

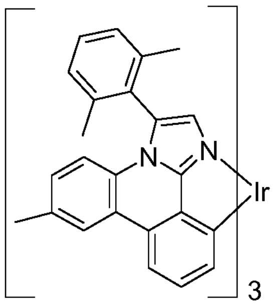
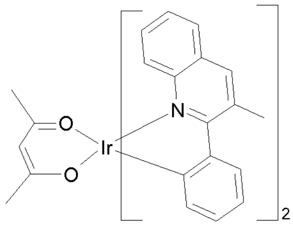
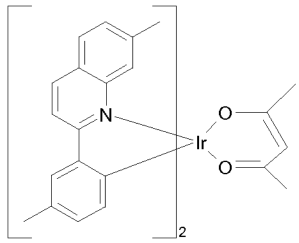

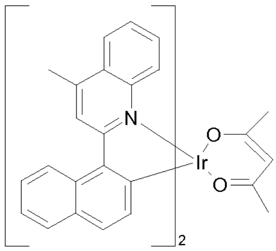
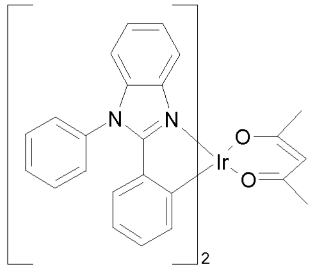
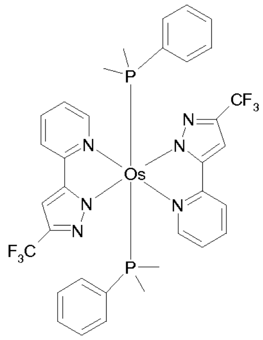

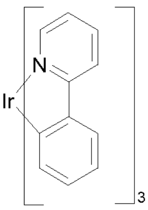
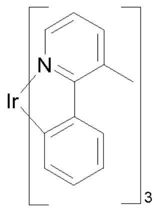
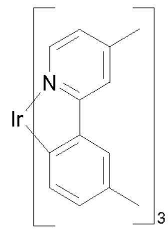

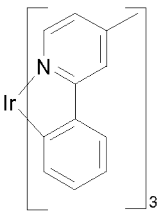
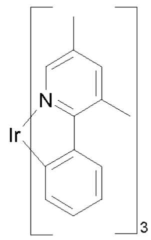
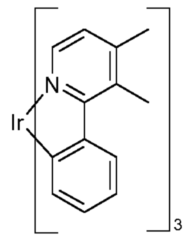

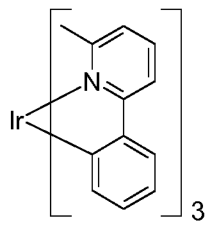
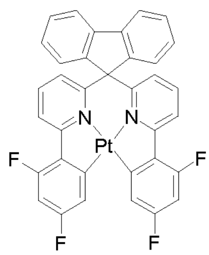
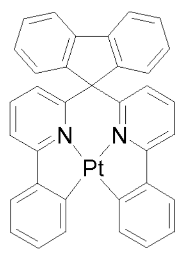

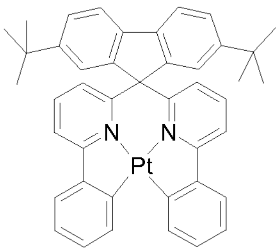
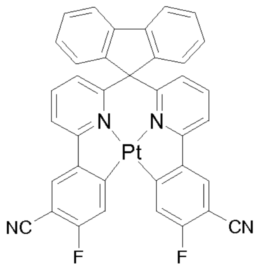
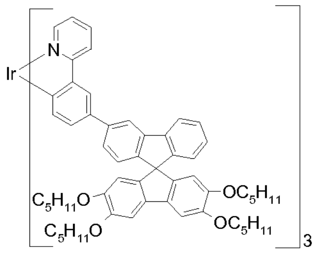
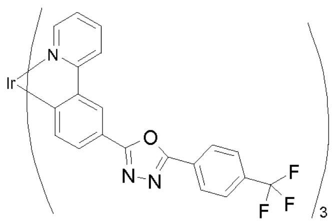
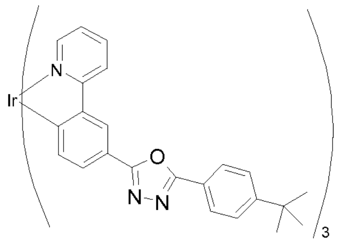
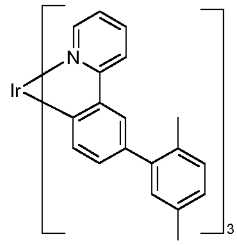
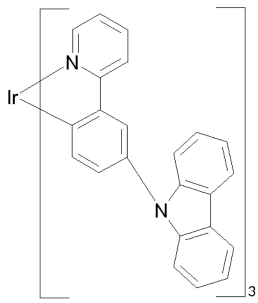
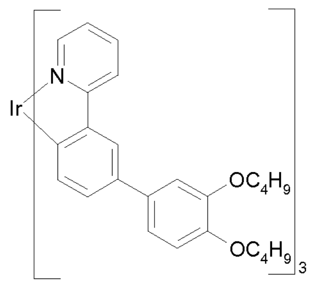
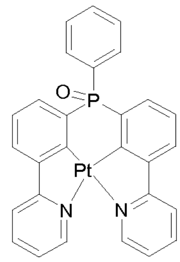
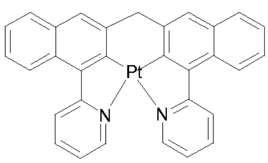
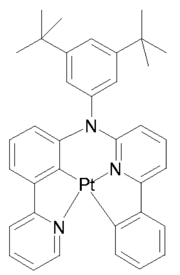
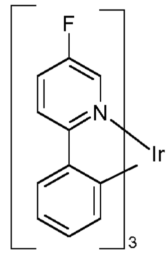
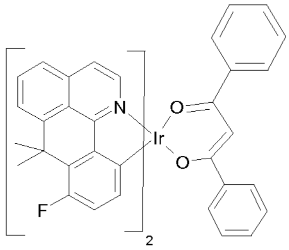
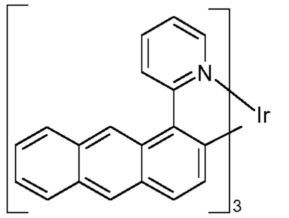
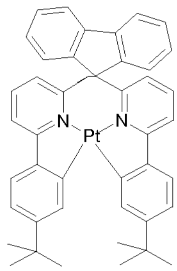

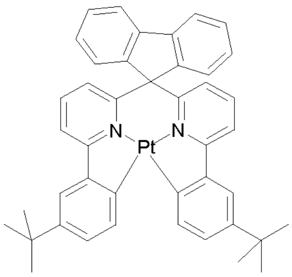
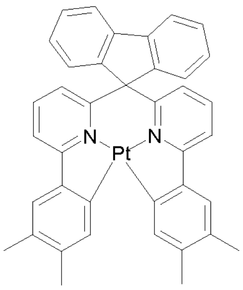
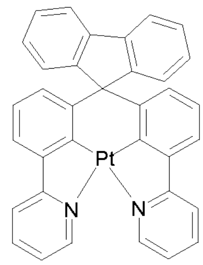

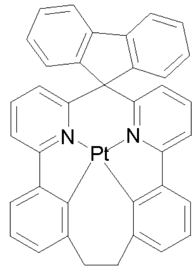
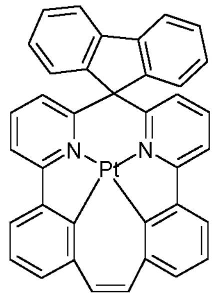
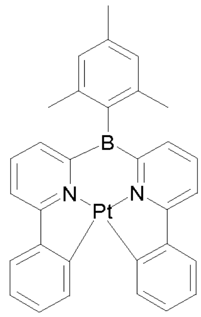

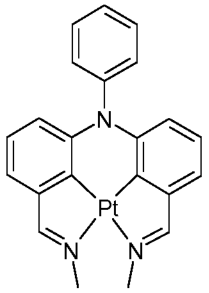
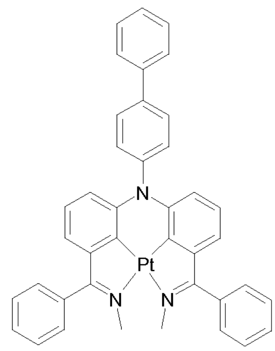
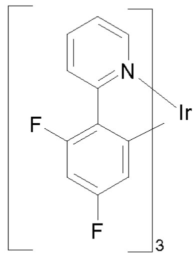

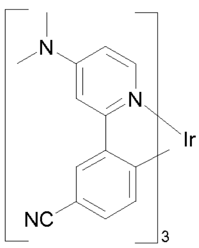
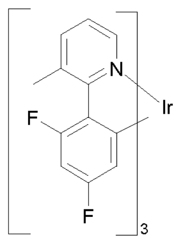
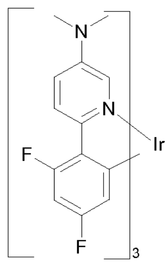

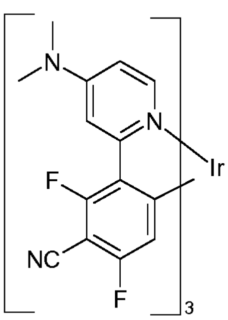
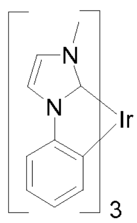
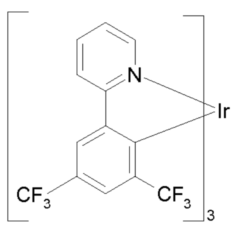

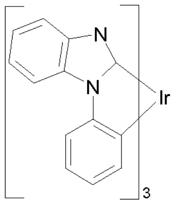
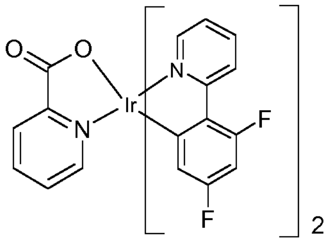
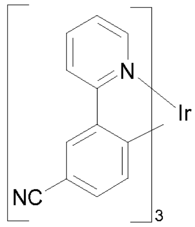

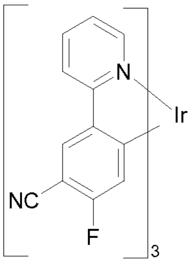
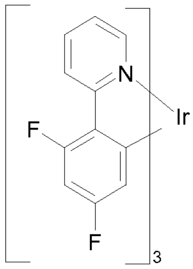
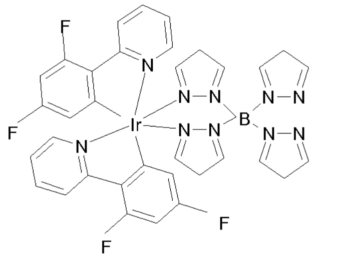

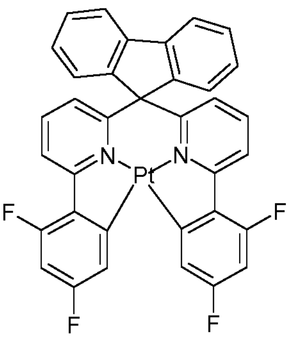
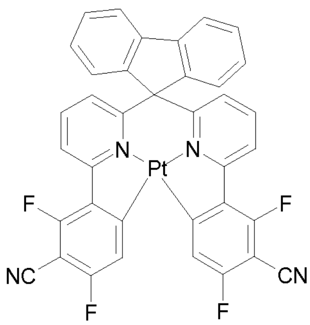
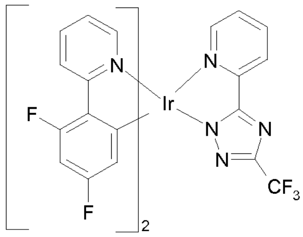

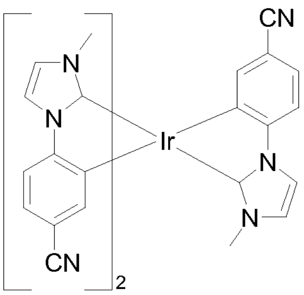
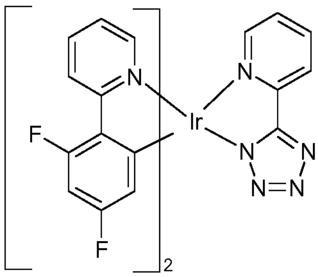
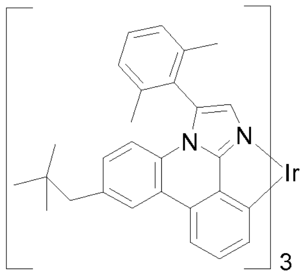

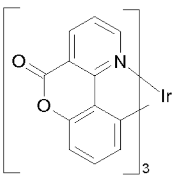
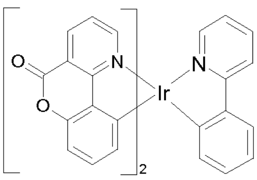
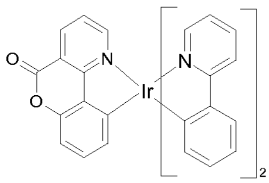

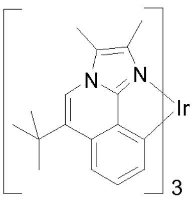
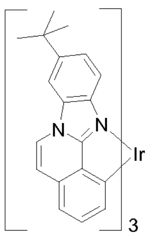
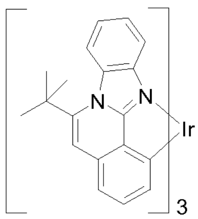

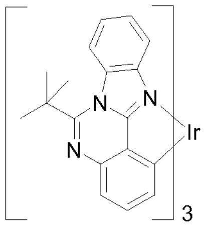 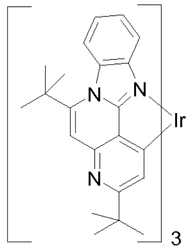 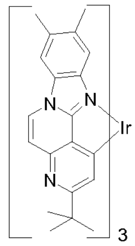

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Als Matrixmaterialien, bevorzugt für fluoreszierende Dotanden, kommen neben den Verbindungen der Formel (1) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß
WO 2006/117052) oder der Benzanthracene (z. B. gemäß
WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den Verbindungen der Formel (1) aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAlQ.

Außer Kathode, Anode und der Schicht enthaltend die Verbindung der Formel (1) kann die elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device

Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957,
WO 2003/060956, WO 2004/028217, WO 2004/080975 und
WO 2010/072300 offenbart werden.

Als Lochtransportmaterialien sind insbesondere bevorzugt Materialien, die in einer Lochtransport-, Lochinjektions- oder Elektronenblockierschicht verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627), und Dihydroacridin-Derivate.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die organische Elektrolumineszenzvorrichtung wird bei der Herstellung entsprechend (je nach Anwendung) strukturiert, kontaktiert und schließlich versiegelt, da sich die Lebensdauer der erfindungsgemäßen Vorrichtungen bei

Anwesenheit von Wasser und/oder Luft verkürzt. In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Gegenstand der Erfindung ist somit weiterhin ein Verfahren zur Herstellung der erfindungsggemäßen organischen Elektrolumineszenzvorrichtung, dadurch gekennzeichnet, dass mindestens eine organische Schicht durch Gasphasenabscheidung oder aus Lösung aufgebracht wird.

Erfindungsgemäß können die organischen Elektrolumineszenzvorrichtungen enthaltend eine oder mehrere Verbindungen gemäß Formel (1) in Displays und als Lichtquellen in Beleuchtungsanwendungen eingesetzt werden.

Die vorliegende Erfindung betrifft auch eine Formulierung enthaltend wenigstens eine Verbindung gemäß Formel (1) oder wenigstens eine der oben genannten Zusammensetzungen sowie wenigstens ein Lösungsmittel.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Vorrichtungen enthaltend die Verbindungen nach Formel (1) können sehr vielseitig eingesetzt werden. So können, beispielsweise, elektrolumineszierende Vorrichtungen enthaltend eine oder mehrere Verbindungen nach Formel (1) in Displays für Fernseher, Mobiltelefone, Computer und Kameras eingesetzt werden. Die Vorrichtungen können aber auch in Beleuchtungsanwendungen verwendet werden.

Gegenstand der vorliegenden Erfindung ist daher eine organische Elektrolumineszenzvorrichtung, ganz bevorzugt eine OLED oder OLEC.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:

1. Die erfindungsgemäßen Verbindungen eignen sich sehr gut für den Einsatz in einer Emissionsschicht und zeigen verbesserte Leistungsdaten gegenüber Verbindungen aus dem Stand der Technik.

2. Die erfindungsgemäßen Verbindungen weisen eine relativ niedrige Sublimationstemperatur, eine hohe Temperaturstabilität und lassen sich daher unzersetzt und rückstandsfrei sublimieren. Weiterhin weisen sie eine hohe Oxidationsstabilität und eine hohe Glasübergangstemperatur auf, was sowohl für die Prozessierbarkeit, beispielweise aus Lösung oder aus der Gasphase, als auch für die Verbendung in elektronischen Vorrichtungen vorteilhaft ist.

3. Die Verwendung der erfindungsgemäßen Verbindungen in organischen Elektrolumineszenzvorrichtungen, insbesondere eingesetzt als Elektronentransport- oder Elektroneninjektionsmaterial, aber auch als Matrixmaterial, führen zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern

dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

## Beispiel 1

**Synthese von 3-Dibenzofuran-4-yl-9-phenyl-9H-carbazol**

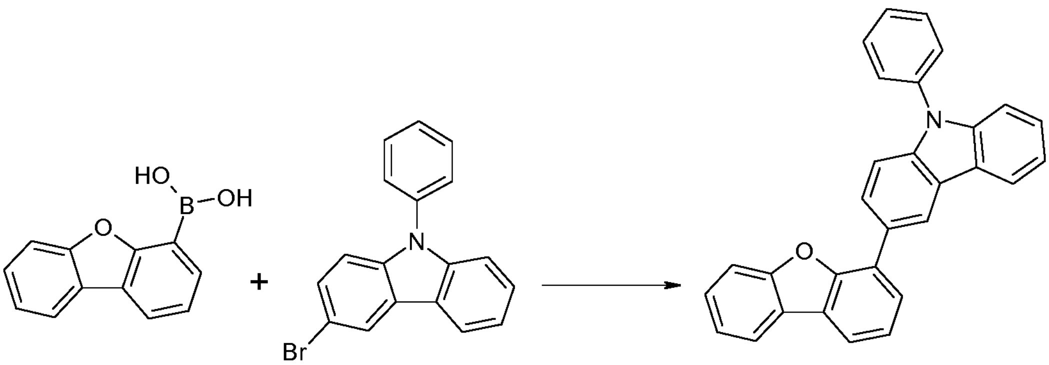

[89827-45-2] [1153-85-1]

28,9 g (136 mmol) Dibenzofuran-4-boronsäure, 40 g (124,1 mmol) 3-Bromo-9-phenyl-9H-carbazole, 78,9 ml (158 mmol) $Na_2CO_3$ (2 M-Lösung) werden in 120 mL Toulol, 120 mL Ethanol und 100 mL Wasser suspendiert. Zu dieser Suspension werden 2,6 g (2.2 mmol) $Pd(PPh_3)_4$ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 49,7 g (121 mmol), entsprechend 97% der Theorie.

Analog können folgende Verbindungen erhalten werden:

| Edukt 1 | Edukt 2 | Produkt | Ausbeute |
|---|---|---|---|
| 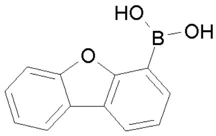 [89827-45-2] | 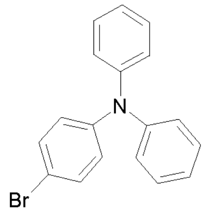 [36809-26-4 ] | 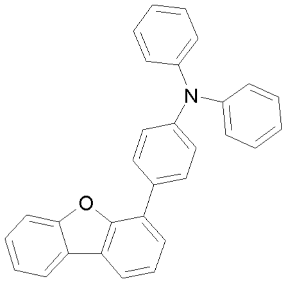 | 90% |
| 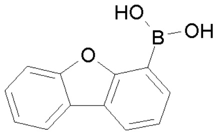 [89827-45-2] | 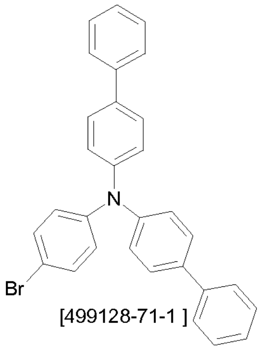 [499128-71-1 ] | 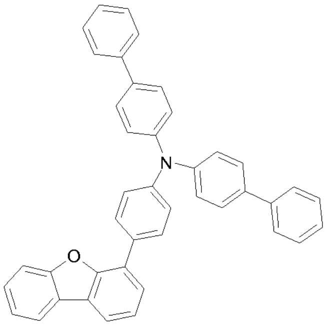 | 92% |
| 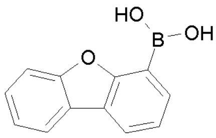 [89827-45-2] | 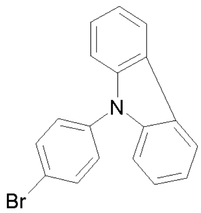 [57102-42-8] | 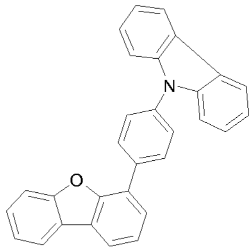 | 89% |
| 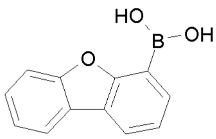 [89827-45-2] | 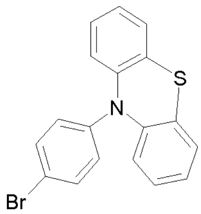 [63524-03-8] | 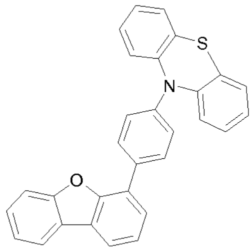 | 92% |
| 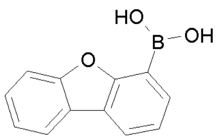 [89827-45-2] | 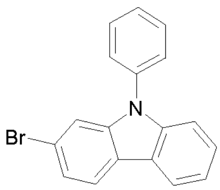 [94994-62-4 ] | 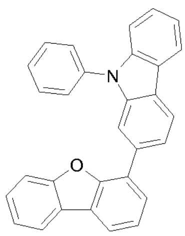 | 86% |
| 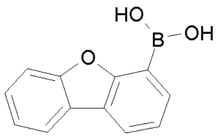 [89827-45-2] | 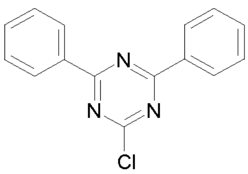 [3842-55-5] | 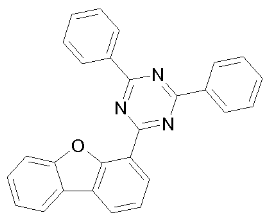 | 69% |

(fortgesetzt)

| Edukt 1 | Edukt 2 | Produkt | Ausbeute |
|---|---|---|---|
| 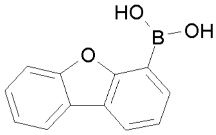 [89827-45-2] | 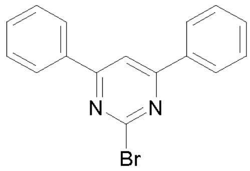 [56181-49-8] | 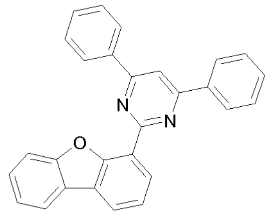 | 72% |

**Beispiel 2**

**Synthese von Bis-biphenyl-4-yl-dibenzofuran-4-yl-amin**

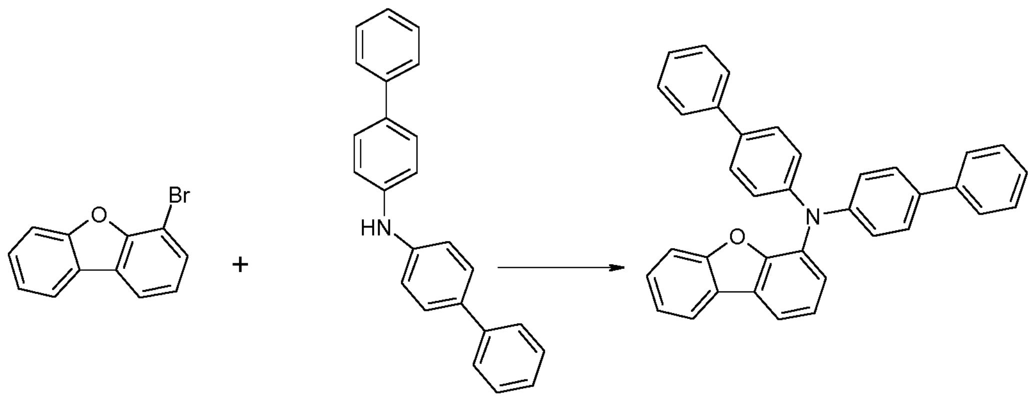

[89827-45-2] [102113-98-4]

Eine entgaste Lösung von 36,6 g (147 mmol) 4-Brom-Dibenzofuran und 39,5 g (123 mmol) Bis-biphenyl-4-yl-amin in 600 mL Toluol wird 1 h mit $N_2$ gesättigt. Danach wird die Lösung zuerst mit 2.09 mL (8.6 mmol) P(*t*Bu)$_3$, dann mit 1.38 g (6.1 mmol) Palladium(II)acetat versetzt und anschließend 17.7 g (185 mmol) NaOtBu im festen Zustand zugegeben. Die Reaktionsmischung wird 1 h unter Rückfluss erhitzt. Nach Abkühlen auf Raumtemperatur werden vorsichtig 500 mL Wasser zugesetzt. Die wässrige Phase wird mit 3x 50 mL Toluol gewaschen, über $MgSO_4$ getrocknet und das Lösungsmittel im Vakuum entfernt. Danach wird das Rohprodukt über Kieselgel mit Heptan/Essigsäureester (20:1) chromatographisch gereinigt. Die Ausbeute beträgt 57,7 g (118 mmol), entsprechend 80 % der Theorie.

Analog können folgende Verbindungen erhalten werden:

| Edukt 1 | Edukt 2 | Produkt | Ausbeute |
|---|---|---|---|
| 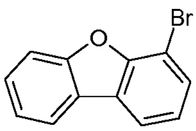 [89827-45-2] | 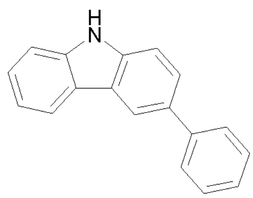 [103012-26-6 ] | 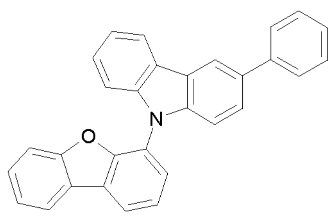 | 90% |

(fortgesetzt)

| Edukt 1 | Edukt 2 | Produkt | Ausbeute |
|---|---|---|---|
| 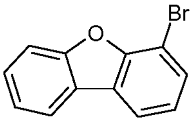<br>[89827-45-2] | 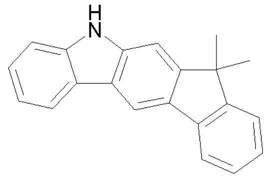<br>[1257220-47-5] | 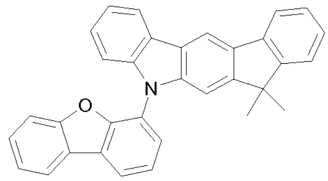 | 87% |
| 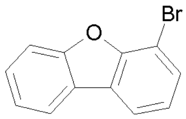<br>[89827-45-2] | 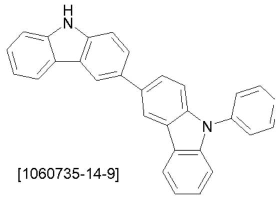<br>[1060735-14-9] | 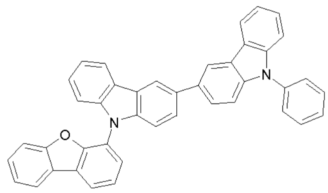 | 83% |
| 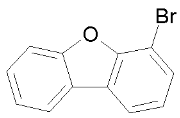<br>[89827-45-2] | 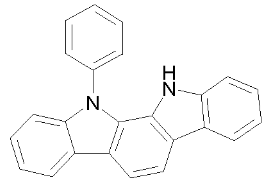<br>[1024598-06-8] | 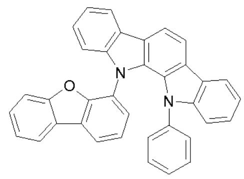 | 57% |
| 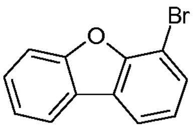<br>[89827-45-2] | 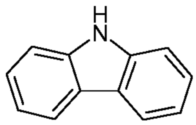 | 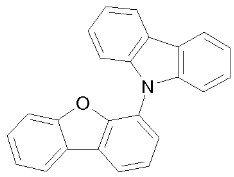 | 93% |

**Beispiel 3**

**Synthese von 9-Phenyl-3-(6-trimethylsilanyl-dibenzofuran-4-yl)-9H-carbazol**

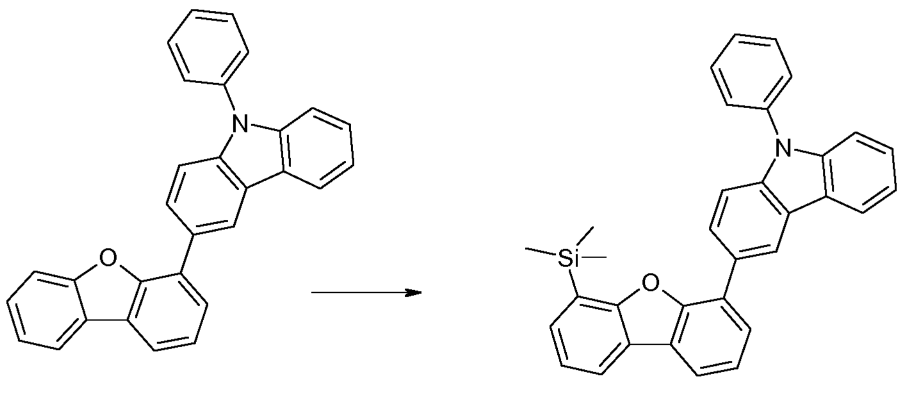

Eine auf 20°C gekühlte Lösung von 49 g (121 mmol) 3-Dibenzofuran-4-yl-9-phenyl-9H-carbazole und 28 g (242 mmol)TMEDA in 1000 ml THF wird tropfenweise mit 127 ml (225,4 mmol) n-Buthyllithium (2.5 M in Hexan) versetzt. Die Reaktionsmischung wird 3 h bei Raumtemperatur gerührt, dann kühlt man auf 0°C ab und tropft innerhalb 30 min. 26 g (242 mmol) Chlortrimetylsilan hinzu und rührt 8 h bei Raumtemperatur. Anschließend wird das Lösungsmittel im Vakuum entfernt und der Rückstand chromatographisch über Kieselgel mit Chloroform als Laufmittel gereinigt. Ausbeute: 34 g (72 mmol), 60% der Theorie.

Analog können die folgenden Verbindungen erhalten werden:

| Edukt 1 | Produkt | Ausbeute |
|---|---|---|
| 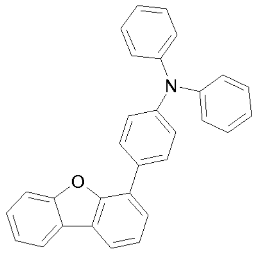 | 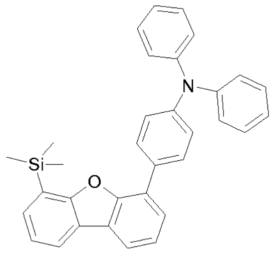 | 81% |
| 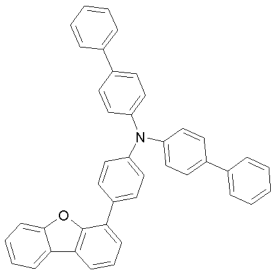 | 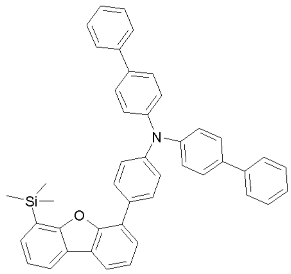 | 83% |
| 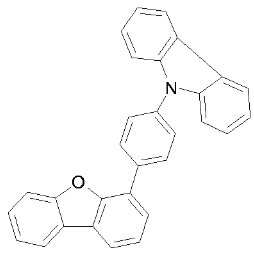 | 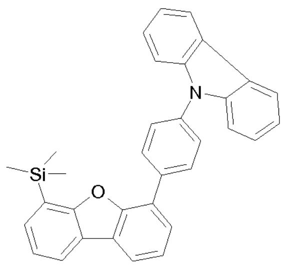 | 88% |
| 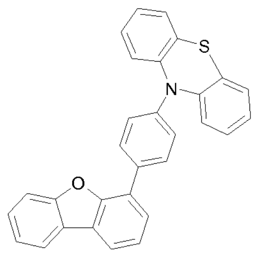 | 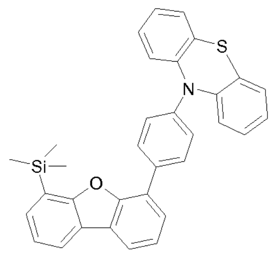 | 84% |
| 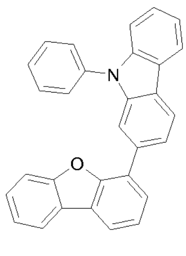 | 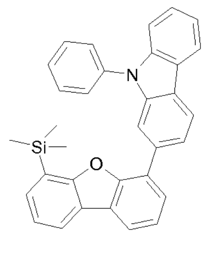 | 88% |
| 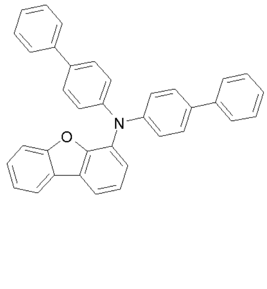 | 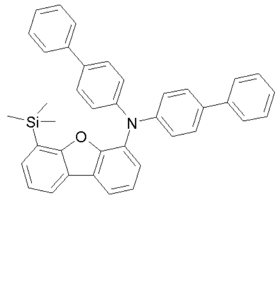 | 70% |

(fortgesetzt)

| Edukt 1 | Produkt | Ausbeute |
|---|---|---|
| 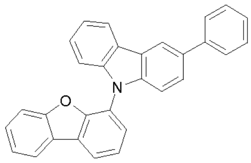 | 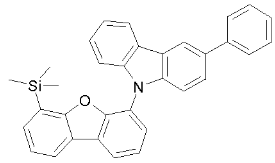 | 86% |
| 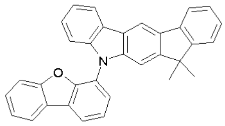 | 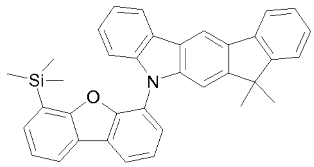 | 79% |
| 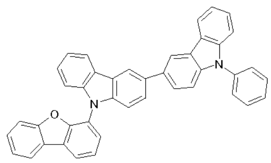 | 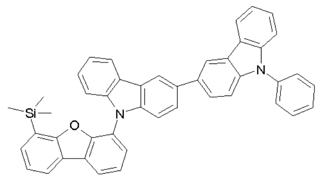 | 75% |
| 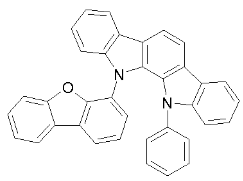 | 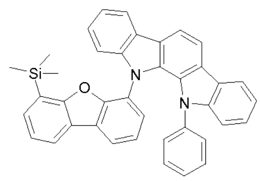 | 72% |
| 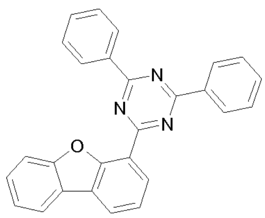 | 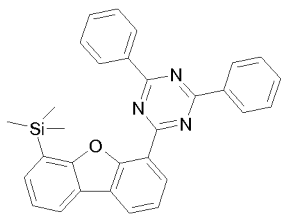 | 65% |
| 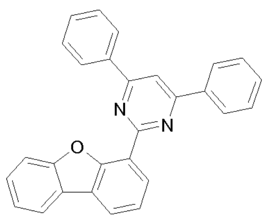 | 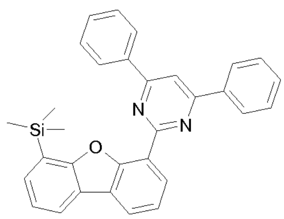 | 64% |
| 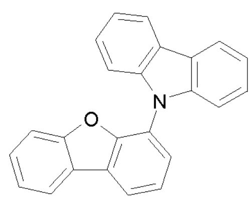 | 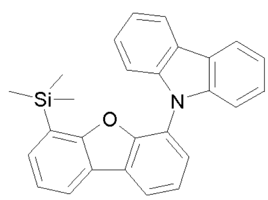 | 63% |

## Beispiel 4

**Synthese von 6-(Phenyl-9H-carbazol-3-yl)-4-dibenzofuranyl]-boronsäure**

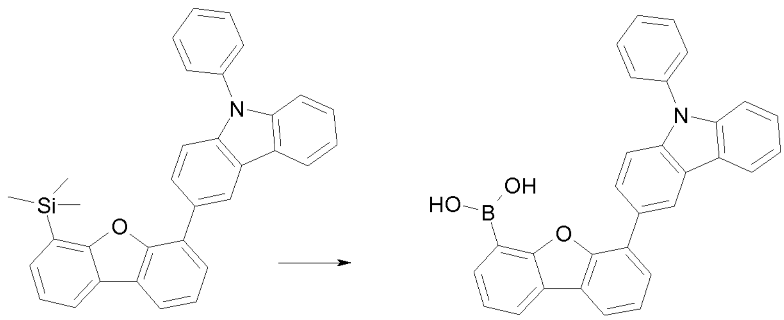

Unter Schutzgas wird eine Lösung von 34 g (72 mmol) -Phenyl-3-(6-trimethylsilanyl-dibenzofuran-4-yl)-9H-carbazol in 500 ml Dichlormethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 28 g (62 mmol), entsprechend 86% der Theorie.

Analog können die folgenden Verbindungen erhalten werden.

| Edukt 1 | Produkt | Ausbeute |
|---|---|---|
| 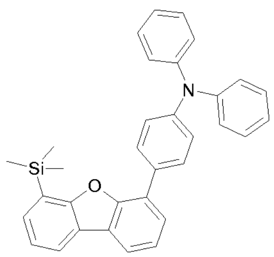 | 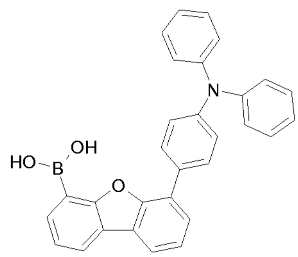 | 84% |
| 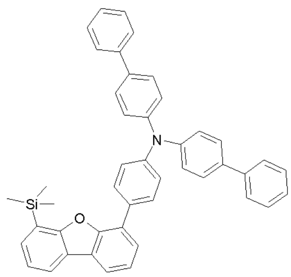 | 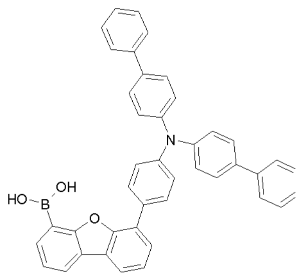 | 84% |
| 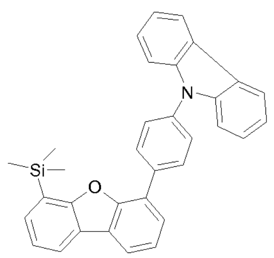 | 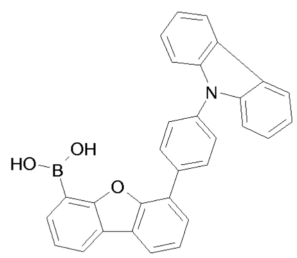 | 81% |
| 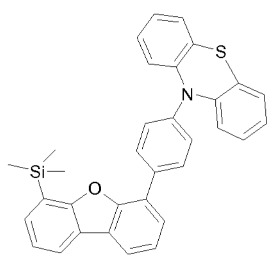 | 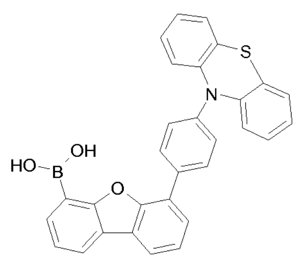 | 87% |

(fortgesetzt)

| Edukt 1 | Produkt | Ausbeute |
| --- | --- | --- |
| 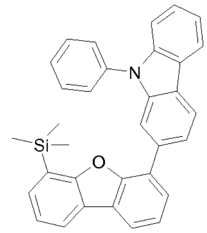 | 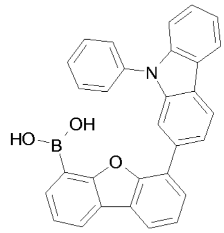 | 86% |
| 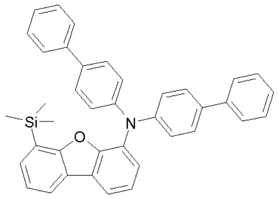 | 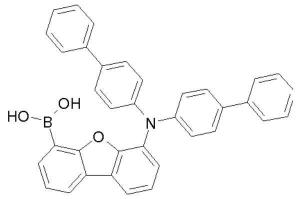 | 79% |
| 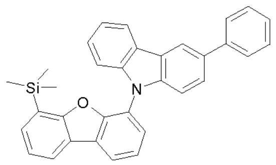 | 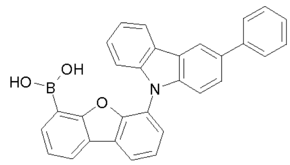 | 78% |
| 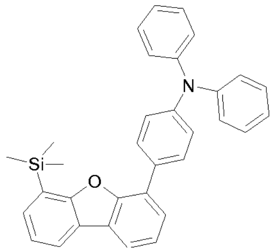 | 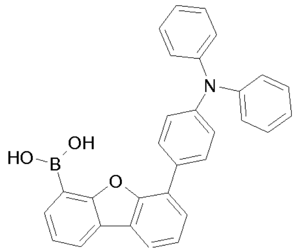 | 83% |
| 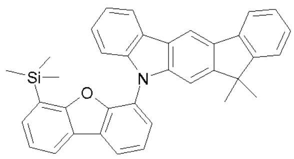 | 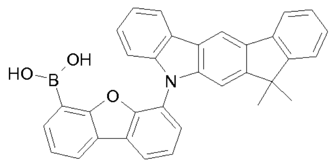 | 85% |
| 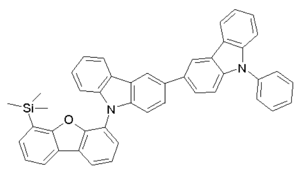 | 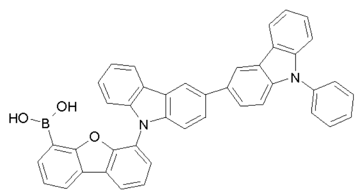 | 81% |
| 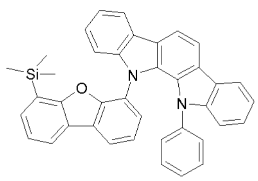 | 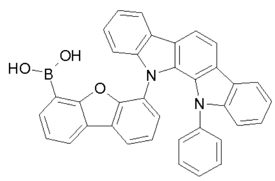 | 78% |
| 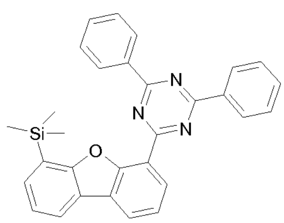 | 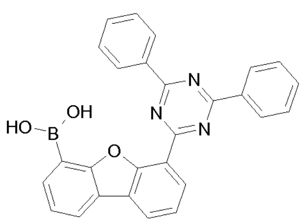 | 69% |

(fortgesetzt)

| Edukt 1 | Produkt | Ausbeute |
|---|---|---|
| 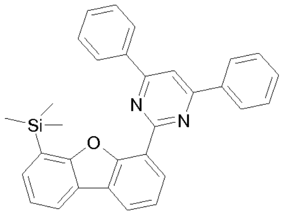 | 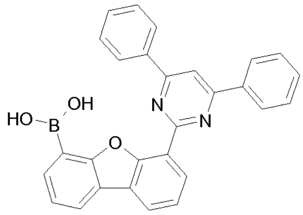 | 78% |
| 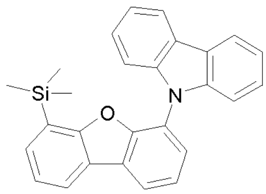 | 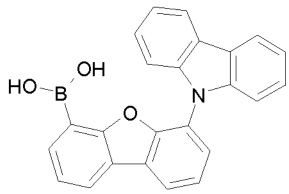 | 78% |

**Beispiel 5**

**Synthese von B-[6-(Phenyl-9H-carbazol-3-yl)-4-dibenzofuranyl]-boronsäure**

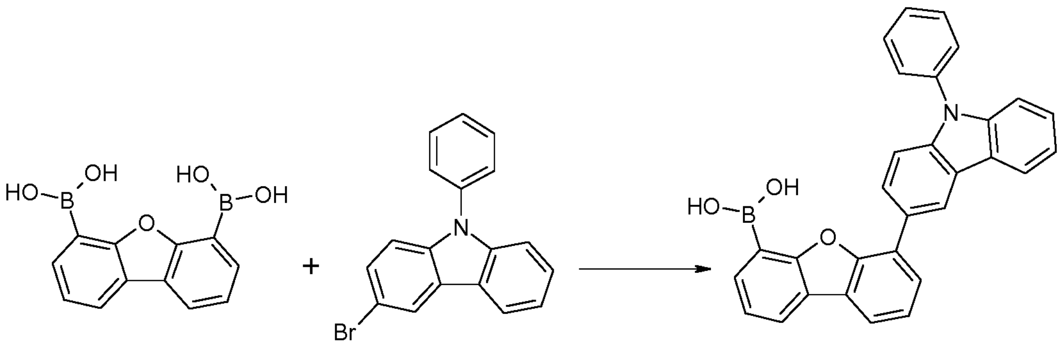

[145238-17-1] [1153-85-1]

9 g (32 mmol) B,B'-4,6-dibenzofurandiylbisboronsäure, 15 g (31,6 mmol) 3-Bromo-9-phenyl-9H-carbazol, 31 ml (63 mmol) $Na_2CO_3$ (2 M-Lösung) werden in 120 mL Toulol, 120 mL Ethanol suspendiert. Zu dieser Suspension werden 0,73 g (0,63 mmol) $Pd(PPh_3)_4$ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 11,1 g (24 mmol), entsprechend 70% der Theorie.

Analog können die folgenden Verbindungen erhalten werden:

| Edukt 1 | Edukt 2 | Produkt | Ausbeute |
|---|---|---|---|
| 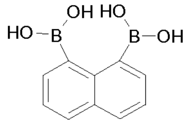 [947617-22-3] | 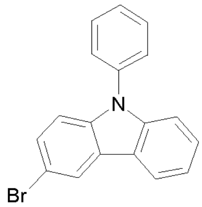 [1153-85-1] | 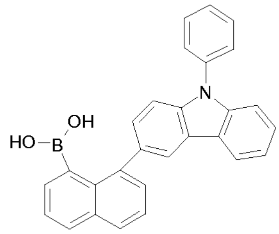 | 85% |

(fortgesetzt)

| Edukt 1 | Edukt 2 | Produkt | Ausbeute |
|---|---|---|---|
| 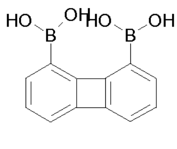 [480438-76-4] | 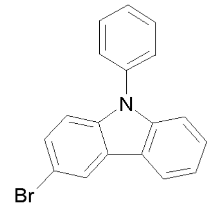 [1153-85-1] | 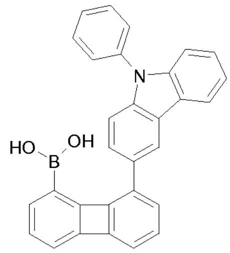 | 69% |
| 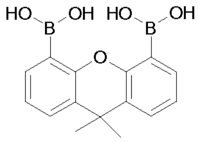 [862159-27-1 ] | 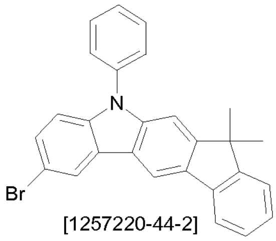 [1257220-44-2] | 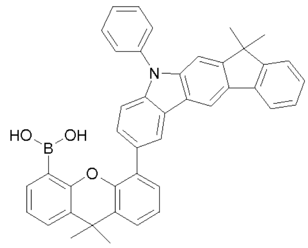 | 74% |

**Beispiel 6**

**Synthese von 3-(6-Bromo-dibenzofuran-4-yl)-9-phenyl-9H-carbazol**

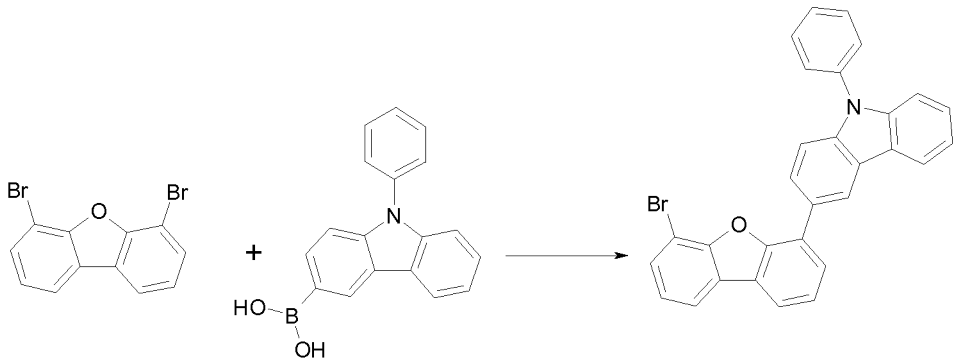

[201138-91-2] [854952-58-2]

10,43 g (32 mmol) B-(9-Phenyl-9H-carbazol-3-yl)boronsäure, 8,9 g (31,6 mmol) 4,6-Dibromdibenzofuran, 31 ml (63 mmol) $Na_2CO_3$ (2 M-Lösung) werden in 120 mL Toulol, 120 mL Ethanol suspendiert. Zu dieser Suspension werden 0,73 g (0,63 mmol) $Pd(PPh_3)_4$ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 11,4 g (23 mmol), entsprechend 73% der Theorie.

Analog können die folgenden Verbindungen erhalten werden.

| Edukt 1 | Edukt 2 | Produkt | Ausbeute |
|---|---|---|---|
| 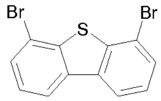<br>[669773-34-6] | 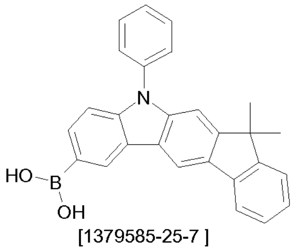<br>[1379585-25-7 ] | 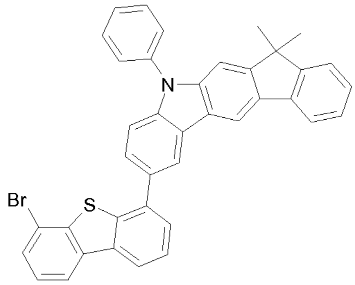 | 51% |
| 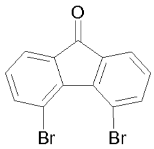<br>[69414-97-7] | 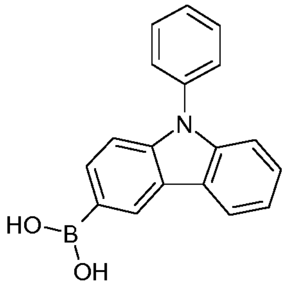<br>[854952-58-2] | 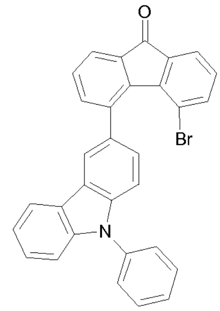 | 65% |
| 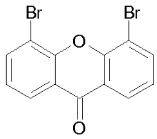<br>[1262398-42-4 ] | 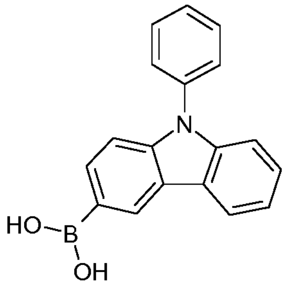<br>[854952-58-2] | 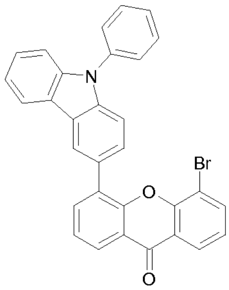 | 69% |
| 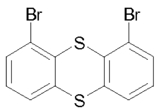<br>[176646-34-7 ] | 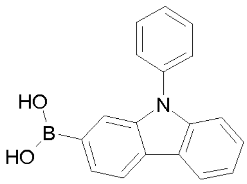<br>[1001911-63-2] | 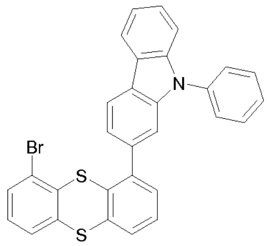 | 67% |
| 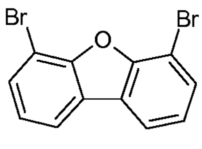<br>[201138-91-2] | 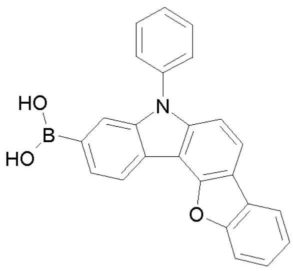<br>[1391729-62-6] | 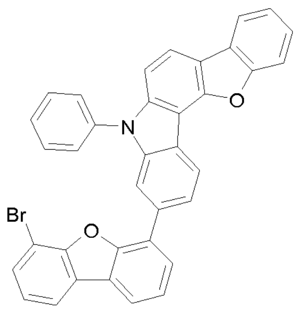 | 62% |

(fortgesetzt)

| Edukt 1 | Edukt 2 | Produkt | Ausbeute |
|---|---|---|---|
| 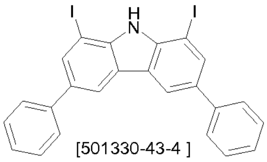 [501330-43-4 ] | 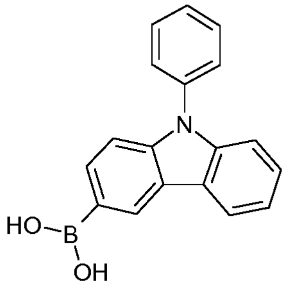 [854952-58-2] | 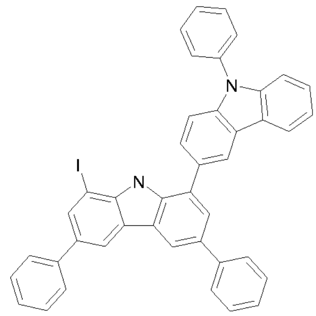 | 62% |
| 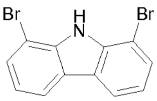 [553663-65-3] | 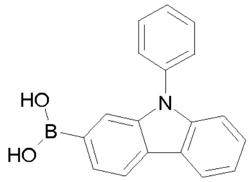 [1001911-63-2] | 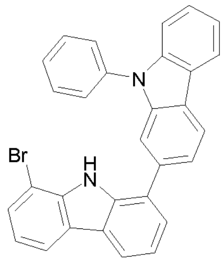 | 61% |
| 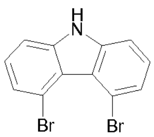 [905702-33-2] | 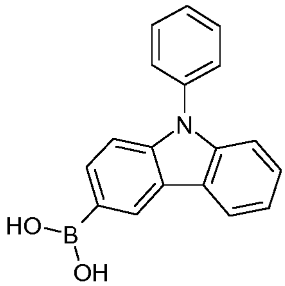 [854952-58-2] | 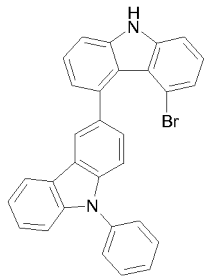 | 64% |
| 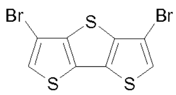 [502764-54-7] | 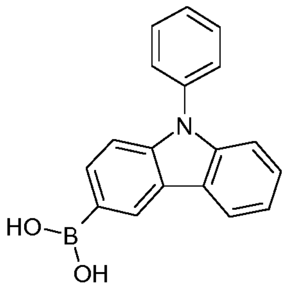 [854952-58-2] | 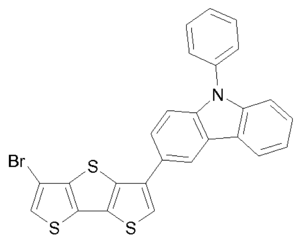 | 66% |
| 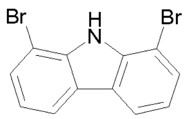 [553663-65-3] | 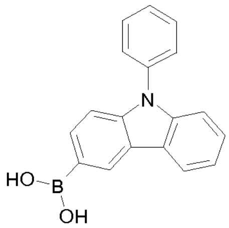 [854952-58-2] | 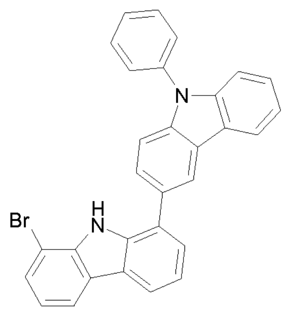 | 56% |

Analog können auch die folgenden Verbindungen durch eine zweite Addition mit den entsprechenden Borosäuren erhalten werden: Der Rückstand wird aus Toluol umkristallisiert und abschließend im Hochvakuum (p = 5 x $10^{-5}$

mbar) sublimiert

| Edukt 1 | Edukt 2 | Produkt | Ausbeute |
|---|---|---|---|
| 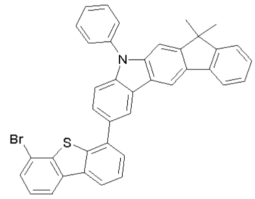 | 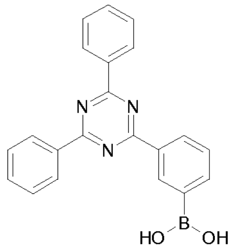 [1269508-31-7 ] | 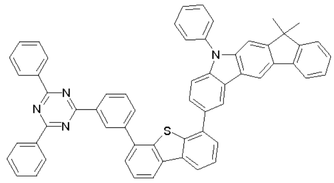 | 80% |
| 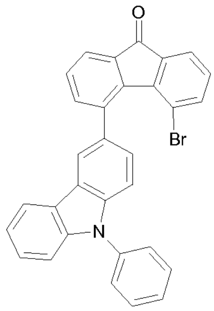 | 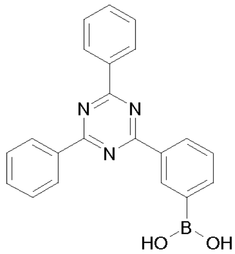 [1269508-31-7 ] | 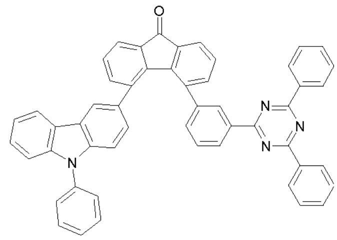 | 79% |
| 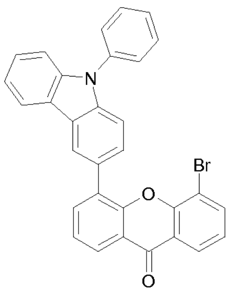 | 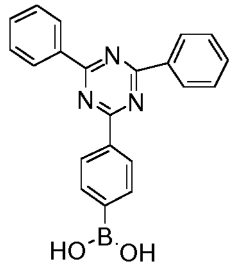 1313018-07-3 | 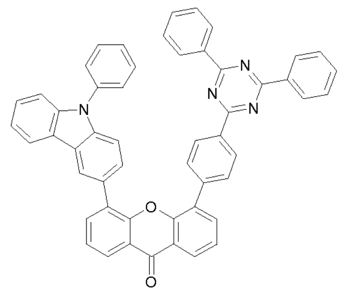 | 68% |
| 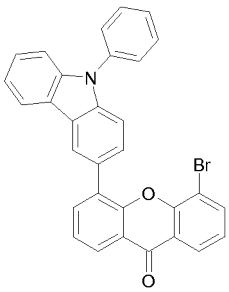 | 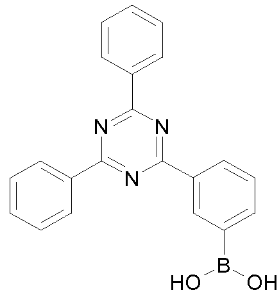 [1269508-31-7 ] | 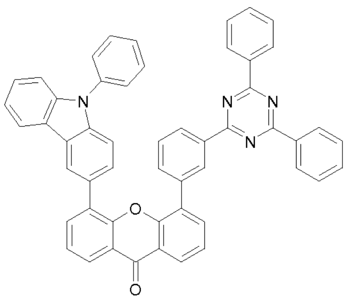 | 78% |
| 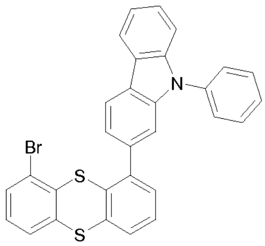 | 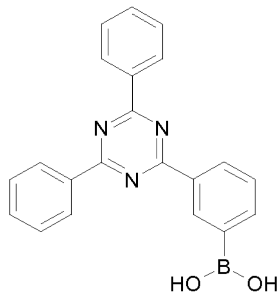 [1269508-31-7 ] | 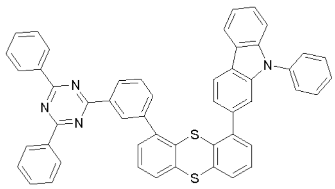 | 87% |

(fortgesetzt)

| Edukt 1 | Edukt 2 | Produkt | Ausbeute |
|---|---|---|---|
| 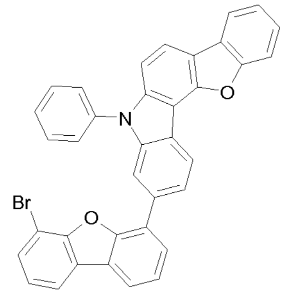 | 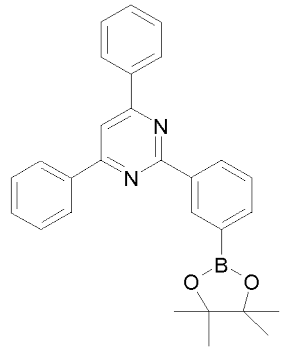 [1381862-91-4 ] | 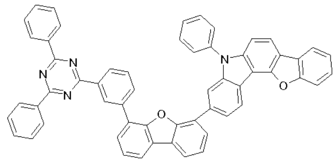 | 89% |
| 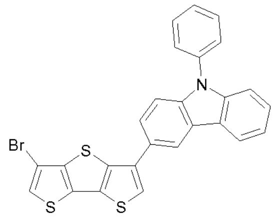 | 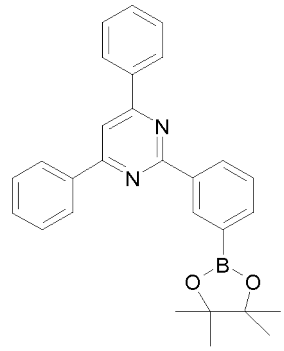 [1381862-91-4 ] | 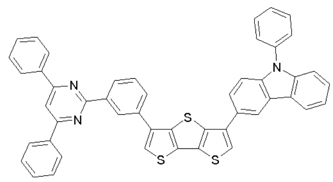 | 87% |
| 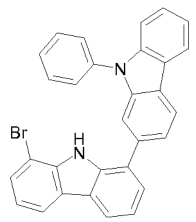 | 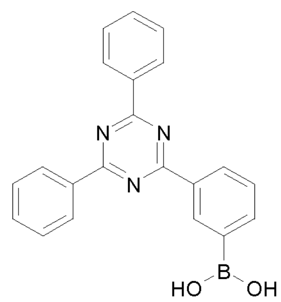 [1269508-31-7 ] | 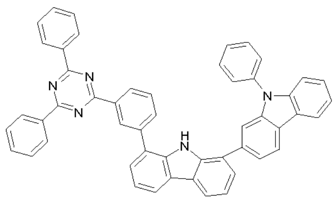 | 83% |
| 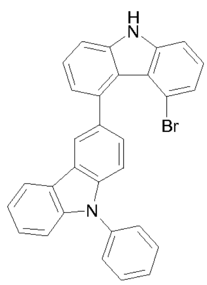 | 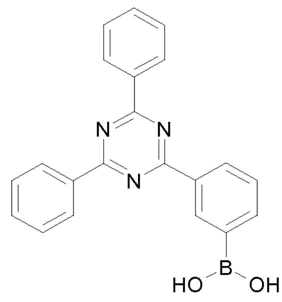 [1269508-31-7 ] | 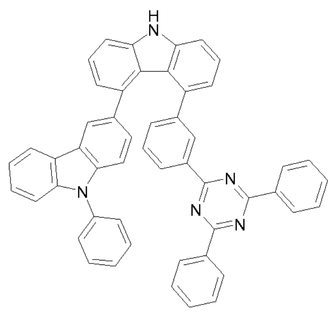 | 80% |

(fortgesetzt)

| Edukt 1 | Edukt 2 | Produkt | Ausbeute |
|---|---|---|---|
| 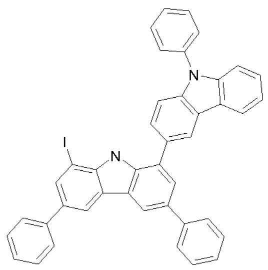 | 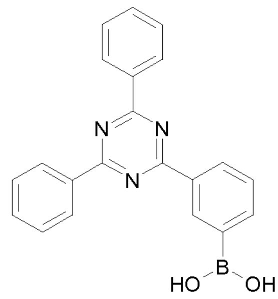 [1269508-31-7 ] | 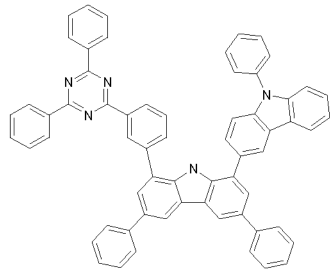 | 79% |
| 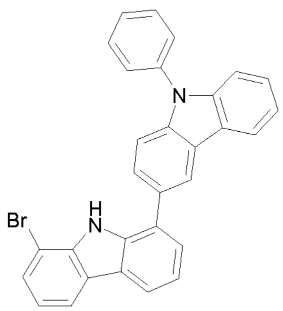 | 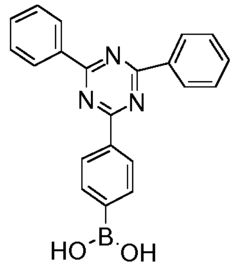 1313018-07-3 | 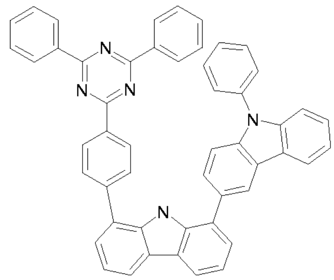 | 79% |

**Beispiel 7**

**Synthese von 3-{6-[3-(4,6-Diphenyl-[1,3,5]triazin-2-yl)-phenyl]-dibenzofuran-4-yl}-9-phenyl-9H-carbazol**

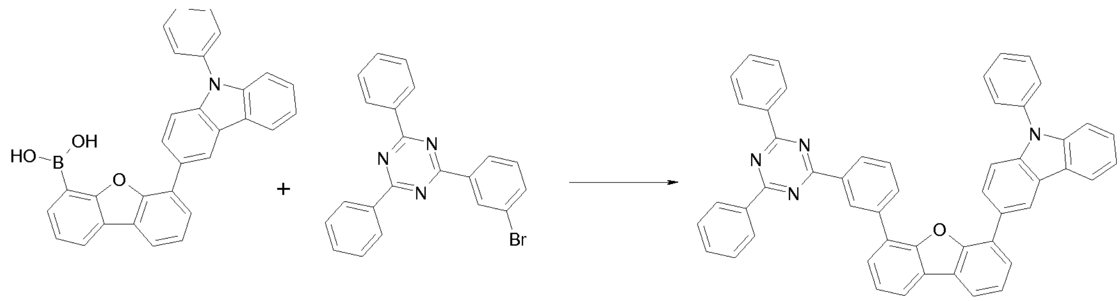

32,1 g (70 mmol) B-[6-(Phenyl-9H-carbazol-3-yl)-4-dibenzofuranyl]-boronsäure, 27 g (70 mmol) 2-(3-Bromophenyl)-4,6-diphenyl-[1,3,5]triazin, 78,9 ml (158 mmol) $Na_2CO_3$ (2 M-Lösung) werden in 120 mL Ethanol und 100 mL Wasser suspendiert. Zu dieser Suspension werden 1,3 g (1,1 mmol) $Pd(PPh_3)_4$ gegeben, und die Reaktionsmischung wird 16 h unter Rückfluss erhitzt. Nach Erkalten wird die Mischung mit Dichloromethan versetzt, die organische Phase abgetrennt, über Kieselgel filtriert. Die Ausbeute beträgt 44 g (61 mmol), entsprechend 87% der Theorie. Der Rückstand wird aus Toluol umkristallisiert und abschließend im Hochvakuum ($p = 5 \times 10^{-5}$ mbar) sublimiert. Die Reinheit beträgt 99.9%.

Analog können die folgenden Verbindungen erhalten werden.

| Edukt 1 | Edukt 2 | Produkt | Ausbeute |
|---|---|---|---|
| 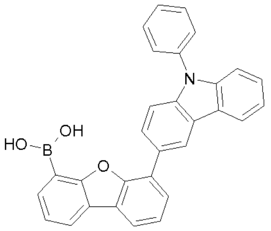 | 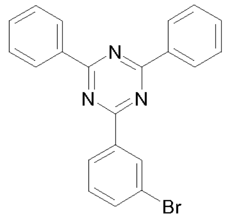 864377-31-1] | 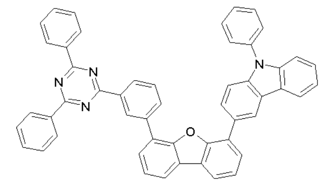 | 79% |
| 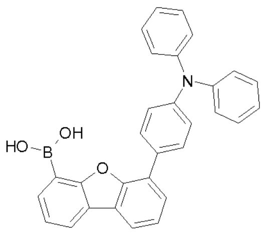 | 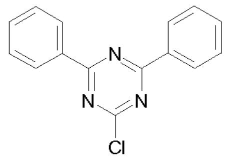 [3842-55-5 ] | 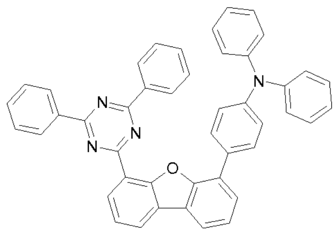 | 83% |
| 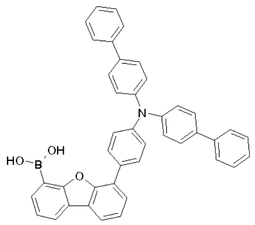 | 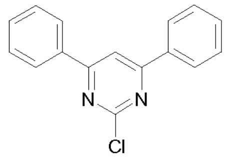 [2915-16-4 ] | 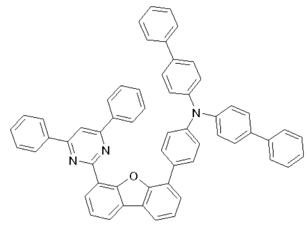 | 86% |
| 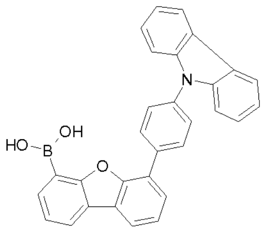 | 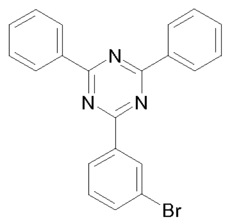 864377-31-1] | 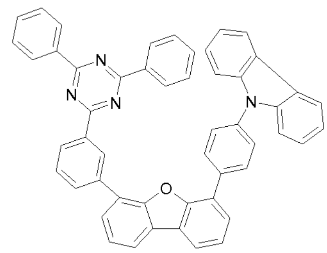 | 89% |
| 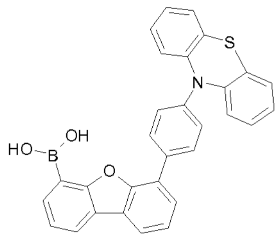 | 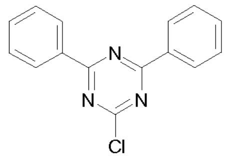 [3842-55-5 ] | 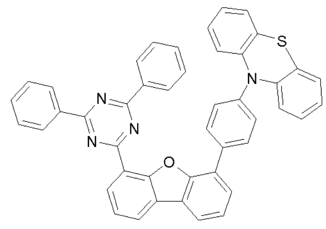 | 80% |
| 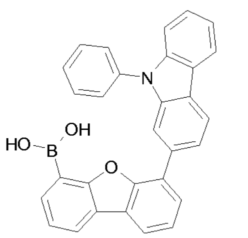 | 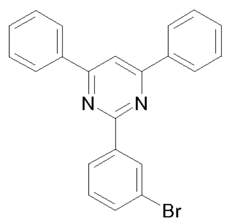 [864377-22-0] | 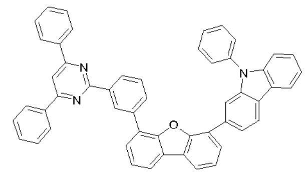 | 79% |

(fortgesetzt)

| Edukt 1 | Edukt 2 | Produkt | Ausbeute |
|---|---|---|---|
| 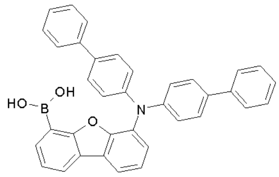 | 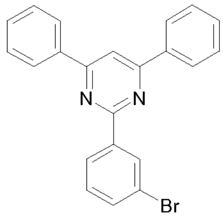 [864377-22-0] | 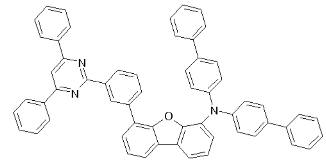 | 84% |
| 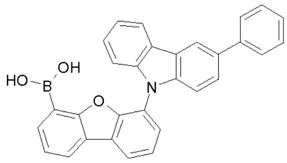 | 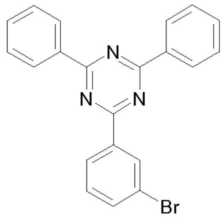 864377-31-1] | 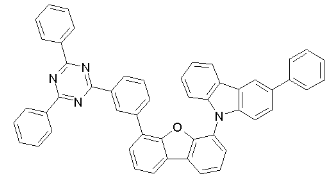 | 79% |
| 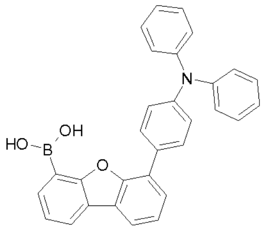 | 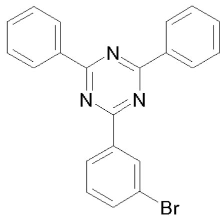 864377-31-1] | 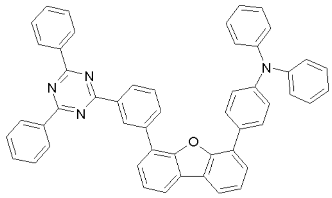 | 77% |
| 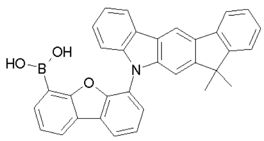 | 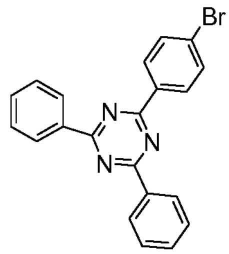 23449-08-3 | 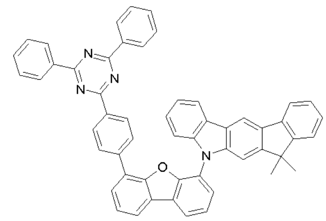 | 78% |
| 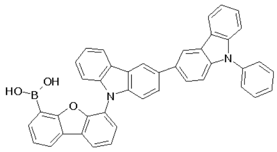 | 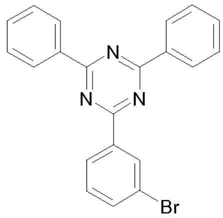 864377-31-1] | 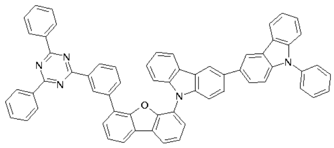 | 79% |

(fortgesetzt)

| Edukt 1 | Edukt 2 | Produkt | Ausbeute |
|---|---|---|---|
| 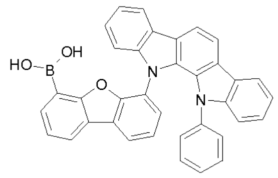 | 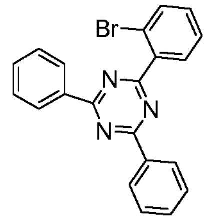 77989-15-2 | 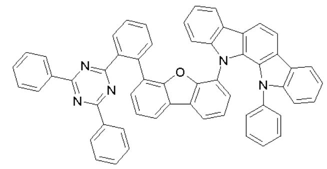 | 75% |
| 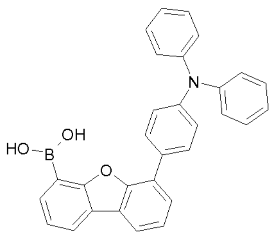 | 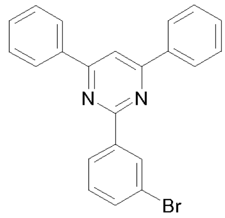 [864377-22-0] | 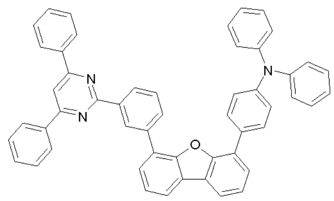 | 74% |
| 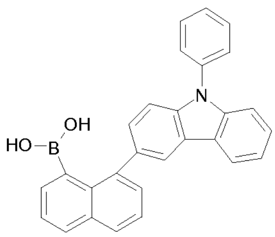 | 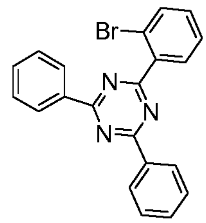 77989-15-2 | 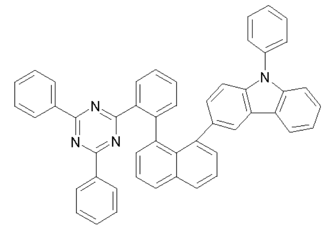 | 59% |
| 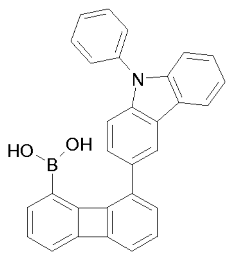 | 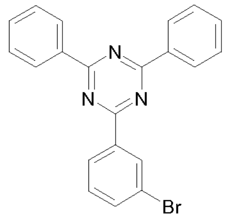 864377-31-1] | 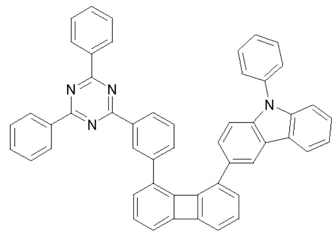 | 67% |
| 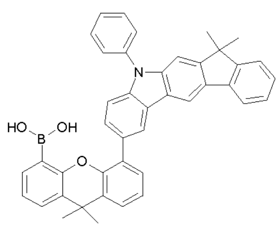 | 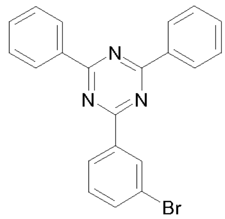 864377-31-1] | 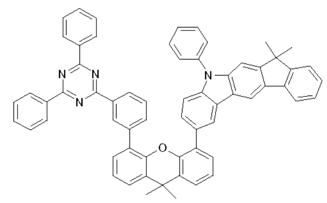 | 68% |

(fortgesetzt)

| Edukt 1 | Edukt 2 | Produkt | Ausbeute |
|---|---|---|---|
| 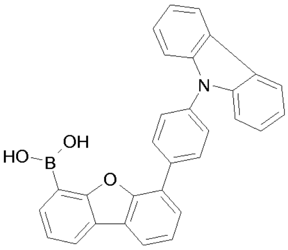 | 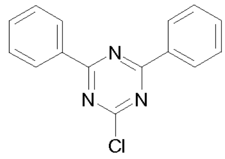 [3842-55-5 ] | 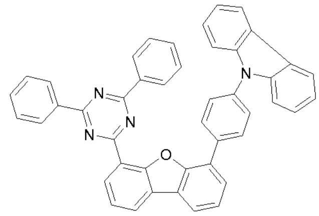 | 70% |
| 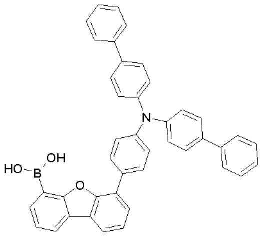 | 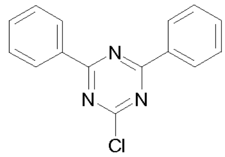 [3842-55-5 ] | 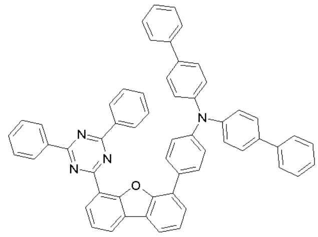 | 71% |
| 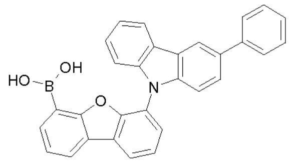 | 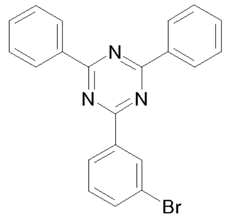 864377-31-1] | 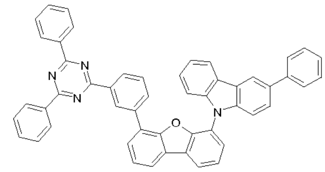 | 78% |
| 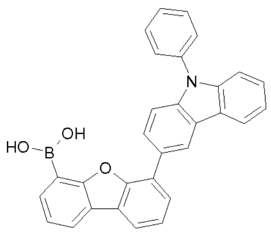 | 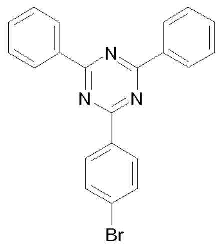 [23449-08-3] | 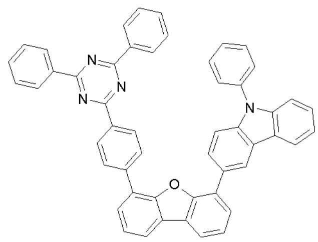 | 78% |
| 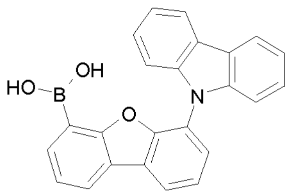 | 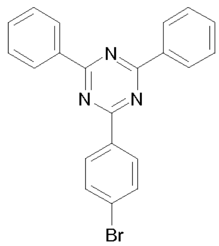 [23449-08-3] | 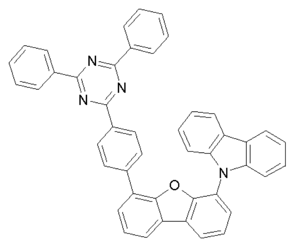 | 73% |
| 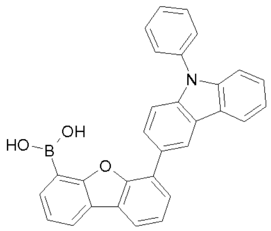 | 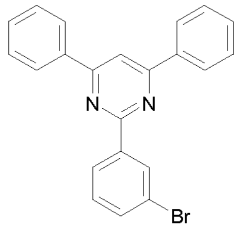 [864377-22-0] | 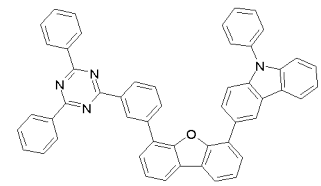 | 79% |

**Beispiel 8**

**Synthese von 9,9'-Diphenyl-8-(3-{4-phenyl-6-[(E)-((Z)-1-propenyl)-buta-1,3-dienyl]-[1,3,5]triazin-2-yl}-phenyl)-9H,9'H-[1,2']bicarbazolyl**

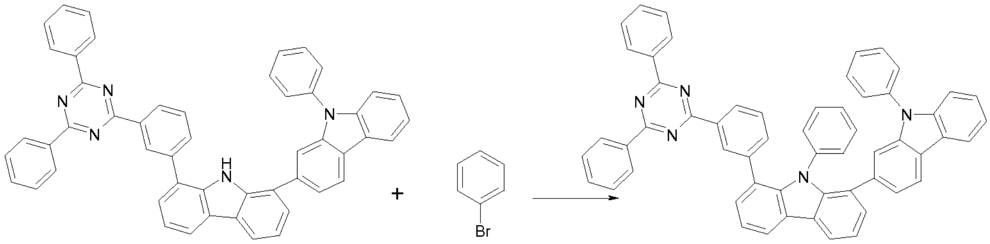

50 g (70,58 mmol) 8-[3-(4,6-Diphenyl-[1,3,5]triazin-2-yl)-phenyl]-9'-phenyl-9H,9'H-[1,2']bicarbazolyl und 16,4 g (105,87 mmol) Brom-benzol werden in Toluol gelöst und mittels Schutzgaseinleitung entgast. Anschließend wird mit 7 mL (7 mmol, 1 M Lösung in Toluol) Tri-tert-butylphosphin, 633,8 mg (2,82 mmol) $Pd(OAc)_2$ und 10,2 g (105,87 mmol) NaOtBu versetzt. Die Feststoffe werden zuvor entgast, die Reaktionsmischung wird nachentgast und anschließend unter Rückfluss für 3 h gerührt. Die warme Reaktionslösung wird über Alox B (Aktivitätsstufe 1) filtriert, mit Wasser gewaschen, getrocknet und eingeengt. Die Ausbeute beträgt 42 g (53 mmol), entsprechend 77% der Theorie. Der Rückstand wird aus Toluol umkristallisiert und abschließend im Hochvakuum ($p = 5 \times 10^{-5}$ mbar) sublimiert. Die Reinheit beträgt 99.9%.

Analog können die folgenden Verbindungen erhalten werden.

| Edukt 1 | Edukt 2 | Produkt | Ausbeute |
|---|---|---|---|
| 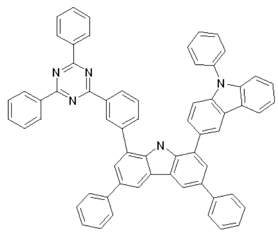 | 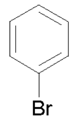 | 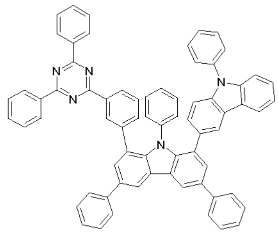 | 79% |
| 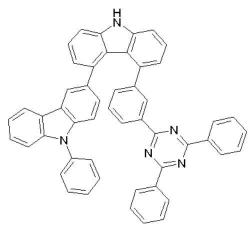 | 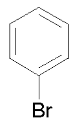 | 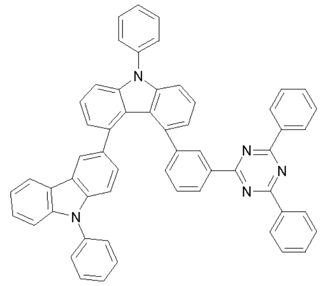 | 80% |
| 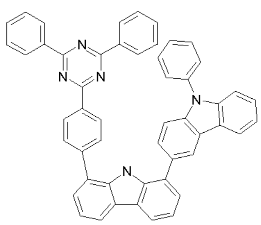 | 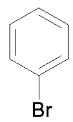 | 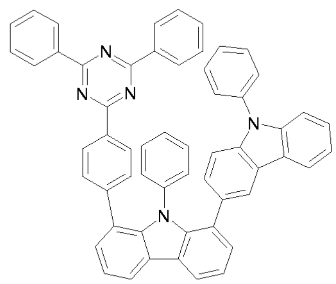 | 80% |

**Beispiel 9**

**Synthese von 2-{6-[4-(2,6-Diphenyl-pyridin-4-yl)-phenyl]-dibenzofuran-4-yl}-4,6-diphenyl-[1,3,5]triazin** (nicht erfindungsgemäß)

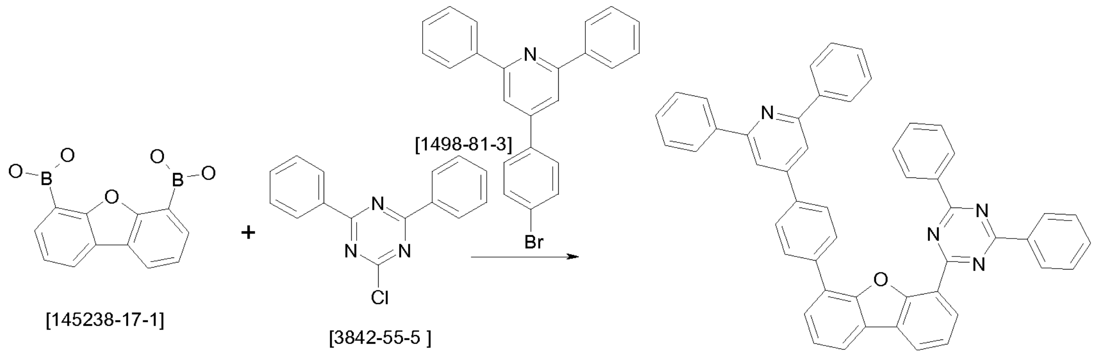


9 g (32 mmol) B,B'-4,6-dibenzofurandiylbisboronsäure, 6,5 g (31,6 mmol) 2-Chlor-4,6-Diphenyl-[1,3,5]triazin, 31 ml (63 mmol) $Na_2CO_3$ (2 M-Lösung) werden in 120 mL Toulol, 120 mL Ethanol suspendiert. Zu dieser Suspension werden 0,73 g (0,63 mmol) $Pd(PPh_3)_4$ gegeben, und die Reaktionsmischung wird 8 h unter Rückfluss erhitzt Anschlißend werden 6,5 g (31,6 mmol) 4-(4-Bromo-phenyl)-2,6-diphenyl-pyridine zugegeben und weiter 8 h unter Rückfluß erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 19,1 g (26 mmol), entsprechend 77% der Theorie.

Analog können die folgenden Verbindungen erhalten werden. Im Fall einer symmetrischen Verbindung werden zuerst 0,5 eq. von Edukt 2 und dann 0,5 eq.Edukt 3 zugegeben.

| Edukt 1 1eq. | Edukt 2 0,5 eq. | Edukt 3 0,5 eq. | Produkt | Ausbeute |
|---|---|---|---|---|
| 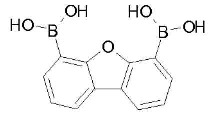 [145238-17-1] | 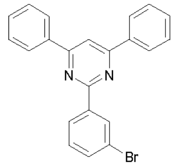 [864377-22-0] | 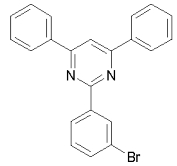 [864377-22-0] | 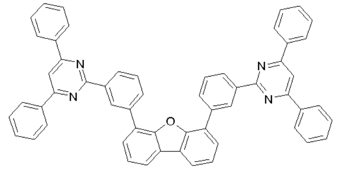 | 83% |
| 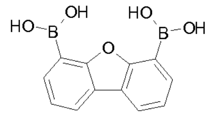 [145238-17-1] | 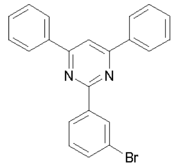 [864377-22-0] | 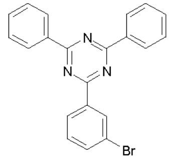 864377-31-1] | 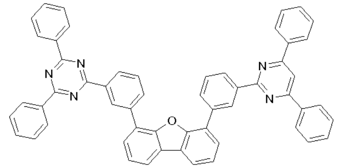 | 79% |
| 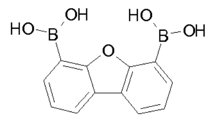 [145238-17-1] | 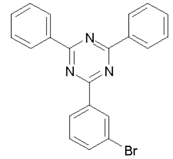 864377-31-1] | 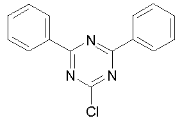 [3842-55-5 ] | 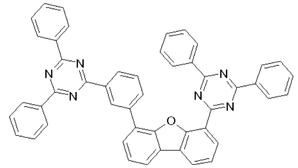 | 69% |
| 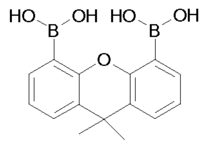 [862159-27-1 ] | 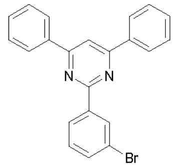 [864377-22-0] | 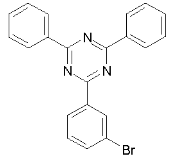 864377-31-1] | 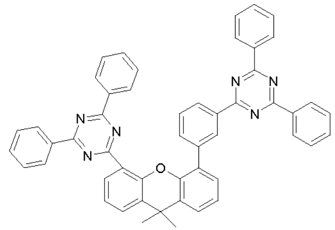 | 71% |

(fortgesetzt)

| Edukt 1 1eq. | Edukt 2 0,5 eq. | Edukt 3 0,5 eq. | Produkt | Ausbeute |
|---|---|---|---|---|
| 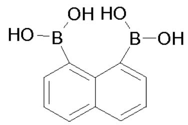 [947617-22-3] | 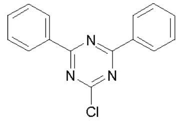 [3842-55-5 ] | 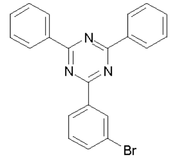 864377-31-1] | 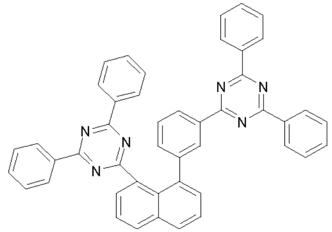 | 60% |
| 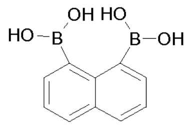 [947617-22-3] | 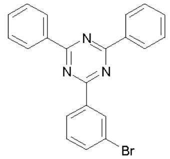 864377-31-1] | 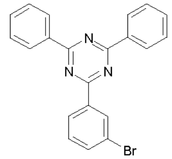 864377-31-1] | 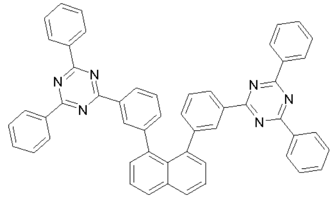 | 59% |
| 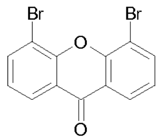 [1262398-42-4 ] | 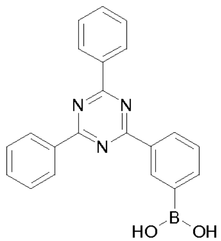 [1269508-31-7 ] | 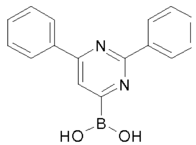 [1251825-66-7 ] | 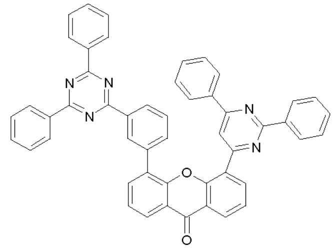 | 65% |
| 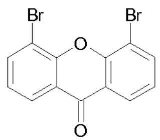 [1262398-42-4 ] | 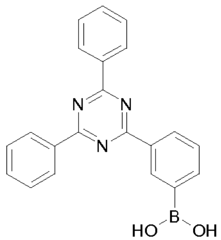 [1269508-31-7 ] | 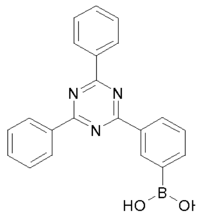 [1269508-31-7 ] | 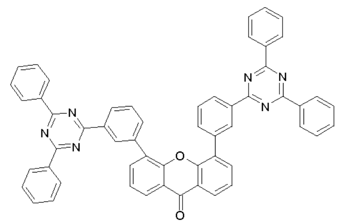 | 76% |

(fortgesetzt)

| Edukt 1 1eq. | Edukt 2 0,5 eq. | Edukt 3 0,5 eq. | Produkt | Ausbeute |
|---|---|---|---|---|
| 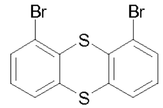 [176646-34-7 ] | 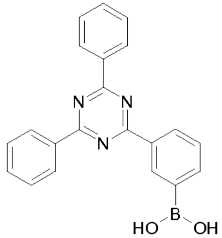 [1269508-31-7 ] | 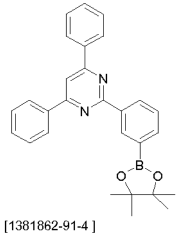 [1381862-91-4 ] | 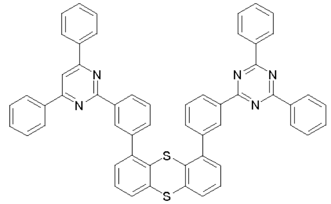 # | 65% |
| 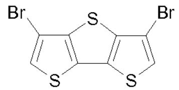 [502764-54-7] | 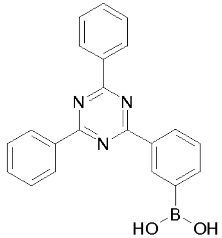 [1269508-31-7 ] | 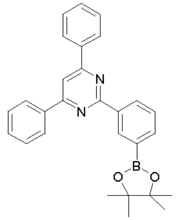 [1381862-91-4 ] | 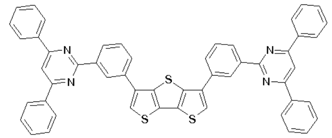 | 56% |
| 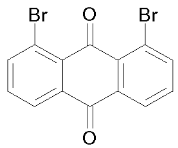 [38313-16-5] | 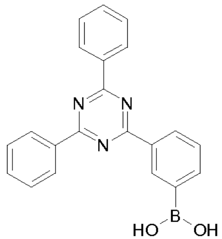 [1269508-31-7 ] | 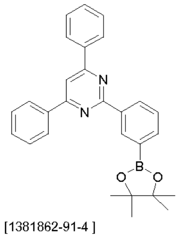 [1381862-91-4 ] | 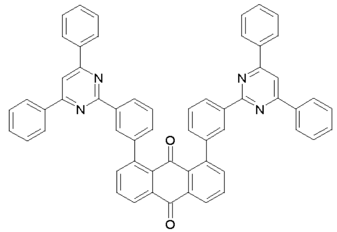 # | 61% |

# nicht erfindungsgemäß

**Beispiel 10**

**Synthese von 2-{6-[4-(2,6-Diphenyl-[1,3,4] triazin-4-yl)-phenyl]-dibenzofuran-4-yl}-4,6-diphenyl-[1,3,5]triazin**

**a) Herstellung von 2-(6-Bromo-dibenzofuran-4-yl)-4,6-diphenyl-[1,3,5]triazin**

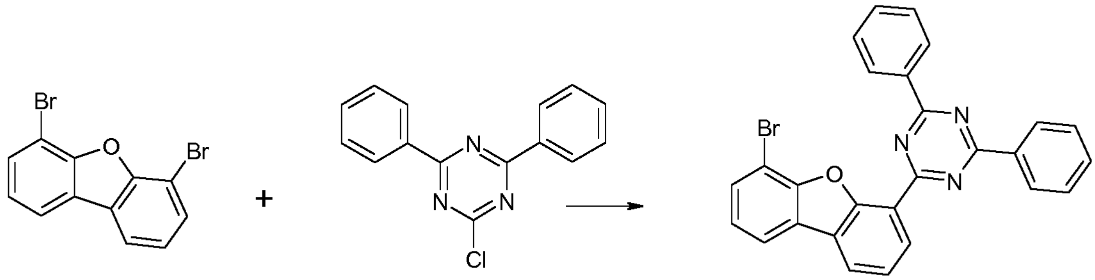

201138-91-2 3842-55-5

80 g (245 mmol) 4,6-Dibromdibenzofuran werden in einem ausgeheizten Kolben in 500 mL getrocknetem THF gelöst. Die Reaktionsmischung wird auf -78°C gekühlt. Bei dieser Temperatur werden 57 mL einer 1,9 M-Lösung n-Phenyllithium in Dibutylether (115 mmol) langsam zugetropft. Der Ansatz wird 1 Stunde bei -73°C nachgerührt. Anschließend werden 65 g 2-Chlor-4,6-diphenyl-1,3,5-triazin (245 mmol) in 150 ml THF gelöst und bei -70°C zugetropft. Nach beendeter Zugabe wird die Reaktionsmischung langsam auf Raumtemperatur erwärmt, über Nacht bei Raumtemperatur gerührt, mit Wasser gequencht und anschließend am Rotationsverdampfer eingeengt. Dabei fällt ein weißer Feststoff aus. Der Ansatz wird nun auf Raumtemperatur abgekühlt und der ausgefallene Feststoff wird abgesaugt und mit Methanol nachgewaschen. Die Ausbeute beträgt 40 g (84 mmol), entsprechend 34% der Theorie.

**b) Herstellung von 2-{6-[4-(2,6-Diphenyl-[1,3,4]triazin-4-yl)-phenyl]-dibenzofuran-4-yl}-4,6-diphenyl-[1,3,5]triazin**

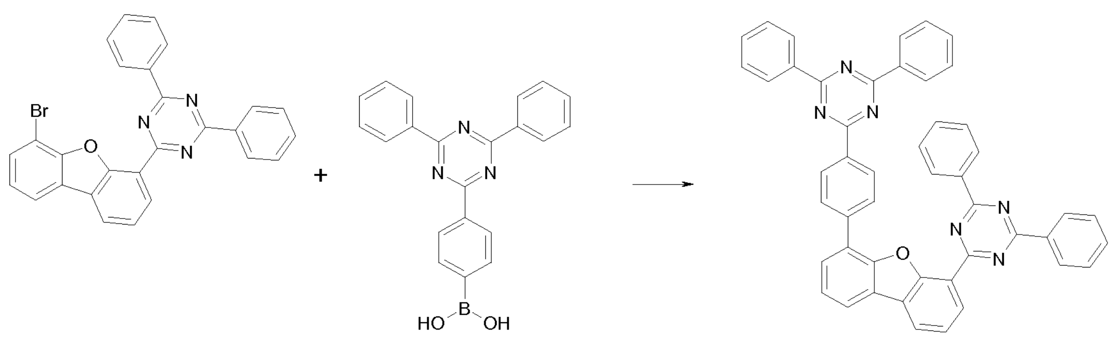

33,4 g (70 mmol) 2-(6-Bromo-dibenzofuran-4-yl)-4,6-diphenyl-[1,3,5]triazin, 24,7 g (70 mmol) 4-(4,6-diphenyl-1,3,5-triazin-2-yl)phenyl-boromsäure, 78,9 mL (158 mmol) $Na_2CO_3$ (2 M-Lösung) werden in 120 mL Ethanol und 100 mL Wasser suspendiert. Zu dieser Suspension werden 1,3 g (1,1 mmol) $Pd(PPh_3)_4$ gegeben, und die Reaktionsmischung wird 16 h unter Rückfluss erhitzt. Nach dem Erkalten wird die Mischung mit Dichloromethan versetzt, die organische Phase abgetrennt, über Kieselgel filtriert und aus Toluol umkristallisiert. Die Ausbeute beträgt 42 g (59 mmol), entsprechend 85% der Theorie.

**Beispiel 11**

**Herstellung von von 2-(4-Dibenzofuran-3-yl-phenyl)-4,6-diphenyl-[1,3,5]triazin**

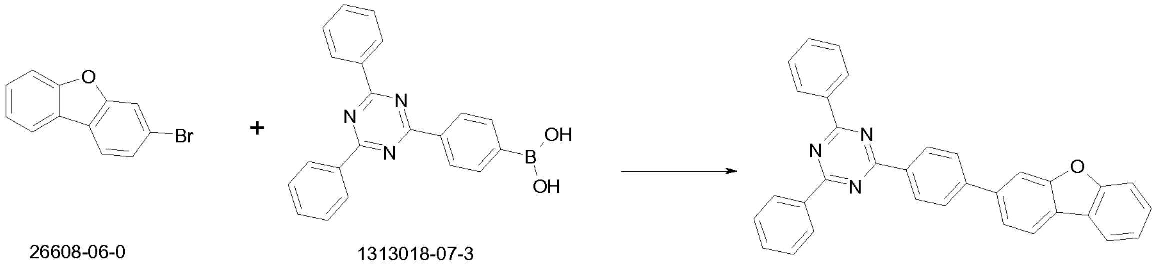

26608-06-0

1313018-07-3

24 g (70 mmol) 4-(4-6-diphenyl-1,3,5-triazin-2yl)phenyl)- boronsäure , 17,3 g (70 mmol) 3-Bromo-Dibenzofuran, 78,9 ml (158 mmol) $Na_2CO_3$ (2 M-Lösung) werden in 120 mL Ethanol und 100 mL Wasser suspendiert. Zu dieser Suspension werden 1,3 g (1,1 mmol) $Pd(PPh_3)_4$ gegeben, und die Reaktionsmischung wird 16 h unter Rückfluss erhitzt. Nach dem Erkalten wird die Mischung mit Dichloromethan versetzt, die organische Phase abgetrennt, über Kieselgel filtriert und aus Toluol umkristallisiert. Die Ausbeute beträgt 28 g (58 mmol), entsprechend 86% der Theorie.

Analog kann die folgende Verbindung hergestellt werden.

| Edukt 1 | Edukt 2 | Produkt | Ausbeute |
|---|---|---|---|
| 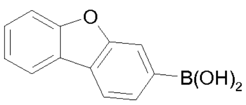 395087-89-5 | 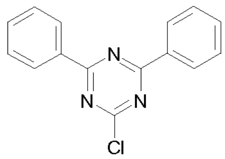 [3842-55-5 ] | 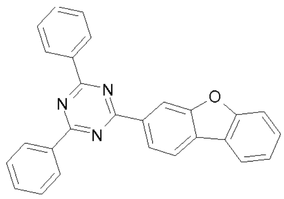 | 87% |

## Beispiel 12

### Herstellung von 2,4-Diphenyl-6-[4-(6-trimethylsilanyl-dibenzofuran-3-yl)-phenyl]-[1,3,5] triazin

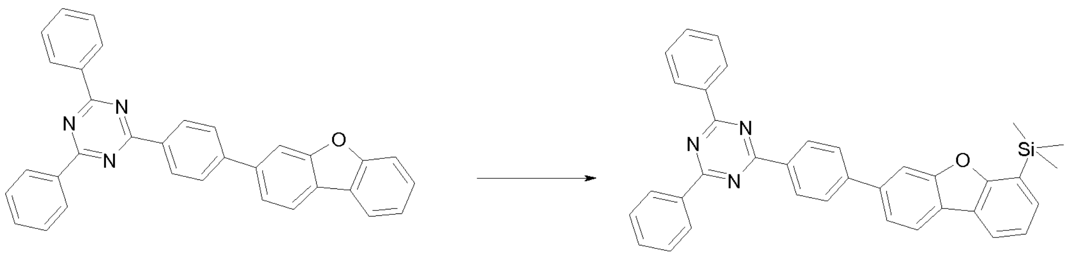

Eine auf 20°C gekühlte Lösung von 57,4 g (121 mmol) 2-(4-Dibenzofuran-3-yl-phenyl)-4,6-diphenyl-[1,3,5]triazin und 28 g (242 mmol) TMEDA in 1000 mL THF wird tropfenweise mit 127 mL (225,4 mmol) n-Buthyllithium (2.5 M in Hexan) versetzt. Die Reaktionsmischung wird 3 h bei Raumtemperatur gerührt, dann auf 0°C abgekühlt und innerhalb von 30 min. 26 g (242 mmol) Chlortrimetylsilan hinzugetropft. Es wird 8 h bei Raumtemperatur gerührt. Anschließend wird das Lösungsmittel im Vakuum entfernt und der Rückstand Chromateographisch über Kieselgel mit Chloroform als Laumittel gereinigt. Ausbeute: 41 g (74 mmol), 63% der Theorie.

Analog kann die folgende Verbindung hergestellt werden.

| Edukt 1 | Produkt | Ausbeute |
|---|---|---|
| 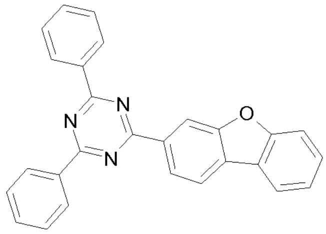 | 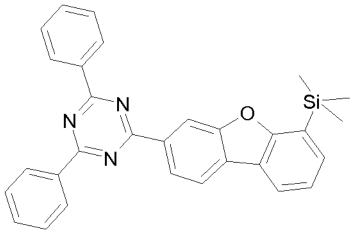 | 87% |

**Beispiel 13**

**Herstellung von 3-[4-(4,6-Diphenyl-[1,3,5]triazin-2-yl)-phenyl]-Dibenzofuran-6-boronsäure**

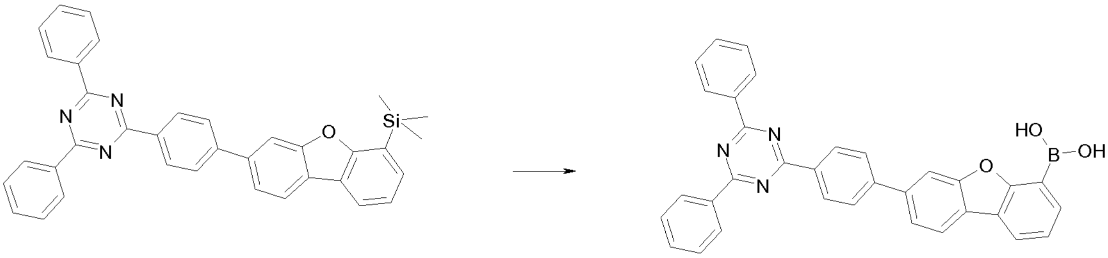

Unter Schutzgas wird eine Lösung von 39 g 2,4-Diphenyl-6-[4-(6-trimethyl-silanyl-dibenzofuran-3-yl)-phenyl]-[1,3,5] in 500 mL Dichloromethan tropfenweise mit 21 g (86 mmol) Bromtribromid versetzt und 10 h bei Raumtemperatur gerührt. Danach wird die Mischung langsam mit etwas Wasser versetzt und der ausgefallene Rückstand abfiltriert und mit Heptan gewaschen. Die Ausbeute beträgt 32 g (62 mmol), entsprechend 87% der Theorie.

Analog kann die folgende Verbindung hergestellt werden.

| Edukt 1 | Produkt | Ausbeute |
|---|---|---|
| 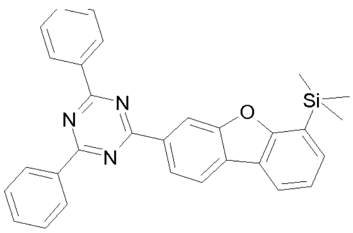 | 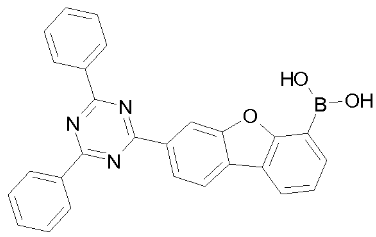 | 90% |

**Beispiel 14**

**Herstellung von 3-{7-[4-(4,6-Diphenyl-[1,3,5]triazin-2-yl)-phenyl]-dibenzofuran-4-yl}-9-phenyl-9H-carbazol**

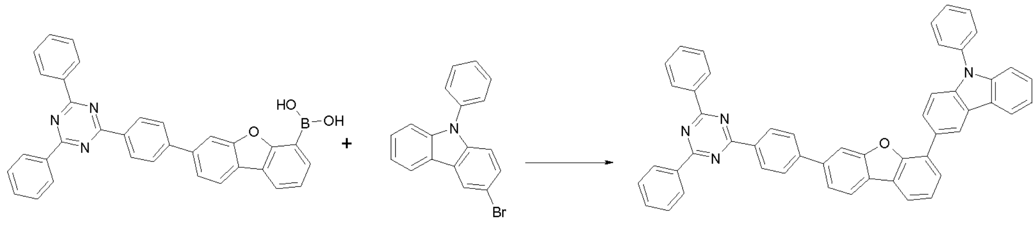

[1153-85-1]

36 g (70 mmol) 3-[4-(4,6-Diphenyl-[1,3,5]triazin-2-yl)-phenyl]-Dibenzofuran- 6- boronsäure 22,5 g (70 mmol) 3-Bromo-Dibenzofuran und 78,9 mL (158 mmol) $Na_2CO_3$ (2 M-Lösung) werden in 120 mL Ethanol und 100 mL Wasser suspendiert. Zu dieser Suspension werden 1,3 g (1,1 mmol) $Pd(PPh_3)_4$ gegeben, und die Reaktionsmischung wird 16 h unter Rückfluss erhitzt. Nach Erkalten wird Die Mischung mit Dichloromethan versetzt, die organische Phase abgetrennt, über Kieselgel filtriert und aus Toluol umkristallisier. Der Rückstand wird aus Toluol umkristallisiert und abschließend im Hochvakuum ($p = 5 \times 10^{-5}$ mbar) sublimiert. Die Ausbeute beträgt 39 g (54 mmol), entsprechend 80% der Theorie.

Analog kann die folgede Verbindung hergestellt werden.

| Edukt 1 | Edukt 2 | Produkt | Ausbeute |
|---|---|---|---|
| 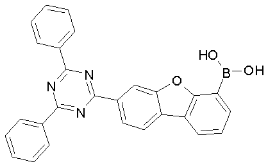 | 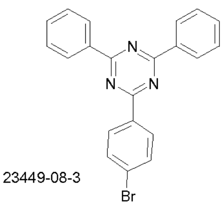 23449-08-3 | 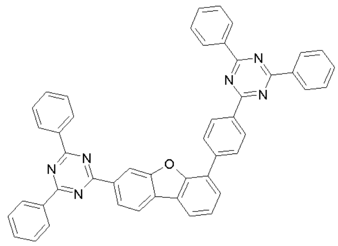 | 87% |

## Beispiel 15

### Herstellung und Charakterisierung der OLEDs

In den folgenden Beispielen V1 bis E22 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie INV-1:IC3:TEG1 (60%:35%:5%) bedeutet hierbei, dass das Material INV-1 in einem Volumenanteil von 60%, IC3 in einem Anteil von 35% und TEG1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich

bezeichnet die externe Quanteneffizienz bei einer Betriebsleuchtdichte von 1000 cd/m$^2$.

Die Daten der verschiedenen OLEDs sind in Tabelle 2 zusammengefasst. Die Beispiel V1-V8 sind Vergleichsbeispiele und zeigen OLEDs, die Materialien gemäß dem Stand der Technik enthalten. Die Beispiele E1-E22 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

**Verwendung von erfindungsgemäßen Verbindungen als Elektronentransportmaterialien**

Im Vergleich zu einer OLED, in denen das Material VG-6 gemäß dem Stand der Technik in der ETL verwendet werden, beobachtet man beim Einsatz der erfindungsgemäßen Materialien INV-5, INV-7, INV-15, INV-21 und INV-20 eine deutliche Verbesserung von Spannung und Effizienz. Insbesondere bei Einsatz der Substanz INV-5 erhält man eine gegenüber VG-6 um 1.5 V verbesserte Spannung, etwa 35% bessere externe Quanteneffizienz und eine mehr als verdoppelte Leistungseffizienz (Beispiele V6, E5)

**Verwendung von erfindungsgemäßen Verbindungen als Matrixmaterialien in phosphoreszierenden OLEDs**

Durch einfügen eines Phenylrings zwischen Pyrimidin und Dibonzofuran lässt sich die EQE um 15%, die Spannung um 0.1 V verbessern (Beispiele V1, E1). Analog gilt dies für Verbindungen mit Triazin (Beispiele V2, E2, E3).

Weiterhin ist es vorteilhaft, wenn eine Triazin- bzw. Carbazolgruppe faceto-face an das Dibenzofuran gebunden sind (Beispiele V4, E2, E3). Analog gilt dies, wenn es sich bei der Verbindungsgruppe zwischen Carbazol und Triazin nicht um ein Dibenzofuran sondern um ein Carbazol handelt (Beispiele V5, E4).

Tabelle 1: Aufbau der OLEDs

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | VG-1:TEG1 (90%:10%) 30nm | ST2 10nm | ST2:LiQ (50%:50%) 30nm | --- |
| V2 | SpA1 70nm | HATCN 5nm | SpMA1 90nm | VG-2:TEG1 (90%:10%) 30nm | ST2 10nm | ST2:LiQ (50%:50%) 30nm | --- |
| V3 | SpA1 70nm | HATCN 5nm | SpMA1 90nm | IC1:TEG1 (90%:10%) 30nm | --- | VG-7 40nm | LiQ 3nm |
| V4 | SpA1 70nm | HATCN 5nm | SpMA1 90nm | VG-4:TEG1 (90%:10%) 30nm | ST2 10nm | ST2:LiQ (50%:50%) 30nm | --- |
| V5 | SpA1 90nm | HATCN 5nm | SpMA1 130nm | VG-5:TER1 (92%:8%) 40nm | --- | ST2:LiQ (50%:50%) 40nm | --- |
| V6 | SpA1 70nm | HATCN 5nm | SpMA1 90nm | IC1:TEG1 (90%:10%) 30nm | --- | VG-6 40nm | LiQ 3nm |
| E1 | SpA1 70nm | HATCN 5nm | SpMA1 90nm | INV-1:TEG1 (90%:10%) 30nm | ST2 10nm | ST2:LiQ (50%:50%) 30nm | --- |
| E2 | SpA1 70nm | HATCN 5nm | SpMA1 90nm | INV-2:TEG1 (90%:10%) 30nm | ST2 10nm | ST2:LiQ (50%:50%) 30nm | --- |
| E3 | SpA1 70nm | HATCN 5nm | SpMA1 90nm | INV-3:TEG1 (90%:10%) 30nm | ST2 10nm | ST2:LiQ (50%:50%) 30nm | --- |
| E4 | SpA1 90nm | HATCN 5nm | SpMA1 130nm | INV-4:TER1 (92%:8%) 40nm | --- | ST2:LiQ (50%:50%) 40nm | --- |

(fortgesetzt)

| Bsp. | HTL Dicke | IL Dicke | EBL Dicke | EML Dicke | HBL Dicke | ETL Dicke | EIL Dicke |
|---|---|---|---|---|---|---|---|
| E5 | SpA1 70nm | HATCN 5nm | SpMA1 90nm | IC1:TEG1 (90%:10%) 30nm | --- | INV-5 40nm | LiQ 3nm |
| E6 | SpA1 70nm | HATCN 5nm | SpMA1 90nm | INV-10:TEG1 (90%:10%) 30nm | ST2 10nm | ST2:LiQ (50%:50%) 30nm | --- |
| E7 | SpA1 70nm | HATCN 5nm | SpMA1 90nm | INV-6:TEG1 (90%:10%) 30nm | ST2 10nm | ST2:LiQ (50%:50%) 30nm | --- |
| E8 | SpA1 70nm | HATCN 5nm | SpMA1 90nm | INV-7:TEG1 (90%:10%) 30nm | ST2 10nm | ST2:LiQ (50%:50%) 30nm | --- |
| E9 | SpA1 70nm | HATCN 5nm | SpMA1 90nm | IC1:TEG1 (90%:10%) 30nm | --- | INV-7 30nm | LiQ 3nm |
| E10 | SpA1 90nm | HATCN 5nm | SpMA1 130nm | INV-8:TER1 (92%:8%) 40nm | --- | ST2:LiQ (50%:50%) 40nm | --- |
| E11 | SpA1 70nm | HATCN 5nm | SpMA1 90nm | INV-9:IC3:TEG1 (60%:35%:5%) 30nm | IC1 10nm | ST2:LiQ (50%:50%) 30nm | --- |
| E12 | SpA1 70nm | HATCN 5nm | SpMA1 90nm | INV-11:IC2:TEG1 (45%:45%:10%) 30nm | IC1 10nm | ST2:LiQ (50%:50%) 30nm | --- |
| E13 | SpA1 70nm | HATCN 5nm | SpMA1 90nm | INV-12:TEG1 (90%:10%) 30nm | ST2 10nm | ST2:LiQ (50%:50%) 30nm | --- |
| E14 | SpA1 70nm | HATCN 5nm | SpMA1 90nm | INV-13:TEG1 (90%:10%) 30nm | ST2 10nm | ST2:LiQ (50%:50%) 30nm | --- |
| E15 | SpA1 70nm | HATCN 5nm | SpMA1 90nm | INV-14:TEG1 (90%:10%) 30nm | ST2 10nm | ST2:LiQ (50%:50%) 30nm | --- |
| E16 | SpA1 70nm | HATCN 5nm | SpMA1 90nm | IC1:TEG1 (90%:10%) 30nm | --- | INV-15 40nm | LiQ 3nm |
| E17 | SpA1 70nm | HATCN 5nm | SpMA1 90nm | INV-16:TEG1 (90%:10%) 30nm | ST2 10nm | ST2:LiQ (50%:50%) 30nm | --- |
| E18 | SpA1 70nm | HATCN 5nm | SpMA1 90nm | INV-17:TEG1 (90%:10%) 30nm | ST2 10nm | ST2:LiQ (50%:50%) 30nm | --- |
| E19 | SpA1 70nm | HATCN 5nm | SpMA1 90nm | IC1:TEG1 (90%:10%) 30nm | INV-18 10nm | ST1:LiQ (50%:50%) 30nm | --- |
| E20 | SpA1 70nm | HATCN 5nm | SpMA1 90nm | IC2:TEG1 (90%:10%) 30nm | INV-19 10nm | ST2:LiQ (50%:50%) 30nm | --- |
| E21 | SpA1 70nm | HATCN 5nm | SpMA1 90nm | IC1:TEG1 (90%:10%) 30nm | --- | INV-20 40nm | LiQ 3nm |
| E22 # | SpA1 70nm | HATCN 5nm | SpMA1 90nm | IC2:TEG1 (90%:10%) 30nm | --- | INV-21 40nm | LiQ 3nm |
| # nicht erfindungsgemäß | | | | | | | |

Tabelle 2: Daten der OLEDs

| Bsp. | U1000 (V) | SE1000 (cd/A) | LE1000 (lm/W) | EQE 1000 | CIE x/y bei 1000 cd/$m^2$ |
|---|---|---|---|---|---|
| V1 | 3.7 | 50 | 43 | 13.7% | 0.33/0.62 |

(fortgesetzt)

| Bsp. | U1000 (V) | SE1000 (cd/A) | LE1000 (lm/W) | EQE 1000 | CIE x/y bei 1000 cd/m$^2$ |
|---|---|---|---|---|---|
| V2 | 3.6 | 53 | 45 | 14.4% | 0.34/0.62 |
| V3 | 3.6 | 56 | 49 | 15.5% | 0.34/0.62 |
| V4 | 3.4 | 52 | 49 | 14.1% | 0.33/0.62 |
| V5 | 4.8 | 9.4 | 6.1 | 10.1% | 0.67/0.33 |
| V6 | 4.4 | 45 | 31 | 12.4% | 0.34/0.62 |
| E1 | 3.6 | 58 | 51 | 15.8% | 0.33/0.62 |
| E2 | 3.4 | 61 | 57 | 16.7% | 0.33/0.62 |
| E3 | 3.5 | 57 | 51 | 15.6% | 0.34/0.62 |
| E4 | 4.6 | 10.5 | 7.1 | 11.3% | 0.67/0.33 |
| E5 | 2.9 | 62 | 68 | 16.9% | 0.34/0.63 |
| E6 | 3.7 | 63 | 54 | 17.6% | 0.33/0.63 |
| E7 | 3.1 | 59 | 59 | 16.4% | 0.34/0.62 |
| E8 | 4.3 | 50 | 36 | 14.0% | 0.39/0.59 |
| E9 | 4.0 | 56 | 45 | 15.2% | 0.33/0.62 |
| E10 | 4.2 | 12.3 | 9.1 | 12.4% | 0.67/0.33 |
| E11 | 3.2 | 61 | 60 | 17.1% | 0.34/0.62 |
| E12 | 3.6 | 47 | 41 | 13.1% | 0.32/0.63 |
| E13 | 3.2 | 51 | 50 | 14.4% | 0.33/0.62 |
| E14 | 3.6 | 59 | 52 | 16.6% | 0.34/0.62 |
| E15 | 3.6 | 56 | 49 | 15.8% | 0.33/0.62 |
| E16 | 2.8 | 58 | 65 | 16.2% | 0.32/0.63 |
| E17 | 3.4 | 56 | 51 | 15.5% | 0.34/0.62 |
| E18 | 3.7 | 53 | 46 | 15.0% | 0.33/0.63 |
| E19 | 3.6 | 58 | 51 | 16.1% | 0.32/0.63 |
| E20 | 3.4 | 49 | 46 | 13.7% | 0.35/0.61 |
| E21 | 3.8 | 55 | 45 | 15.5% | 0.35/0.62 |
| E22 # | 3.0 | 49 | 51 | 14.5% | 0.35/0.61 |
| # nicht erfindungsgemäß | | | | | |

Tabelle 3: Verwendete Materialien

| | |
|---|---|
| 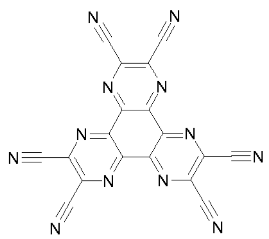 | 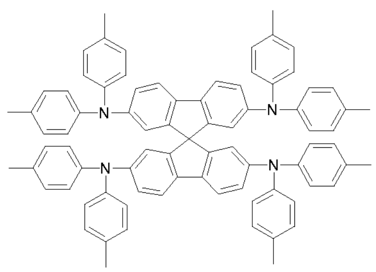 |
| HATCN | SpA1 |

(fortgesetzt)

| | |
|---|---|
| 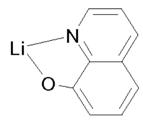 | 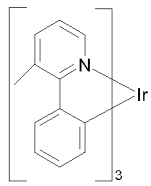 |
| LiQ | TEG1 |
| 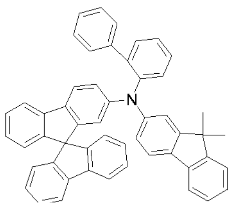 | 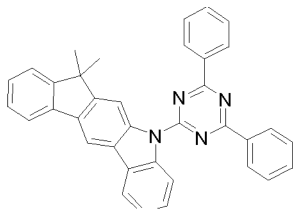 |
| SpMA1 | IC1 |
| 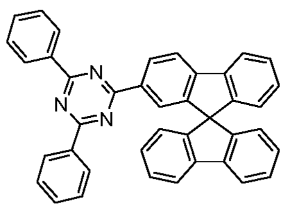 | 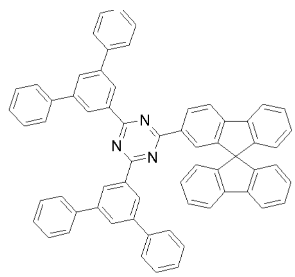 |
| ST1 | ST2 |
| 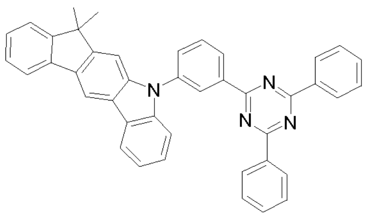 | 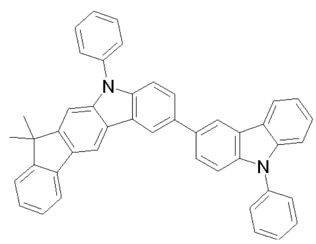 |
| IC2 | IC3 |
| 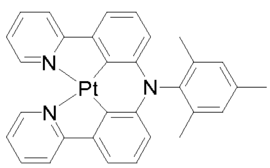 | 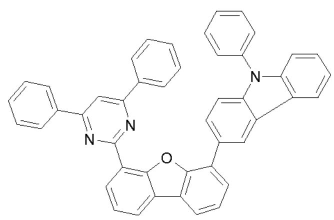 WO 2011/057706 |
| TER1 | VG-1 |

(fortgesetzt)

| | |
|---|---|
| 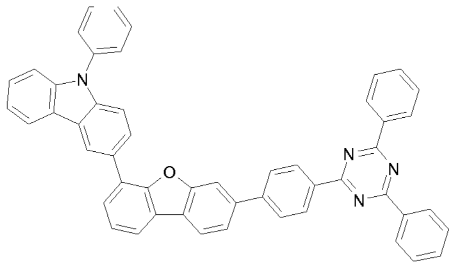 | 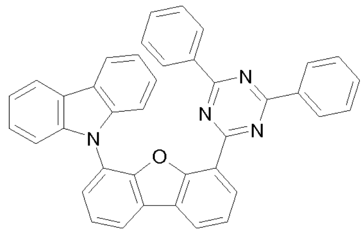 |
| VG-2 | VG-3 |
| 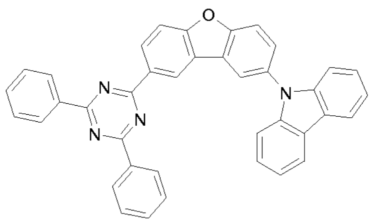 JP 2009/21336 | 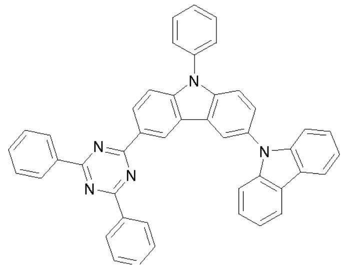 JP 2009/21336 |
| VG-4 | VG-5 |
| 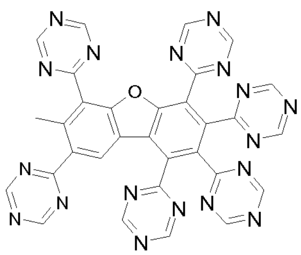 WO 2009/069442 | 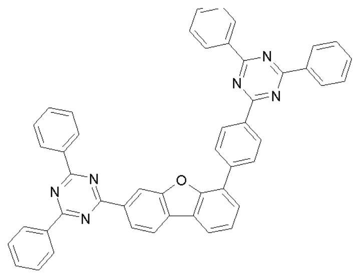 |
| VG-6 | VG-7 |
| 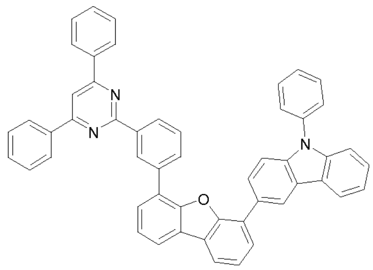 | 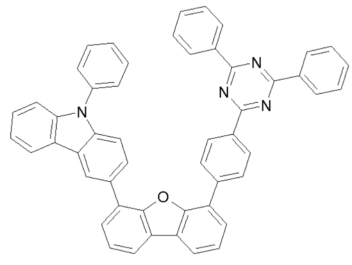 |
| INV-1 | INV-2 |
| 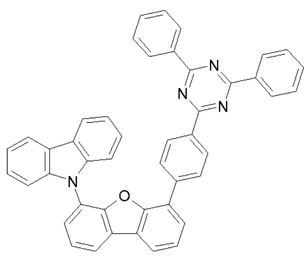 | 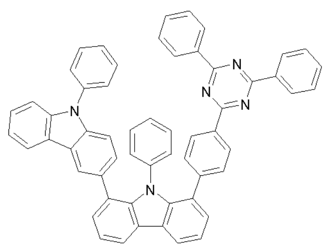 |
| INV-3 | INV-4 |

(fortgesetzt)

| | |
|---|---|
| 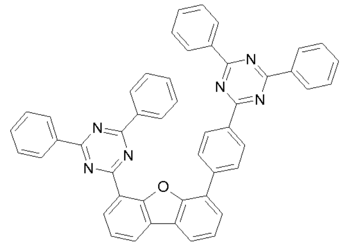 | 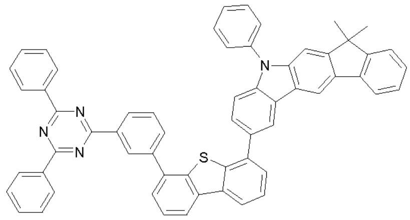 |
| INV-5 | INV-6 |
| 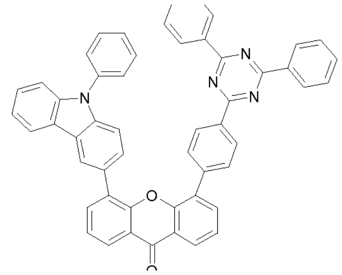 | 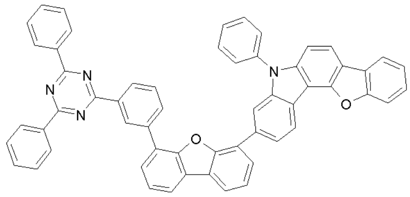 |
| INV-7 | INV-8 |
| 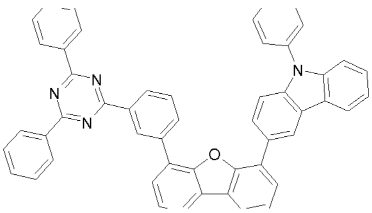 | 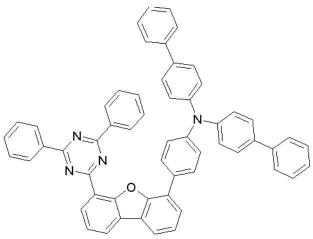 |
| INV-9 | INV-10 |
| 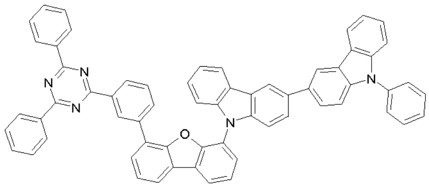 | 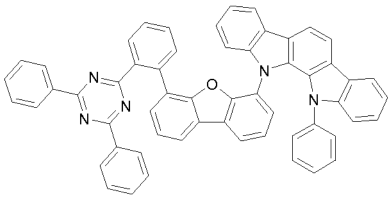 |
| INV-11 | INV-12 |
| 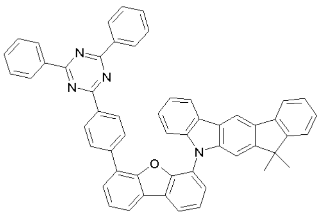 | 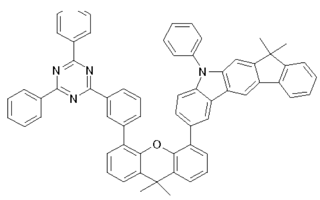 |
| INV-13 | INV-14 |
| 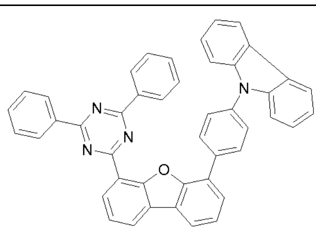 | 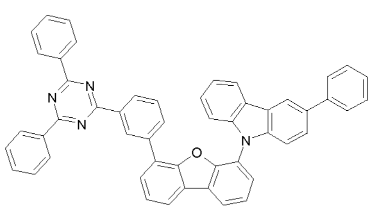 |

(fortgesetzt)

| INV-15 | INV-16 |
|---|---|
| 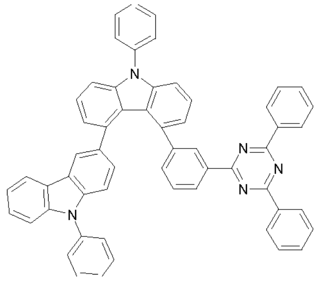 | 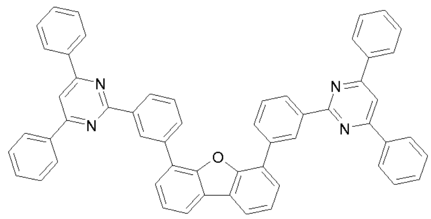 |
| INV-17 | INV-18 |
| 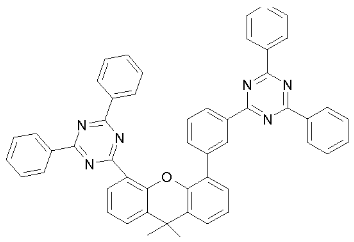 | 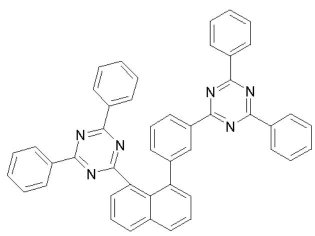 |
| INV-19 | INV-20 |
| 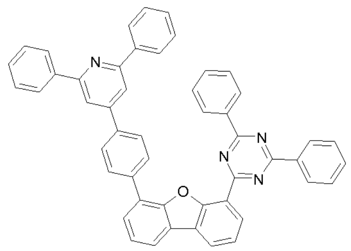 | |
| INV-20 # | |
| # nicht erfindungsgemäß | |

| Edukt 1 | Edukt 2 | Produkt | Ausbeute |
|---|---|---|---|
| 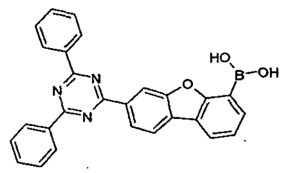 | 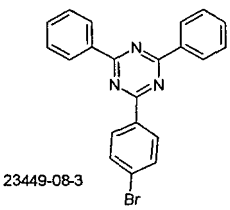 23449-08-3 | 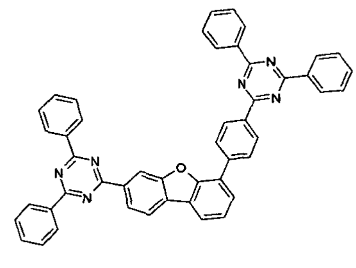 | 87% |

**Beispiel 15**

**Herstellung und Charakterisierung der OLEDs**

In den folgenden Beispielen V1 bis E22 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert). Diese beschichteten Glasplättchen

bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie INV-1:IC3:TEG1 (60%:35%:5%) bedeutet hierbei, dass das Material INV-1 in einem Volumenanteil von 60%, IC3 in einem Anteil von 35% und TEG1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$.

Die Daten der verschiedenen OLEDs sind in Tabelle 2 zusammengefasst. Die Beispiel V1-V8 sind Vergleichsbeispiele und zeigen OLEDs, die Materialien gemäß dem Stand der Technik enthalten. Die Beispiele E1-E22 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

**Verwendung von erfindungsgemäßen Verbindungen als Elektronentransportmaterialien**

Im Vergleich zu einer OLED, in denen das Material VG-6 gemäß dem Stand der Technik in der ETL verwendet werden, beobachtet man beim Einsatz der erfindungsgemäßen MaterialienINV-5, INV-7, INV-15, INV-21 und INV-20 eine deutliche Verbesserung von Spannung und Effizienz. Insbesondere bei Einsatz der Substanz INV-5 erhält man eine gegenüber VG-6 um 1.5 V verbesserte Spannung, etwa 35% bessere externe Quanteneffizienz und eine mehr als verdoppelte Leistungseffizienz (Beispiele V6, E5)

**Verwendung von erfindungsgemäßen Verbindungen als Matrixmaterialien in phosphoreszierenden OLEDs**

Durch einfügen eines Phenylrings zwischen Pyrimidin und Dibonzofuran lässt sich die EQE um 15%, die Spannung um 0.1 V verbessern (Beispiele V1, E1). Analog gilt dies für Verbindungen mit Triazin (Beispiele V2, E2, E3).

Weiterhin ist es vorteilhaft, wenn eine Triazin- bzw. Carbazolgruppe faceto-face an das Dibenzofuran gebunden sind (Beispiele V4, E2, E3). Analog gilt dies, wenn es sich bei der Verbindungsgruppe zwischen Carbazol und Triazin nicht um ein Dibenzofuran sondern um ein Carbazol handelt (Beispiele V5, E4).

Tabelle 1: Aufbau der OLEDs

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | VG-1:TEG1 (90%:10%) 30nm | ST2 10nm | ST2:LiQ (50%:50%) 30nm | --- |
| V2 | SpA1 70nm | HATCN 5nm | SpMA1 90nm | VG-2:TEG1 (90%:10%) 30nm | ST2 10nm | ST2:LiQ (50%:50%) 30nm | --- |
| V3 | SpA1 | HATCN | SpMA1 | IC1:TEG1 | --- | VG-7 | LiQ |

(fortgesetzt)

| Bsp. | HTL Dicke | IL Dicke | EBL Dicke | EML Dicke | HBL Dicke | ETL Dicke | EIL Dicke |
|---|---|---|---|---|---|---|---|
| | 70nm | 5nm | 90nm | (90%:10%) 30nm | | 40nm | 3nm |
| V4 | SpA1 70nm | HATCN 5nm | SpMA1 90nm | VG-4:TEG1 (90%:10%) 30nm | ST2 10nm | ST2:LiQ (50%:50%) 30nm | --- |
| V5 | SpA1 90nm | HATCN 5nm | SpMA1 130nm | VG-5:TER1 (92%:8%) 40nm | --- | ST2:LiQ (50%:50%) 40nm | --- |
| V6 | SpA1 70nm | HATCN 5nm | SpMA1 90nm | IC1:TEG1 (90%:10%) 30nm | --- | VG-6 40nm | LiQ 3nm |
| E1 | SpA1 70nm | HATCN 5nm | SpMA1 90nm | INV-1:TEG1 (90%:10%) 30nm | ST2 10nm | ST2:LiQ (50%:50%) 30nm | --- |
| E2 | SpA1 70nm | HATCN 5nm | SpMA1 90nm | INV-2:TEG1 (90%:10%) 30nm | ST2 10nm | ST2:LiQ (50%:50%) 30nm | --- |
| E3 | SpA1 70nm | HATCN 5nm | SpMA1 90nm | INV-3:TEG1 (90%:10%) 30nm | ST2 10nm | ST2:LiQ (50%:50%) 30nm | --- |
| E4 | SpA1 90nm | HATCN 5nm | SpMA1 130nm | INV-4:TER1 (92%:8%) 40nm | --- | ST2:LiQ (50%:50%) 40nm | --- |
| E5 | SpA1 70nm | HATCN 5nm | SpMA1 90nm | IC1:TEG1 (90%:10%) 30nm | --- | INV-5 40nm | LiQ 3nm |
| E6 | SpA1 70nm | HATCN 5nm | SpMA1 90nm | INV-10:TEG1 (90%:10%) 30nm | ST2 10nm | ST2:LiQ (50%:50%) 30nm | --- |
| E7 | SpA1 70nm | HATCN 5nm | SpMA1 90nm | INV-6:TEG1 (90%:10%) 30nm | ST2 10nm | ST2:LiQ (50%:50%) 30nm | --- |
| E8 | SpA1 70nm | HATCN 5nm | SpMA1 90nm | INV-7:TEG1 (90%:10%) 30nm | ST2 10nm | ST2:LiQ (50%:50%) 30nm | --- |
| E9 | SpA1 70nm | HATCN 5nm | SpMA1 90nm | IC1:TEG1 (90%:10%) 30nm | --- | INV-7 30nm | LiQ 3nm |
| E10 | SpA1 90nm | HATCN 5nm | SpMA1 130nm | INV-8:TER1 (92%:8%) 40nm | --- | ST2:LiQ (50%:50%) 40nm | --- |
| E11 | SpA1 70nm | HATCN 5nm | SpMA1 90nm | INV-9:IC3:TEG1 (60%:35%:5%) 30nm | IC1 10nm | ST2:LiQ (50%:50%) 30nm | --- |
| E12 | SpA1 70nm | HATCN 5nm | SpMA1 90nm | INV-11:IC2:TEG1 (45%:45%:10%) 30nm | IC1 10nm | ST2:LiQ (50%:50%) 30nm | --- |
| E13 | SpA1 70nm | HATCN 5nm | SpMA1 90nm | INV-12:TEG1 (90%:10%) 30nm | ST2 10nm | ST2:LiQ (50%:50%) 30nm | --- |
| E14 | SpA1 70nm | HATCN 5nm | SpMA1 90nm | INV-13:TEG1 (90%:10%) 30nm | ST2 10nm | ST2:LiQ (50%:50%) 30nm | --- |
| E15 | SpA1 70nm | HATCN 5nm | SpMA1 90nm | INV-14:TEG1 (90%:10%) 30nm | ST2 10nm | ST2:LiQ (50%:50%) 30nm | --- |
| E16 | SpA1 70nm | HATCN 5nm | SpMA1 90nm | IC1:TEG1 (90%:10%) 30nm | --- | INV-15 40nm | LiQ 3nm |
| E17 | SpA1 70nm | HATCN 5nm | SpMA1 90nm | INV-16:TEG1 (90%:10%) 30nm | ST2 10nm | ST2:LiQ (50%:50%) 30nm | --- |
| E18 | SpA1 | HATCN | SpMA1 | INV-17:TEG1 | ST2 | ST2:LiQ | --- |

(fortgesetzt)

| Bsp. | HTL Dicke | IL Dicke | EBL Dicke | EML Dicke | HBL Dicke | ETL Dicke | EIL Dicke |
|---|---|---|---|---|---|---|---|
| | 70nm | 5nm | 90nm | (90%:10%) 30nm | 10nm | (50%:50%) 30nm | |
| E19 | SpA1 70nm | HATCN 5nm | SpMA1 90nm | IC1:TEG1 (90%:10%) 30nm | INV-18 10nm | ST1:LiQ (50%:50%) 30nm | --- |
| E20 | SpA1 70nm | HATCN 5nm | SpMA1 90nm | IC2:TEG1 (90%:10%) 30nm | INV-19 10nm | ST2:LiQ (50%:50%) 30nm | --- |
| E21 | SpA1 70nm | HATCN 5nm | SpMA1 90nm | IC1:TEG1 (90%:10%) 30nm | --- | INV-20 40nm | LiQ 3nm |
| E22 | SpA1 70nm | HATCN 5nm | SpMA1 90nm | IC2:TEG1 (90%:10%) 30nm | --- | INV-21 40nm | LiQ 3nm |

Tabelle 2: Daten der OLEDs

| Bsp. | U1000 (V) | SE1000 (cd/A) | LE1000 (lm/W) | EQE 1000 | CIE x/y bei 1000 cd/$m^2$ |
|---|---|---|---|---|---|
| V1 | 3.7 | 50 | 43 | 13.7% | 0.33/0.62 |
| V2 | 3.6 | 53 | 45 | 14.4% | 0.34/0.62 |
| V3 | 3.6 | 56 | 49 | 15.5% | 0.34/0.62 |
| V4 | 3.4 | 52 | 49 | 14.1% | 0.33/0.62 |
| V5 | 4.8 | 9.4 | 6.1 | 10.1% | 0.67/0.33 |
| V6 | 4.4 | 45 | 31 | 12.4% | 0.34/0.62 |
| E1 | 3.6 | 58 | 51 | 15.8% | 0.33/0.62 |
| E2 | 3.4 | 61 | 57 | 16.7% | 0.33/0.62 |
| E3 | 3.5 | 57 | 51 | 15.6% | 0.34/0.62 |
| E4 | 4.6 | 10.5 | 7.1 | 11.3% | 0.67/0.33 |
| E5 | 2.9 | 62 | 68 | 16.9% | 0.34/0.63 |
| E6 | 3.7 | 63 | 54 | 17.6% | 0.33/0.63 |
| E7 | 3.1 | 59 | 59 | 16.4% | 0.34/0.62 |
| E8 | 4.3 | 50 | 36 | 14.0% | 0.39/0.59 |
| E9 | 4.0 | 56 | 45 | 15.2% | 0.33/0.62 |
| E10 | 4.2 | 12.3 | 9.1 | 12.4% | 0.67/0.33 |
| E11 | 3.2 | 61 | 60 | 17.1% | 0.34/0.62 |
| E12 | 3.6 | 47 | 41 | 13.1% | 0.32/0.63 |
| E13 | 3.2 | 51 | 50 | 14.4% | 0.33/0.62 |
| E14 | 3.6 | 59 | 52 | 16.6% | 0.34/0.62 |
| E15 | 3.6 | 56 | 49 | 15.8% | 0.33/0.62 |
| E16 | 2.8 | 58 | 65 | 16.2% | 0.32/0.63 |
| E17 | 3.4 | 56 | 51 | 15.5% | 0.34/0.62 |
| E18 | 3.7 | 53 | 46 | 15.0% | 0.33/0.63 |
| E19 | 3.6 | 58 | 51 | 16.1% | 0.32/0.63 |
| E20 | 3.4 | 49 | 46 | 13.7% | 0.35/0.61 |

(fortgesetzt)

| Bsp. | U1000 (V) | SE1000 (cd/A) | LE1000 (lm/W) | EQE 1000 | CIE x/y bei 1000 cd/$m^2$ |
|---|---|---|---|---|---|
| E21 | 3.8 | 55 | 45 | 15.5% | 0.35/0.62 |
| E22 | 3.0 | 49 | 51 | 14.5% | 0.35/0.61 |

Tabelle 3: Verwendete Materialien

| | |
|---|---|
| 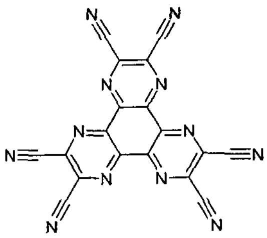 | 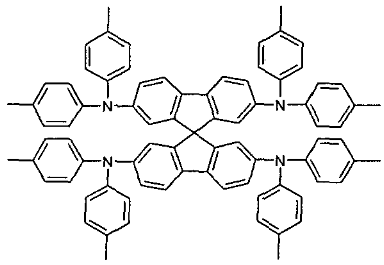 |
| HATCN | SpA1 |
| 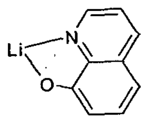 | 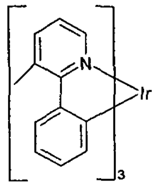 |
| LiQ | TEG1 |
| 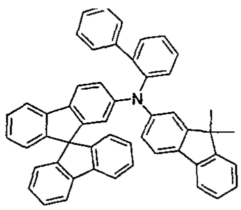 | 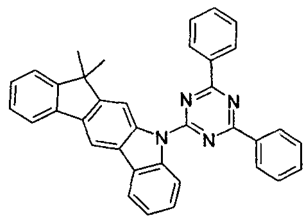 |
| SpMA1 | IC1 |
| 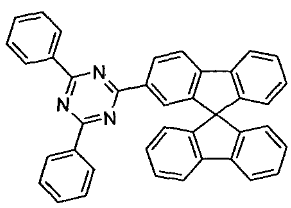 | 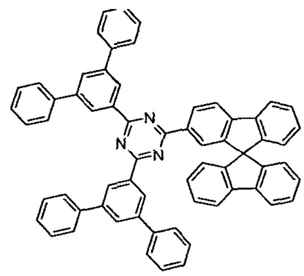 |
| ST1 | ST2 |
| 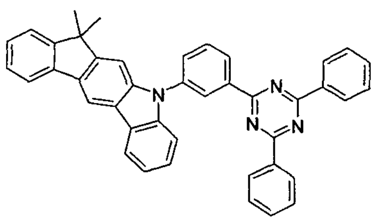 | 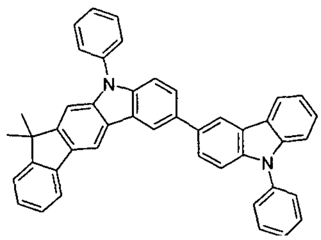 |
| IC2 | IC3 |

(fortgesetzt)

| | |
|---|---|
| 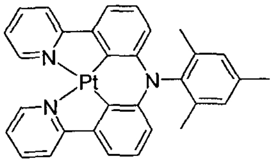 | 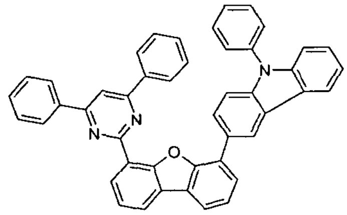 WO 2011/057706 |
| TER1 | VG-1 |
| | |
| 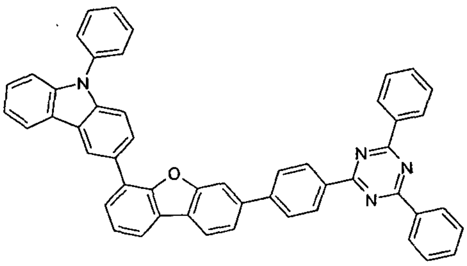 | 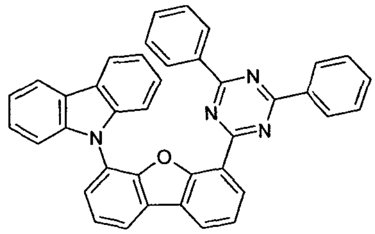 |
| VG-2 | VG-3 |
| 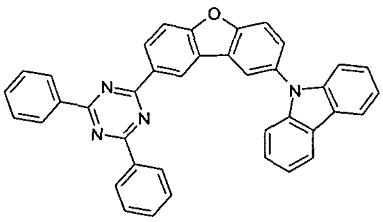 JP 2009/21336 | 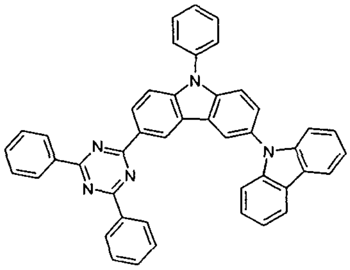 JP 2009/21336 |
| VG-4 | VG-5 |
| 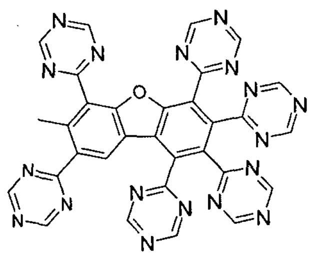 WO 2009/069442 | 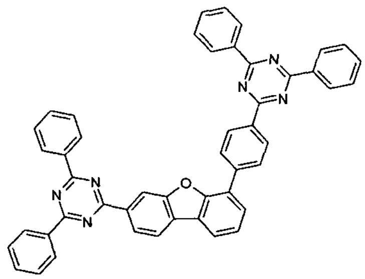 |
| VG-6 | VG-7 |
| 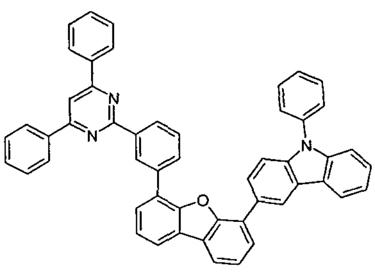 | 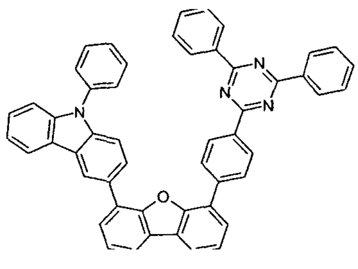 |
| INV-1 | INV-2 |

(fortgesetzt)

| | |
|---|---|
| 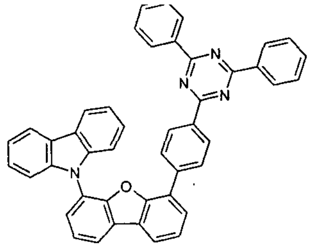 | 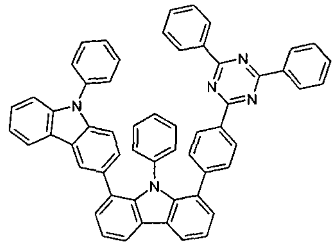 |
| INV-3 | INV-4 |
| 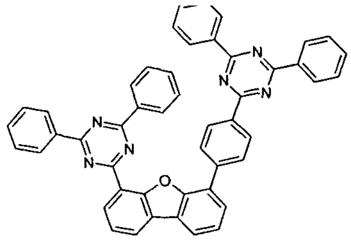 | 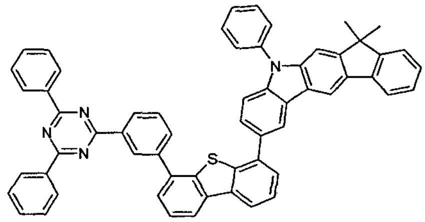 |
| INV-5 | INV-6 |
| 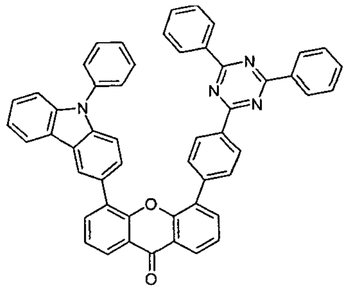 | 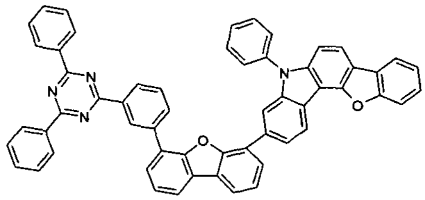 |
| INV-7 | INV-8 |
| 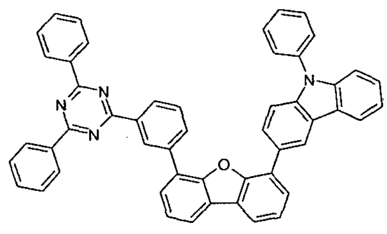 | 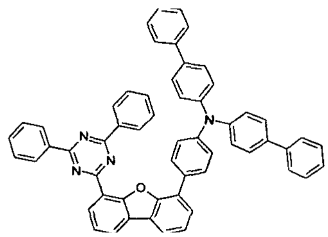 |
| INV-9 | INV-10 |
| 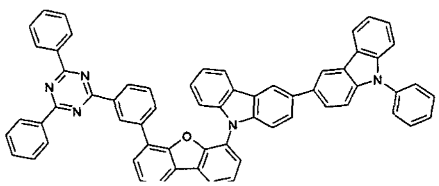 | 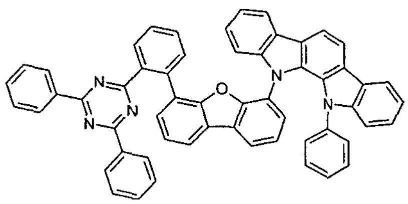 |
| INV-11 | INV-12 |

(fortgesetzt)

| | |
|---|---|
| 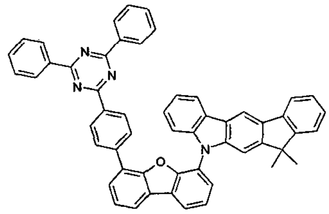 | 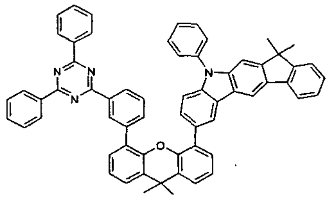 |
| INV-13 | INV-14 |
| 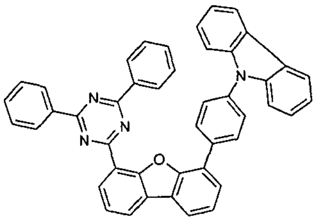 | 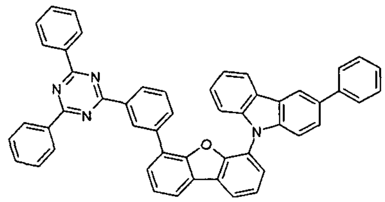 |
| INV-15 | INV-16 |
| 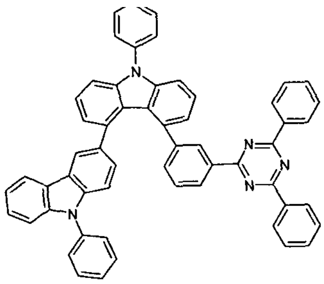 | 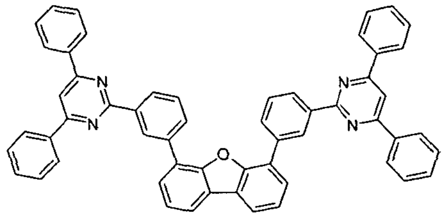 |
| INV-17 | INV-18 |
| 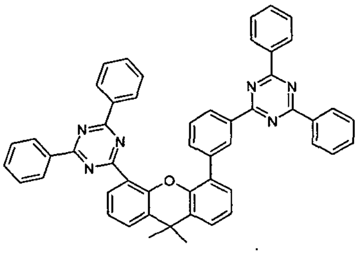 | 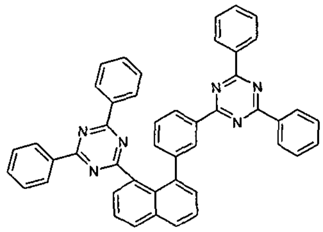 |
| INV-19 | INV-20 |
| 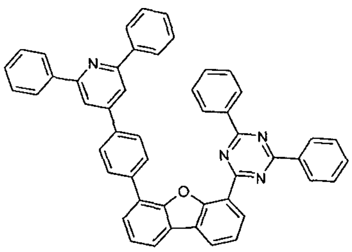 | |
| INV-20 | |

## Patentansprüche

**1.** Verbindung der allgemeinen Formel (1)

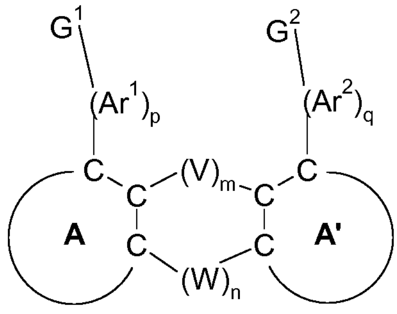

Formel (1)

wobei für die verwendeten Symbole und Indizes gilt:

A und A' sind gleich oder verschieden voneinander, ein aromatischer oder heteroaromatischer Ring mit 5 oder 6 Ringatomen, die mit einem oder mehreren Resten $R^1$, die unabhängig voneinander sein können, substituiert sein können;

$G^1$, $G^2$ sind bei jedem Auftreten gleich oder verschieden

eine organische elektronentransportierende Gruppe (ETG) aus der Gruppe der elektronenarmen heteroaromatischen Gruppen aus der Gruppe der Triazine, Pyrimidine und Pyrazine;
oder eine elektronenreiche organische Gruppe, die Löcher leitet (LTG), wobei die LTG ausgewählt ist aus der Gruppe der Arylamine,Triaryl-amine, der verbrückten Amine, bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole, wobei wenigstens eine der beiden Gruppen $G^1$ oder $G^2$ eine elektronentransportierende Gruppe (ETG) sein muss und wobei die Gruppen $G^1$ und $G^2$ mit einem oder mehreren voneinander unabhängigen Resten $R^1$ substituiert sein können;
$Ar^1$ ist, wenn $G^1$ eine elektonentransportierende Gruppe ist, ein bivalenter aromatischer oder heteroaromatischer, bevorzugt ein aromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist, wenn $Ar^1$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist
oder, wenn $G^1$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^1$ eine Phenylen-, Biphenylen- oder Terphenylengruppe ist und eine Phenylengruppe besonders bevorzugt ist;
$Ar^2$ ist, wenn $G^2$ eine elektonentransportierende Gruppe ist, ein bivalenter aromatischer oder heteroaromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist wenn $Ar^2$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist
oder, wenn $G^2$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^2$ eine Phenylen-, Biphenylen- oder Terphenylengruppe ist und eine Phenylengruppe besonders bevorzugt ist;
V ist eine Einfachbindung, $NAr^3$, O, S, , wobei im Fall einer Einfachbindung die Kohlenstoffatome der Ringe A und A' direkt durch eine Einfachbindung miteinander verbunden sind, wobei eine Einfachbindung, $NAr^3$, O und S bevorzugt sind, wobei eine Einfachbindung, O und S ganz bevorzugt sind, wobei O und S ganz besonders bevorzugt sind, wobei O insbesondere bevorzugt ist;
W ist eine Einfachbindung, C=O, $C(R^1)_2$ oder $NR^1$, wobei im Fall einer Einfachbindung die Kohlenstoffatome der Ringe A und A' direkt durch eine Einfachbindung miteinander verbunden sind, wobei eine Einfachbindung, $C(R^1)_2$ oder $NR^1$ bevorzugt sind, wobei eine Einfachbindung oder $C(R^1)_2$ ganz bevorzugt sind;
wobei weiterhin bevorzugt ist, dass V eine Einfachbindung ist sofern W keine Einfachbindung ist bzw. dass W eine Einfachbindung ist sofern V keine Einfachbindung ist;

wobei weiterhin ganz bevorzugt ist, dass V eine Einfachbindung ist sofern W gleich O oder S ist bzw. dass W eine Einfachbindung ist sofern V gleich O oder S ist;
wobei weiterhin ganz besonders bevorzugt ist, dass V eine Einfachbindung ist sofern W gleich O ist bzw. dass W eine Einfachbindung ist sofern V gleich O ist;
m ist entweder 0 oder 1;
n ist entweder 0 oder 1,
wobei m = n gilt;
p ist entweder 0 oder 1;
q ist entweder 0 oder 1, wobei gilt, dass p + q entweder 1 oder 2 ist;
$Ar^3$ ist ein aromatischer Ring oder Ringsystem mit 6 bis 30 Ring-atomen, wobei der Ring oder das jeweils durch einen oder mehrere Reste $R^2$ substi-tuiert sein kann, die durch einen oder mehrere Reste $R^3$ substituiert sein können, wobei zwei oder mehr Reste $R^2$ miteinander einen Ringschluss bilden können;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;
$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;
$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden;

mit der Maßgabe, dass maximal ein Substituent $R^1$ des Rings A und maximal ein Substituent $R^1$ des Rings A' eine aromatische oder heteroaromatische Gruppe mit 5 bis 30 Ringatomen enthält;
wobei die folgenden Verbindungen vom Schutzumfang ausgenommen sind

| Compound 1-b-1 | Compound 1-b-2 |
|---|---|

(fortgesetzt)

| | |
|---|---|
| 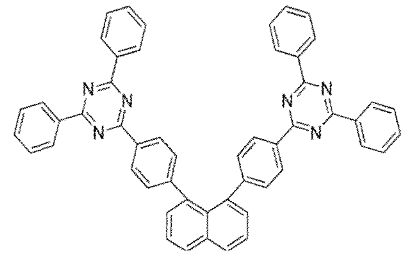 | 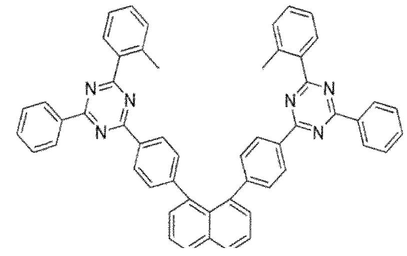 |
| Compound 1-b-3 | Compound 1-b-4 |
| 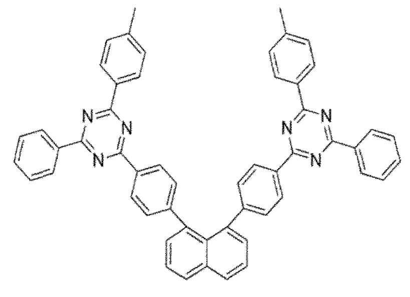 | 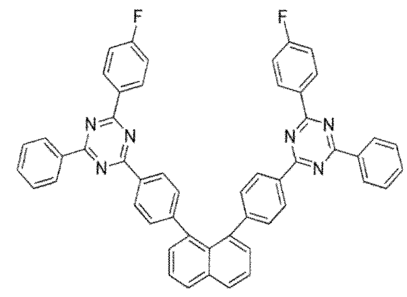 |
| Compound 1-b-5 | Compound 1-b-6 |
| 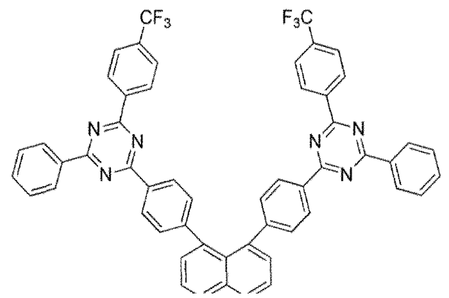 | 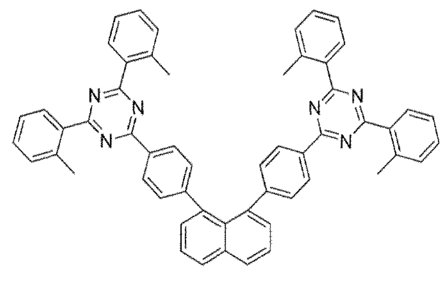 |
| Compound 1-b-7 | Compound 1-b-8 |
| 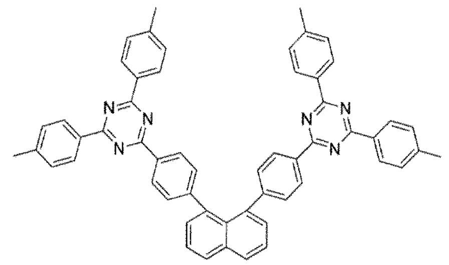 | 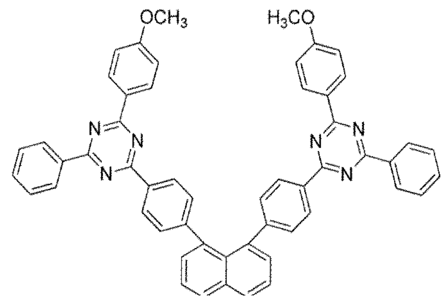 |
| Compound 1-b-9 | Compound 1-b-10 |
| | |
| 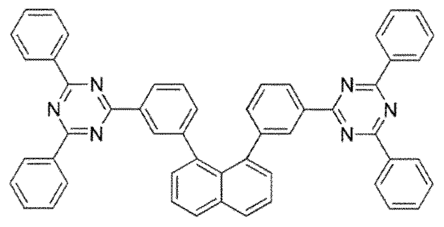 | 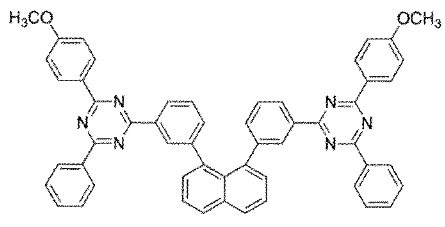 |
| Compound 1-b-11 | Compound 1-b-12 |

(fortgesetzt)

| | |
|---|---|
| 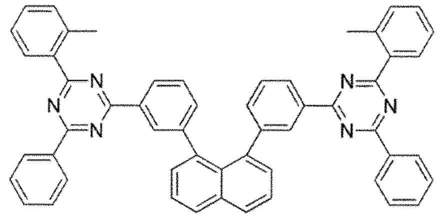 | 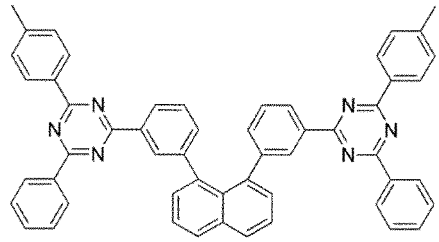 |
| Compound 1-b-13 | Compound 1-b-14 |
| 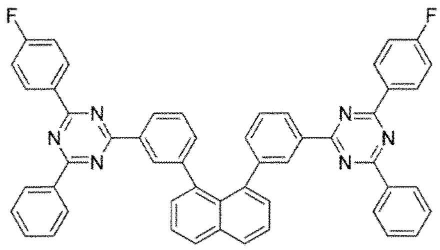 | 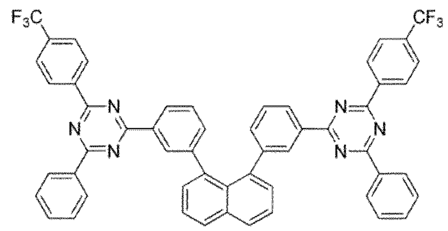 |
| Compound 1-b-15 | Compound 1-b-16 |
| 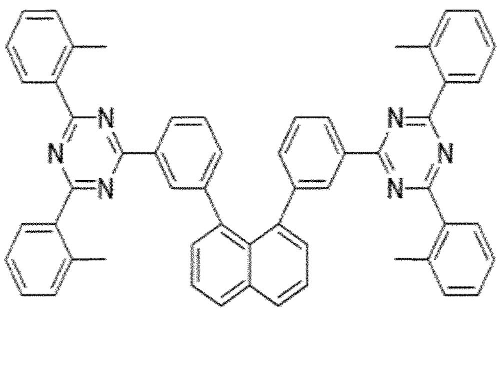 | 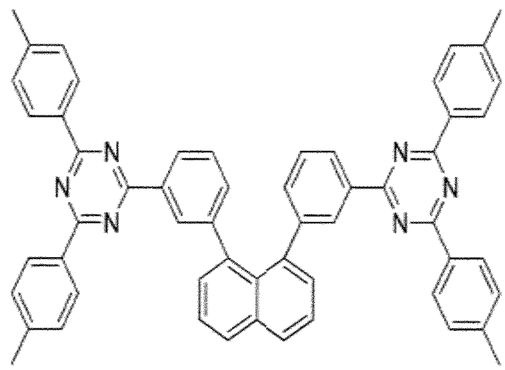 |

| | |
|---|---|
| Compound 2-b-1 | Compound 2-b-2 |
| 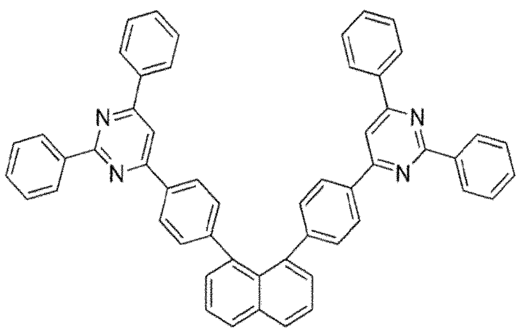 | 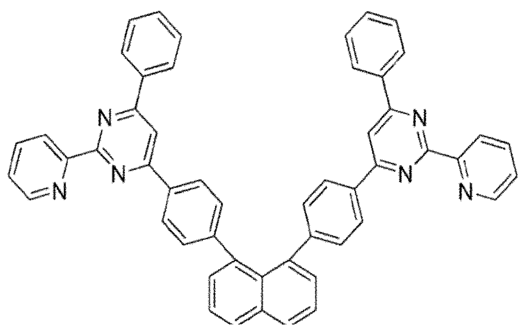 |
| Compound 2-b-3 | Compound 2-b-4 |

(fortgesetzt)

| | |
|---|---|
| 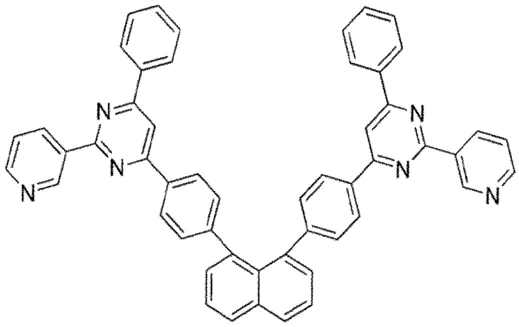 | 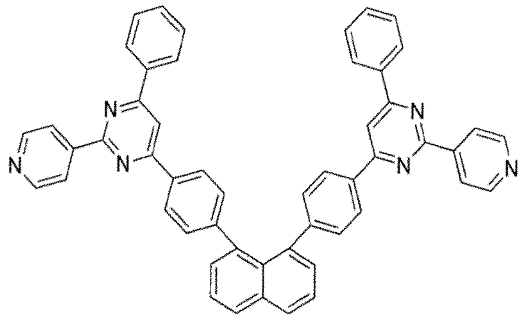 |
| Compound 2-b-5 | Compound 2-b-6 |
| 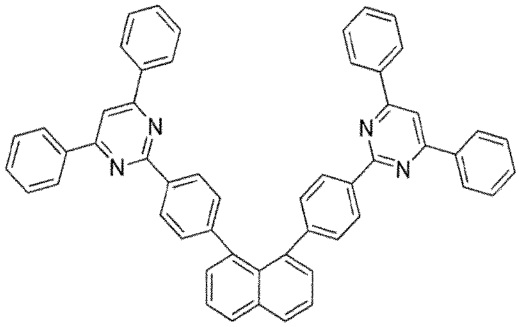 | 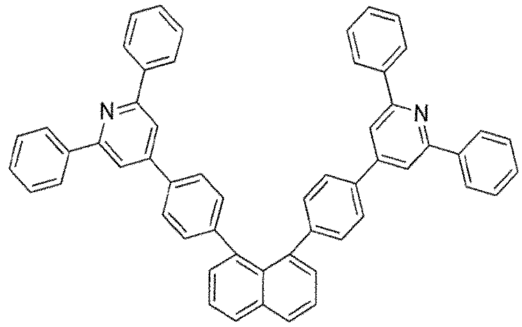 |
| Compound 2-b-7 | Compound 2-b-8 |
| 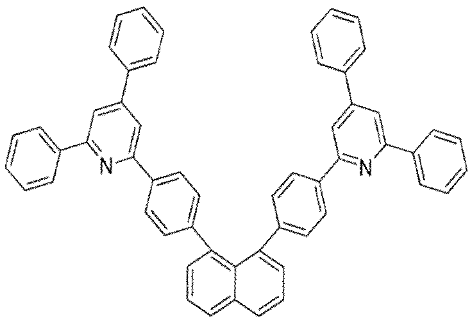 | 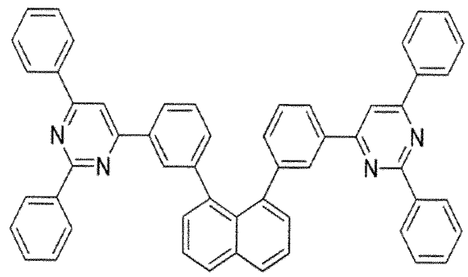 |
| Compound 2-b-9 | Compound 2-b-10 |
| 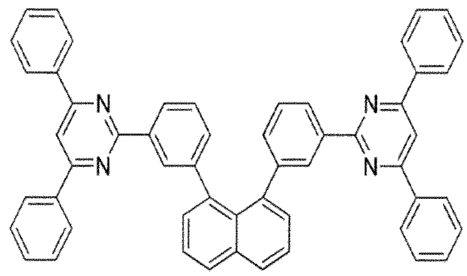 | 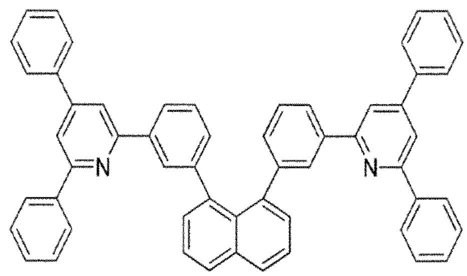 |

(fortgesetzt)

| Compound 2-b-11 | |
|---|---|
| 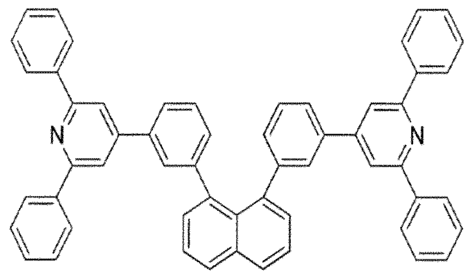 | |

2. Verbindung gemäß Anspruch 1, dadurch charakterisiert, dass sie die allgemeine Formel (2) hat

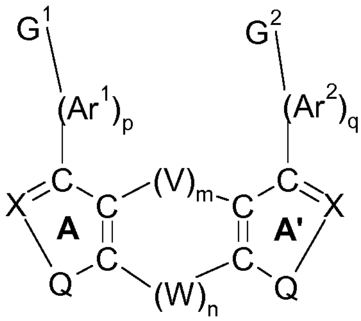

Formel (2)

wobei für die zusätzlich verwendeten Symbole gilt:

X ist gleich oder verscheiden bei jeden Auftreten N oder $CR^1$;
Q ist gleich oder verschieden bei jedem Auftreten X=X, S, O oder $NR^1$, bevorzugt X=X, S und O, ganz bevorzugt X=X und S und ganz besonders bevorzugt X=X.

3. Verbindung gemäß Anspruch 1 oder 2, dadurch charakterisiert, dass sie eine der folgenden allgemeinen Formeln (3) bis (11) hat

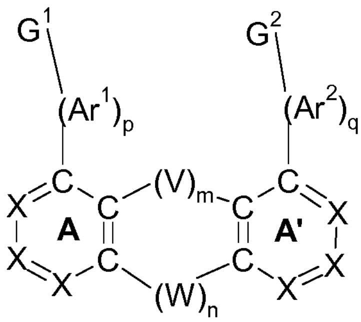

Formel (3)

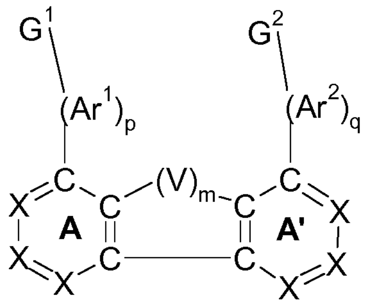

Formel (4)

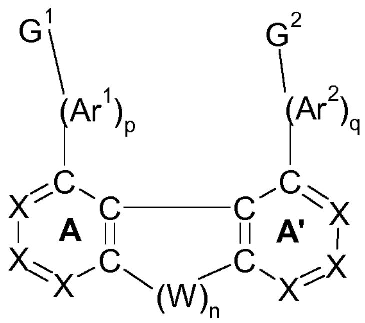

Formel (5)

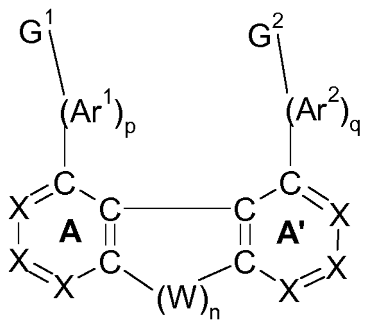

Formel (6)

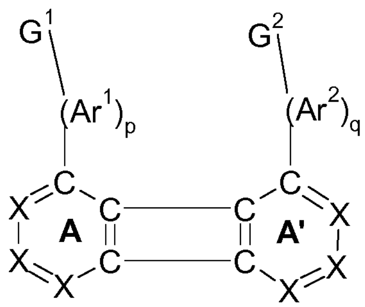

Formel (7)

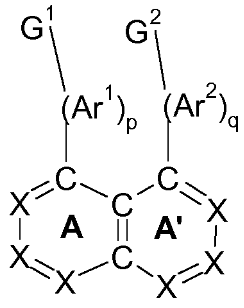

Formel (8)

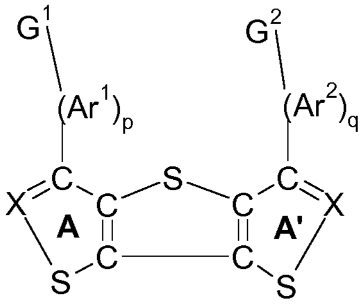

Formel (9)

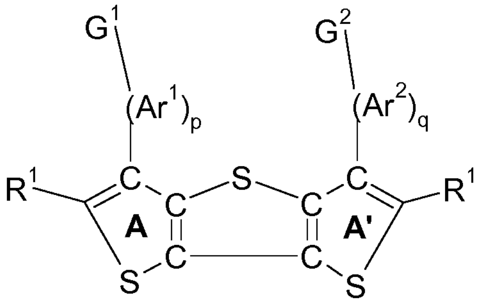

Formel (10)

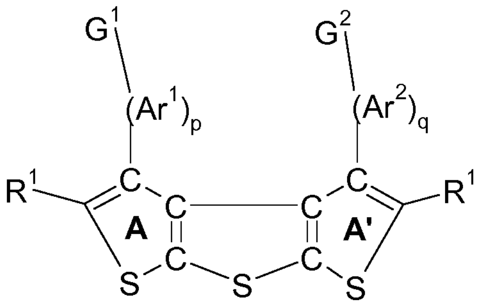

Formel (11)

4. Verbindung gemäß einem oder mehreren der Ansprüche 1 bis 3, dadurch charakterisiert, dass sie die allgemeine Formel (4) hat.

5. Verbindung gemäß einem oder mehreren der Ansprüche 1 bis 4, dadurch charakterisiert, dass sie die allgemeine Formel (4) hat und wobei X gleich $CR^1$ und m gleich 1 sind.

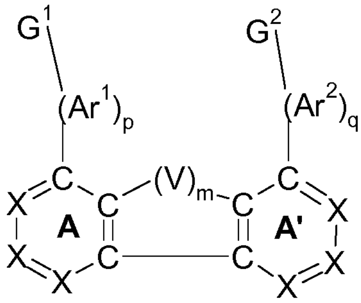

Formel (4)

6. Verbindung gemäß einem oder mehreren der Ansprüche 1 bis 5, **dadurch gekennzeichnet, dass** sie die allgemeine Formel (4) hat und X gleich $CR^1$, m gleich 1, p gleich 0 und q gleich 1 sind.

7. Verbindung gemäß einem oder mehreren der Ansprüche 1 bis 6, **dadurch gekennzeichnet, dass** sie die allgemeine Formel (13) hat,

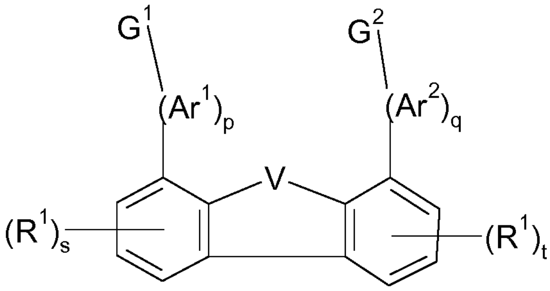

Formel (13)

wobei V gleich O oder S ist und wobei und wobei die aromatischen Ringe jeweils maximal einen Substituenten $R^1$ haben, d.h, s ist gleich 0 oder 1 und t ist gleich 0 oder 1, wobei s + t gleich 0, 1 oder 2 sein kann.

8. Verbindung gemäß einem oder mehreren der Ansprüche 1 bis 7, dadurch charakterisiert, dass sie die allgemeine Formel (15) hat

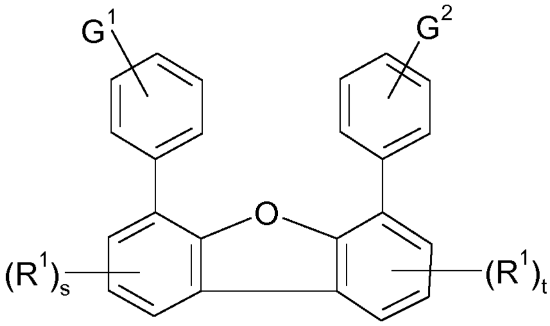

Formel (15)

9. Verbindung gemäß einem oder mehreren der Ansprüche 1 bis 8, dadurch charakterisiert, dass sie die allgemeine Formel (16) hat

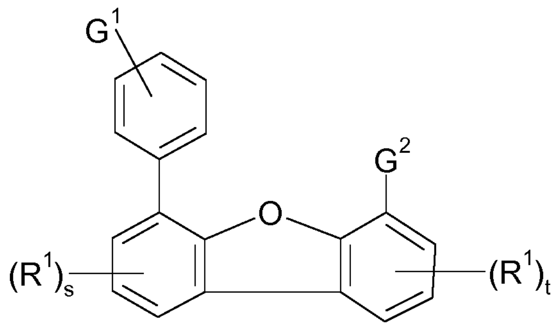

Formel (16)

**10.** Verbindung gemäß einem oder mehreren der Ansprüche 1 bis 9, dadurch charakterisiert, dass sie die allgemeine Formel (16a) hat

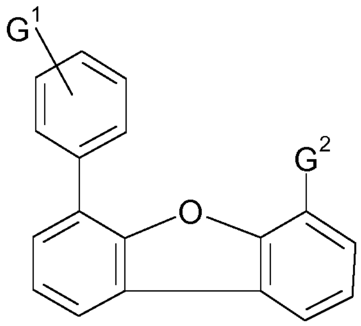

Formel (16a)

**11.** Verbindung gemäß einem oder mehreren der Ansprüche 1 bis 10, dadurch charakterisiert, dass eine der beiden Gruppen $G^1$ oder $G^2$ eine ETG und die andere eine LTG ist.

**12.** Zusammensetzung enthaltend wenigstens eine zusätzliche Verbindung der allgemeinen Formel (1)

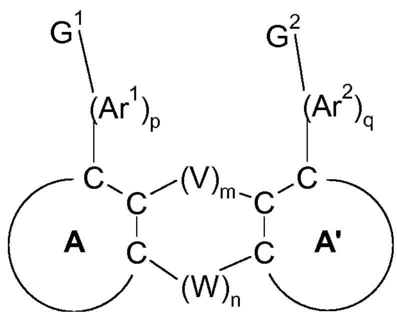

Formel (1)

wobei für die verwendeten Symbole und Indizes gilt:

A und A' sind gleich oder verschieden voneinander, ein aromatischer oder heteroaromatischer Ring mit 5 oder 6 Ringatomen, die mit einem oder mehreren Resten $R^1$, die unabhängig voneinander sein können, substituiert sein können;

$G^1$, $G^2$ sind bei jedem Auftreten gleich oder verschieden

eine organische elektronentransportierende Gruppe (ETG) aus der Gruppe der elektronenarmen heteroaromatischen Gruppen aus der Gruppe der Triazine, Pyrimidine und Pyrazine;

oder eine elektronenreiche organische Gruppe, die Löcher leitet (LTG), wobei die LTG ausgewählt ist aus der Gruppe der Arylamine,Triaryl-amine, der verbrückten Amine, bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole,

wobei wenigstens eine der beiden Gruppen $G^1$ oder $G^2$ eine elektronentransportierende Gruppe (ETG) sein muss und wobei die Gruppen $G^1$ und $G^2$ mit einem oder mehreren voneinander unabhängigen Resten $R^1$ substituiert sein können;

$Ar^1$ ist, wenn $G^1$ eine elektonentransportierende Gruppe ist, ein bivalenter aromatischer oder heteroaromatischer, bevorzugt ein aromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist, wenn $Ar^1$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist

oder, wenn $G^1$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^1$ eine Phenylen-, Biphenylen- oder Terphenylengruppe ist und eine Phenylengruppe besonders bevorzugt ist;

$Ar^2$ ist, wenn $G^2$ eine elektonentransportierende Gruppe ist, ein bivalenter aromatischer oder heteroaromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist wenn $Ar^2$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist

oder, wenn $G^2$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^2$ eine Phenylen-, Biphenylen- oder Terphenylengruppe ist und eine Phenylengruppe besonders bevorzugt ist;

V ist eine Einfachbindung, $NAr^3$, O, S, , wobei im Fall einer Einfachbindung die Kohlenstoffatome der Ringe A und A' direkt durch eine Einfachbindung miteinander verbunden sind, wobei eine Einfachbindung, $NAr^3$, O und S bevorzugt sind, wobei eine Einfachbindung, O und S ganz bevorzugt sind, wobei O und S ganz besonders bevorzugt sind, wobei O insbesondere bevorzugt ist;

W ist eine Einfachbindung, C=O, $C(R^1)_2$ oder $NR^1$, wobei im Fall einer Einfachbindung die Kohlenstoffatome der Ringe A und A' direkt durch eine Einfachbindung miteinander verbunden sind, wobei eine Einfachbindung, $C(R^1)_2$ oder $NR^1$ bevorzugt sind, wobei eine Einfachbindung oder $C(R^1)_2$ ganz bevorzugt sind;

wobei weiterhin bevorzugt ist, dass V eine Einfachbindung ist sofern W keine Einfachbindung ist bzw. dass W eine Einfachbindung ist sofern V keine Einfachbindung ist;

wobei weiterhin ganz bevorzugt ist, dass V eine Einfachbindung ist sofern W gleich O oder S ist bzw. dass W eine Einfachbindung ist sofern V gleich O oder S ist;

wobei weiterhin ganz besonders bevorzugt ist, dass V eine Einfachbindung ist sofern W gleich O ist bzw. dass W eine Einfachbindung ist sofern V gleich O ist;

m ist entweder 0 oder 1;

n ist entweder 0 oder 1,

wobei m = n gilt;

p ist entweder 0 oder 1;

q ist entweder 0 oder 1, wobei gilt, dass p + q entweder 1 oder 2 ist;

$Ar^3$ ist ein aromatischer Ring oder Ringsystem mit 6 bis 30 Ring-atomen, wobei der Ring oder das jeweils durch einen oder mehrere Reste $R^2$ substi-tuiert sein kann, die durch einen oder mehrere Reste $R^3$ substituiert sein können, wobei zwei oder mehr Reste $R^2$ miteinander einen Ringschluss bilden können;

$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^2$ substituiert sein kann, oder eine

Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C{=}CR^3$, C≡C, $Si(R^3)_2$, $Ge(R^3)_2$, $Sn(R^3)_2$, C=O, C=S, C=Se, $C{=}NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden;

mit der Maßgabe, dass maximal ein Substituent $R^1$ des Rings A und maximal ein Substituent $R^1$ des Rings A' eine aromatische oder heteroaromatische Gruppe mit 5 bis 30 Ringatomen enthält;

sowie wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Loch-blockiermaterialien

wobei die Zusammensetzung enthaltend Liq und die im Folgenden abgebildete Verbindung ausgenommen ist

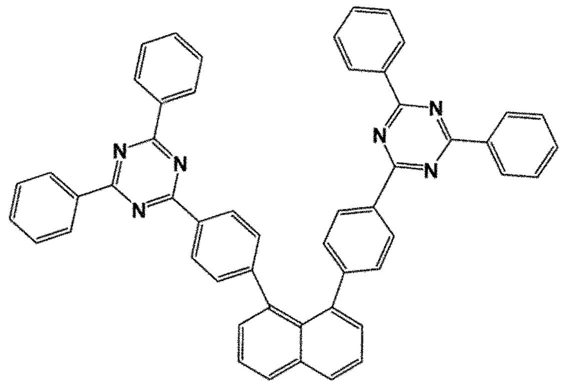

.

**13.** Zusammensetzung gemäß Anspruch 12, dadurch charakterisiert, dass die zusätzliche Verbindung ein Host- oder Matrixmaterial ist.

**14.** Zusammensetzung gemäß Anspruch 12 oder 13, dadurch charakterisiert, dass die zusätzliche Verbindung ein band gap von 2.5 eV oder mehr, bevorzugt 3.0 eV oder mehr, ganz bevorzugt von 3.5 eV oder mehr aufweist.

**15.** Formulierung enthaltend wenigstens eine Verbindung der allgemeinen Formel (1)

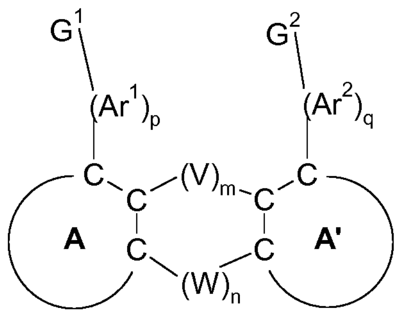

Formel (1)

wobei für die verwendeten Symbole und Indizes gilt:

A und A' sind gleich oder verschieden voneinander, ein aromatischer oder heteroaromatischer Ring mit 5 oder 6 Ringatomen, die mit einem oder mehreren Resten $R^1$, die unabhängig voneinander sein können, substituiert sein können;

$G^1$, $G^2$ sind bei jedem Auftreten gleich oder verschieden eine organische elektronentransportierende Gruppe (ETG) aus der Gruppe der elektronenarmen heteroaromatischen Gruppen aus der Gruppe der Triazine, Pyrimidine und Pyrazine;

oder eine elektronenreiche organische Gruppe, die Löcher leitet (LTG), wobei die LTG ausgewählt ist aus der Gruppe der Arylamine,Triaryl-amine, der verbrückten Amine, bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole,
wobei wenigstens eine der beiden Gruppen $G^1$ oder $G^2$ eine elektronentransportierende Gruppe (ETG) sein muss und wobei die Gruppen $G^1$ und $G^2$ mit einem oder mehreren voneinander unabhängigen Resten $R^1$ substituiert sein können;
$Ar^1$ ist, wenn $G^1$ eine elektonentransportierende Gruppe ist, ein bivalenter aromatischer oder heteroaromatischer, bevorzugt ein aromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist, wenn $Ar^1$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist
oder, wenn $G^1$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^1$ eine Phenylen-, Biphenylen- oder Terphenylengruppe ist und eine Phenylengruppe besonders bevorzugt ist;
$Ar^2$ ist, wenn $G^2$ eine elektonentransportierende Gruppe ist, ein bivalenter aromatischer oder heteroaromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist wenn $Ar^2$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist
oder, wenn $G^2$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^2$ eine Phenylen-, Biphenylen- oder Terphenylengruppe ist und eine Phenylengruppe besonders bevorzugt ist;
V ist eine Einfachbindung, $NAr^3$, O, S, , wobei im Fall einer Einfachbindung die Kohlenstoffatome der Ringe A und A' direkt durch eine Einfachbindung miteinander verbunden sind, wobei eine Einfachbindung, $NAr^3$, O und S bevorzugt sind, wobei eine Einfachbindung, O und S ganz bevorzugt sind, wobei O und S ganz besonders bevorzugt sind, wobei O insbesondere bevorzugt ist;
W ist eine Einfachbindung, C=O, $C(R^1)_2$ oder $NR^1$, wobei im Fall einer Einfachbindung die Kohlenstoffatome der Ringe A und A' direkt durch eine Einfachbindung miteinander verbunden sind, wobei eine Einfachbindung, $C(R^1)_2$ oder $NR^1$ bevorzugt sind, wobei eine Einfachbindung oder $C(R^1)_2$ ganz bevorzugt sind;
wobei weiterhin bevorzugt ist, dass V eine Einfachbindung ist sofern W keine Einfachbindung ist bzw. dass W

eine Einfachbindung ist sofern V keine Einfachbindung ist;
wobei weiterhin ganz bevorzugt ist, dass V eine Einfachbindung ist sofern W gleich O oder S ist bzw. dass W eine Einfachbindung ist sofern V gleich O oder S ist;
wobei weiterhin ganz besonders bevorzugt ist, dass V eine Einfachbindung ist sofern W gleich O ist bzw. dass W eine Einfachbindung ist sofern V gleich O ist;
m ist entweder 0 oder 1;
n ist entweder 0 oder 1,
wobei m = n gilt;
p ist entweder 0 oder 1;
q ist entweder 0 oder 1, wobei gilt, dass p + q entweder 1 oder 2 ist;
$Ar^3$ ist ein aromatischer Ring oder Ringsystem mit 6 bis 30 Ring-atomen, wobei der Ring oder das jeweils durch einen oder mehrere Reste $R^2$ substi-tuiert sein kann, die durch einen oder mehrere Reste $R^3$ substituiert sein können, wobei zwei oder mehr Reste $R^2$ miteinander einen Ringschluss bilden können;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;
$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;
$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden;

mit der Maßgabe, dass maximal ein Substituent $R^1$ des Rings A und maximal ein Substituent $R^1$ des Rings A' eine aromatische oder heteroaromatische Gruppe mit 5 bis 30 Ringatomen enthält;
oder wenigstens einer Zusammensetzung gemäß einem oder mehreren der Ansprüche 12 bis 14,
sowie wenigstens ein Lösungsmittel;
wobei die die Formulierung enthaltend Tetrahydrofuran und folgende Verbindung ausgenommen ist

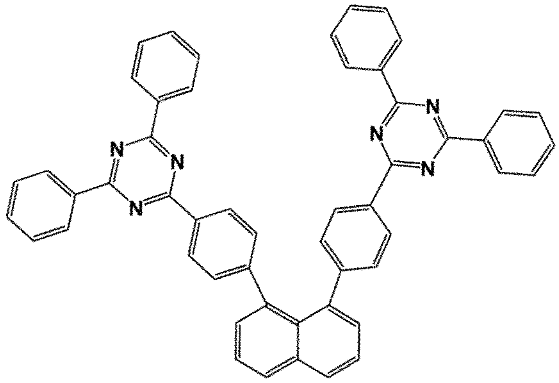

.

**16.** Verwendung wenigstens einer Verbindung gemäß einem oder mehreren der Ansprüche 1 bis 11 oder wenigstens einer Zusammensetzung gemäß einem oder mehreren der Ansprüche 12 bis 14 in einer organischen Elektrolumineszenzvorrichtung, bevorzugt in einer organischen lichtemittierenden Diode (OLED) oder organischen lichtemittierenden elektrochemischen Zelle (OLEC, LEEC, LEC), ganz besonders bevorzugt in einer OLED, bevorzugt in einer Elektronentransportschicht (ETL) und in einer Lochblockierschicht (HBL).

**17.** Verwendung wenigstens einer Verbindung der allgemeinen Formel (1)

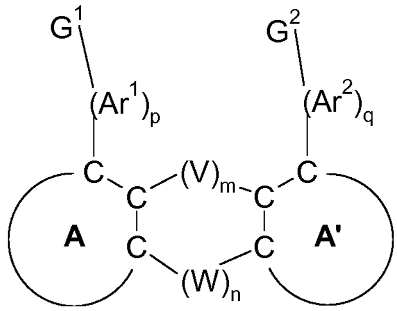

Formel (1)

wobei für die verwendeten Symbole und Indizes gilt:

A und A' sind gleich oder verschieden voneinander, ein aromatischer oder heteroaromatischer Ring mit 5 oder 6 Ringatomen, die mit einem oder mehreren Resten $R^1$, die unabhängig voneinander sein können, substituiert sein können;

$G^1$, $G^2$ sind bei jedem Auftreten gleich oder verschieden eine organische elektronentransportierende Gruppe (ETG) aus der Gruppe der elektronenarmen heteroaromatischen

Gruppen aus der Gruppe der Triazine, Pyrimidine und Pyrazine;
oder eine elektronenreiche organische Gruppe, die Löcher leitet (LTG), wobei die LTG ausgewählt ist aus der Gruppe der Arylamine,Triaryl-amine, der verbrückten Amine, bevorzugte verbrückte Amine sind dabei Dihydroacridine, Dihydrophenazine, Phenoxazione und Phenothiazine, Carbazole, verbrückten Carbazole, Biscarbazole, Indenocarbazole und Indolocarbazole,
wobei wenigstens eine der beiden Gruppen $G^1$ oder $G^2$ eine elektronentransportierende Gruppe (ETG) sein muss und wobei die Gruppen $G^1$ und $G^2$ mit einem oder mehreren voneinander unabhängigen Resten $R^1$ substituiert sein können;
$Ar^1$ ist, wenn $G^1$ eine elektonentransportierende Gruppe ist, ein bivalenter aromatischer oder heteroaromatischer, bevorzugt ein aromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist, wenn $Ar^1$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist

oder, wenn $G^1$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^1$ eine Phenylen-, Biphenylen- oder Terphenylengruppe ist und eine Phenylengruppe besonders bevorzugt ist;

$Ar^2$ ist, wenn $G^2$ eine elektonentransportierende Gruppe ist, ein bivalenter aromatischer oder heteroaromatischer Ring oder Ringsystem mit 5 bis 60 Ringatomen, wobei der Ring oder das Ringsystem weder mit dem Ringsystem enthaltend die Ringe A und A' noch mit der ETG verbrückt ist, wobei bevorzugt ist wenn $Ar^2$ eine Pyridylen-, Pyrimidylen-, Phenylen-, Biphenylen- oder Fluoren-, Spiro-, Terphenylen-, Thiophen- oder Furangruppe ist, wobei eine Phenylen-, Biphenylen- oder Terphenylengruppe besonders und eine Phenylengruppe ganz besonders bevorzugt ist

oder, wenn $G^2$ eine lochtransportierende Gruppe ist, ein aromatischer Ring oder Ringsystem mit 5 bis 60 Ringatomen, wobei der Ring oder das Ringsystem weder mit dem Ringsystem enthaltend die Ringe A und A' noch mit der LTG verbrückt ist, wobei bevorzugt ist wenn $Ar^2$ eine Phenylen-, Biphenylen- oder Terphenylengruppe ist und eine Phenylengruppe besonders bevorzugt ist;

V ist eine Einfachbindung, $NAr^3$, O, S, , wobei im Fall einer Einfachbindung die Kohlenstoffatome der Ringe A und A' direkt durch eine Einfachbindung miteinander verbunden sind, wobei eine Einfachbindung, $NAr^3$, O und S bevorzugt sind, wobei eine Einfachbindung, O und S ganz bevorzugt sind, wobei O und S ganz besonders bevorzugt sind, wobei O insbesondere bevorzugt ist;

W ist eine Einfachbindung, C=O, $C(R^1)_2$ oder $NR^1$, wobei im Fall einer Einfachbindung die Kohlenstoffatome der Ringe A und A' direkt durch eine Einfachbindung miteinander verbunden sind, wobei eine Einfachbindung, $C(R^1)_2$ oder $NR^1$ bevorzugt sind, wobei eine Einfachbindung oder $C(R^1)_2$ ganz bevorzugt sind;

wobei weiterhin bevorzugt ist, dass V eine Einfachbindung ist sofern W keine Einfachbindung ist bzw. dass W eine Einfachbindung ist sofern V keine Einfachbindung ist;

wobei weiterhin ganz bevorzugt ist, dass V eine Einfachbindung ist sofern W gleich O oder S ist bzw. dass W eine Einfachbindung ist sofern V gleich O oder S ist;

wobei weiterhin ganz besonders bevorzugt ist, dass V eine Einfachbindung ist sofern W gleich O ist bzw. dass W eine Einfachbindung ist sofern V gleich O ist;

m ist entweder 0 oder 1;

n ist entweder 0 oder 1,

wobei m = n gilt;

p ist entweder 0 oder 1;

q ist entweder 0 oder 1, wobei gilt, dass p + q entweder 1 oder 2 ist;

$Ar^3$ ist ein aromatischer Ring oder Ringsystem mit 6 bis 30 Ring-atomen, wobei der Ring oder das jeweils durch einen oder mehrere Reste $R^2$ substi-tuiert sein kann, die durch einen oder mehrere Reste $R^3$ substituiert sein können, wobei zwei oder mehr Reste $R^2$ miteinander einen Ringschluss bilden können;

$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^3$ substituiert sein kann, oder eine

Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden;

mit der Maßgabe, dass maximal ein Substituent $R^1$ des Rings A und maximal ein Substituent $R^1$ des Rings A' eine aromatische oder heteroaromatische Gruppe mit 5 bis 30 Ringatomen enthält;
in einer organischen Elektrolumineszenzvorrichtung in einer Emissionsschicht (EML).

**18.** Organische Elektrolumineszenzvorrichtung enthaltend wenigstens eine Verbindung gemäß einem oder mehreren der Ansprüche 1 bis 11 oder wenigstens eine Zusammensetzung gemäß einem oder mehreren der Ansprüche 12 bis 14, bevorzugt in einer Elektronentransportschicht (ETL) und in einer Lochblockierschicht (HBL), ganz bevorzugt in einer ETL.

**19.** Organische Elektrolumineszenzvorrichtung enthaltend in der Emissionsschicht wenigstens eine Verbindung der allgemeinen Formel (1)

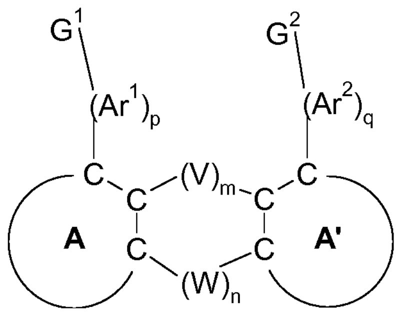

Formel (1)

wobei für die verwendeten Symbole und Indizes die in Anspruch 17 angegebenen Definitionen gelten, oder wenigstens eine Zusammensetzung gemäß einem oder mehreren der Ansprüche 12 bis 14.

**20.** Organischen Elektrolumineszenzvorrichtung gemäß einem oder mehreren der Ansprüche 18 bis 19, **dadurch gekennzeichnet, dass** sie ausgewählt ist aus der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

**21.** Verfahren zur Herstellung einer organischen Elektrolumineszenzvorrichtung gemäß einem oder mehreren der Ansprüche 18 bis 20, **dadurch gekennzeichnet, dass** mindestens eine organische Schicht durch Gasphasenabscheidung oder aus Lösung aufgebracht wird.

## Claims

**1.** Compound of the general formula (1)

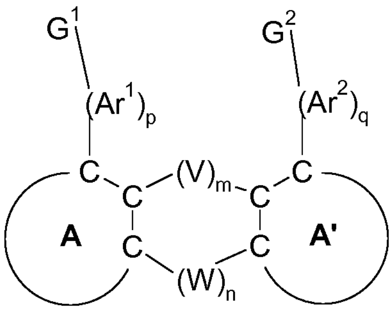

formula (1)

where the following applies to the symbols and indices used:

A and A' are, identically or differently from one another, an aromatic or heteroaromatic ring having 5 or 6 ring atoms, which may be substituted by one or more radicals $R^1$, which may be independent of one another;

$G^1$, $G^2$ are on each occurrence, identically or differently, an organic electron-transporting group (ETG) from the group of the electron-deficient heteroaromatic groups from the group of the triazines, pyrimidines and pyrazines; or an electron-rich organic hole-transporting group (HTG), where the HTG is selected from the group of the arylamines, triarylamines, bridged amines, where preferred bridged amines are dihydroacridines, dihydrophenazines, phenoxazines and phenothiazines, carbazoles, bridged carbazoles, biscarbazoles, indenocarbazoles and indolocarbazoles, where at least one of the two groups $G^1$ and $G^2$ must be an electron-transporting group (ETG) and where the groups $G^1$ and $G^2$ may be substituted by one or more radicals $R^1$, which are independent of one another;

$Ar^1$, if $G^1$ is an electron-transporting group, is a divalent aromatic or heteroaromatic, preferably aromatic, ring or ring system having 5 to 60 ring atoms, where the ring or ring system is bridged neither to the ring system containing the rings A and A' nor to the ETG, where it is preferred if $Ar^1$ is a pyridylene, pyrimidylene, phenylene, biphenylene or fluorene, spiro, ter-phenylene, thiophene or furan group, where a phenylene, bi-phenylene or terphenylene group is particularly preferred and a phenylene group is very particularly preferred,

or, if $G^1$ is a hole-transporting group, is an aromatic ring or ring system having 5 to 60 ring atoms, where the ring or ring system is bridged neither to the ring system containing the rings A and A' nor to the HTG, where it is preferred if $Ar^1$ is a phenylene, biphenylene or terphenylene group and a phenylene group is particularly preferred;

$Ar^2$, if $G^2$ is an electron-transporting group, is a divalent aromatic or heteroaromatic ring or ring system having 5 to 60 ring atoms, where the ring or ring system is bridged neither to the ring system containing the rings A and A' nor to the ETG, where it is preferred if $Ar^2$ is a pyridylene, pyrimidylene, phenylene, biphenylene or fluorene, spiro, terphenylene, thiophene or furan group, where a phenylene, biphenylene or terphenylene group is particularly preferred and a phenylene group is very particularly preferred,

or, if $G^2$ is a hole-transporting group, is an aromatic ring or ring system having 5 to 60 ring atoms, where the ring or ring system is bridged neither to the ring system containing the rings A and A' nor to the HTG, where it is preferred if $Ar^2$ is a phenylene, biphenylene or terphenylene group and a phenylene group is particularly preferred;

V is a single bond, $NAr^3$, O or S, where, in the case of a single bond, the carbon atoms of the rings A and A' are connected directly to one another by a single bond, where a single bond, $NAr^3$, O and S are preferred, where a single bond, O and S are very preferred, where O and S are very particularly preferred, where O is especially preferred;

W is a single bond, C=O, $C(R^1)_2$ or $NR^1$, where, in the case of a single bond, the carbon atoms of the rings A and A' are connected directly to one another by a single bond, where a single bond, $C(R^1)_2$ and $NR^1$ are preferred, where a single bond or $C(R^1)_2$ are very preferred;

where it is furthermore preferred that V is a single bond if W is not a single bond or that W is a single bond if V is not a single bond;

where it is furthermore very preferred that V is a single bond if W is equal to O or S or that W is a single bond if V is equal to O or S;

where it is furthermore very particularly preferred that V is a single bond if W is equal to O or that W is a single bond if V is equal to O;

m is either 0 or 1;

n is either 0 or 1,

where m = n;
p is either 0 or 1;
q is either 0 or 1, where p + q is either 1 or 2;
$Ar^3$ is an aromatic ring or ring system having 6 to 30 ring atoms, where the ring or ring system may in each case be substituted by one or more radicals $R^2$, which may be substituted by one or more radicals $R^3$, where two or more radicals $R^2$ may form a ring closure with one another;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups or a crosslinkable group Q;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be sub-stituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroaryl-amino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another;
$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic aliphatic or aromatic ring system with one another;

with the proviso that a maximum of one substituent $R^1$ of ring A and a maximum of one substituent $R^1$ of ring A' contains an aromatic or heteroaromatic group having 5 to 30 ring atoms;
where the following compounds are excluded from the scope of protection

| Compound 1-b-1 | Compound 1-b-2 |
| --- | --- |
| 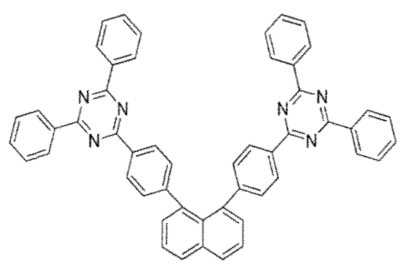 | 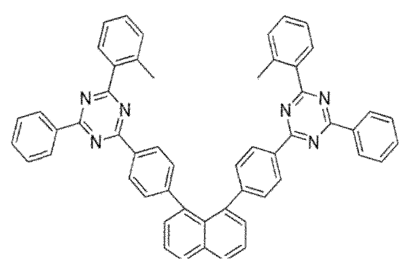 |
| Compound 1-b-3 | Compound 1-b-4 |

(continued)

| | |
|---|---|
| 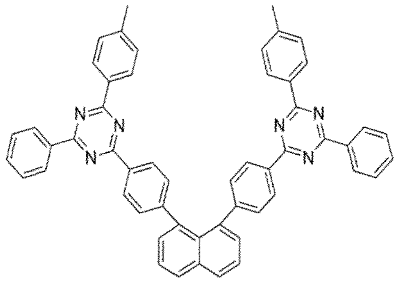 | 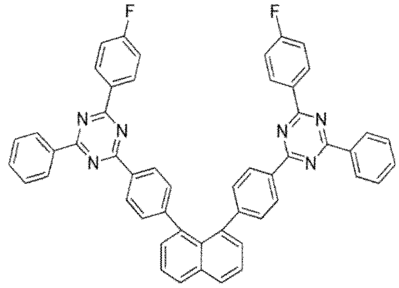 |
| Compound 1-b-5 | Compound 1-b-6 |
| 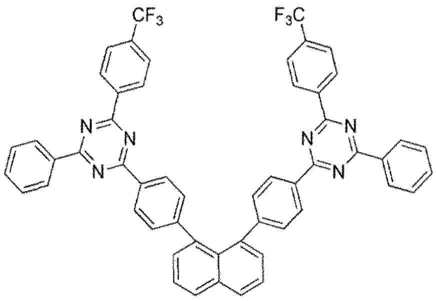 | 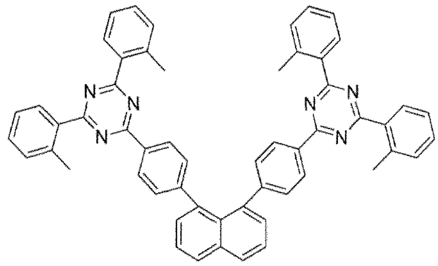 |
| Compound 1-b-7 | Compound 1-b-8 |
| 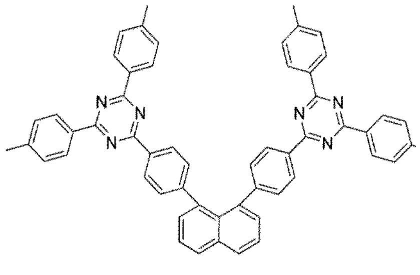 | 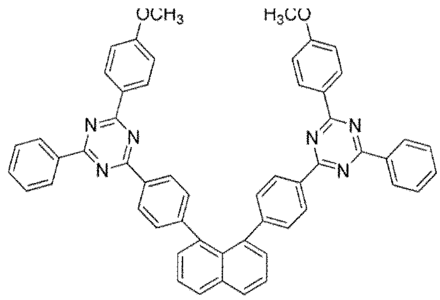 |
| Compound 1-b-9 | Compound 1-b-10 |
| 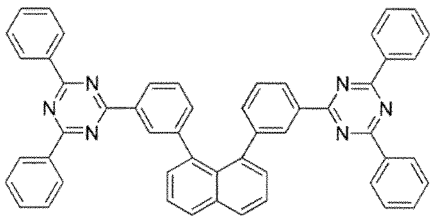 | 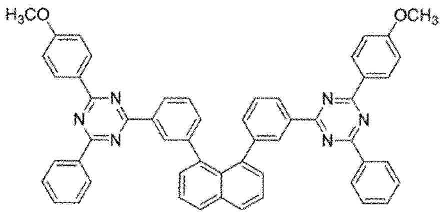 |
| Compound 1-b-11 | Compound 1-b-12 |
| 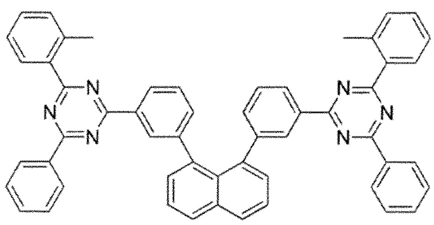 | 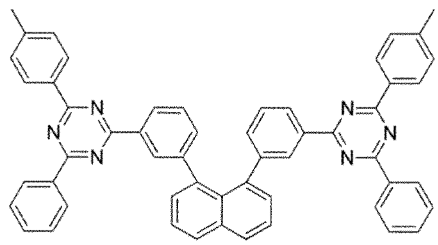 |
| Compound 1-b-13 | Compound 1-b-14 |

(continued)

| | |
|---|---|
| 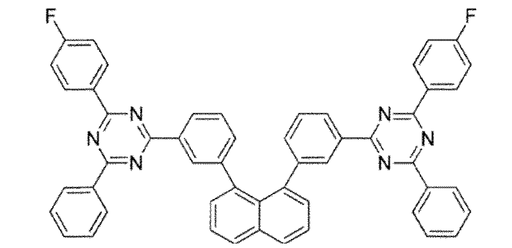 | 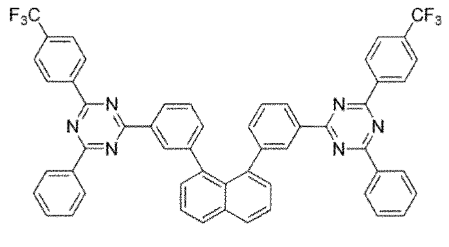 |
| Compound 1-b-15 | Compound 1-b-16 |
| 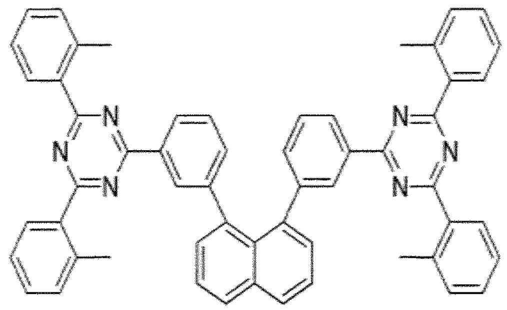 | 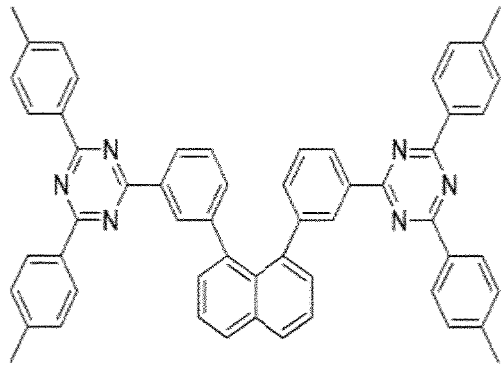 |
| Compound 2-b-1 | Compound 2-b-2 |
| 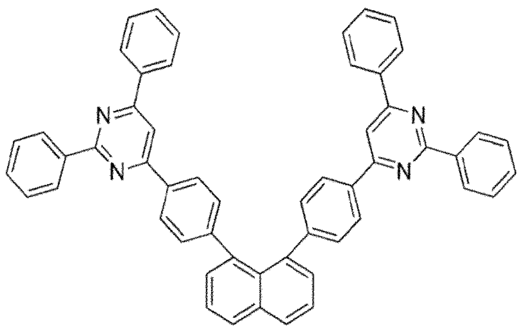 | 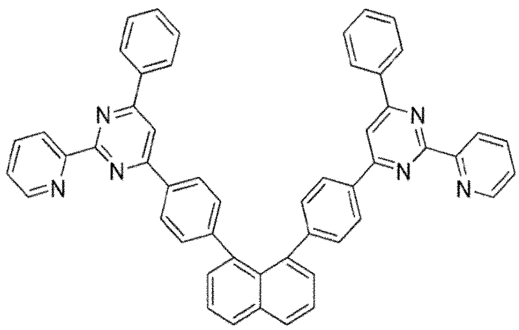 |
| Compound 2-b-3 | Compound 2-b-4 |
| 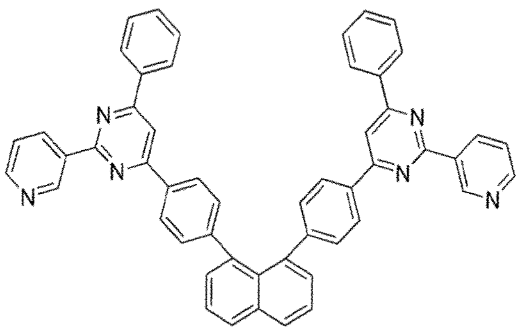 | 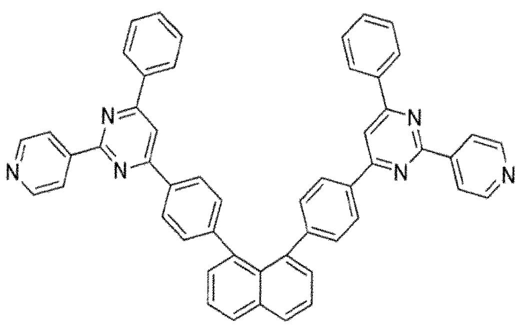 |
| Compound 2-b-5 | Compound 2-b-6 |

(continued)

| | |
|---|---|
| 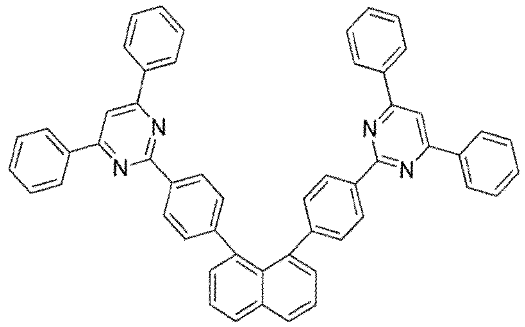 | 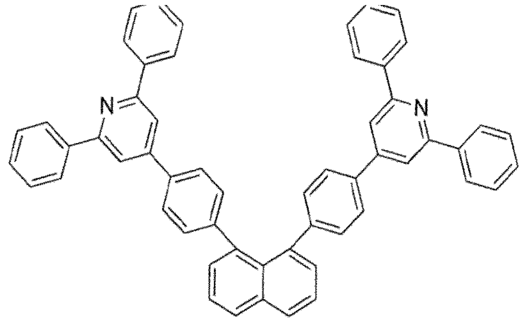 |
| Compound 2-b-7 | Compound 2-b-8 |
| 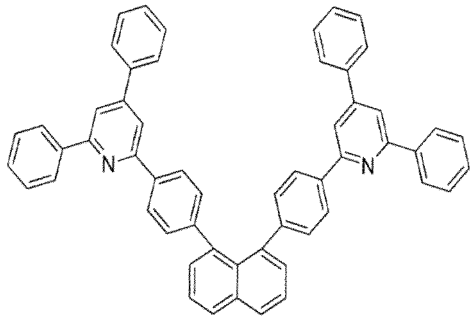 | 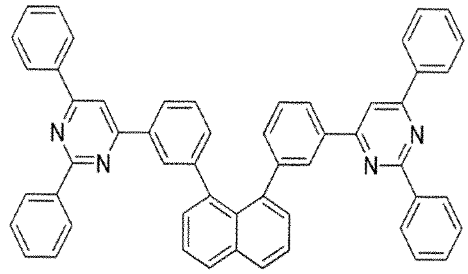 |
| Compound 2-b-9 | Compound 2-b-10 |
| 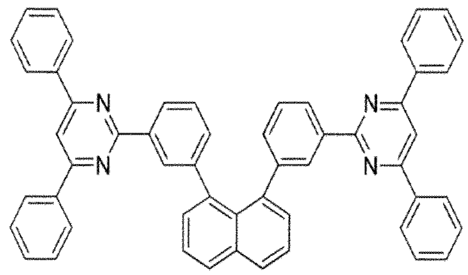 | 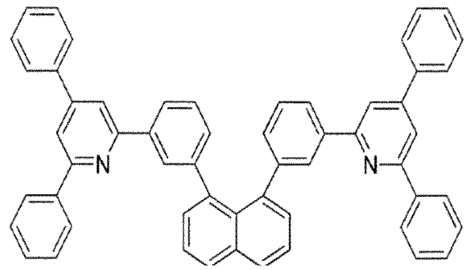 |
| Compound 2-b-11 | |
| 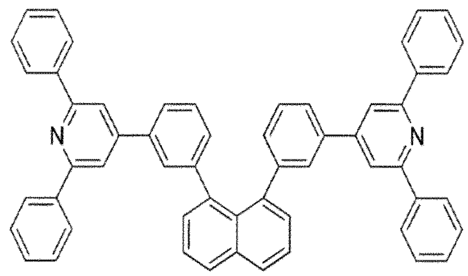 | |

**2.** Compound according to Claim 1, **characterised in that** it has the general formula (2)

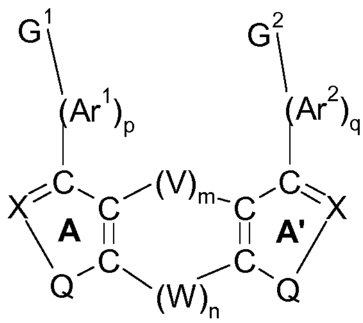

formula (2)

where the following applies to the symbols additionally used:

X is, identically or differently on each occurrence, N or $CR^1$;
Q is, identically or differently on each occurrence, X=X, S, O or $NR^1$, preferably X=X, S or O, very preferably X=X or S and very particularly preferably X=X.

**3.** Compound according to Claim 1 or 2, **characterised in that** it has one of the following general formulae (3) to (11)

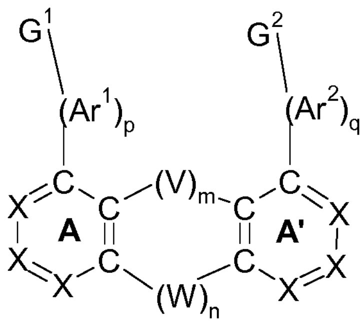

formula (3)

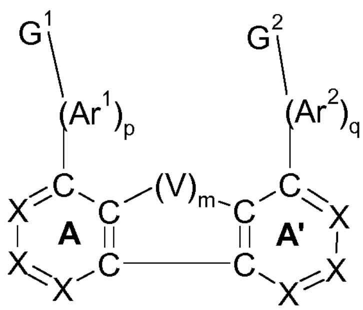

formula (4)

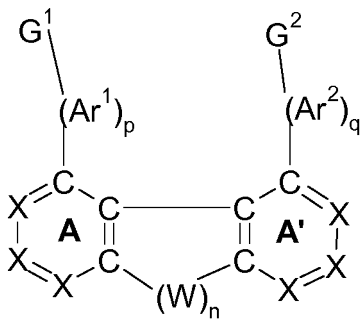

formula (5)

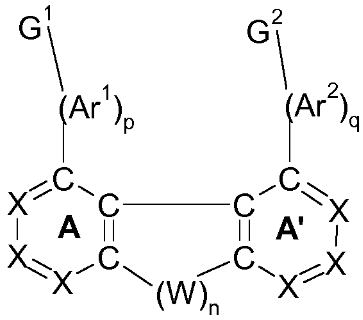

formula (6)

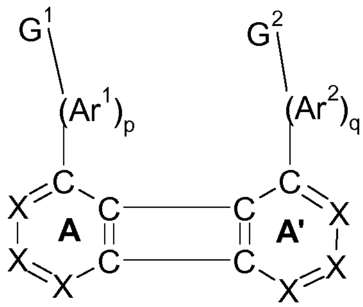

formula (7)

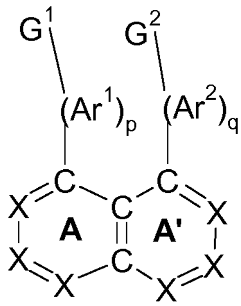

formula (8)

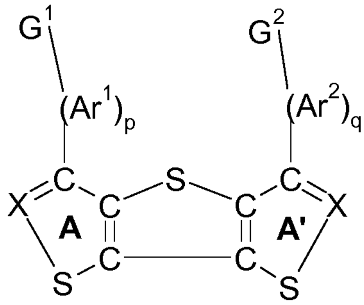

formula (9)

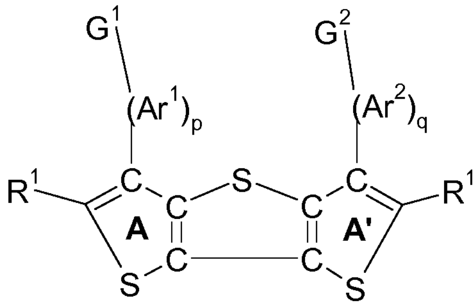

formula (10)

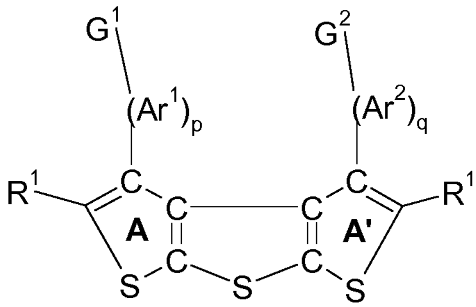

formula (11)

**4.** Compound according to one or more of Claims 1 to 3, **characterised in that** it has the general formula (4).

**5.** Compound according to one or more of Claims 1 to 4, **characterised in that** it has the general formula (4) and where X is equal to $CR^1$ and m is equal to 1.

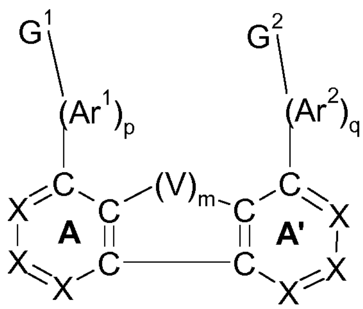

formula (4)

**6.** Compound according to one or more of Claims 1 to 5, **characterised in that** it has the general formula (4) and X

is equal to $CR^1$, m is equal to 1, p is equal to 0 and q is equal to 1.

**7.** Compound according to one or more of Claims 1 to 6, **characterised in that** it has the general formula (13),

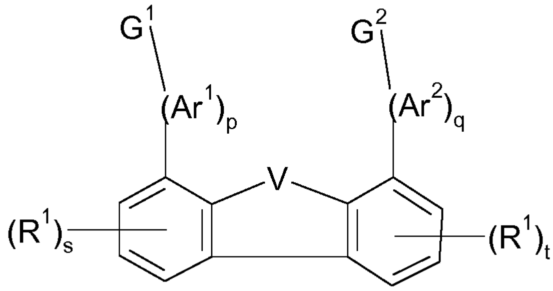

formula (13)

where V is equal to O or S and where the aromatic rings in each case have a maximum of one substituent $R^1$, i.e. s is equal to 0 or 1 and t is equal to 0 or 1, where s + t can be equal to 0, 1 or 2.

**8.** Compound according to one or more of Claims 1 to 7, **characterised in that** it has the general formula (15)

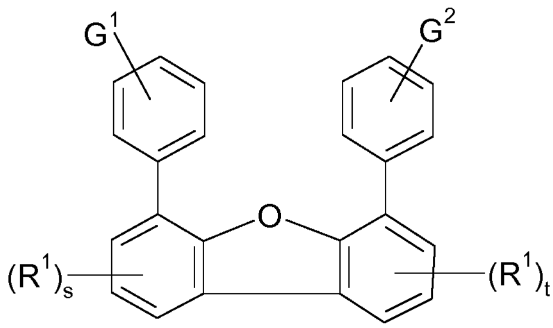

formula (15)

**9.** Compound according to one or more of Claims 1 to 8, **characterised in that** it has the general formula (16)

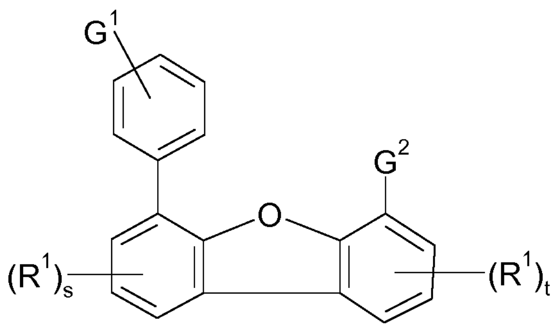

formula (16)

**10.** Compound according to one or more of Claims 1 to 9, **characterised in that** it has the general formula (16a)

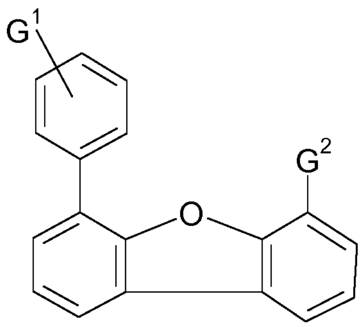

formula (16a)

**11.** Compound according to one or more of Claims 1 to 10, **characterised in that** one of the two groups $G^1$ and $G^2$ is an ETG and the other is an HTG.

**12.** Composition comprising at least one additional compound of the general formula (1)

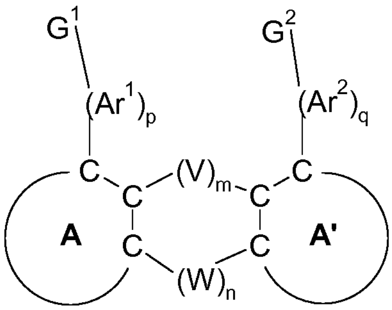

formula (1)

where the following applies to the symbols and indices used:

A and A' are, identically or differently from one another, an aromatic or heteroaromatic ring having 5 or 6 ring atoms, which may be substituted by one or more radicals $R^1$, which may be independent of one another;

$G^1$, $G^2$ are on each occurrence, identically or differently,

an organic electron-transporting group (ETG) from the group of the electron-deficient heteroaromatic groups from the group of the triazines, pyrimidines and pyrazines;

or an electron-rich organic hole-transporting group (HTG), where the HTG is selected from the group of the arylamines, triarylamines, bridged amines, where preferred bridged amines are dihydroacridines, dihydrophenazines, phenoxazines and phenothiazines, carbazoles, bridged carbazoles, biscarbazoles, indenocarbazoles and indolocarbazoles,

where at least one of the two groups $G^1$ and $G^2$ must be an electron-transporting group (ETG) and where the groups $G^1$ and $G^2$ may be substituted by one or more radicals $R^1$, which are independent of one another;

$Ar^1$, if $G^1$ is an electron-transporting group, is a divalent aromatic or heteroaromatic, preferably aromatic, ring or ring system having 5 to 60 ring atoms, where the ring or ring system is bridged neither to the ring system containing the rings A and A' nor to the ETG, where it is preferred if $Ar^1$ is a pyridylene, pyrimidylene, phenylene, biphenylene or fluorene, spiro, ter-phenylene, thiophene or furan group, where a phenylene, biphenylene or terphenylene group is particularly preferred and a phenylene group is very particularly preferred,

or, if $G^1$ is a hole-transporting group, is an aromatic ring or ring system having 5 to 60 ring atoms, where the ring or ring system is bridged neither to the ring system containing the rings A and A' nor to the HTG, where it is preferred if $Ar^1$ is a phenylene, biphenylene or terphenylene group and a phenylene group is particularly preferred;

$Ar^2$, if $G^2$ is an electron-transporting group, is a divalent aromatic or heteroaromatic ring or ring system having 5 to 60 ring atoms, where the ring or ring system is bridged neither to the ring system containing the rings A and A' nor to the ETG, where it is preferred if $Ar^2$ is a pyridylene, pyrimidylene, phenylene, biphenylene or fluorene, spiro, terphenylene, thiophene or furan group, where a phenylene, biphenylene or terphenylene group

is particularly preferred and a phenylene group is very particularly preferred,

or, if $G^2$ is a hole-transporting group, is an aromatic ring or ring system having 5 to 60 ring atoms, where the ring or ring system is bridged neither to the ring system containing the rings A and A' nor to the HTG, where it is preferred if $Ar^2$ is a phenylene, biphenylene or terphenylene group and a phenylene group is particularly preferred;

V is a single bond, $NAr^3$, O or S, where, in the case of a single bond, the carbon atoms of the rings A and A' are connected directly to one another by a single bond, where a single bond, $NAr^3$, O and S are preferred, where a single bond, O and S are very preferred, where O and S are very particularly preferred, where O is especially preferred;

W is a single bond, C=O, $C(R^1)_2$ or $NR^1$, where, in the case of a single bond, the carbon atoms of the rings A and A' are connected directly to one another by a single bond, where a single bond, $C(R^1)_2$ or $NR^1$ are preferred, where a single bond or $C(R^1)_2$ are very preferred;

where it is furthermore preferred that V is a single bond if W is not a single bond or that W is a single bond if V is not a single bond;

where it is furthermore very preferred that V is a single bond if W is equal to O or S or that W is a single bond if V is equal to O or S;

where it is furthermore very particularly preferred that V is a single bond if W is equal to O or that W is a single bond if V is equal to O;

m is either 0 or 1;

n is either 0 or 1,

where m = n;

p is either 0 or 1;

q is either 0 or 1, where p + q is either 1 or 2;

$Ar^3$ is an aromatic ring or ring system having 6 to 30 ring atoms, where the ring or ring system may in each case be substituted by one or more radicals $R^2$, which may be substituted by one or more radicals $R^3$, where two or more radicals $R^2$ may form a ring closure with one another;

$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylhetero-arylamino group having 10 to 40 aromatic ring atoms, which may be substituted by one or more radicals $R^2$, or a combination of two or more of these groups or a crosslinkable group Q;

$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroaryl-amino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another;

$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic aliphatic or aromatic ring system with one another;

with the proviso that a maximum of one substituent $R^1$ of ring A and a maximum of one substituent $R^1$ of ring A' contains an aromatic or heteroaromatic group having 5 to 30 ring atoms;

and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials,
where the composition comprising Liq and the compound depicted below is excluded

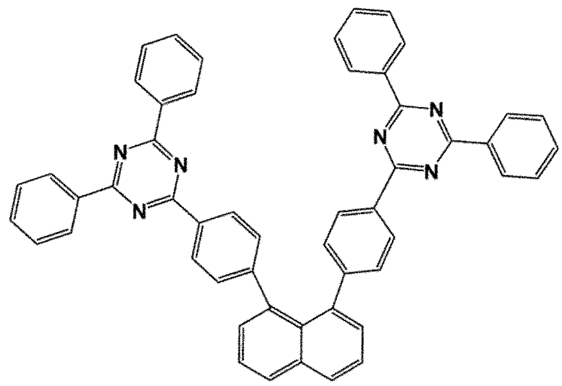

**13.** Composition according to Claim 12, **characterised in that** the additional compound is a host or matrix material.

**14.** Composition according to Claim 12 or 13, **characterised in that** the additional compound has a band gap of 2.5 eV or more, preferably 3.0 eV or more, very preferably 3.5 eV or more.

**15.** Formulation comprising at least one compound of the general formula (1)

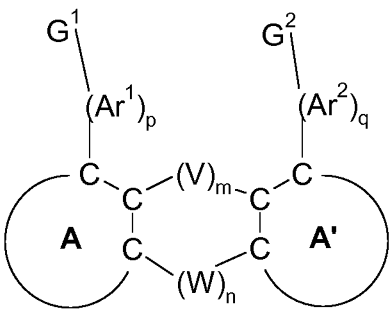

formula (1)

where the following applies to the symbols and indices used:

A and A' are, identically or differently from one another, an aromatic or heteroaromatic ring having 5 or 6 ring atoms, which may be substituted by one or more radicals $R^1$, which may be independent of one another;
$G^1$, $G^2$ are on each occurrence, identically or differently,
an organic electron-transporting group (ETG) from the group of the electron-deficient heteroaromatic groups from the group of the triazines, pyrimidines and pyrazines;
or an electron-rich organic hole-transporting group (HTG), where the HTG is selected from the group of the arylamines, triarylamines, bridged amines, where preferred bridged amines are dihydroacridines, dihydrophenazines, phenoxazines and phenothiazines, carbazoles, bridged carbazoles, biscarbazoles, indenocarbazoles and indolocarbazoles,
where at least one of the two groups $G^1$ and $G^2$ must be an electron-transporting group (ETG) and where the groups $G^1$ and $G^2$ may be substituted by one or more radicals $R^1$, which are independent of one another;
$Ar^1$, if $G^1$ is an electron-transporting group, is a divalent aromatic or heteroaromatic, preferably aromatic, ring or ring system having 5 to 60 ring atoms, where the ring or ring system is bridged neither to the ring system containing the rings A and A' nor to the ETG, where it is preferred if $Ar^1$ is a pyridylene, pyrimidylene, phenylene, biphenylene or fluorene, spiro, ter-phenylene, thiophene or furan group, where a phenylene, biphenylene or terphenylene group is particularly preferred and a phenylene group is very particularly preferred,
or, if $G^1$ is a hole-transporting group, is an aromatic ring or ring system having 5 to 60 ring atoms, where the

ring or ring system is bridged neither to the ring system containing the rings A and A' nor to the HTG, where it is preferred if $Ar^1$ is a phenylene, biphenylene or terphenylene group and a phenylene group is particularly preferred;

$Ar^2$, if $G^2$ is an electron-transporting group, is a divalent aromatic or heteroaromatic ring or ring system having 5 to 60 ring atoms, where the ring or ring system is bridged neither to the ring system containing the rings A and A' nor to the ETG, where it is preferred if $Ar^2$ is a pyridylene, pyrimidylene, phenylene, biphenylene or fluorene, spiro, terphenylene, thiophene or furan group, where a phenylene, biphenylene or terphenylene group is particularly preferred and a phenylene group is very particularly preferred,

or, if $G^2$ is a hole-transporting group, is an aromatic ring or ring system having 5 to 60 ring atoms, where the ring or ring system is bridged neither to the ring system containing the rings A and A' nor to the HTG, where it is preferred if $Ar^2$ is a phenylene, biphenylene or terphenylene group and a phenylene group is particularly preferred;

V is a single bond, $NAr^3$, O or S, where, in the case of a single bond, the carbon atoms of the rings A and A' are connected directly to one another by a single bond, where a single bond, $NAr^3$, O and S are preferred, where a single bond, O and S are very preferred, where O and S are very particularly preferred, where O is especially preferred;

W is a single bond, C=O, $C(R^1)_2$ or $NR^1$, where, in the case of a single bond, the carbon atoms of the rings A and A' are connected directly to one another by a single bond, where a single bond, $C(R^1)_2$ and $NR^1$ are preferred, where a single bond or $C(R^1)_2$ are very preferred;

where it is furthermore preferred that V is a single bond if W is not a single bond or that W is a single bond if V is not a single bond;

where it is furthermore very preferred that V is a single bond if W is equal to O or S or that W is a single bond if V is equal to O or S;

where it is furthermore very particularly preferred that V is a single bond if W is equal to O or that W is a single bond if V is equal to O;

m is either 0 or 1;

n is either 0 or 1,

where m = n;

p is either 0 or 1;

q is either 0 or 1, where p + q is either 1 or 2;

$Ar^3$ is an aromatic ring or ring system having 6 to 30 ring atoms, where the ring or ring system may in each case be substituted by one or more radicals $R^2$, which may be substituted by one or more radicals $R^3$, where two or more radicals $R^2$ may form a ring closure with one another;

$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylhetero-arylamino group having 10 to 40 aromatic ring atoms, which may be substituted by one or more radicals $R^2$, or a combination of two or more of these groups or a crosslinkable group Q;

$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, $C\equiv C$, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroaryl-amino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another;

$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic aliphatic or aromatic ring system with one another;

with the proviso that a maximum of one substituent $R^1$ of ring A and a maximum of one substituent $R^1$ of ring A' contains an aromatic or heteroaromatic group having 5 to 30 ring atoms;
or at least one composition according to one or more of Claims 12 to 14,
and at least one solvent;
where the formulation comprising tetrahydrofuran and the following compound is excluded

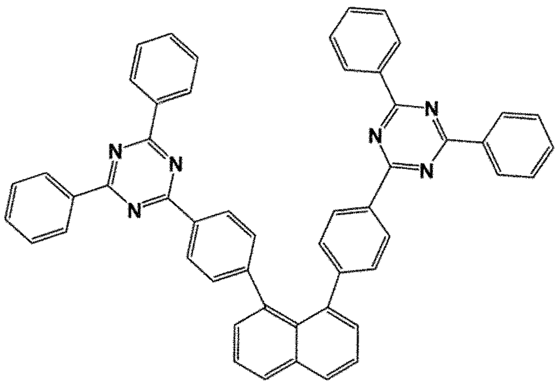

**16.** Use of at least one compound according to one or more of Claims 1 to 11 or at least one composition according to one or more of Claims 12 to 14 in an organic electroluminescent device, preferably in an organic light-emitting diode (OLED) or organic light-emitting electrochemical cell (OLEC, LEEC, LEC), very particularly preferably in an OLED, preferably in an electron-transport layer (ETL) and in a hole-blocking layer (HBL).

**17.** Use of at least one compound of the general formula (1)

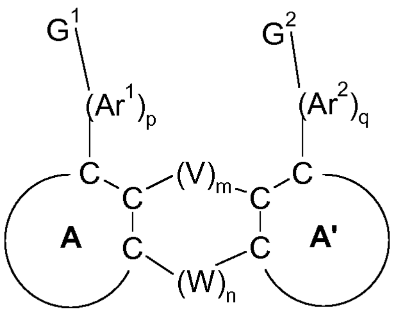

formula (1)

where the following applies to the symbols and indices used:

A and A' are, identically or differently from one another, an aromatic or heteroaromatic ring having 5 or 6 ring atoms, which may be substituted by one or more radicals $R^1$, which may be independent of one another;
$G^1$, $G^2$ are on each occurrence, identically or differently,
an organic electron-transporting group (ETG) from the group of the electron-deficient heteroaromatic groups from the group of the triazines, pyrimidines and pyrazines;
or an electron-rich organic hole-transporting group (HTG), where the HTG is selected from the group of the arylamines, triarylamines, bridged amines, where preferred bridged amines are dihydroacridines, dihydrophenazines, phenoxazines and phenothiazines, carbazoles, bridged carbazoles, biscarbazoles, indenocarbazoles and indolocarbazoles,
where at least one of the two groups $G^1$ and $G^2$ must be an electron-transporting group (ETG) and where the groups $G^1$ and $G^2$ may be substituted by one or more radicals $R^1$, which are independent of one another;

$Ar^1$, if $G^1$ is an electron-transporting group, is a divalent aromatic or heteroaromatic, preferably aromatic, ring or ring system having 5 to 60 ring atoms, where the ring or ring system is bridged neither to the ring system containing the rings A and A' nor to the ETG, where it is preferred if $Ar^1$ is a pyridylene, pyrimidylene, phenylene, biphenylene or fluorene, spiro, ter-phenylene, thiophene or furan group, where a phenylene, biphenylene or terphenylene group is particularly preferred and a phenylene group is very particularly preferred,

or, if $G^1$ is a hole-transporting group, is an aromatic ring or ring system having 5 to 60 ring atoms, where the ring or ring system is bridged neither to the ring system containing the rings A and A' nor to the HTG, where it is preferred if $Ar^1$ is a phenylene, biphenylene or terphenylene group and a phenylene group is particularly preferred;

$Ar^2$, if $G^2$ is an electron-transporting group, is a divalent aromatic or heteroaromatic ring or ring system having 5 to 60 ring atoms, where the ring or ring system is bridged neither to the ring system containing the rings A and A' nor to the ETG, where it is preferred if $Ar^2$ is a pyridylene, pyrimidylene, phenylene, biphenylene or fluorene, spiro, terphenylene, thiophene or furan group, where a phenylene, biphenylene or terphenylene group is particularly preferred and a phenylene group is very particularly preferred,

or, if $G^2$ is a hole-transporting group, is an aromatic ring or ring system having 5 to 60 ring atoms, where the ring or ring system is bridged neither to the ring system containing the rings A and A' nor to the HTG, where it is preferred if $Ar^2$ is a phenylene, biphenylene or terphenylene group and a phenylene group is particularly preferred;

V is a single bond, $NAr^3$, O or S, where, in the case of a single bond, the carbon atoms of the rings A and A' are connected directly to one another by a single bond, where a single bond, $NAr^3$, O and S are preferred, where a single bond, O and S are very preferred, where O and S are very particularly preferred, where O is especially preferred;

W is a single bond, C=O, $C(R^1)_2$ or $NR^1$, where, in the case of a single bond, the carbon atoms of the rings A and A' are connected directly to one another by a single bond, where a single bond, $C(R^1)_2$ and $NR^1$ are preferred, where a single bond or $C(R^1)_2$ are very preferred;

where it is furthermore preferred that V is a single bond if W is not a single bond or that W is a single bond if V is not a single bond;

where it is furthermore very preferred that V is a single bond if W is equal to O or S or that W is a single bond if V is equal to O or S;

where it is furthermore very particularly preferred that V is a single bond if W is equal to O or that W is a single bond if V is equal to O;

m is either 0 or 1;

n is either 0 or 1,

where m = n;

p is either 0 or 1;

q is either 0 or 1, where p + q is either 1 or 2;

$Ar^3$ is an aromatic ring or ring system having 6 to 30 ring atoms, where the ring or ring system may in each case be substituted by one or more radicals $R^2$, which may be substituted by one or more radicals $R^3$, where two or more radicals $R^2$ may form a ring closure with one another;

$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylhetero-arylamino group having 10 to 40 aromatic ring atoms, which may be substituted by one or more radicals $R^2$, or a combination of two or more of these groups or a crosslinkable group Q;

$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring

system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroaryl-amino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another;
$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic aliphatic or aromatic ring system with one another;

with the proviso that a maximum of one substituent $R^1$ of ring A and a maximum of one substituent $R^1$ of ring A' contains an aromatic or heteroaromatic group having 5 to 30 ring atoms;
in an emission layer (EML) in an organic electroluminescent device.

**18.** Organic electroluminescent device containing at least one compound according to one or more of Claims 1 to 11 or at least one composition according to one or more of Claims 12 to 14, preferably in an electron-transport layer (ETL) and in a hole-blocking layer (HBL), very preferably in an ETL.

**19.** Organic electroluminescent device containing at least one compound of the general formula (1)

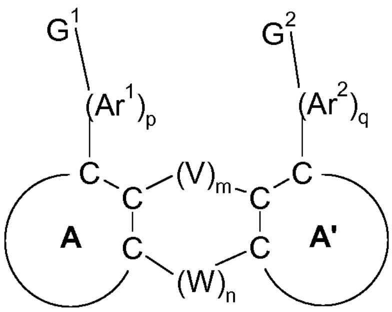

formula (1)

where the definitions indicated in Claim 17 apply to the symbols and indices used,
or at least one composition according to one or more of Claims 12 to 14
in the emission layer.

**20.** Organic electroluminescent device according to one or more of Claims 18 to 19, **characterised in that** it is selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

**21.** Process for the production of an organic electroluminescent device according to one or more of Claims 18 to 20, **characterised in that** at least one organic layer is applied by gas-phase deposition or from solution.

**Revendications**

**1.** Composé de la formule générale (1)

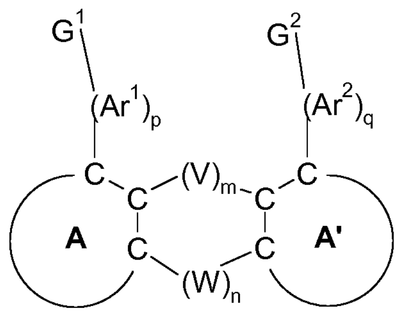

formule (1)

où ce qui suit s'applique aux symboles et aux indices qui sont utilisés :

A et A' sont, de manière identique ou différente l'un par rapport à l'autre, un cycle aromatique ou hétéroaromatique qui comporte 5 ou 6 atomes de cycle, lequel peut être substitué par un radical ou par plusieurs radicaux $R^1$, lesquels peuvent être indépendants les uns des autres ;

$G^1$, $G^2$ sont pour chaque occurrence, de manière identique ou différente,

un groupe de transport d'électrons (ETG) organique qui est pris parmi le groupe qui est constitué par les groupes hétéroaromatiques déficients en électrons qui sont pris parmi le groupe des triazines, des pyrimidines et des pyrazines ;

ou un groupe de transport de trous (HTG) organique riche en électrons, dans lequel le HTG est sélectionné parmi le groupe des arylamines, des triarylamines, des amines pontées, où les amines pontées préférées sont les dihydroacridines, les dihydrophénazines, les phénoxazines et les phénothiazines, les carbazoles, les carbazoles pontés, les biscarbazoles, les indénocarbazoles et les indolocarbazoles,

où au moins l'un des deux groupes $G^1$ et $G^2$ doit être un groupe de transport d'électrons (ETG) et où les groupes $G^1$ et $G^2$ peuvent être substitués par un radical ou par plusieurs radicaux $R^1$, lesquels sont indépendants les uns des autres ;

$Ar^1$ si $G^1$ est un groupe de transport d'électrons, est un cycle ou un système de cycle divalent aromatique ou hétéroaroma-tique, de préférence aromatique, qui comporte de 5 à 60 atomes de cycle, où le cycle ou le système de cycle n'est ponté ni sur le système de cycle qui contient les cycles A et A', ni sur l'ETG, où il est préférable que $Ar^1$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,

ou, si $G^1$ est un groupe de transport de trous, est un cycle ou un système de cycle aromatique qui comporte de 5 à 60 atomes de cycle, où le cycle ou le système de cycle n'est ponté ni sur le système de cycle qui contient les cycles A et A', ni sur le HTG, où il est préférable que $Ar^1$ soit un groupe phénylène, biphénylène ou terphénylène et un groupe phénylène a particulièrement la préférence ;

$Ar^2$ si $G^2$ est groupe de transport d'électrons, est un cycle ou un système de cycle divalent aromatique ou hétéroaromatique qui comporte de 5 à 60 atomes de cycle, où le cycle ou le système de cycle n'est ponté ni sur le système de cycle qui contient les cycles A et A', ni sur l'ETG, où il est préférable que $Ar^2$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,

ou, si $G^2$ est un groupe de transport de trous, est un cycle ou un système de cycle aromatique qui comporte de 5 à 60 atomes de cycle aromatique, où le cycle ou le système de cycle n'est ni ponté sur le système de cycle qui contient les cycles A et A', ni sur le HTG, où il est préférable que $Ar^2$ soit un groupe phénylène, biphénylène ou terphénylène et un groupe phénylène a particulièrement la préférence ;

V est une liaison simple, $NAr^3$, O ou S, où, dans le cas d'une liaison simple, les atomes de carbone des cycles A et A' sont connectés directement les uns aux autres au moyen d'une liaison simple, où une liaison simple, $NAr^3$, O et S ont la préférence, où une liaison simple, O et S ont plus particulièrement la préférence, où O et S sont tout particulièrement préférés, où O a tout spécialement la préférence ;

W est une liaison simple, C=O, $C(R^1)_2$ ou $NR^1$, où, dans le cas d'une liaison simple, les atomes de carbone des cycles A et A' sont connectés directement les uns aux autres au moyen d'une liaison simple, où une liaison simple, $C(R^1)_2$ et $NR^1$ ont la préférence, où une liaison simple ou $C(R^1)_2$ ont plus particulièrement la préférence ; où il est en outre préférable que V soit une liaison simple si W n'est pas une liaison simple ou que W soit une liaison simple si V n'est pas une liaison simple ;

où il est en outre particulièrement préférable que V soit une liaison simple si W est égal à O ou à S ou que W soit une liaison simple si V est égal à O ou à S ;
où il est en outre plus particulièrement préférable que V soit une liaison simple si W est égal à O ou que W soit une liaison simple si V est égal à O ;
m est soit 0, soit 1 ;
n est soit 0, soit 1,
où m = n ;
p est soit 0, soit 1 ;
q est soit 0, soit 1, où p + q est soit 1, soit 2 ;
$Ar^3$ est un cycle ou un système de cycle aromatique qui comporte de 6 à 30 atomes de cycle, où le cycle ou le système de cycle peut dans chaque cas être substitué par un radical ou par plusieurs radicaux $R^2$, lesquels peuvent être substitués par un radical ou par plusieurs radicaux $R^3$, où deux radicaux $R^2$ ou plus peuvent former une fermeture de cycle l'un avec l'autre ou les uns avec les autres ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux ou plus de ces groupes ou un groupe réticulable Q ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux ou plus de ces groupes ; deux radicaux $R^2$ adjacents ou plus peuvent former un système de cycle aliphatique ou aromatique mono- ou polycyclique l'un avec l'autre ou les uns avec les autres ;
$R^3$ est, de manière identique ou différente pour chaque occurence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants $R^3$ ou plus peuvent également former un système de cycle aliphatique ou aromatique mono- ou polycyclique l'un avec l'autre ou les uns avec les autres ;

étant entendu qu'un maximum d'un substituant $R^1$ du cycle A et qu'un maximum d'un substituant $R^1$ du cycle A' contiennent un groupe aromatique ou hétéroaromatique qui comporte de 5 à 30 atomes de cycle ;
où les composés qui suivent sont exclus du cadre de protection :
*nota : compound = composé*

| Composé 1-b-1 | Composé 1-b-2 |
|---|---|

(suite)

| | |
|---|---|
| 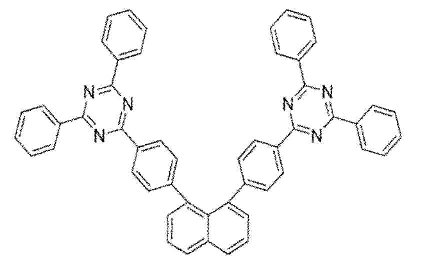 | 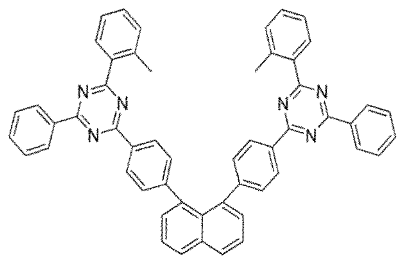 |
| Composé 1-b-3 | Composé 1-b-4 |
| 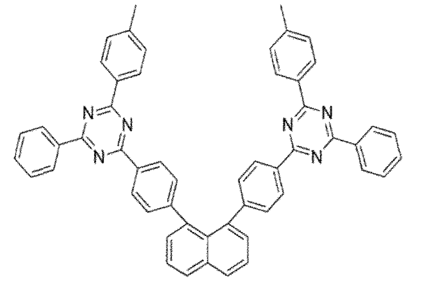 | 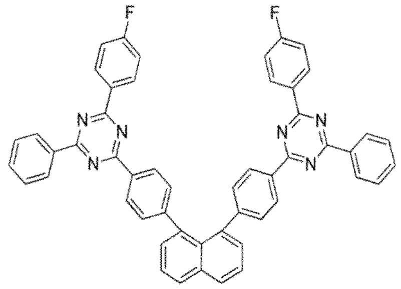 |
| Composé 1-b-5 | Composé 1-b-6 |
| 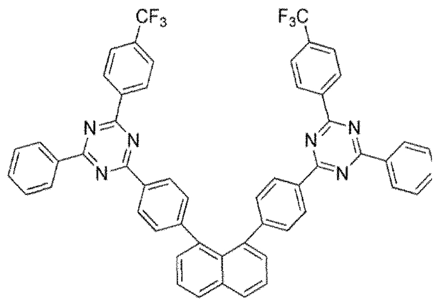 | 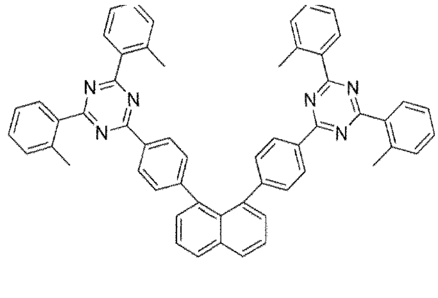 |
| Composé 1-b-7 | Composé 1-b-8 |
| 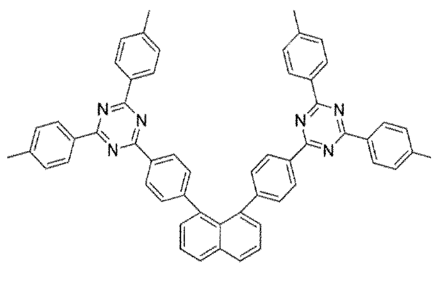 | 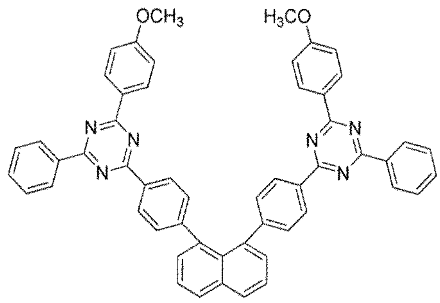 |
| Composé 1-b-9 | Composé 1-b-10 |
| 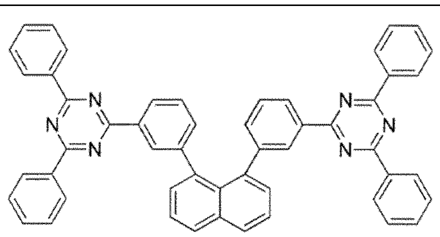 | 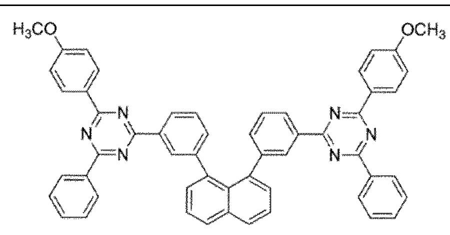 |
| Composé 1-b-11 | Composé 1-b-12 |

(suite)

| | |
|---|---|
| 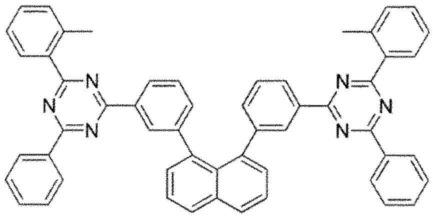 | 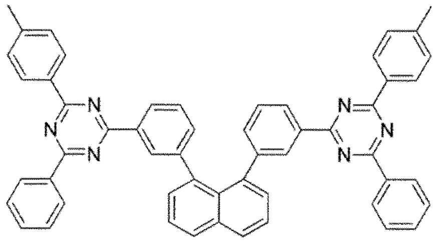 |
| Composé 1-b-13 | Composé 1-b-14 |
| 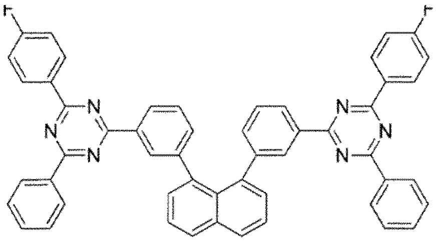 | 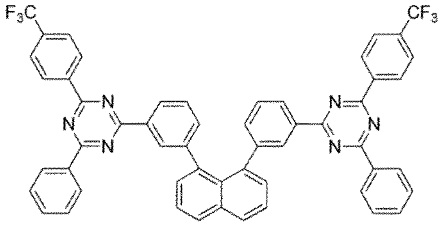 |
| Composé 1-b-15 | Composé 1-b-16 |
| 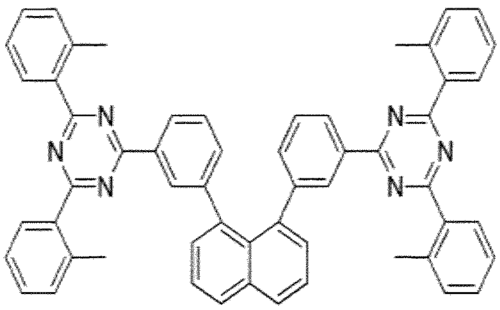 | 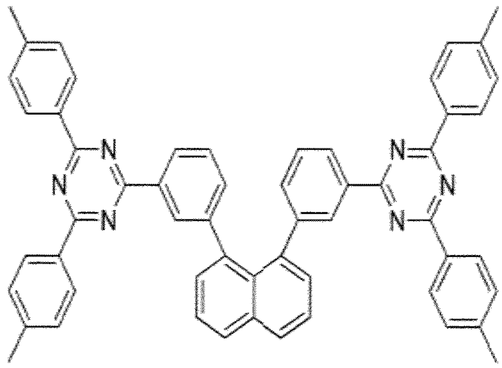 |
| Composé 2-b-1 | Composé 2-b-2 |
| 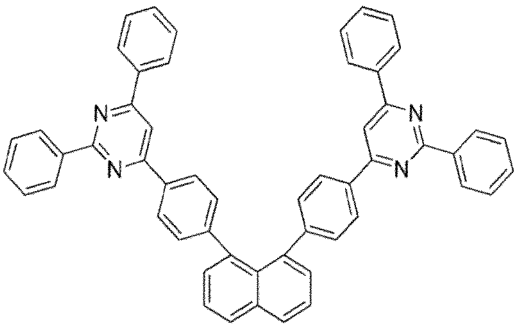 | 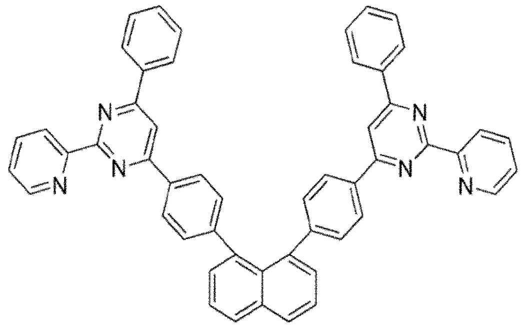 |
| Composé 2-b-3 | Composé 2-b-4 |

(suite)

| | |
|---|---|
| 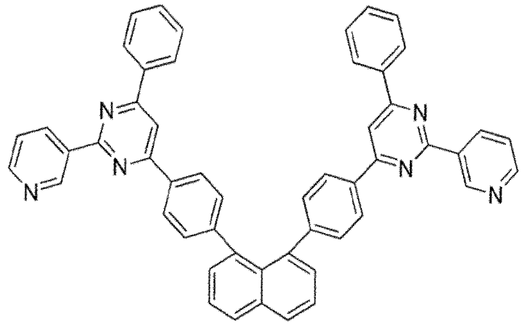 | 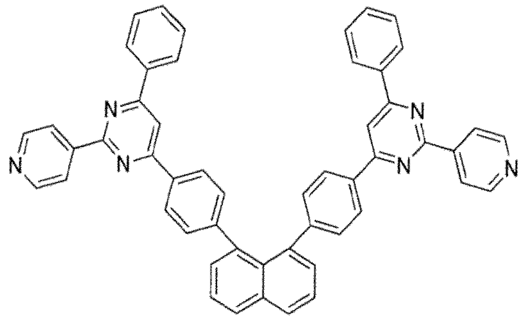 |
| Composé 2-b-5 | Composé 2-b-6 |
| 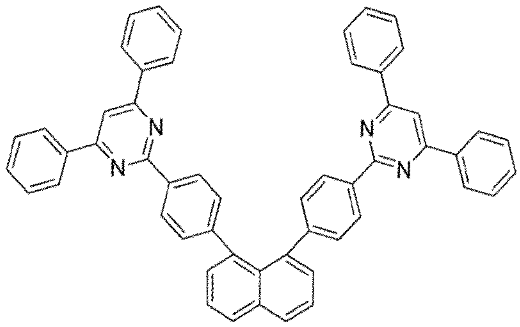 | 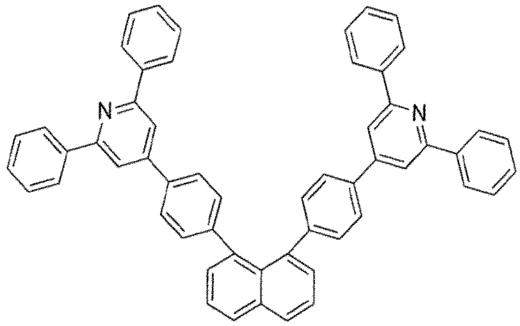 |
| Composé 2-b-7 | Composé 2-b-8 |
| 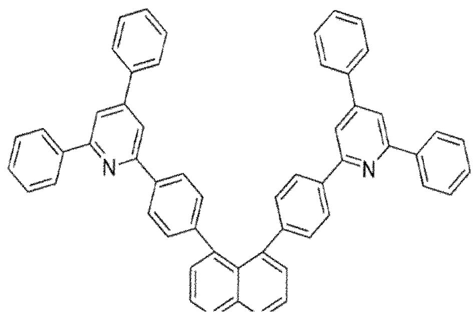 | 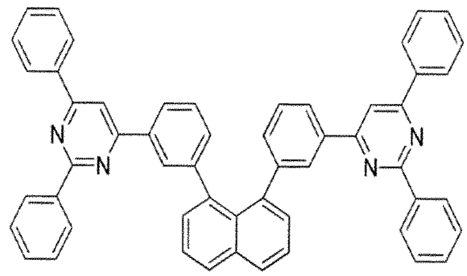 |
| Composé 2-b-9 | Composé 2-b-10 |
| 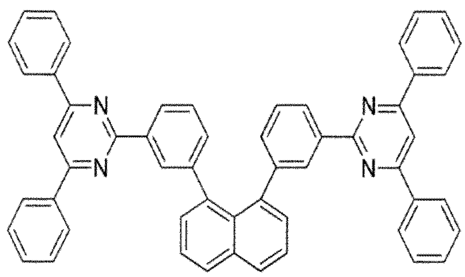 | 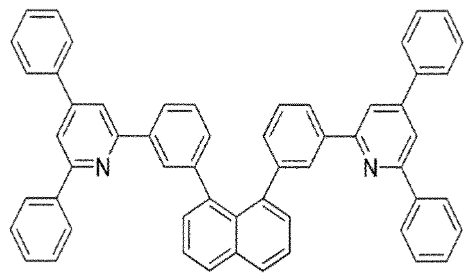 |

(suite)

| Composé 2-b-11 | |
|---|---|
| 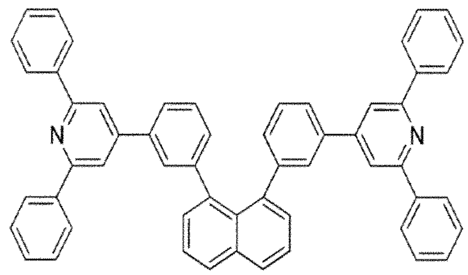 | |

2. Composé selon la revendication 1, **caractérisé en ce qu'**il présente la formule générale (2)

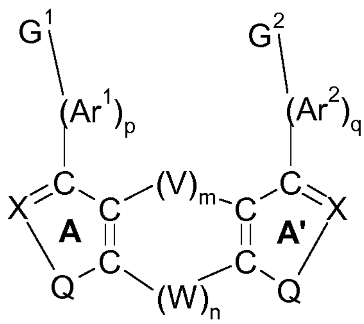

formule (2)

où ce qui suit s'applique aux symboles qui sont utilisés de façon additionnelle :

X est, de manière identique ou différente pour chaque occurrence, N ou $CR^1$ ;
Q est, de manière identique ou différente pour chaque occurrence, X=X, S, O ou $NR^1$, de préférence X=X, S ou O, de façon très préférable, X=X ou S et de façon tout particulièrement préférable, X=X.

3. Composé selon la revendication 1 ou 2, **caractérisé en ce qu'**il présente l'une des formules générales qui suivent (3) à (11)

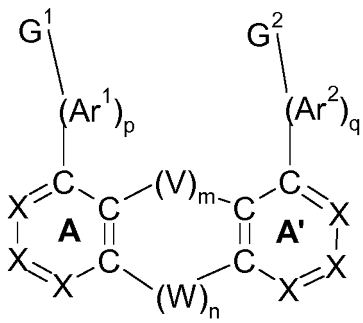

formule (3)

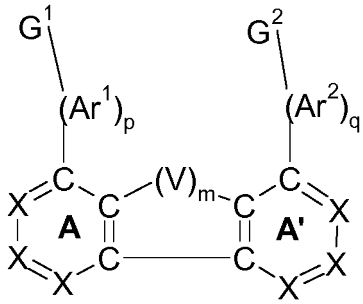

formule (4)

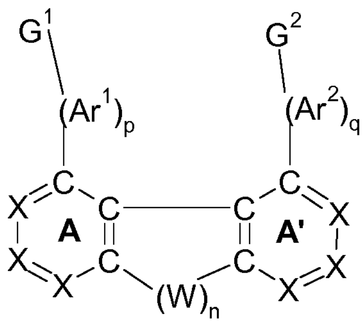

formule (5)

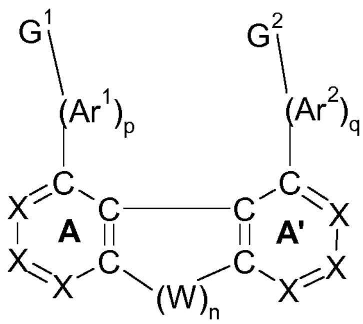

formule (6)

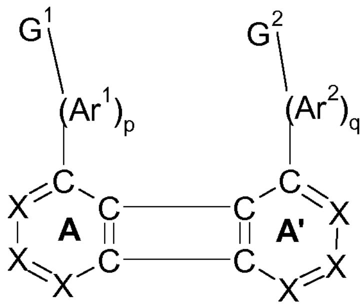

formule (7)

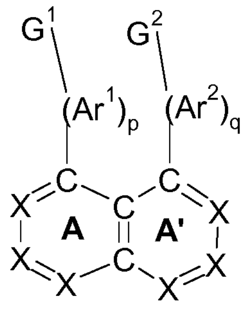

formule (8)

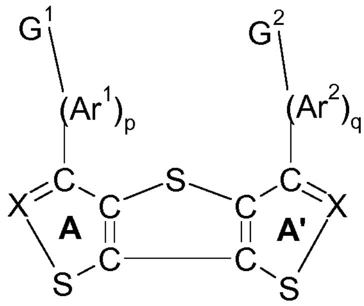

formule (9)

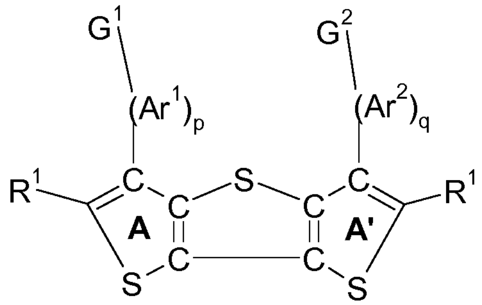

formule (10)

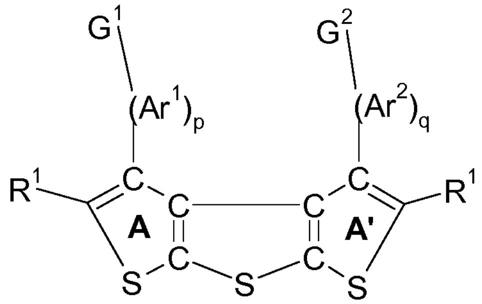

formule (11)

4. Composé selon une ou plusieurs des revendications 1 à 3, **caractérisé en ce qu'**il présente la formule générale (4).

5. Composé selon une ou plusieurs des revendications 1 à 4, **caractérisé en ce qu'**il présente la formule générale (4) et où X est égal à $CR^1$ et m est égal à 1.

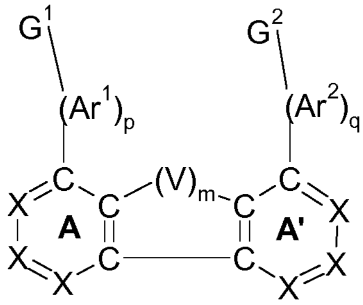

formule (4)

6. Composé selon une ou plusieurs des revendications 1 à 5, **caractérisé en ce qu'**il présente la formule générale (4) et X est égal à $CR^1$, m est égal à 1, p est égal à 0 et q est égal à 1.

7. Composé selon une ou plusieurs des revendications 1 à 6, **caractérisé en ce qu'**il présente la formule générale (13),

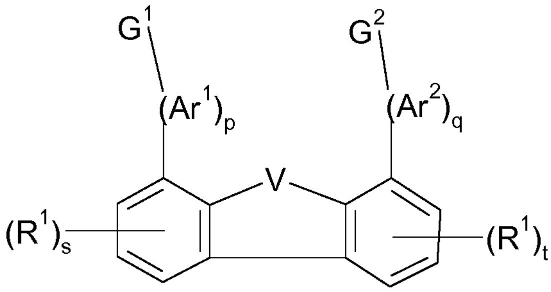

formule (13)

dans laquelle V est égal à O ou à S et dans laquelle les cycles aromatiques comportent dans chaque cas un maximum d'un substituant $R^1$, c'est-à-dire que s est égal à 0 ou à 1 et t est égal à 0 ou à 1, où s + t peut être égal à 0, à 1 ou à 2.

8. Composé selon une ou plusieurs des revendications 1 à 7, **caractérisé en ce qu'**il présente la formule générale (15)

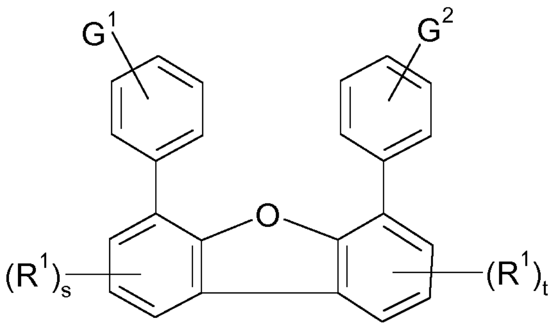

formule (15)

9. Composé selon une ou plusieurs des revendications 1 à 8, **caractérisé en ce qu'**il présente la formule générale (16)

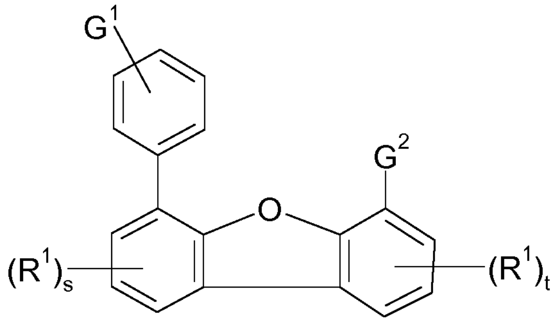

formule (16)

**10.** Composé selon une ou plusieurs des revendications 1 à 9, **caractérisé en ce qu'**il présente la formule générale (16a)

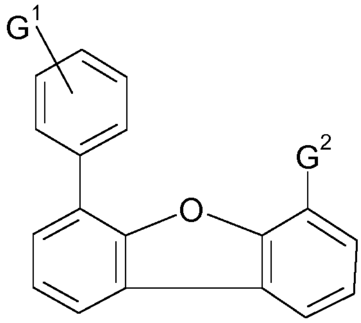

formule (16a)

**11.** Composé selon une ou plusieurs des revendications 1 à 10, **caractérisé en ce que** l'un des deux groupes $G^1$ et $G^2$ est un ETG et l'autre est un HTG.

**12.** Composition comprenant au moins un composé additionnel de la formule générale (1)

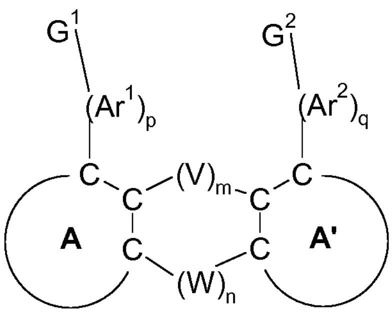

formule (1)

où ce qui suit s'applique aux symboles et aux indices qui sont utilisés :

A et A' sont, de manière identique ou différente l'un par rapport à l'autre, un cycle aromatique ou hétéroaromatique qui comporte 5 ou 6 atomes de cycle, lequel peut être substitué par un radical ou par plusieurs radicaux $R^1$, lesquels peuvent être indépendants les uns des autres ;
$G^1$, $G^2$ sont pour chaque occurrence, de manière identique ou différente,
un groupe de transport d'électrons (ETG) organique qui est pris parmi le groupe qui est constitué par les groupes hétéroaromatiques déficients en électrons qui sont pris parmi le groupe des triazines, des pyrimidines et des pyrazines ;
ou un groupe de transport de trous (HTG) organique riche en électrons, dans lequel le HTG est sélectionné

parmi le groupe des arylamines, des triarylamines, des amines pontées, où les amines pontées préférées sont les dihydroacridines, les dihydrophénazines, les phénoxazines et les phénothiazines, les carbazoles, les carbazoles pontés, les biscarbazoles, les indénocarbazoles et les indolocarbazoles,
où au moins l'un des deux groupes $G^1$ et $G^2$ doit être un groupe de transport d'électrons (ETG) et où les groupes $G^1$ et $G^2$ peuvent être substitués par un radical ou par plusieurs radicaux $R^1$, lesquels sont indépendants les uns des autres ;
$Ar^1$ si $G^1$ est un groupe de transport d'électrons, est un cycle ou un système de cycle divalent aromatique ou hétéroaroma-tique, de préférence aromatique, qui comporte de 5 à 60 atomes de cycle, où le cycle ou le système de cycle n'est ponté ni sur le système de cycle qui contient les cycles A et A', ni sur l'ETG, où il est préférable que $Ar^1$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,
ou, si $G^1$ est un groupe de transport de trous, est un cycle ou un système de cycle aromatique qui comporte de 5 à 60 atomes de cycle, où le cycle ou le système de cycle n'est ponté ni sur le système de cycle qui contient les cycles A et A', ni sur le HTG, où il est préférable que $Ar^1$ soit un groupe phénylène, biphénylène ou terphénylène et un groupe phénylène a particulièrement la préférence ;
$Ar^2$ si $G^2$ est groupe de transport d'électrons, est un cycle ou un système de cycle divalent aromatique ou hétéroaromatique qui comporte de 5 à 60 atomes de cycle, où le cycle ou le système de cycle n'est ponté ni sur le système de cycle qui contient les cycles A et A', ni sur l'ETG, où il est préférable que $Ar^2$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,
ou, si $G^2$ est un groupe de transport de trous, est un cycle ou un système de cycle aromatique qui comporte de 5 à 60 atomes de cycle aromatique, où le cycle ou le système de cycle n'est ni ponté sur le système de cycle qui contient les cycles A et A', ni sur le HTG, où il est préférable que $Ar^2$ soit un groupe phénylène, biphénylène ou terphénylène et un groupe phénylène a particulièrement la préférence ;
V est une liaison simple, $NAr^3$, O ou S, où, dans le cas d'une liaison simple, les atomes de carbone des cycles A et A' sont connectés directement les uns aux autres au moyen d'une liaison simple, où une liaison simple, $NAr^3$, O et S ont la préférence, où une liaison simple, O et S ont plus particulièrement la préférence, où O et S sont tout particulièrement préférés, où O a tout spécialement la préférence ;
W est une liaison simple, C=O, $C(R^1)_2$ ou $NR^1$, où, dans le cas d'une liaison simple, les atomes de carbone des cycles A et A' sont connectés directement les uns aux autres au moyen d'une liaison simple, où une liaison simple, $C(R^1)_2$ et $NR^1$ ont la préférence, où une liaison simple ou $C(R^1)_2$ ont plus particulièrement la préférence ;
où il est en outre préférable que V soit une liaison simple si W n'est pas une liaison simple ou que W soit une liaison simple si V n'est pas une liaison simple ;
où il est en outre particulièrement préférable que V soit une liaison simple si W est égal à O ou à S ou que W soit une liaison simple si V est égal à O ou à S ;
où il est en outre plus particulièrement préférable que V soit une liaison simple si W est égal à O ou que W soit une liaison simple si V est égal à O ;
m est soit 0, soit 1 ;
n est soit 0, soit 1,
où m = n ;
p est soit 0, soit 1 ;
q est soit 0, soit 1, où p + q est soit 1, soit 2 ;
$Ar^3$ est un cycle ou un système de cycle aromatique qui comporte de 6 à 30 atomes de cycle, où le cycle ou le système de cycle peut dans chaque cas être substitué par un radical ou par plusieurs radicaux $R^2$, lesquels peuvent être substitués par un radical ou par plusieurs radicaux $R^3$, où deux radicaux $R^2$ ou plus peuvent former une fermeture de cycle l'un avec l'autre ou les uns avec les autres ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy,

arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux ou plus de ces groupes ou un groupe réticulable Q ;

$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux ou plus de ces groupes ; deux radicaux $R^2$ adjacents ou plus peuvent former un système de cycle aliphatique ou aromatique mono- ou polycyclique l'un avec l'autre ou les uns avec les autres ;

$R^3$ est, de manière identique ou différente pour chaque occurence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants $R^3$ ou plus peuvent également former un système de cycle aliphatique ou aromatique mono- ou polycyclique l'un avec l'autre ou les uns avec les autres ;

étant entendu qu'un maximum d'un substituant $R^1$ du cycle A et qu'un maximum d'un substituant $R^1$ du cycle A' contiennent un groupe aromatique ou hétéroaromatique qui comporte de 5 à 30 atomes de cycle ;
et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous ;
où la composition qui comprend Liq et le composé qui est représenté ci-dessous est exclue

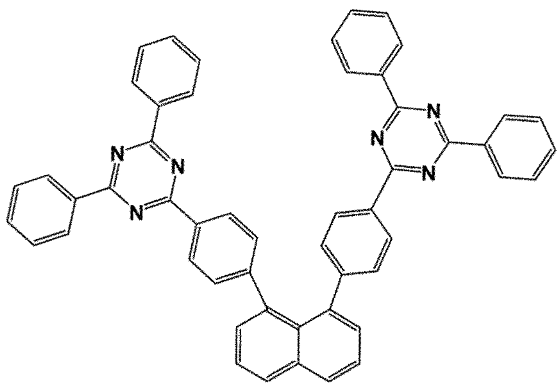

13. Composition selon la revendication12, **caractérisée en ce que** le composé additionnel est un matériau hôte ou de matrice.

14. Composition selon la revendication 12 ou 13, **caractérisée en ce que** le composé additionnel présente une bande interdite de 2,5 eV ou plus, de préférence de 3,0 eV ou plus, de façon très préférable, de 3,5 eV ou plus.

15. Formulation comprenant au moins un composé de la formule générale (1)

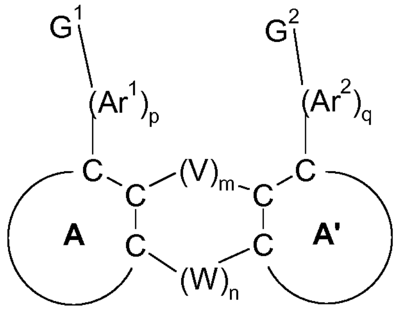

formule (1)

où ce qui suit s'applique aux symboles et aux indices qui sont utilisés :

A et A' sont, de manière identique ou différente l'un par rapport à l'autre, un cycle aromatique ou hétéroaromatique qui comporte 5 ou 6 atomes de cycle, lequel peut être substitué par un radical ou par plusieurs radicaux $R^1$, lesquels peuvent être indépendants les uns des autres ;

$G^1$, $G^2$ sont pour chaque occurrence, de manière identique ou différente,
un groupe de transport d'électrons (ETG) organique qui est pris parmi le groupe qui est constitué par les groupes hétéroaromatiques déficients en électrons qui sont pris parmi le groupe des triazines, des pyrimidines et des pyrazines ;
ou un groupe de transport de trous (HTG) organique riche en électrons, dans lequel le HTG est sélectionné parmi le groupe des arylamines, des triarylamines, des amines pontées, où les amines pontées préférées sont les dihydroacridines, les dihydrophénazines, les phénoxazines et les phénothiazines, les carbazoles, les carbazoles pontés, les biscarbazoles, les indénocarbazoles et les indolocarbazoles,
où au moins l'un des deux groupes $G^1$ et $G^2$ doit être un groupe de transport d'électrons (ETG) et où les groupes $G^1$ et $G^2$ peuvent être substitués par un radical ou par plusieurs radicaux $R^1$, lesquels sont indépendants les uns des autres ;

$Ar^1$ si $G^1$ est un groupe de transport d'électrons, est un cycle ou un système de cycle divalent aromatique ou hétéroaromatique, de préférence aromatique, qui comporte de 5 à 60 atomes de cycle, où le cycle ou le système de cycle n'est ponté ni sur le système de cycle qui contient les cycles A et A', ni sur l'ETG, où il est préférable que $Ar^1$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence, ou, si $G^1$ est un groupe de transport de trous, est un cycle ou un système de cycle aromatique qui comporte de 5 à 60 atomes de cycle, où le cycle ou le système de cycle n'est ponté ni sur le système de cycle qui contient les cycles A et A', ni sur le HTG, où il est préférable que $Ar^1$ soit un groupe phénylène, biphénylène ou terphénylène et un groupe phénylène a particulièrement la préférence ;

$Ar^2$ si $G^2$ est groupe de transport d'électrons, est un cycle ou un système de cycle divalent aromatique ou hétéroaromatique qui comporte de 5 à 60 atomes de cycle, où le cycle ou le système de cycle n'est ponté ni sur le système de cycle qui contient les cycles A et A', ni sur l'ETG, où il est préférable que $Ar^2$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,
ou, si $G^2$ est un groupe de transport de trous, est un cycle ou un système de cycle aromatique qui comporte de 5 à 60 atomes de cycle aromatique, où le cycle ou le système de cycle n'est ni ponté sur le système de cycle qui contient les cycles A et A', ni sur le HTG, où il est préférable que $Ar^2$ soit un groupe phénylène, biphénylène ou terphénylène et un groupe phénylène a particulièrement la préférence ;

V est une liaison simple, $NAr^3$, O ou S, où, dans le cas d'une liaison simple, les atomes de carbone des cycles A et A' sont connectés directement les uns aux autres au moyen d'une liaison simple, où une liaison simple, $NAr^3$, O et S ont la préférence, où une liaison simple, O et S ont plus particulièrement la préférence, où O et S sont tout particulièrement préférés, où O a tout spécialement la préférence ;

W est une liaison simple, C=O, $C(R^1)_2$ ou $NR^1$, où, dans le cas d'une liaison simple, les atomes de carbone des cycles A et A' sont connectés directement les uns aux autres au moyen d'une liaison simple, où une liaison simple, $C(R^1)_2$ et $NR^1$ ont la préférence, où une liaison simple ou $C(R^1)_2$ ont plus particulièrement la préférence ;
où il est en outre préférable que V soit une liaison simple si W n'est pas une liaison simple ou que W soit une liaison simple si V n'est pas une liaison simple ;

où il est en outre particulièrement préférable que V soit une liaison simple si W est égal à O ou à S ou que W soit une liaison simple si V est égal à O ou à S ;
où il est en outre plus particulièrement préférable que V soit une liaison simple si W est égal à O ou que W soit une liaison simple si V est égal à O ;
m est soit 0, soit 1 ;
n est soit 0, soit 1,
où m = n ;
p est soit 0, soit 1 ;
q est soit 0, soit 1, où p + q est soit 1, soit 2 ;
$Ar^3$ est un cycle ou un système de cycle aromatique qui comporte de 6 à 30 atomes de cycle, où le cycle ou le système de cycle peut dans chaque cas être substitué par un radical ou par plusieurs radicaux $R^2$, lesquels peuvent être substitués par un radical ou par plusieurs radicaux $R^3$, où deux radicaux $R^2$ ou plus peuvent former une fermeture de cycle l'un avec l'autre ou les uns avec les autres ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux ou plus de ces groupes ou un groupe réticulable Q ;
$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux ou plus de ces groupes ; deux radicaux $R^2$ adjacents ou plus peuvent former un système de cycle aliphatique ou aromatique mono- ou polycyclique l'un avec l'autre ou les uns avec les autres ;
$R^3$ est, de manière identique ou différente pour chaque occurence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants $R^3$ ou plus peuvent également former un système de cycle aliphatique ou aromatique mono- ou polycyclique l'un avec l'autre ou les uns avec les autres ;

étant entendu qu'un maximum d'un substituant $R^1$ du cycle A et qu'un maximum d'un substituant $R^1$ du cycle A' contiennent un groupe aromatique ou hétéroaromatique qui comporte de 5 à 30 atomes de cycle ;
ou au moins une composition selon une ou plusieurs des revendications 12 à 14,
et au moins un solvant ;
où la formulation qui comprend du tétrahydrofurane et le composé qui suit est exclue

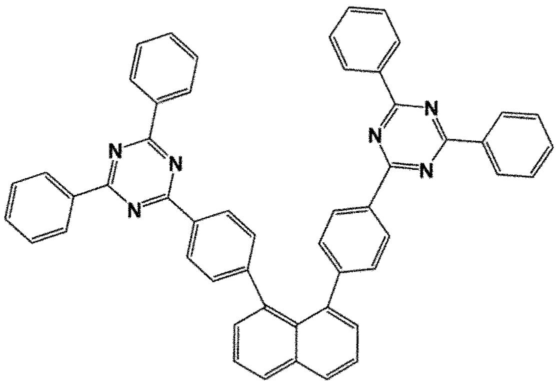

**16.** Utilisation d'au moins un composé selon une ou plusieurs des revendications 1 à 11 ou d'au moins une composition selon une ou plusieurs des revendications 12 à 14 dans un dispositif électroluminescent organique, de préférence dans une diode à émission de lumière organique (OLED) ou dans une cellule électrochimique à émission de lumière organique (OLEC, LEEC, LEC), de façon tout particulièrement préférable, dans une OLED, de façon préférable, dans une couche de transport d'électrons (ETL) et dans une couche de blocage de trous (HBL).

**17.** Utilisation d'au moins un composé de la formule générale (1)

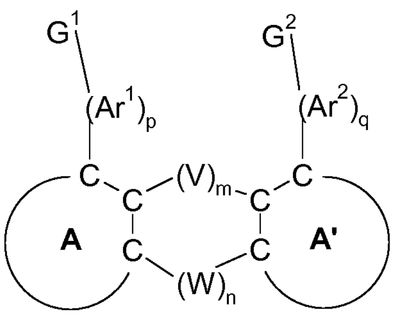

formule (1)

où ce qui suit s'applique aux symboles et aux indices qui sont utilisés :

A et A' sont, de manière identique ou différente l'un par rapport à l'autre, un cycle aromatique ou hétéroaromatique qui comporte 5 ou 6 atomes de cycle, lequel peut être substitué par un radical ou par plusieurs radicaux $R^1$, lesquels peuvent être indépendants les uns des autres ;
$G^1$, $G^2$ sont pour chaque occurrence, de manière identique ou différente,
un groupe de transport d'électrons (ETG) organique qui est pris parmi le groupe qui est constitué par les groupes hétéroaromatiques déficients en électrons qui sont pris parmi le groupe des triazines, des pyrimidines et des pyrazines ;
ou un groupe de transport de trous (HTG) organique riche en électrons, dans lequel le HTG est sélectionné parmi le groupe des arylamines, des triarylamines, des amines pontées, où les amines pontées préférées sont les dihydroacridines, les dihydrophénazines, les phénoxazines et les phénothiazines, les carbazoles, les carbazoles pontés, les biscarbazoles, les indénocarbazoles et les indolocarbazoles,
où au moins l'un des deux groupes $G^1$ et $G^2$ doit être un groupe de transport d'électrons (ETG) et où les groupes $G^1$ et $G^2$ peuvent être substitués par un radical ou par plusieurs radicaux $R^1$, lesquels sont indépendants les uns des autres ;
$Ar^1$ si $G^1$ est un groupe de transport d'électrons, est un cycle ou un système de cycle divalent aromatique ou hétéroaromatique, de préférence aromatique, qui comporte de 5 à 60 atomes de cycle, où le cycle ou le système de cycle n'est ponté ni sur le système de cycle qui contient les cycles A et A', ni sur l'ETG, où il est préférable que $Ar^1$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,
ou, si $G^1$ est un groupe de transport de trous, est un cycle ou un système de cycle aromatique qui comporte

de 5 à 60 atomes de cycle, où le cycle ou le système de cycle n'est ponté ni sur le système de cycle qui contient les cycles A et A', ni sur le HTG, où il est préférable que $Ar^1$ soit un groupe phénylène, biphénylène ou terphénylène et un groupe phénylène a particulièrement la préférence ;

$Ar^2$ si $G^2$ est groupe de transport d'électrons, est un cycle ou un système de cycle divalent aromatique ou hétéroaromatique qui comporte de 5 à 60 atomes de cycle, où le cycle ou le système de cycle n'est ponté ni sur le système de cycle qui contient les cycles A et A', ni sur l'ETG, où il est préférable que $Ar^2$ soit un groupe pyridylène, pyrimidylène, phénylène, biphénylène ou fluorène, spiro, terphénylène, thiophène ou furane, où un groupe phénylène, biphénylène ou terphénylène a particulièrement la préférence et un groupe phénylène a plus particulièrement la préférence,

ou, si $G^2$ est un groupe de transport de trous, est un cycle ou un système de cycle aromatique qui comporte de 5 à 60 atomes de cycle aromatique, où le cycle ou le système de cycle n'est ni ponté sur le système de cycle qui contient les cycles A et A', ni sur le HTG, où il est préférable que $Ar^2$ soit un groupe phénylène, biphénylène ou terphénylène et un groupe phénylène a particulièrement la préférence ;

V est une liaison simple, $NAr^3$, O ou S, où, dans le cas d'une liaison simple, les atomes de carbone des cycles A et A' sont connectés directement les uns aux autres au moyen d'une liaison simple, où une liaison simple, $NAr^3$, O et S ont la préférence, où une liaison simple, O et S ont plus particulièrement la préférence, où O et S sont tout particulièrement préférés, où O a tout spécialement la préférence ;

W est une liaison simple, C=O, $C(R^1)_2$ ou $NR^1$, où, dans le cas d'une liaison simple, les atomes de carbone des cycles A et A' sont connectés directement les uns aux autres au moyen d'une liaison simple, où une liaison simple, $C(R^1)_2$ et $NR^1$ ont la préférence, où une liaison simple ou $C(R^1)_2$ ont plus particulièrement la préférence ;

où il est en outre préférable que V soit une liaison simple si W n'est pas une liaison simple ou que W soit une liaison simple si V n'est pas une liaison simple ;

où il est en outre particulièrement préférable que V soit une liaison simple si W est égal à O ou à S ou que W soit une liaison simple si V est égal à O ou à S ; où il est en outre plus particulièrement préférable que V soit une liaison simple si W est égal à O ou que W soit une liaison simple si V est égal à O ;

m est soit 0, soit 1 ;

n est soit 0, soit 1,

où m = n ;

p est soit 0, soit 1 ;

q est soit 0, soit 1, où p + q est soit 1, soit 2 ;

$Ar^3$ est un cycle ou un système de cycle aromatique qui comporte de 6 à 30 atomes de cycle, où le cycle ou le système de cycle peut dans chaque cas être substitué par un radical ou par plusieurs radicaux $R^2$, lesquels peuvent être substitués par un radical ou par plusieurs radicaux $R^3$, où deux radicaux $R^2$ ou plus peuvent former une fermeture de cycle l'un avec l'autre ou les uns avec les autres ;

$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, C≡C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux ou plus de ces groupes ou un groupe réticulable Q ;

$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $R^3C=CR^3$, C≡C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique,

lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux ou plus de ces groupes ; deux radicaux $R^2$ adjacents ou plus peuvent former un système de cycle aliphatique ou aromatique mono- ou polycyclique l'un avec l'autre ou les uns avec les autres ;

$R^3$ est, de manière identique ou différente pour chaque occurence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants $R^3$ ou plus peuvent également former un système de cycle aliphatique ou aromatique mono- ou polycyclique l'un avec l'autre ou les uns avec les autres ;

étant entendu qu'un maximum d'un substituant $R^1$ du cycle A et qu'un maximum d'un substituant $R^1$ du cycle A' contiennent un groupe aromatique ou hétéroaromatique qui comporte de 5 à 30 atomes de cycle ;
dans une couche d'émission (EML) dans un dispositif électroluminescent organique.

**18.** Dispositif électroluminescent organique contenant au moins un composé selon une ou plusieurs des revendications 1 à 11 ou au moins une composition selon une ou plusieurs des revendications 12 à 14, de préférence dans une couche de transport d'électrons (ETL) et dans une couche de blocage de trous (HBL), de façon très préférable, dans une ETL.

**19.** Dispositif électroluminescent organique comprenant dans la couche d'émission au moins un composé de la formule générale (1)

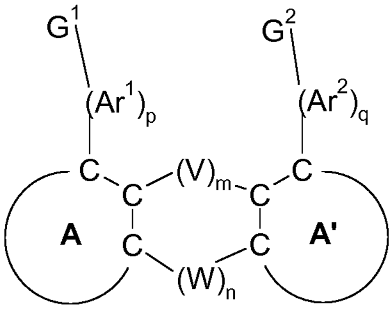

formule (1)

où les définitions qui ont été indiquées selon la revendication 17 s'appliquent aux symboles et aux indices qui sont utilisés,
ou au moins une composition selon une ou plusieurs des revendications 12 à 14.

**20.** Dispositif électroluminescent organique selon une ou plusieurs des revendications 18 et 19, **caractérisé en ce qu'**il est sélectionné parmi le groupe qui est constitué par les transistors à émission de lumière organiques (OLET), les dispositifs à extinction de champ organiques (OFQD), les cellules électrochimiques à émission de lumière organiques (OLEC, LEC, LEEC), les diodes laser organiques (O-laser) et les diodes à émission de lumière organiques (OLED), de préférence les OLEC et les OLED, de façon très préférable, les OLED.

**21.** Procédé pour la fabrication d'un dispositif électroluminescent organique selon une ou plusieurs des revendications 18 à 20, **caractérisé en ce qu'**au moins une couche organique est appliquée au moyen d'un dépôt en phase gazeuse ou à partir d'une solution.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2004093207 A
- WO 2010006680 A
- WO 2005003253 A
- WO 2008056746 A
- EP 0906947 A
- EP 0908787 A
- EP 0906948 A
- US 5935721 A
- WO 03095445 A
- CN 1362464
- WO 01076323 A
- WO 01021729 A
- WO 2004013073 A
- WO 2004018588 A
- WO 2003087023 A
- WO 2004018587 A
- WO 2004016575 A
- WO 2008145239 A
- WO 2005039246 A
- US 20050069729 A
- JP 2004288381 A
- EP 1205527 A
- WO 2008086851 A
- WO 2010136109 A
- WO 2011000455 A
- WO 2010015306 A
- WO 2007063754 A
- WO 2009069442 A
- JP 2009021336 A
- WO 2011057706 A
- EP 2873667 A1
- US 2011006670 A1
- WO 2010108579 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2006108497 A
- WO 2006122630 A
- WO 2008006449 A
- WO 2007140847 A
- WO 2010012328 A
- EP 676461 A
- WO 2004081017 A
- WO 2004058911 A
- WO 2005084081 A
- WO 2005084082 A
- WO 2006048268 A
- WO 2006117052 A
- WO 2011088877 A
- WO 2011128017 A
- EP 1617710 A
- EP 1617711 A
- EP 1731584 A
- JP 2005347160 A
- WO 2007137725 A
- WO 2005111172 A
- EP 652273 A
- WO 2009062578 A
- WO 2010054729 A
- WO 2010054730 A
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A


### In der Beschreibung aufgeführte Nicht-Patentliteratur


- **M. A. BALDO et al.** *Appl. Phys. Lett.,* 1999, vol. 75, 4-6

- **T. MATSUMOTO ; T. NAKADA ; J. ENDO ; K. MORI ; N. KAWAMURA ; A. YOKOI ; J. KIDO.** *Multiphoton Organic EL Device Having Charge Generation Layer*
- **Y. SHIROTA et al.** *Chem. Rev.,* 2007, vol. 107 (4), 953-1010
- **M. S. ARNOLD et al.** *Appl. Phys. Lett.,* 2008, vol. 92, 053301